(19) Europäisches Patentamt
European Patent Office
Office européen des brevets

(11) Veröffentlichungsnummer : **0 220 297 B1**

(12) # EUROPÄISCHE PATENTSCHRIFT

(45) Veröffentlichungstag der Patentschrift : **11.12.91 Patentblatt 91/50**

(21) Anmeldenummer : **86903300.1**

(22) Anmeldetag : **23.04.86**

(86) Internationale Anmeldenummer : **PCT/EP86/00248**

(87) Internationale Veröffentlichungsnummer : **WO 86/06373 06.11.86 Gazette 86/24**

(51) Int. Cl.$^5$ : **C07D 239/30**, C07D 239/34, C07D 239/26, C07D 241/12, C07D 241/18, C07D 213/30, C09K 19/34

(54) **STICKSTOFFHALTIGE HETEROCYCLEN.**

(30) Priorität : **27.04.85 DE 3515373**

(43) Veröffentlichungstag der Anmeldung : **06.05.87 Patentblatt 87/19**

(45) Bekanntmachung des Hinweises auf die Patenterteilung : **11.12.91 Patentblatt 91/50**

(84) Benannte Vertragsstaaten : **AT BE CH DE FR GB IT LI NL SE**

(56) Entgegenhaltungen :
EP-A- 0 131 373
EP-A- 0 152 697
EP-A- 0 160 416
EP-A- 0 175 591
WO-A-86/00087
US-A- 4 309 539

(73) Patentinhaber : **MERCK PATENT GESELLSCHAFT MIT BESCHRÄNKTER HAFTUNG**
**Frankfurter Strasse 250 Postfach 4119**
**W-6100 Darmstadt (DE)**

(72) Erfinder : **KRAUSE, Joachim**
**Samuel-Morse-Strasse 14**
**W-6110 Dieburg (DE)**
Erfinder : **EIDENSCHINK, Rudolf**
**Kornblumenstrasse 1**
**W-6115 Münster (DE)**
Erfinder : **BOFINGER, Klaus**
**Eberstädter Strasse 7**
**W-6109 Mühltal (DE)**
Erfinder : **HOPF, Reinhard**
**Wolfsgasse 3**
**W-6432 Heringen/Werra (DE)**
Erfinder : **REIFFENRATH, Volker**
**Pfungstädterstrasse 31**
**W-6100 Darmstadt (DE)**
Erfinder : **POETSCH, Eike**
**Am Buchwald 4**
**W-6109 Mühltal 6 (DE)**
Erfinder : **SCHEUBLE, Bernhard**
**Am Grenzweg 18**
**W-6136 Alsbach (DE)**
Erfinder : **GEELHAAR, Thomas**
**Kurt-Schumacher-Strasse 93**
**W-6500 Mainz (DE)**

EP 0 220 297 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft stickstoffhaltige Heterocyclen der Formel I

$$R^1\text{-}A^1\text{-}Z^1\text{-}A^2\text{-}R^2 \quad I$$

worin

einer der Reste

$R^1$ und $R^2$ H, eine unsubstituierte oder substituierte Alkylgruppe mit 1-15 C-Atomen, worin auch eine oder zwei nicht benachbarte $CH_2$-Gruppen durch mindestens eine Gruppierung aus der Gruppe -O-, -CO-, -O-CO-, -CO-O- und -CH=CH- ersetzt sein können, F, Cl, Br, -CN, -NCS oder $R^3\text{-}(A^3)_p\text{-}Z^2\text{-}$,

der andere Rest

$R^1$ oder

$R^2$ ein optisch aktiver organischer Rest der Formel

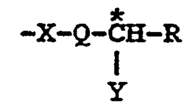

worin

X -CO-O-, -O-CO-, -O-CO-O-, -CO-, -O-, -S-, -CH=CH-, -CH=CH-COO- oder eine Einfachbindung,

Q Alkylen mit 1 bis 5 C-Atomen, worin auch eine nicht mit X verknüpfte $CH_2$-Gruppe durch -O-, -CO-, -O-CO-, -CO-O- oder -CH=CH- ersetzt sein kann, oder eine Einfachbindung,

Y CN, Halogen, Methyl oder Methoxy, und

R eine von Y verschiedene Alkylgruppe mit 1 bis 10 C-Atomen, worin auch eine oder zwei nicht benachbarte $CH_2$-Gruppen durch -O-, -CO-, -O-CO-, -CO-O- und/oder -CH=CH- ersetzt sein können,

bedeutet,

$A^1$ -A-, $\text{-}A^4\text{-}Z^3\text{-}A\text{-}$ oder $\text{-}A\text{-}Z^3\text{-}A^4\text{-}$,

A eine 1,4-Phenylengruppe, worin mindestens eine CH-Gruppe durch N ersetzt ist,

$A^2$, $A^3$ und $A^4$ jeweils unsubstituiertes oder durch ein oder zwei F- und/oder Cl-Atome und/oder $CH_3$-Gruppen und/oder CN-Gruppen substituiertes 1,4-Phenylen, worin auch eine oder zwei CH-Gruppen durch N-Atome ersetzt sein können, 1,4-Cyclohexylen, worin auch eine oder zwei nicht benachbarte $CH_2$-Gruppen durch O-Atome und/oder S-Atome ersetzt sein können, Piperidin-1,4-diyl, 1,4-Bi-cyclo(2,2,2)-octylen, unsubstituiertes oder durch CN unsubstituiertes Decahydro-naphthalin-2,6-diyl oder 1,2,3,4-Tetrahydronaphthalin-2,6-diyl,

$Z^1$, $Z^2$ und $Z^3$ jeweils -CO-O-, -O-CO-, $\text{-}OCH_2\text{-}$, $\text{-}CH_2O\text{-}$, $\text{-}CH_2CH_2\text{-}$, substituiertes Ethylen oder eine Einfachbindung,

$R^3$ H, eine unsubstituierte oder substituierte Alkylgruppe mit 1-15 C-Atomen, worin auch eine oder zwei nicht benachbarte $CH_2$-Gruppen durch eine Gruppierung aus der Gruppe -O-, -CO-, -O-CO-, -CO-O und -CH=CH- ersetzt sein können, F, Cl, Br, -NCS oder -CN, und

P 1 oder 2

bedeutet, wobei für p = 2 die Gruppen $A^3$ gleich der voneinander verschieden sein können, mit den Maßgaben, daß

a) im Falle A = Pyrimidin-2,5-diyl einer der Reste $R^1$ und $R^2$ ein optisch aktiver organischer Rest und der andere Rest $R^1$ oder $R^2$ eine unsubstituierte oder substituierte Alkylgruppe mit 1-15 C-Atomen, worin auch eine oder zwei nicht benachbarte $CH_2$-Gruppen durch mindestens eine Gruppierung aus der Gruppe -O-, -CO-, -O-CO-, -CO-O- und -CH=CH- ersetzt sein können, bedeutet.

b) Verbindungen der Formel

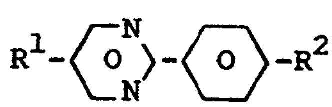

wobei einer der Reste $R^1$ und $R^2$ Alkyl, Alkoxy oder Alkoxycarbonyl mit 1 bis 15 C-Atomen und der andere Rest $R^1$ oder $R^2$ ein optisch aktiver Rest der Formel,

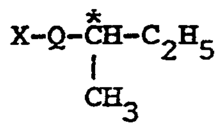

worin
X -CO-O-, -O-, -O-CO-, -CO- oder eine Einfachbindung und
Q Alkylen mit 1 bis 5 C-Atomen, worin auch eine nicht mit X verknüpfte $CH_2$-Gruppe durch -O- ersetzt sein kann,
bedeutet,
für die Vertvagsstaaten DE, GB, CH, LI, FR und SE ausgenommen sind,
c) (S)-5-n-Octyl-2-[4-(3-chlorpentyloxy)phenyl] pyrimidin,
(S)-5-n-Undecyl-2-[4-(3-cyanopentyloxy)phenyl] pyrimidin, und
(S)-5-(n-Octyloxy)-2-[4-(2-methyloctyl)-phenyl] pyrimidin
für die Vertragsstaaten DE, GB, CH, LI, FR und SE ausgenommen sind,
d) 3-(2-Methylbutoxy)-6-(4-pentylphenyl)-pyridazin,
3-(2-Methylbutoxy)-6-(4-nonylphenyl)-pyridazin,
3-(2-Methylbutoxy)-6-(4-dodecylphenyl)-pyridazin,
3-(2-Methylbutoxy)-6-(4-pentadecylphenyl)-pyridazin,
3-(2-Methylbutoxy)-6-(4-octyloxyphenyl)-pyridazin,
3-(2-Methylbutoxy)-6-(4-decyloxyphenyl)-pyridazin,
3-(2-Methylbutoxy)-6-(4-tetradecyloxyphenyl)-pyridazin,
3-(1-Methylheptyloxy)-6-(4-pentylphenyl)-pyridazin,
3-(1-Methylheptyloxy)-6-(4-dodecylphenyl)-pyridazin,
3-(1-Methylheptyloxy)-6-(4-decyloxyphenyl)-pyridazin und
3-(1-Methylbutoxy)-6-(4-octyloxyphenyl)-pyridazin
für die Vertragsstaaten DE, GB, CH, LI ausgenommen sind,
e) 4-[5-(4'-Butylphenyl)pyrimidin-2-yl]benzoesäure-(2'-dodecyloxypropyl-ester) und
4-(5-Heptylpyrimidin-2-yl)benzoesäure-(2'-decyloxypropyl-ester)
für die Vertragsstaaten DE, GB, CH, LI, FR, NL ausgenommen sind, und
f) die Verbindungen der Formel

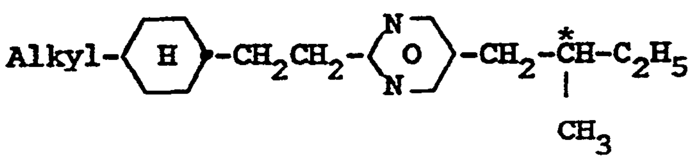

worin
Alkyl Alkyl mit 1 bis 12 C-Atomen bedeutet,
für die Vertragsstaaten DE, GB, CH, LI, FR ausgenommen sind.

Der Einfachheit halber bedeuten im folgenden Cy eine 1,4-Cyclohexylengruppe, Dio eine 1,3-Dioxan-2,5-diylgruppe, Dit eine 1,3-Dithian-2,5-diylgruppe, Bi eine 1,4-Bicyclo(2,2,2)-octylengruppe, Phe eine 1,4-Phenylengruppen und Pyr eine 1,4-Phenylengruppe, worin mindestens eine CH-Gruppe durch N ersetzt ist, wobei diese Gruppen, insbesondere Cy und Phe, unsubstituiert oder durch ein oder zwei F- und/oder Cl-Atome und/oder $CH_3$-Gruppen und/oder CN-Gruppen substituiert sein können.

Ähnliche Verbindungen sind z. B. aus der EP-OS 0 131 373 bekannt. Die dort angegebenen Verbindungen enthalten jedoch im Gegensatz zu den vorliegenden keine stickstoffhaltigen heterocyclischen Ringe.

Die Verbindungen der Formel I können wie ähnliche Verbindungen als chirale Dotierstoffe für flüssigkristalline Phasen, insbesondere jedoch als Komponenten ferroelektrischer flüssigkristalliner Phasen verwendet werden. Diese Phasen eignen sich für Displays, die auf dem Prinzip der verdrillten Zelle (TN-Displays), dem Guest-Host-Effekt, dem Effekt der Deformation aufgerichteter Phasen oder dem Effekt der dynamischen Streuung beruhen, insbesondere jedoch für ferroelektrische Displays beispielsweise nach N.A. Clark und S.T. Lagerwall, Applied Phys. Lett. 36, 899 (1980).

Aus der US-A-4,309,539 sind optisch aktive Pyrimidin-Derivate bekannt, welche eine chirale Alkyl- oder Alkoxy-Gruppe und eine Cyano-Gruppe als Flügelgruppen aufweisen. Diese Verbindungen eignen sich nicht als Dotierstoffe für chiral getiltete smektische, flüssigkristalline Phasen, sondern werden als Dotierstoffe zur

Erzeugung einer cholesterischen Phase nematischen Flüssigkristallmischungen zugesetzt.

Der Erfindung lag die Aufgabe zugrunde, neue stabile flüssigkristalline oder mesogene Verbindungen aufzufinden, die als Komponenten derartiger Phasen geeignet sind.

Es wurde gefunden, daß die Verbindungen der Formel I als Komponenten flüssigkristalliner Phasen vorzüglich geeignet sind. Insbesondere sind mit ihrer Hilfe stabile chirale $S_c$ Phasen in einem für die elektrooptische Anwendung günstig gelegenen Temperaturbereich herstellbar.

Überraschenderweise zeigte sich beim Zusatz von Verbindungen der Formel I zu unpolaren flüssigkristallinen Phasen, daß selbst kleinere Zusätze bereits die spontane Polarisation beträchtlich erhöhen können.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung ferroelektrischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie und/oder die spontane Polarisation einer solchen Phase zu variieren. Die Verbindungen der Formel I eignen sich ferner als Zwischenprodukte zur Herstellung anderer Substanzen, die sich als Bestandteile flüssigkristalliner Phasen verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I sowie die Verwendung der Verbindungen der Formel I als Komponenten flüssigkristalliner Phasen. Gegenstand der Erfindung sind ferner flüssigkristalline Phasen, insbesondere ferroelektrische flüssigkristalline Phasen, mit einem Gehalt an mindestens einer Verbindung der Formel I sowie Flüssigkristallanzeigeelemente, insbesondere elektrooptische Anzeigeelemente, die derartige Phasen enthalten.

Vor- und nachstehend haben $R^1$, $R^2$, $R^3$, A, $A^1$, $A^2$, $A^3$, $A^4$, $Z^1$, $Z^2$, $Z^3$ und p die angegebene Bedeutung, sofern nicht ausdrücklich etwas anderes vermerkt ist.

Die Verbindungen der Formel I umfassen dementsprechend insbesondere Verbindungen der Teilformeln I1 und I2 (mit zwei Ringen), I3-I20 (mit 3 Ringen), I21-I71 (mit 4 Ringen), sowie I72-I94 (mit 5 Ringen):

| | |
|---|---|
| $R^1$-A-$A^2$-$R^2$ | I1 |
| $R^1$-A-$Z^1$-$A^2$-$R^2$ | I2 |
| $R^1$-$A^4$-A-$A^2$-$R^2$ | I3 |
| $R^1$-A-$A^4$-$A^2$-$R^2$ | I4 |
| $R^3$-$A^3$-A-$A^2$-$R^2$ | I5 |
| $R^1$-A-$A^2$-$A^3$-$R^3$ | I6 |
| $R^1$-$A^4$-$Z^3$-A-$A^2$-$R^2$ | I7 |
| $R^1$-A-$Z^3$-$A^4$-$A^2$-$R^2$ | I8 |
| $R^1$-A-$Z^1$-$A^2$-$A^3$-$R^3$ | I9 |
| $R^1$-$A^4$-$Z^3$-A-$A^2$-$R^2$ | I10 |
| $R^1$-A-$Z^3$-$A^4$-$A^2$-$R^2$ | I11 |
| $R^1$-$A^4$-A-$Z^1$-$A^2$-$R^2$ | I12 |
| $R^1$-A-$A^4$-$Z^1$-$A^2$-$R^2$ | I13 |
| $R^3$-$A^3$-A-$Z^1$-$A^2$-$R^2$ | I14 |
| $R^1$-A-$A^2$-$Z^2$-$A^3$-$R^3$ | I15 |
| $R^3$-$A^3$-$Z^2$-A-$A^2$-$R^2$ | I16 |
| $R^3$-$A^3$-$Z^2$-A-$Z^1$-$A^2$-$R^2$ | I17 |
| $R^1$-A-$Z^1$-$A^2$-$Z^2$-$A^3$-$R^3$ | I18 |
| $R^1$-$A^4$-$Z^3$-A-$Z^1$-$A^2$-$R^2$ | I19 |
| $R^1$-A-$Z^3$-$A^4$-$Z^1$-$A^2$-$R^2$ | I20 |
| $R^3$-$A^3$-$A^4$-A-$A^2$-$R^2$ | I21 |
| $R^3$-$A^3$-A-$A^4$-$A^2$-$R^2$ | I22 |
| $R^1$-$A^4$-A-$A^2$-$A^3$-$R^3$ | I23 |
| $R^1$-A-$A^4$-$A^2$-$A^3$-$R^3$ | I24 |
| $R^3$-$A^3$-A-$A^2$-$A^3$-$R^3$ | I25 |
| $R^3$-$A^3$-$A^3$-A-$A^2$-$R^3$ | I26 |
| $R^1$-A-$A^2$-$A^3$-$A^3$-$R^3$ | I27 |
| $R^3$-$A^3$-$2^2$-A-$A^2$-$A^3$-$R^3$ | I28 |

| | |
|---|---|
| $R^3$-$A^3$-A-$Z^1$-$A^2$-$A^3$-$R^3$ | I29 |
| $R^3$-$A^3$-A-$Z^1$-$A^2$-$A^3$-$R^3$ | I30 |
| $R^3$-$A^3$-A-$A^2$-$Z^2$-$A^3$-$R^3$ | I31 |
| $R^1$-A-$A^4$-$Z^1$-$A^2$-$A^3$-$R^3$ | I32 |
| $R^1$-$A^4$-A-$Z^1$-$A^2$-$A^3$-$R^3$ | I33 |
| $R^3$-$A^3$-$A^4$-A-$Z^1$-$A^2$-$R^2$ | I34 |
| $R^3$-$A^3$-A-$A^4$-$Z^1$-$A^2$-$R^2$ | I35 |
| $R^1$-$A^4$-A-$A^2$-$Z^2$-$A^3$-$R^3$ | I36 |
| $R^1$-A-$A^4$-$A^2$-$Z^2$-$A^3$-$R^3$ | I37 |
| $R^3$-$A^3$-$Z^2$-$A^4$-A-$A^2$-$R^2$ | I38 |
| $R^3$-$A^3$-$Z^2$-A-$A^4$-$A^2$-$R^2$ | I39 |
| $R^3$-$A^3$-A-$A^2$-$Z^2$-$A^3$-$R^3$ | I40 |
| $R^3$-$A^3$-$Z^2$-A-$A^2$-$A^3$-$R^3$ | I41 |
| $R^3$-$A^3$-$A^3$-A-$Z^1$-$A^2$-$R^2$ | I42 |
| $R^3$-$A^3$-$A^4$-$Z^3$-A-$A^2$-$R^2$ | I43 |
| $R^3$-$A^3$-A-$Z^3$-$A^4$-$A^2$-$R^2$ | I44 |
| $R^1$-A-$A^2$-$Z^2$-$A^3$-$A^3$-$R^3$ | I45 |
| $R^1$-$A^4$-$Z^3$-A-$A^2$-$A^3$-$R^3$ | I46 |
| $R^1$-A-$Z^3$-$A^4$-$A^2$-$A^3$-$R^3$ | I47 |
| $R^3$-$A^3$-$A^3$-$Z^2$-A-$A^2$-$R^2$ | I48 |
| $R^1$-A-$Z^1$-$A^2$-$A^3$-$A^3$-$R^3$ | I49 |
| $R^3$-$A^3$-$Z^2$-$A^4$-A-$Z^1$-$A^2$-$R^2$ | I50 |
| $R^3$-$A^3$-$Z^2$-A-$A^4$-$Z^1$-$A^2$-$R^2$ | I51 |
| $R^1$-$A^4$-A-$Z^1$-$A^2$-$Z^2$-$A^3$-$R^3$ | I52 |
| $R^1$-A-$A^4$-$Z^1$-$A^2$-$Z^2$-$A^3$-$R^3$ | I53 |
| $R^3$-$A^3$-A-$Z^1$-$A^2$-$Z^2$-$A^3$-$R^3$ | I54 |
| $R^3$-$A^3$-$Z^2$-A-$A^2$-$Z^2$-$A^3$-$R^3$ | I55 |
| $R^3$-$A^3$-$Z^2$-A-$Z^1$-$A^2$-$A^3$-$R^2$ | I56 |
| $R^3$-$A^3$-$A^4$-$Z^3$-A-$Z^1$-$A^2$-$R^2$ | I57 |
| $R^3$-$A^3$-A-$Z^3$-$A^4$-$Z^1$-$A^2$-$R^2$ | I58 |
| $R^1$-$A^4$-$Z^3$-A-$A^2$-$Z^2$-$A^3$-$R^3$ | I59 |
| $R^1$-A-$Z^3$-$A^4$-$A^2$-$Z^2$-$A^3$-$R^3$ | I60 |
| $R^3$-$A^3$-$Z^2$-$A^4$-$Z^3$-A-$A^2$-$R^2$ | I61 |
| $R^3$-$A^3$-$Z^2$-A-$Z^3$-$A^4$-$A^2$-$R^2$ | I62 |
| $R^1$-$A^4$-$Z^3$-A-$Z^1$-$A^2$-$A^3$-$R^3$ | I63 |
| $R^1$-A-$Z^3$-$A^4$-$Z^1$-$A^2$-$A^3$-$R^3$ | I64 |
| $R^3$-$A^3$-$A^3$-$Z^2$-A-$Z^1$-$A^2$-$R^2$ | I65 |
| $R^1$-A-$Z^1$-$A^2$-$Z^2$-$A^3$-$A^3$-$R^3$ | I66 |
| $R^3$-$A^3$-$Z^2$-A-$Z^1$-$A^2$-$Z^2$-$A^3$-$R^3$ | I67 |
| $R^3$-$A^3$-$Z^2$-$A^4$-$Z^3$-A-$Z^1$-$A^2$-$R^2$ | I68 |
| $R^3$-$A^3$-$Z^2$-A-$Z^3$-$A^4$-$Z^1$-$A^2$-$R^2$ | I69 |
| $R^1$-$A^4$-$Z^3$-A-$Z^1$-$A^2$-$Z^2$-$A^3$-$R^3$ | I70 |
| $R^1$-A-$Z^3$-$A^4$-$Z^1$-$A^2$-$Z^2$-$A^3$-$R^3$ | I71 |
| $R^3$-$A^3$-$A^4$-A-$A^2$-$A^3$-$R^3$ | I72 |
| $R^3$-$A^3$-A-$A^4$-$A^2$-$A^3$-$R^3$ | I73 |
| $R^3$-$A^3$-$A^4$-A-$A^2$-$A^3$-$R^3$ | I74 |
| $R^3$-$A^3$-A-$A^4$-$A^2$-$A^3$-$R^3$ | I75 |
| $R^1$-A-$A^4$-$A^2$-$A^3$-$A^3$-$R^3$ | I76 |
| $R^3$-$A^3$-$A^4$-A-$A^2$-$Z^2$-$A^3$-$R^3$ | I77 |
| $R^3$-$A^3$-A-$A^4$-$A^2$-$Z^2$-$A^3$-$R^3$ | I78 |
| $R^3$-$A^3$-$A^4$-A-$Z^1$-$A^2$-$A^3$-$R^3$ | I79 |
| $R^3$-$A^3$-A-$A^4$-$Z^1$-$A^2$-$A^3$-$R^3$ | I80 |
| $R^3$-$A^3$-$Z^2$-$A^4$-A-$A^2$-$A^3$-$R^3$ | I81 |
| $R^3$-$A^3$-$Z^2$-A-$A^4$-$A^2$-$A^3$-$R^3$ | I82 |
| $R^3$-$A^3$-$A^4$-A-$Z^1$-$A^2$-$Z^2$-$A^3$-$R^3$ | I83 |
| $R^3$-$A^3$-A-$A^4$-$Z^1$-$A^2$-$Z^2$-$A^3$-$R^3$ | I84 |
| $R^3$-$A^3$-$Z^2$-$A^4$-A-$A^2$-$Z^2$-$A^3$-$R^3$ | I85 |
| $R^3$-$A^3$-$Z^2$-A-$A^4$-$A^2$-$Z^2$-$A^3$-$R^3$ | I86 |

$R^3-A^3-Z^2-A^4-A-Z^1-A^2-A^3-R^3$ I87
$R^3-A^3-Z^2-A-A^4-Z^1-A^2-A^3-R^3$ I88
$R^3-A^3-Z^2-A^4-A-Z^1-A^2-Z^2-A^3-R^3$ I89
$R^3-A^3-Z^2-A-A^4-Z^1-A^2-Z^2-A^3-R^3$ I90
$R^1-A^4-Z^3-A-Z^1-A^2-Z^2-A^3-A^3-R^3$ I91
$R^1-A-Z^3-A^4-Z^1-A^2-Z^2-A^3-A^3-R^3$ I92
$R^3-A^3-A^3-Z^2-A-Z^1-A^2-Z^2-A^3-R^3$ I93
$R^3-A^3-Z^2-A-Z^1-A^2-Z^2-A^3-A^3-R^3$ I94

Besonders bevorzugte kleinere Gruppen von Verbindungen sind diejenigen der Formeln I001 bis I022:

$R^1$-Phe-$Z^1$-A-$R^2$ I001
$R^1$-Cy-$Z^1$-A-$R^2$ I002
$R^1$-Dio-$Z^1$-A-$R^2$ I003
$R^1$-Pip-$Z^1$-A-$R^2$ I004
$R^1$-Bi-$Z^1$-A-$R^2$ I005
$R^1$-Pyr-$Z^1$-A-$R^2$ I006
$R^1$-Phe-Z-A-$Z^2$-Phe-$R^2$ I007
$R^1$-Dio-$Z^1$-A-$Z^2$-Cy-$R^2$ I008
$R^1$-Cy-$Z^1$-A-$Z^2$-Phe-$R^2$ I009
$R^1$-Cy-$Z^1$-A-$Z^2$-Cy-$R^2$ I010
$R^1$-Phe-Phe-$Z^1$-A-$R^2$ I011
$R^1$-Phe-Cy-$Z^1$-A-$R^2$ I012
$R^1$-Cy-Phe-$Z^1$-A-$R^2$ I013
$R^1$-Cy-Cy-$Z^1$-A-$R^2$ I014
$R^1$-Phe-Phe-$Z^1$-A-$Z^2$-Phe-$R^2$ I015
$R^1$-Phe-Phe-$Z^1$-A-$Z^2$-Cy-$R^2$ I016
$R^1$-Phe-Cy-$Z^1$-A-$Z^2$-Phe-$R^2$ I017
$R^1$-Phe-Cy-$Z^1$-A-$Z^2$-Cy-$R^2$ I018
$R^1$-Cy-Phe-$Z^1$-A-$Z^2$-Phe-$R^2$ I019
$R^1$-Cy-Phe-$Z^1$-A-$Z^2$-Cy-$R^2$ I020
$R^1$-Cy-Cy-$Z^1$-A-$Z^2$-Phe-$R^2$ I021
$R^1$-Cy-Cy-$Z^1$-A-$Z^2$-Cy-$R^2$ I022

In den Verbindungen der vorstehenden Teilformeln I001 bis I022 bedeuten $Z^1$ und $2^2$ jeweils vorzugsweise eine Einfachbindung. Die Teilformeln I001, I009 und I013 sind besonders bevorzugt.

In den Verbindungen der vor- und nachstehenden Formeln bedeutet eine substituierte Alkylgruppe bzw. substituiertes Ethylen eine durch Halogen, vorzugsweise Fluor oder Chlor, oder CN ein oder mehrfach an verschiedenen C-Atomen substituierte Alkylgruppe bzw. $-CH_2CH_2-$ (Ethylen) -Gruppe. Vorzugsweise ist die Alkylgruppe nur einfach durch Halogen oder CN substituiert. Das mit Halogen oder CN verknüpfte C-Atom ist vorzugsweise ein asymmetrisches Kohlenstoffatom.

Einer der Reste $R^1$ und $R^2$ bedeutet vorzugsweise Alkyl, -0-Alkyl oder Oxaalkyl, -C00-Alkyl, -0C0-Alkyl, -C0- Alkyl oder Alkenyl.

Alkenylgruppen in den Verbindungen der Formel I sind vorzugsweise geradkettige trans-Alkenylgruppen der Formel

$$CH_3-(CH_2)_{n1}-CH=HC-(CH_2)_{n2}-$$

worin

n2 0 bis 10, vorzugsweise 2 bis 10, und
n1 0 bis 5, vorzugsweise 0, bedeutet.

Die Alkylreste, in denen eine ("Alkoxy" bzw. "0xaalkyl") oder zwei ("Alkoxyalkoxy" bzw. "Dioxaalkyl") nicht benachbarte $CH_2$-Grugpen durch 0-Atome ersetzt sein können, können geradkettig oder verzweigt sein. Vorzugsweise sind sie geradkettig, haben 2, 3, 4, 5, 6, 7, 8, 9, 10, 11, 12, 13, 14 oder 15 C-Atome und bedeuten demnach bevorzugt 2-Oxapropyl (= Methoxymethyl), 2- (= Ethoxymethyl) oder 3-Oxabutyl (= 2-Methoxymethyl), 2-, 3- oder 4-Oxapentyl, 2-, 3-, 4- oder 5-Oxahexyl, 2-, 3-, 4-, 5-, oder 6-Oxaheptyl, 2-, 3,- 4,- 5,- 6,- oder 8-Oxaoctyl, 2-, 3,- 4,- 5,- 6,- oder 9-Oxanonyl, 2-, 3,- 4,- 5,- 6,- oder 10- Oxadecyl, 2-, 3,- 4,- 5,- 6,- oder 11-Oxaundecyl, 2-, 3,- 4,- 5,- 6,- oder 12-Oxadodecyl, 2-, 3,- 4,- 5,- 6,- oder 13-Oxatridecyl, 2-, 3,- 4,- 5,- 6,- oder

14-Oxatetradecyl, 2-, 3,- 4,- 5,- 6,- oder 15-Oxapentadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5,- 3,6- oder 4,6-Dioxaheptyl.

Die Alkylrest in den Gruppen $R^1$ und/oder $R^2$ können geradkettig oder verzweigt sein. Vorzugsweise sind sie geradkettig, haben 2, 3, 4, 5, 6, 7, 8, 9, 10, 11 oder 12, 13, 14 oder 15 C-Atome und bedeuten demnach bevorzugt Ethyl, Propyl, Butyl, Pentyl, Hexyl, Heptyl, Octyl, Nonyl, Decyl, Undecyl, Dodecyl, Tridecyl, Tetradecyl oder Pentadecyl, ferner Methyl.

Der andere Rest $R^1$ oder $R^2$ ist ein optisch aktiver organischer Rest mit einem asymmetrischen Kohlenstoffatom. Vorzugsweise ist das asymmetrische Kohlenstoffatom mit zwei unterschiedlich substituierten C-Atomen, einem H-Atom und einem Substituenten ausgewählt aus der Gruppe Halogen (insbesondere F, Cl oder Br), Alkyl oder Alkoxy mit jeweils 1 bis 5 C-Atomen und CN verknüpft. Der optisch aktive organische Rest hat vorzugsweise die Formel,

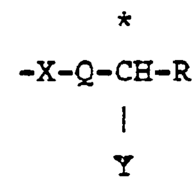

worin X -CO-O-, -O-CO-, -O-CO-O-, -CO-, -O-, -S-, -CH=CH-, -CH=CH-COO- oder eine Einfachbindung,

Q Alkylen mit 1 bis 5 C-Atomen, worin auch eine nicht mit X verknüpfte $CH_2$-Gruppe durch -O-, -CO-, -O-CO-, -CO-O- oder -CH=CH- ersetzt sein kann, oder eine Einfachbindung,

Y CN, Halogen, Methyl oder Methoxy, und

R eine von Y verschiedene Alkylgruppe mit 1 bis 18 C-Atomen, worin auch eine oder zwei nicht benachbarte $CH_2$-Gruppen durch -O-, -CO-, -O-CO-, -CO-O- und/oder -CH=CH- ersetzt sein können,

bedeutet.

X ist vorzugsweise -CO-O-, -O-CO-, -O-, -CH=CH-COO- (trans) oder eine Einfachbindung. Besonders bevorzugt sind -O-, -CO-O- und -O-CO-.

Q ist vorzugsweise Alkylen mit 1 bis 5 C-Atomen oder eine Einfachbindung, insbesondere bevorzugt $-CH_2-$, $-CH_2CH_2-$ oder eine Einfachbindung.

Y ist vorzugsweise $CH_3$, -CN oder Cl, insbesondere bevorzugt -CN oder Cl.

R ist vorzugsweise geradkettiges Alkyl mit 1 bis 10, insbesondere mit 1 bis 7, C-Atomen, worin gegebenenfalls die mit dem asymmetrischen C-Atom verknüpfte $CH_2$-Gruppe durch -O-, -O-CO- oder -CO-O- ersetzt sein kann.

Vorzugsweise ist $R^2$ in formel I der optisch aktive Rest.

$A^2$, $A^3$ und $A^4$ sind bevorzugt unabhängig voneinander Cy, Dio oder Phe, ferner bevorzugt Dit, Pyr oder Pip; bevorzugt enthält die Verbindung der Formel I nicht mehr als einen der Reste Dio, Dit, Pip, Bi, Pyn oder Pyr.

$A^1$ ist vorzugsweise -A- oder $-A^4-Z^3-A-$.

A ist vorzugsweise Pyrimidin-2,5-diyl, Pyridin-2,5-diyl, Pyridazin-3,6-diyl oder Pyrazin-2,5-diyl, insbesondere bevorzugt Pyrimidin-2,5-diyl oder Pyridin-2,5-diyl.

$Z^1$, $Z^2$ und $Z^3$ sind bevorzugt unabhängig voneinander -0-C0-, -C0-0-Gruppen oder Einfachbindungen.

$-A^1-Z^1-A^2-$ ist vorzugsweise ein Strukturelement ausgewählt aus der Gruppe der Formeln 1 bis 4:

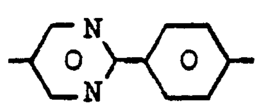,

1

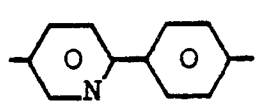,

2

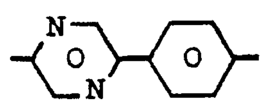,

3

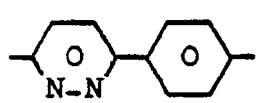

4

Gruppen der Formeln 1 und 2 sind besonders bevorzugt.

Unter den Verbindungen der Formeln I sowie der vor- und nachstehenden Teilformeln sind diejenigen bevorzugt, in denen mindestens einer der darin enthaltenen Reste eine der angegebenen bevorzugten Bedeutungen hat. Besonders bevorzugte kleinere Gruppen von Verbindungen sind diejenigen der Formeln I023 bis I031:

| | |
|---|---|
| R°-Pyr-Phe-R* | I023 |
| R°-Pyr-Cy-R* | I024 |
| R°-Pyr-$CH_2CH_2$-Cy-R* | I025 |
| R°-Pyr-COO-Phe-R* | I026 |
| R°-Pyr-OCO-Phe-R* | I027 |
| R°-Pyr-COO-Cy-R* | I028 |
| R°-Pyr-Phe-$CH_2CH_2$-Cy-R* | I029 |
| R°-Pyr-Phe-O-$CH_2$-Cy-R* | I030 |
| R°-Pyr-Phe-O-$CH_2$-Phe-R* | I031 |

worin R° vorzugsweise geradkettiges Alkyl oder Alkoxy mit jeweils 2 bis 12 C-Atomen bedeutet, Pyr vorzugsweise Pyrimidin-2,5-diyl ist und R* vorzugsweise eine der für den optisch aktiven organischen Rest angegebenen bevorzugten Bedeutungen hat.

Weitere besonders bevorzugte kleinere Gruppen von Verbindungen sind diejenigen der Formeln I032 bis I096:

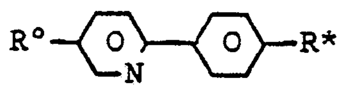

I032

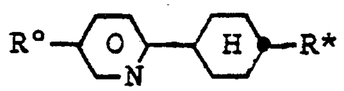

I033

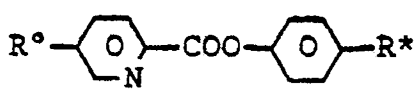

I034a

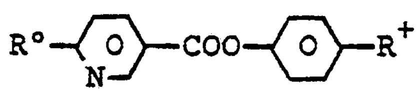

I034b

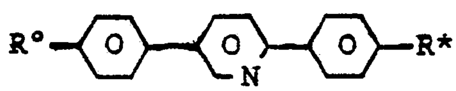

I035

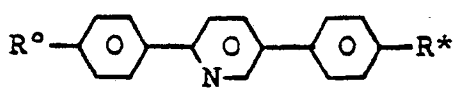

I036

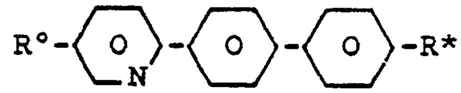 I037

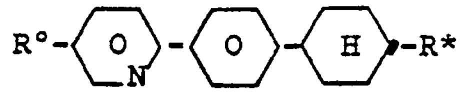 I038

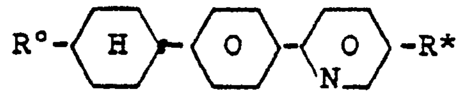 I039

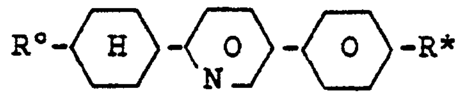 I040

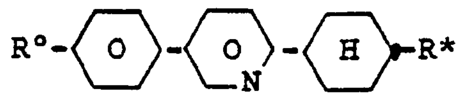 I041

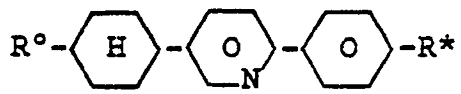 I042

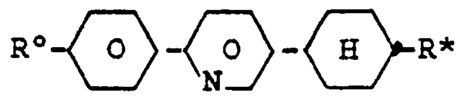 I043

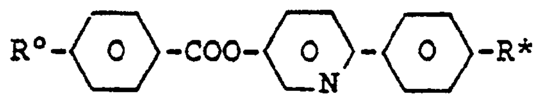 I044

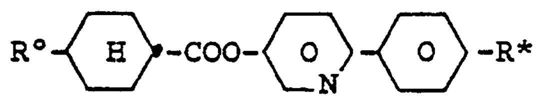 I045

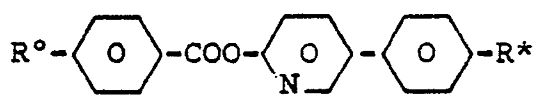 I046

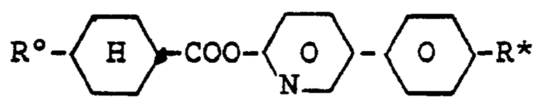 I047

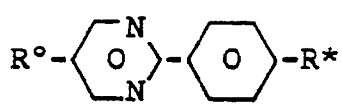 I048

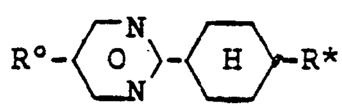 I049

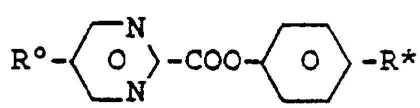 I050

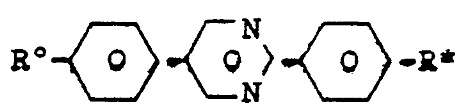 I051

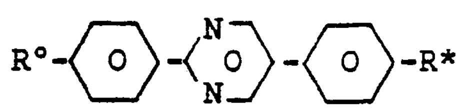 I052

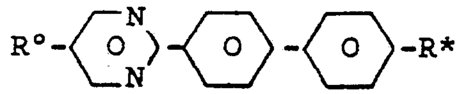

I053

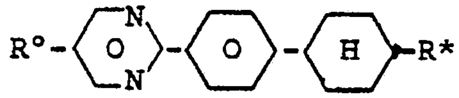

I054

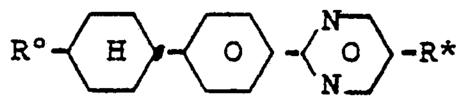

I055

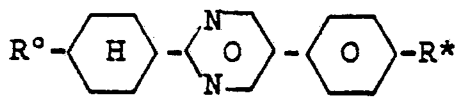

I056

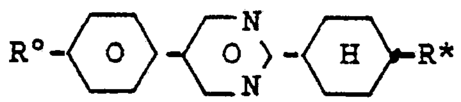

I057

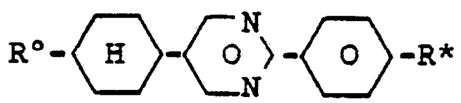

I058

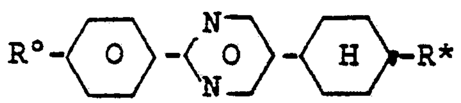

I059

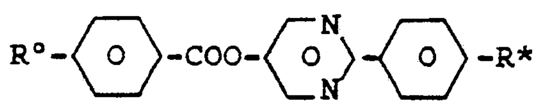

I060

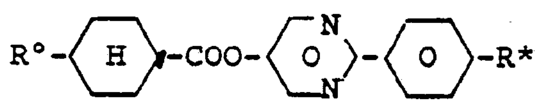

I061

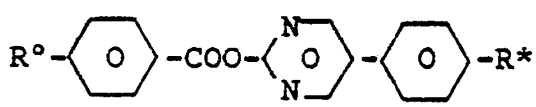

I062

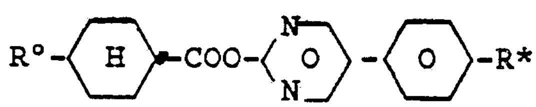

I063

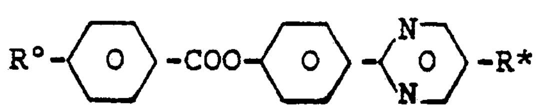

I064

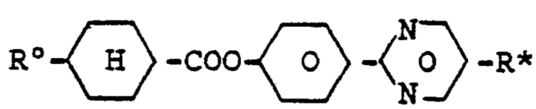

I065

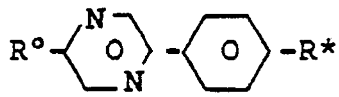

I066

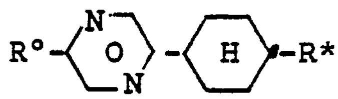

I067

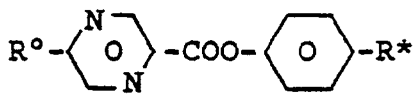

I068

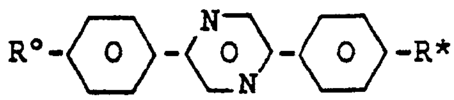

I069

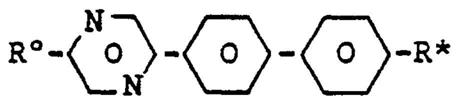

I070

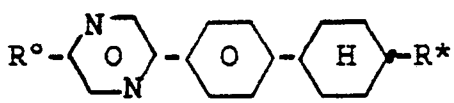

I071

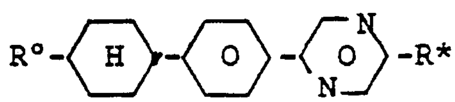

I072

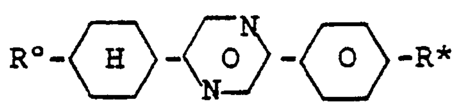

I073

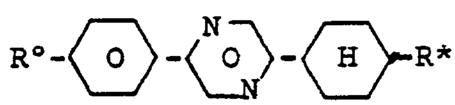

I074

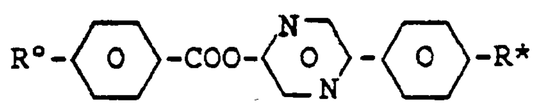

I075

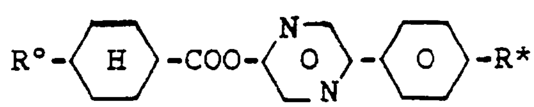

I078

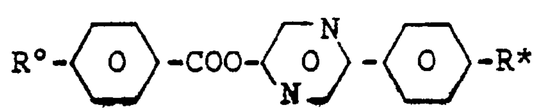

I079

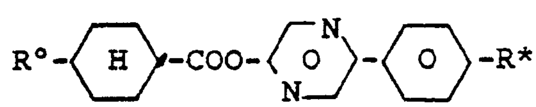

I080

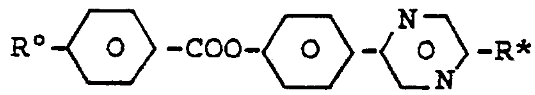 I081

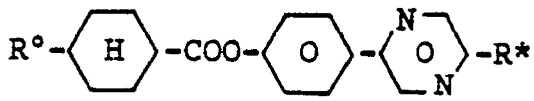 I082

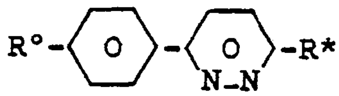 I083

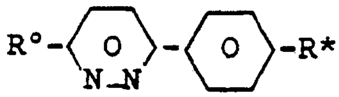 I084

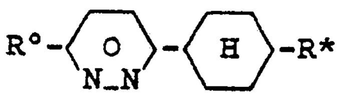 I085

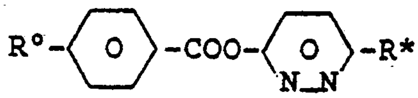 I086

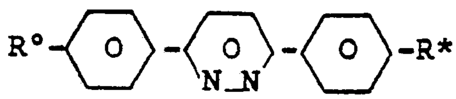 I087

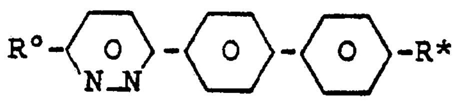 I088

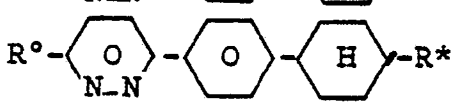 I089

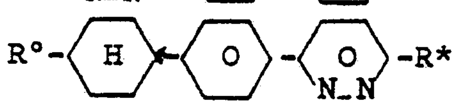 I090

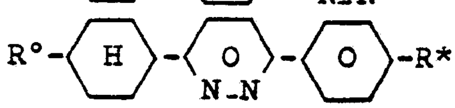 I091

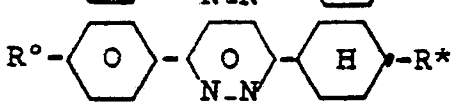 I092

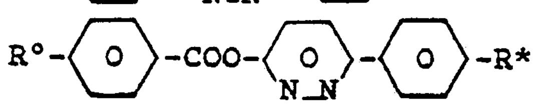 I093

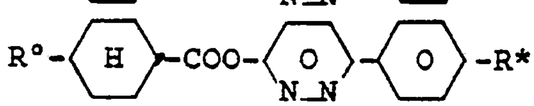 I094

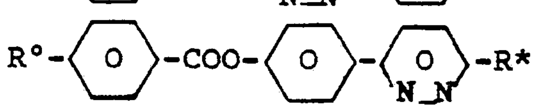 I095

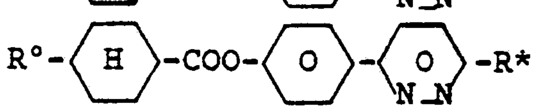 I096

Weitere besonders bevorzugte Pyridinverbindungen sind diejenigen der Formeln I097 bis I117:

| | |
|---|---|
| R°-Pyr-Bi-R* | I097 |
| R°-Bi-Pyr-R* | I098 |
| R°-Pyr-OCO-Phe-R* | I099 |
| R°-Phe-COO-Pyr-R* | I100 |
| R°-Pyr-Phe-Cy-R* | I101 |
| R°-Cy-Phe-Pyr-R* | I102 |

R°-Pyr-Phe-Phe-R* I103
R°-Phe-Phe-Pyr-R* I104
R°-Pyr-Phe-Bi-R* I105
R°-Bi-Phe-Pyr-R* I106
R°-Pyr-Bi-Phe-R* I107
R°-Phe-Bi-Pyr-R* I108
R°-Pyr-Cy-Cy-R* I109
R°-Cy-Cy-Pyr-R* I110
R°-Cy-Pyr-Cy-R* I111
R°-Pyr-Phe-$CH_2CH_2$-Cy-R* I112
R°-Cy-$CH_2CH_2$-Phe-Pyr-R* I113
R°-Pyr-Cy-$CH_2CH_2$-Cy-R* I114
R°-Cy-$CH_2CH_2$-Cy-Pyr-R* I115
R°-Phe-Pyr-Cy-R* I116
R°-Cy-Pyr-Phe-R* I117

Pyr bedeutet in den Formeln I097 bis I117 Pyridin-2,5-diyl.

Besonders bevorzugt sind optisch aktive Pyridin-Verbindungen der Formel I'

$$R^1\text{-}A^1\text{-}Z^1\text{-}A^2\text{-}R^2 \quad I'$$

worin

einer der Reste $R^1$ und $R^2$

H, eine unsubstituierte oder substituierte Alkyl-gruppe mit 1-15 C-Atomen, worin auch eine oder zwei nicht benachbarte $CH_2$-Gruppen durch mindestens eine Gruppierung aus der Gruppe -O-, -CO-, -O-CO-, -CO-O- und -CH=CH- ersetzt sein können, F, Cl, Br, -CN, -NCS oder $R^3$-$(A^3)_p$-$Z^2$-,

der andere Rest $r^1$ oder $R^2$

ein optisch aktiver organischer rest mit einem asymmetrischen Kohlenstoffatom,

$A^1$ -A-, -$A^4$-$Z^3$-A- oder -A-$Z^3$-$A^4$-,

A 2,5-Pyridindiyl,

$A^2$, $A^3$ und $A^4$ jeweils unsubstituiertes oder durch ein oder zwei F- und/oder Cl-Atome und/oder $CH_3$-Gruppen und/oder CN-Gruppen substituiertes 1,4-Phenylen, worin auch eine oder zwei CH-Gruppen durch N-Atome ersetzt sein können, 1,4-Cyclohexylen, worin auch eine oder zwei nicht benachbarte $CH_2$-Gruppen durch O-Atome und/oder S-Atome ersetzt sein können, Piperidin-1,4-diyl, 1,4-Bicyclo(2,2,2)-octylen, unsubstituiertes oder durch CN substituiertes Decahydronaphthalin-2,6-diyl oder 1,2,3,4-Tetrahydronaphthalin-2,6-diyl,

$Z^1$, $Z^2$ und $Z^3$ jeweils -CO-O, -O-CO-, -$OCH_2$-, -$CH_2O$-, -$CH_2CH_2$-, substituiertes Ethylen oder eine Einfachbindung,

$R^3$ H, eine unsubstituierte oder substituierte Alkylgruppe mit 1-15 C-Atomen, worin auch eine oder zwei nicht benachbarte $CH_2$-Gruppen durch eine Gruppierung aus der Gruppe -O-, -CO-, -O-CO-, -CO-O und -CH=CH- ersetzt sein können, F, Cl, Br, -NCS oder -CN, und

p 1 oder 2

bedeutet, wobei für p = 2 die Gruppen $A^3$ gleich oder voneinander verschieden sein können, sowie flüssigkristalline ferroelektrische Phasen mit einem Gehalt an mindestens einer Verbindung der Formel I' und diese Phasen enthaltende elektrooptische Anzeigeelemente. Die für Formel I angegebenen bevorzugten Bedeutungen für $R^1$, $R^2$, $R^3$, $A^1$, $A^2$, $A^3$, $A^4$, $Z^1$, $Z^2$, $Z^3$ und p gelten analog für Verbindungen der Formel I'.

Unter den Verbindungen der Formel I sind diejenigen Stereoisomeren bevorzugt, in denen die gesättigten Ringe (z.B. Cy, Dio, Dit) trans-1,4-disubstituiert sind.

Im Falle $R^1$-$A^1$-$Z^1$-$A^2$-$R^2$ =

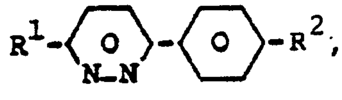

wobei

$R^1$ eine optisch aktive Alkoxygruppe mit einem asymmetrischen C-Atom ist, bedeutet $R^2$ vorzugsweise eine unsubtuierte oder substituierte Alkylgruppe mit 1-15 C-Atomen, worin eine oder zwei nicht benachbarte $CH_2$-Gruppen durch mindestens eine Gruppierung aus der Gruppe -CO-, -O-CO-, -CO-O und -CH=CH- ersetzt sein können, F, Cl, Br, -CN-, -NCS oder $R^3$-$(A^3)_p$-$Z^2$-.

Im Falle $R^1$-$A^1$-$Z^1$-$A^2$-$R^2$ =

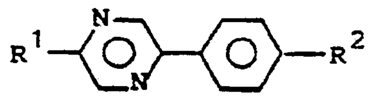,

wobei

$R^2$ eine optisch aktive Alkoxygruppe mit einem asymmetrischen C-Atom ist, bedeutet $R^1$ vorzugsweise eine unsubstituierte oder substituierte Alkylgruppe mit 1-15 C-Atomen, worin auch eine oder zwei nicht benachbarte $CH_2$-Gruppen durch -O- oder -CH=CH- ersetzt sein können. Eine besonders bevorzugte Bedeutung für $R^1$ ist hier geradkettiges Alkyl oder Alkoxy, insbesondere geradkettiges Alkyl, mit jeweils 5 - 12 C-Atomen.

Im falle $R^1$-$A^1$-$Z^1$-$A^2$-$R^2$ =

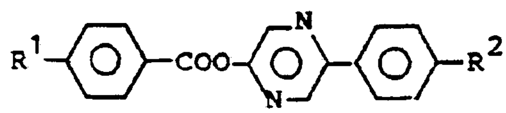

oder $R^1$

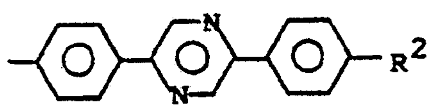,

wobei $R^2$ eine optisch aktive Alkoxygruppe mit einem asymmetrischen C-Atom ist, bedeutet $R^1$ vorzugsweise eine unsubstituierte oder substituierte Alkylgruppe mit 1-15 C-Atomen, worin zwei nicht benachbarte $CH_2$-Gruppen durch -O-, -CO-, -O-CO-, -CO-O- und/oder -CH=CH- ersetzt sind.

Im falle A =

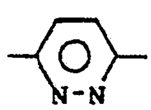 , 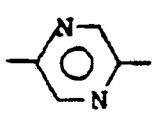 oder 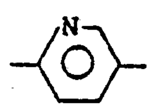 ,

insbesondere im falle A =

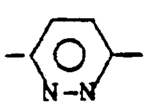 oder 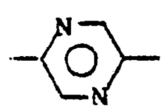

bedeutet der optisch aktive Rest $R^1$ oder $R^2$ vorzugsweise eine Alkylgruppe, worin zwei nicht benachbarte $CH_2$-Gruppen durch mindestens eine Gruppierung aus der Gruppe -O-, -CO-, -O-CO-, -CO-O- und -CH=CH- ersetzt sind. Besonders bevorzugte optisch aktive Reste entsprechen hier der formel

$$-X-Q-\underset{\displaystyle Y}{\overset{*}{C}H}-R,$$

worin X

-O-, -CO-O- oder -O-CO-, Q -$CH_2$- oder eine Einfachbindung, Y $CH_3$ und R geradkettiges Alkyl mit 1 bis 7 C-Atomen bedeutet, worin die mit den asymmetrischen C-Atomen verknüpfte $CH_2$ -Gruppe durch -O-, -CO-O- oder -O-CO- ersetzt ist.

Die Verbindungen der Formel I werden nach an sich bekannten Methoden hergestellt. Wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten

Gebrauch machen.

Der Fachmann kann durch Routinemethoden entsprechende Synthesemethoden aus dem Stand der Technik entnehmen (z.B. DE-OS 23 44 732, 24 50 088, 24 29 093, 25 02 904, 26 36 684, 27 01 591 und 27 52 975 betreffend Verbindungen mit 1,4-Cyclohexylen und 1,4-Phenylen-Gruppen; DE-PS 26 41 724 betreffend Verbindungen mit Pyrimidin-2,5-diyl-Gruppen; DE-OS 29 44 905 und 32 27 916 betreffend Verbindungen mit 1,3-Dioxan-2,5-diyl-Gruppen); DD 160 061 betreffend Verbindungen mit 1,3-Dithian-2,5-diyl-Gruppen; US 4,261,652 und 4,219,256 betreffend Verbindungen mit 1,4-Bicyclo(2,2,2)-octylen-Gruppen; und DE-OS 32 01 721 betreffend Verbindungen mit $-CH_2CH_2$-Brückengliedern.

Die Ausgangsstoffe können gewünschtenfalls auch in situ gebildet werden, derart, daß man sie aus dem Reaktions-gemisch nicht isoliert, sondern sofort weiter zu den Verbindungen der Formel I umsetzt.

Im allgemeinen werden zwei entsprechende Teilverbindungen (z.B. (1) und (2) (Schema 1) oder (3) und (4) (Schema 2) zu Verbindungen der Formel I kondensiert:

Schema 1:

$R'-M^1L^1$ + $L^2M^2-R''$

(1) (2)

↓ $-L^1L^2$

$R'-M^1M^1-R''$ I ($M^1M^2 = A$)

Schema 2:

$R'-M^3L^3$ + $L^4M^4-R''$

(3) (4)

↓ $-L^3L^4$

$R'-M^3M^4-R''$ I

($M^3M^4 = Z$)

$-M^1L^1$ und $-M^2L^2$ sind kondensationsfähige Bausteine, die z.B. Malonsäurederivaten (z.B. Malondialdehyd), Amidinen, Aldehyden, 1,3-Propandiolen und/oder 1,3-Propandithiolen entsprechen. $L^1L^2$ sind eine oder mehrere abgespaltene Gruppen.

$-M^3L^3$ und $-M^4L^4$ sind kondensationsfähige Bausteine, z.B. ausgewählt aus der Gruppe -COOH, -COHalogen, -OH, -OMetall, $-CH_2$-Halogen, $-CH_2$-Metall, $-CH_2$-OH, $-CH_2$-O-Metall, -Metall, -Halogen.

$L^3$ und $L^4$ sind Abgangsgruppen. $L^3L^4$ ist eine abgespaltene Gruppe wie z.B. $H_2O$, H-Halogen, Metall-Halogen.

Weiterhin können jedoch auch entsprechende intramolekulare Kondensationen zur Synthese von Verbindungen der Formel I durchgeführt werden (z.B. Kondensation von 1,4-Diketonen mit Hydrazin (z.B. DE-OS 32 38 350) oder Umsetzung eines Butadienderivates z.B. mit Acetylendicarbonsäurederivaten (z.B. JP-OS 58-144327; JP-OS 58-146543).

Die Ausgangsstoffe sind bekannt oder können nach analogen Methoden wie die bekannten Verbindungen

erhalten werden. Der Fachmann kann durch Routinemethoden entsprechende Ausgangsstoffe und/oder Methoden zu deren Synthese aus dem Stand der Technik entnehmen.

Die Verbindungen der Formel I können ferner hergestellt werden, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise Carbonyl-gruppen in Betracht, insbesondere Ketogruppen, ferner z.B. freie oder veresterte Hydrogruppen oder aromatisch gebundene Halogenatome. Bevorzugte Ausgangsstoffe für die Reduktion entsprechen der Formel I, können aber an Stelle eines Cyclohexanrings einen Cyclohexenring oder Cyclohexanonring und/oder an Stelle einer -$CH_2CH_2$-Gruppe eine -CH=CH-Gruppe und/oder an Stelle einer -$CH_2$-Gruppe eine -CO-Gruppe und/oder an Stelle eines H-Atomes eine freie oder eine funktionell (z.B. in Form ihres p-Toluolsulfonats) abgewandelte OH-Gruppe enthalten.

Die Reduktion kann z.B. erfolgen durch katalytische Hydrierung bei Temperaturen zwischen etwa 0° und etwa 200° sowie Drucken zwischen etwa 1 und 200 bar in einem inerten Lösungsmittel, z.B. einem Alkohol wie Methanol, Ethanol oder Isopropranol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z.B. $Pt0_2$, Pd0), auf einem Träger (z.B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120 °) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperaturen zwischen etwa 100 und 200°) zu den entsprechenden Verbindungen der Formel I, die Alkylgruppen und/oder -$CH_2CH_2$-Brücken enthalten, reduziert werden.

Weiterhin sind Reduktionen mit komplexen Hydriden möglich. Beispielsweise können Arylsulfonyloxygruppen mit $LiAlH_4$ reduktiv entfernt werden, insbesondere p-Toluolsulfonyloxymethylgruppen zu Methylgruppen reduziert werden, zweckmäßig in einem inerten Lösungsmittel wie Diethylether oder THF bei Temperaturen zwischen etwa 0 und 100°. Doppelbindungen können (auch in Gegenwart von CN-Gruppen!) mit $NaBH_4$ oder Tributylzinnhydrid in Methanol hydriert werden; so entstehen z.B. aus 1-Cyancyclohexen-derivaten die entsprechenden Cyclohexanderivate.

Ester der Formel I ($R^1$ und/oder $R^2$ = Alkyl, worin eine oder zwei $CH_2$-Gruppen durch -0-C0- und/oder -C0-0-Gruppen ersetzt sind oder $Z^1$ und/oder $Z^2$ = -C0-0- oder -0-C0-) können auch durch Veresterung entsprechender Carbonsäuren (oder ihren reaktionsfähigen Derivaten mit Alkoholen bzw. Phenolen (oder ihrer reaktionsfähigen Derivaten erhalten werden.

Als reaktionsfähige Derivate der genannten Carbonsäuren eignen sich insbesondere die Säurehalogenide, vor allem die Chloride und Bromide, ferner die Anhydride, Azide oder Ester, insbesondere Alkylester mit 1 - 4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls wie Na oder K, in Betracht.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z.B. Pyridin, Chinolin oder Triethylamin als Lösungsmittel für die Veresterung angewandt werden. Die Veresterung kann auch in Abwesenheit eines Lösungsmittels, z.B. durch einfaches Erhitzen der Komponenten in Gegenwart von Natriumacetat, durchgeführt werden. Dit Reaktionstemperatur liegt gewöhnlich swischen -50° und +250°, vorzugsweise zwischen -20° und +80°. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie

Triethylamin, Pyridin, Lutidin, Kollidin oder Chinolin von Bedeutung sind. Eine weitere bevorzuge Ausführungsform der Veresterung besteht darin, daß man den Alkohol bzw. das Phenol zunächst in das Natrium- oder Kaliumalkoholat bzw. -phenolat überführt, z.B. durch Behandlung mit ethanolischer Natron- oder Kalilauge, diese isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylehter, Aceton oder DMF versetzt, zweckmäßig bei Temperaturen zwischen etwa -25° und +20°.

Dioxanderivate bzw. Dithianderivate der Formel I (worin eine der Gruppen $A^1$ und/oder $A^2$ und/oder $A^3$ eine 1,3-Dioxan-2,5-diyl-Gruppe bzw. eine 1,3-Dithian-2,5-diyl- Gruppt bedeutet) werden zweckmäßig durch Reaktion eines entsprechenden Aldehyds mit einem entsprechenden 1,3-Diol bzw. einem entsprechenden 1,3-Dithiol (oder einem seiner reaktionsfähigen Derivate) hergestellt, vorzugsweise in Gegenwart eines inerten Lösungsmittels wie Benzol oder Toluol und/oder eines Katalysators, z.B. einer starken Säure wie Schwefelsäure, Benzol- oder p-Toluolsulfonsäure, bei Temperaturen zwischen etwa 20° und etwa 150°, vorzugsweise zwischen 80° und 120°. Als reaktionsfähige Derivate der Ausgangsstoffe eignen sich in erster Linie Acetale.

Die genannten Aldehyde, 1-3-Diole und 1,3-Dithiole sowie ihre reaktionsfähigen Derivate sind zum Teil bekannt, zum Teil können sie ohne Schwierigkeiten nach Standard-verfahren der organischen Chemie aus literaturbekannten Verbindungen hergestellt werden. Beispielsweise sind die Aldehyde durch Oxydation entsprechender Carbonsäuren oder ihrer Derivate, die Diole durch Reduktion entsprechender Diester, sowie Dithiole durch Umsetzung entsprechender Dihalogenide mit NaSH erhältlich.

Zur Herstellung von Nitrilen der Formel I (worin $R^1$ und/ oder $R^2$ CN bedeutet) können entsprechende Säureamide dehydratisiert werden. Die Amide sind z.B. aus entsprechenden Estern oder Säurehalogeniden durch Umsetzung mit Ammoniak erhältlich. Als wasserabspaltende Mittel eigenen sich beispielsweise anorganische Säurechloride wie $SOCl_2$, $PCl_3$, $PCl_5$, $POCl_3$, $SO_2Cl_2$, $COCl_2$, ferner $P_2O_5$, $P_2S_5$, $AlCl_3$ (z.B. als Doppelbindung mit NaCl), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten; als Lösungsmittel kommen z.B. Basen wie Pyridin oder Triethylamin, aromatische Kohlenwasserstoffe wie Benzol, Toluol oder Xylol oder Amide wie DMF in Betracht.

Zur Herstellung der vorstehend genannten Nitrile der Formel I kann man auch entsprechende Säurehalogenide, vorzugsweise die Chloride, mit Sulfamid umsetzen, zweckmäßig in einem inerten Lösungsmittel wie Tetramethylensulfon bei Temperaturen zwischen etwa 80° und 150°, vorzugsweise bei 120°. Nach üblicher Aufarbeitung kann man direkt die Nitrile inolieren.

Ether der Formel I (worin $R^1$ und/oder $R^2$ eine Alkylgruppe bedeutet, worin eine oder zwei $CH_2$-Gruppen durch O-Atome ersetzt sind, und/oder worin $Z^1$ und/oder $Z^2$ eine-$OCH_2$- oder eine-$CH_2O$-Gruppe ist) sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, ,z.B. durch Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann mit dem entsprechenden Alkylhalogenid, -sulfonat oder Dialkylsulfat umgesetzt werden, zweckmäßig in einem inerten Lösungsmittel wie Aceton, 1,2-Dimethoxyethan, DMF oder Dimethylsulfoxid oder auch einem Überschuß an wäßriger oder wäßrig-alkoholischer NaOH oder KOH bei Temperaturen zwischen etwa 20° und 100°.

Die erfindungsgemäßen ferroelektrischen flüssigkristallinen Phasen bestehen aus 2 bis 15, vorzugsweise 3 bis 12 Komponenten, darunter mindestens einer Verbindung der Formel I. Die anderen Bestandteile werden vorzugsweise ausgewählt aus den Verbindungen der Formeln II bis IV,

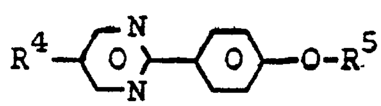
II

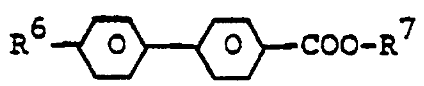
III

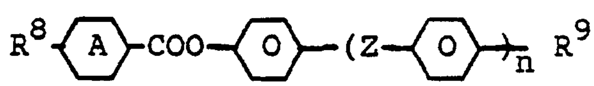
IV

worin $R^4$ und $R^5$ jeweils unabhängig voneinander n-Alkyl mit 5 bis 12 C-Atomen bedeutet und $R^6$, $R^7$, $R^8$ und $R^9$ jeweils unabhängig voneinander geradkettige oder verzweigte, ggf. chirale, Alkyl-, Alkoxy-, Alkoxycarbonyl- oder Alkanoyloxygruppen mit 5 bis 12, insbesondere mit 6 bis 10 C-Atomen bedeutet. Ring A ist 1,4-Phenylen oder trans-1,4-Cyclohexylen. n ist 0 oder 1.

Alle diese Substanzen sind nach literaturbekannten Methoden herstellbar.

Weiterhin bevorzugt sind erfindungsgemäße ferroelektrische Phasen enthaltend mindestens eine Verbindung der Formel V

$$R^1\text{-}Q^1\text{-}A\text{-}(Q^2)_q\text{-}R^2 \quad V$$

worin

$R^1$ und $R^2$ jeweils unabhängig voneinander eine geradkettige Alkylgruppe mit 1 bis 15 C-Atomen, worin auch eine oder mehrere nicht benachbarte $CH_2$-Gruppen durch -O-, -S-, -CO-, $CHCH_3$-O-, -$CHCH_3$-, -CH-Halogen-, CHCN-, -O-CO-, -O-COO-, -CO-O- und/oder -CH=CH- ersetzt sein können,

A 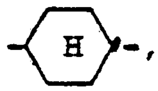 , 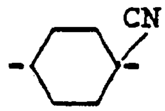 , 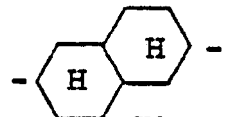 ,

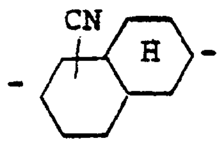 , 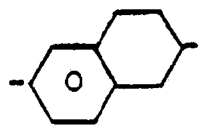 , 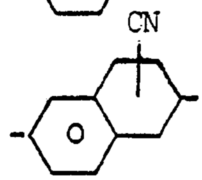 ,

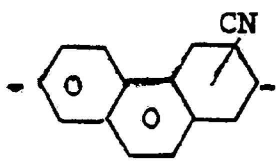 , 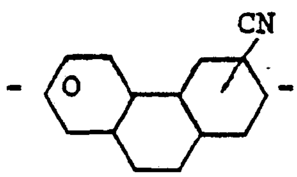 ,

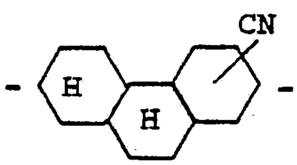 , 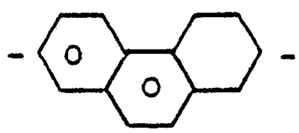 ,

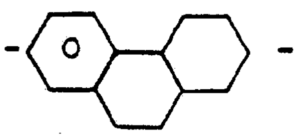 , 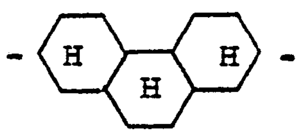 ,

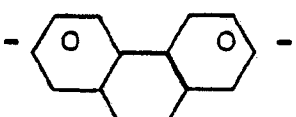 , 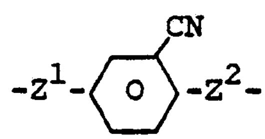 oder

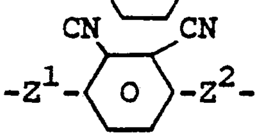

q 0 oder 1,

$Q^1$ und $Q^2$ jeweils unabhängig voneinander, $-(A^\circ\text{-}Z^\circ)_p-$, wobei

A° unsubstituiertes oder ein- oder mehrfach durch Halogenatome, $CH_3$- und/oder Nitril-Gruppen substituiertes 1,4-Cyclohexylen, worin auch eine oder zwei nicht benachbarte $CH_2$-Gruppen durch -O- und/oder -S- und/oder eine

$$\overset{|}{-CH}-CH_2-$$

Gruppierung durch

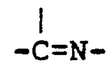

ersetzt sein können (Cy), oder unsubstituiertes oder ein- oder mehrfach durch Halogenatome, $CH_3$- und/oder Nitril-Gruppen substituiertes 1,4-Phenylen, worin auch eine oder mehrere CH-Gruppen durch N ersetzt sein können (Ph) bedeutet, einer der Reste A° auch 2,6-Naphthylen (Na) oder Tetrahydro-2,6-naphthylen (4H-Na), gegebenenfalls durch Halogen oder CN substituiert,

Z°, $Z^1$ jeweils unabhängig voneinander -CO-O-, -O-CO-

und $Z^2$ -$CH_2O$-, $OCH_2$-, -$CH_2CH_2$-, -$CHCNCH_2$-, -$CH_2$-CHCN- oder eine Einfachbindung, und

p 1, 2 oder 3, oder im Falle A = Tetra- oder Octaphenanthren auch 0 bedeutet, wobei im Falle A =

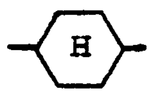,
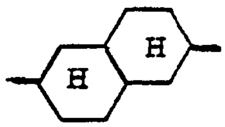,
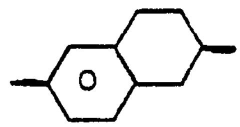,

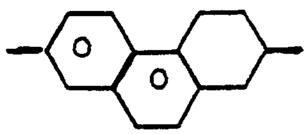,
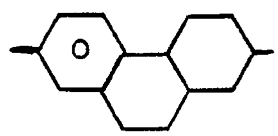,

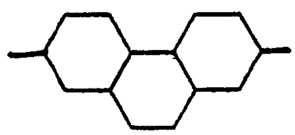
oder
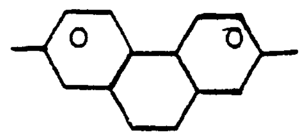

mindestens eine Gruppe Z° -$CHCNCH_2$- oder -$CH_2$CHCN- bedeutet und/oder in mindestens einer der Gruppen $R^1$ und $R^2$ mindestens eine $CH_2$-Gruppe durch -CHCN- ersetzt ist.

Die Verbindungen der Formel V können geradkettige oder verzweigte Flügelgruppen $R^1$ und/oder $R^2$ haben. Verbindungen mit verzweigten Flügelgruppen können in Form des Racemates oder als optisch aktive Verbindungen eingesetzt werden. Achirale Basismischungen aus Verbindungen der Formel V und ggf. weiteren achiralen Komponenten können mit chiralen Verbindungen der formel I oder auch zusätzlich mit anderen chiralen Verbindungen dotiert werden, um chiral getiltete smektische Phasen zu erhalten.

Besonders bevorzugte kleinere Gruppen von Verbindungen sind diejenigen der Formeln V1 bis V18:

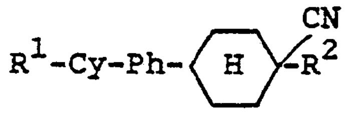

V1

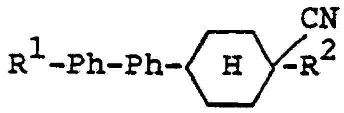

V2

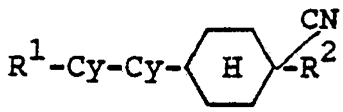

V3

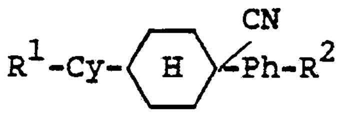

V4

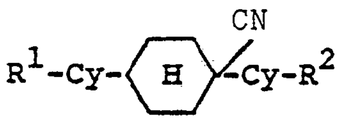

V5

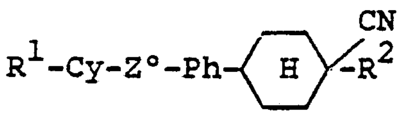

V6

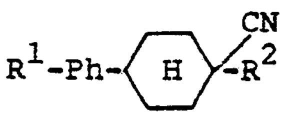

V7

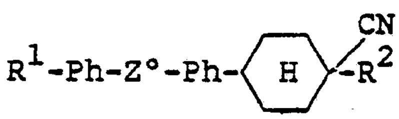

V8

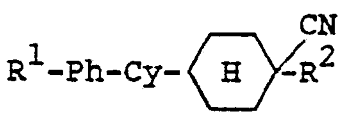

V9

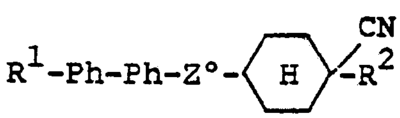

V10

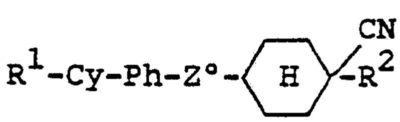

V11

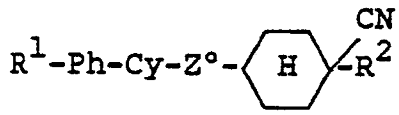 V12

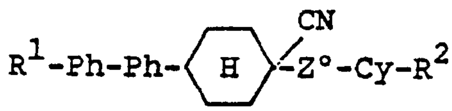 V13

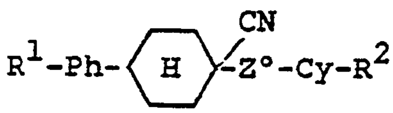 V14

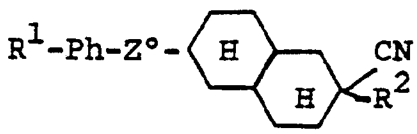 V15

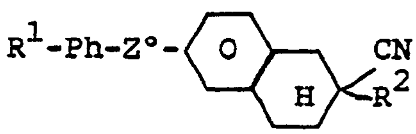 V16

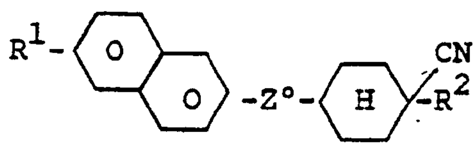 V17

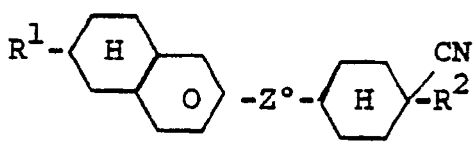 V18

Eine weitere besonders bevorzugte kleinere Gruppe von Verbindungen sind diejenigen der Formeln V19 bis V22:

$$R^1\text{-}A°\text{-}Cy\text{-}(CH_2)_r\text{-}CHCN\text{-}C_sH_{2s+1} \quad V19$$

$$R^1\text{-}A°\text{-}A°\text{-}Cy\text{-}(CH_2)_r\text{-}CHCN\text{-}C_sH_{2s+1} \quad V20$$

$$R^1\text{-}A°\text{-}A°\text{-}CHCN\text{-}CH_2\text{-}Cy\text{-}R^2 \quad V21$$

$$R^1\text{-}A°\text{-}A°\text{-}CH_2\text{-}CHCN\text{-}Cy\text{-}R^2 \quad V22$$

worin r 0, 1, 2 oder 3 bedeutet und (r+s) 1 bis 14 ist.

Verbindungen der Formel V, die keine $S_c$-Phasen aufweisen, eignen sich ebenfalls als Komponenten erfindungsgemäßer smektischer Phasen.

Besonders bevorzugte Verbindungen der Formel V sind im folgenden angegeben:

4-(4-Cyano-4-butylcyclohexyl)-4'-heptylbiphenyl, F. 56°, K. 122°
4-(4-Cyano-4-butylcyclohexyl)-4'-octylbiphenyl, F. 42°, K. 118°
4-(4-Cyano-4-butylcyclohexyl)-4'-nonylbiphenyl
4-(4-Cyano-4-butylcyclohexyl)-4'-butoxybiphenyl

4-(4-Cyano-4-butylcyclohexyl)-4'-pentoxybiphenyl
4-(4-Cyano-4-butylcyclohexyl)-4'-hexoxybiphenyl
4-(4-Cyano-4-butylcyclohexyl)-4'-heptoxybiphenyl, F. 93°, K. 156°
4-(4-Cyano-4-butylcyclohexyl)-4'-octoxybiphenyl, F. 80°, K. 154°
4-(4-Cyano-4-butylcyclohexyl)-4'-nonoxybiphenyl, F. 89°, K. 150°
4-(4-Cyano-4-pentylcyclohexyl)-4'-ethylbiphenyl
4-(4-Cyano-4-pentylcyclohexyl)-4'-propylbiphenyl
4-(4-Cyano-4-pentylcyclohexyl)-4'-butylbiphenyl, F. 75°, K. 128°
4-(4-Cyano-4-pentylcyclohexyl)-4'-pentylbiphenyl
4-(4-Cyano-4-pentylcyclohexyl)-4'-hexylbiphenyl
4-(4-Cyano-4-pentylcyclohexyl)-4'-heptylbiphenyl, F. 54°, K 127°
4-(4-Cyano-4-pentylcyclohexyl)-4'-octylbiphenyl, F. 43°, K. 115°
4-(4-Cyano-4-pentylcyclohexyl)-4'-nonylbiphenyl
4-(4-Cyano-4-pentylcyclohexyl)-4'-butoxybiphenyl
4-(4-Cyano-4-pentylcyclohexyl)-4'-pentoxybiphenyl
4-(4-Cyano-4-pentylcyclohexyl)-4'-hexoxybiphenyl
4-(4-Cyano-4-pentylcyclohexyl)-4'-heptoxybiphenyl, F 91°, K. 161°
4-(4-Cyano-4-pentylcyclohexyl)-4'-octyloxybiphenyl, F. 93°, K. 160°
4-(4-Cyano-4-pentylcyclohexyl)-4'-nonoxybiphenyl, F. 83°, K. 156°
4-[4-Cyano-4-(2-methylbutylcyclohexyl)]-4'-ethylbiphenyl
4-[4-Cyano-4-(2-methylbutylcyclohexyl)]-4'-propylbiphenyl
4-[4-Cyano-4-(2-methylbutylcyclohexyl)]-4'-butylbiphenyl
4-[4-Cyano-4-(2-methylbutylcyclohexyl)]-4'-pentylbiphenyl
4-[4-Cyano-4-(2-methylbutylcyclohexyl)]-4'-hexylbiphenyl
4-[4-Cyano-4-(2-methylbutylcyclohexyl)]-4'-heptylbiphenyl
4-[4-Cyano-4-(2-methylbutylcyclohexyl)]-4'-octylbiphenyl
4-[4-Cyano-4-(2-methylbutylcyclohexyl)]-4'-nonylbiphenyl
4-[4-Cyano-4-(2-methylbutylcyclohexyl)]-4'-butoxybiphenyl
4-[4-Cyano-4-(2-methylbutylcyclohexyl)]-4'-pentoxybiphenyl
4-[4-Cyano-4-(2-methylbutylcyclohexyl)]-4'-hexoxybiphenyl
4-[4-Cyano-4-(2-methylbutylcyclohexyl)]-4'-heptoxybiphenyl, F 66°, K. 131,4°, K131,0° Ch/Bp
4-[4-Cyano-4-(2-methylbutylcyclohexyl)]-4'-octyloxybiphenyl,
4-[4-Cyano-4-(2-methylbutylcyclohexyl)]-4'-nonoxybiphenyl,
4-(4-Cyano-4-hexylcyclohexyl)-4'-propylbiphenyl
4-(4-Cyano-4-hexylcyclohexyl)-4'-butylbiphenyl
4-(4-Cyano-4-hexylcyclohexyl)-4'-pentylbiphenyl
4-(4-Cyano-4-hexylcyclohexyl)-4'-hexylbiphenyl
4-(4-Cyano-4-hexylcyclohexyl)-4'-heptylbiphenyl, F. 66°, K. 125°
4-(4-Cyano-4-hexylcyclohexyl)-4'-octylbiphenyl
4-(4-Cyano-4-hexylcyclohexyl)-4'-nonylbiphenyl
4-(4-Cyano-4-hexylcyclohexyl)-4'-butoxybiphenyl
4-(4-Cyano-4-hexylcyclohexyl)-4'-pentoxybiphenyl
4-(4-Cyano-4-hexylcyclohexyl)-4'-hexoxybiphenyl
4-(4-Cyano-4-hexylcyclohexyl)-4'-heptoxybiphenyl, F 88°, K. 156°
4-(4-Cyano-4-hexylcyclohexyl)-4'-octoxybiphenyl, 90°, K. 155°
4-(4-Cyano-4-hexylcyclohexyl)-4'-nonoxybiphenyl, F. 87°, K. 152°
4-(4-Cyano-4-heptylcyclohexyl)-4'-ethylbiphenyl
4-(4-Cyano-4-heptylcyclohexyl)-4'-propylbiphenyl
4-(4-Cyano-4-heptylcyclohexyl)-4'-butylbiphenyl
4-(4-Cyano-4-heptylcyclohexyl)-4'-pentylbiphenyl
4-(4-Cyano-4-heptylcyclohexyl)-4'-hexylbiphenyl
4-(4-Cyano-4-heptylcyclohexyl)-4'-heptylbiphenyl, F. 61°, K. 124°
4-(4-Cyano-4-heptylcyclohexyl)-4'-octylbiphenyl, F. 64°, K. 125°
4-(4-Cyano-4-heptylcyclohexyl)-4'-nonylbiphenyl
4-(4-Cyano-4-heptylcyclohexyl)-4'-butoxybiphenyl
4-(4-Cyano-4-heptylcyclohexyl)-4'-pentoxybiphenyl
4-(4-Cyano-4-heptylcyclohexyl)-4'-hexoxybiphenyl
4-(4-Cyano-4-heptylcyclohexyl)-4'-heptoxybiphenyl, F 87°, K. 155°

4-(4-Cyano-4-heptylcyclohexyl)-4'-octoxybiphenyl, 83°, K. 154°
4-(4-Cyano-4-heptylcyclohexyl)-4'-nonoxybiphenyl, F. 81°, K. 152°
4-(4-Cyano-4-octylcyclohexyl)-4'-ethylbiphenyl
4-(4-Cyano-4-octylcyclohexyl)-4'-propylbiphenyl
4-(4-Cyano-4-octylcyclohexyl)-4'-butylbiphenyl
4-(4-Cyano-4-octylcyclohexyl)-4'-pentylbiphenyl, F. 52°, K. 124°
4-(4-Cyano-4-octylcyclohexyl)-4'-hexylbiphenyl
4-(4-Cyano-4-octylcyclohexyl)-4'-heptylbiphenyl, F. 61°, K. 122°
4-(4-Cyano-4-octylcyclohexyl)-4'-octylbiphenyl, F. 65°, K. 125°
4-(4-Cyano-4-octylcyclohexyl)-4'-nonylbiphenyl
4-(4-Cyano-4-octylcyclohexyl)-4'-butoxybiphenyl
4-(4-Cyano-4-octylcyclohexyl)-4'-pentoxybiphenyl
4-(4-Cyano-4-octylcyclohexyl)-4'-hexoxybiphenyl
4-(4-Cyano-4-octylcyclohexyl)-4'-heptoxybiphenyl, F 85°, K. 151°
4-(4-Cyano-4-octylcyclohexyl)-4'-octoxybiphenyl, 81°, K. 150°
4-(4-Cyano-4-octylcyclohexyl)-4'-nonoxybiphenyl, F. 72°, K. 149°
4-(4-Cyano-4-nonylcyclohexyl)-4'-ethylbiphenyl
4-(4-Cyano-4-nonylcyclohexyl)-4'-propylbiphenyl
4-(4-Cyano-4-nonylcyclohexyl)-4'-butylbiphenyl
4-(4-Cyano-4-nonylcyclohexyl)-4'-pentylbiphenyl
4-(4-Cyano-4-nonylcyclohexyl)-4'-heptylbiphenyl
4-(4-Cyano-4-nonylcyclohexyl)-4'-octylbiphenyl
4-(4-Cyano-4-nonylcyclohexyl)-4'-nonylbiphenyl
4-(4-Cyano-4-nonylcyclohexyl)-4'-butoxybiphenyl
4-(4-Cyano-4-nonylcyclohexyl)-4'-pentoxybiphenyl
4-(4-Cyano-4-nonylcyclohexyl)-4'-hexoxybiphenyl
4-(4-Cyano-4-nonylcyclohexyl)-4'-heptoxybiphenyl
4-(4-Cyano-4-nonylcyclohexyl)-4'-octoxybiphenyl
4-(4-Cyano-4-nonylcyclohexyl)-4'-nonoxybiphenyl

Alle Verbindungen der Formel V werden nach an sich bekannten Methoden hergestellt wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Formel V umfaßt zum überwiegenden Teil bekannte Verbindungen, wie beispielsweise die bevorzugten, in DOS 32 31 707, 33 19 781, 33 20 024, 34 07 013, 34 43 029, 33 32 690, 33 32 691, 33 32 692, 29 33 563, 28 53 728, 26 13 293, 34 01 320, 31 36 624, 30 40 632, 32 05 766, 22 40 864, 29 37 700, 34 10 734, 33 24 686, EP-OS 0 085 995, EP-OS 0 084 194, DD 116 732, FR 24 25 469, FR 24 19 966, USP 4 237 026, USP 3 953 491, USP 4,225,454 bzw. in H.J. Deutscher et al., J. prakt. Chemie, 321, 569 (1979) und J.C. Dubois et al. Mol. Cryst. Liq. Cryst. 47, 193 (1978) beschriebenen Verbindungen.

Als Komponenten der erfindungsgemäßen Phasen kommen ferner Verbindungen der formel

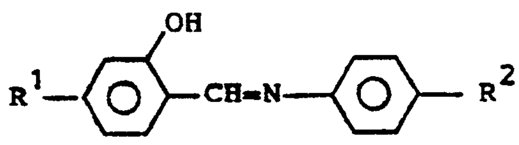

in Frage, worin $R^1$ und $R^2$ die bei Formel V angegebene Bedeutung haben.

Besonders bevorzugt sind erfindungsgemäße chiral getiltete smektische flüssigkristalline Phasen, deren achirale Basismischung neben Verbindungen der Formel I mindestens eine andere Komponente mit negativer oder betragsmäßig kleiner positiver dielektrischer Anisotropie enthält. Als weitere Komponenten mit betragsmäßig kleiner positiver oder negativer dielektrischer Anisotropie eignen sich Verbindungen der Teilformeln Va bis Vp:

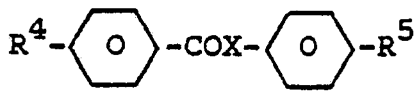
Va

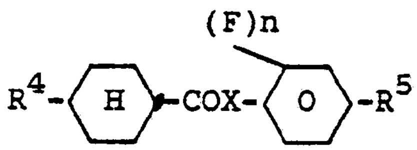
Vb

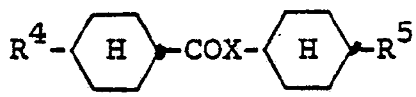
Vc

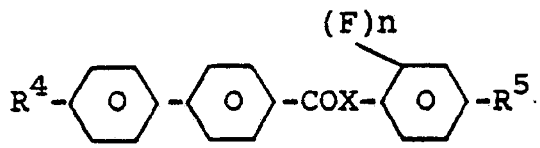
Vd

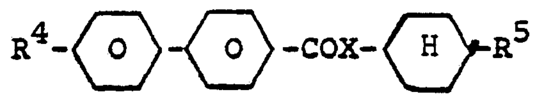
Ve

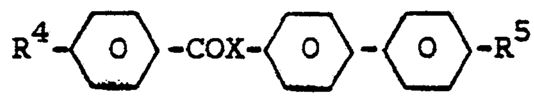
Vf

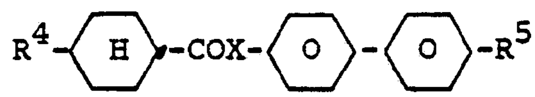
Vg

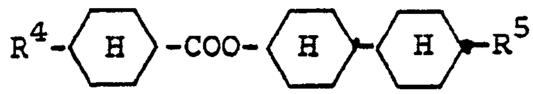 Vh

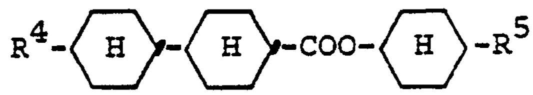 Vi

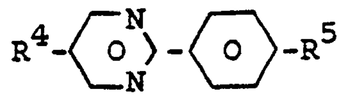 Vj

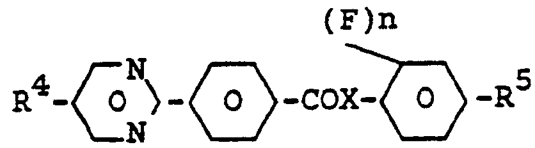 Vk

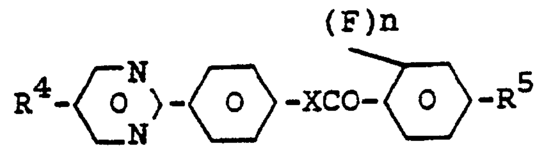 Vl

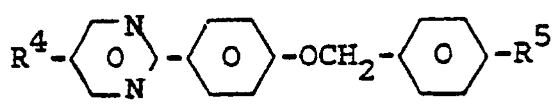 Vm

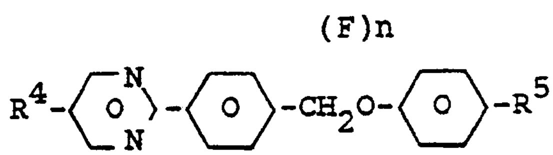 Vn

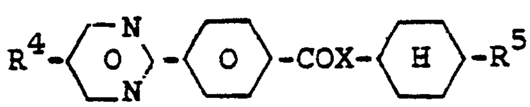 Vo

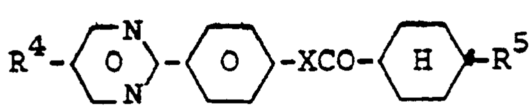 Vp

$R^4$ und $R^5$ sind jeweils vorzugsweise geradkettiges Alkyl, Alkoxy, Alkanoyloxy oder Alkoxycarbonyl mit jeweils 3 bis 12 C-Atomen. X ist vorzugsweise O. n ist 0 oder 1.

Besonders bevorzugt sind die Verbindungen der Teilformeln Va, Vb, Vd und Vf, worin $R^4$ und $R^5$ jeweils geradkettiges Alkyl oder Alkoxy mit jeweils 5 bis 10 C-Atomen bedeutet.

Die Verbindungen der Teilformeln Vc, Vh und Vi eignen sich als Zusätze zur Schmelzpunkterniedrigung und werden normalerweise den Basismischungen mit nicht mehr als 5 %, vorzugsweise 1 bis 3 %, zugesetzt. $R^4$ und $R^5$ bedeuten in den Verbindungen der Teilformeln Vc, Vh und Vi vorzugsweise geradkettiges Alkyl mit 2 bis 7, vorzugsweise 3 bis 5, C-Atomen. Eine weitere zur Schmelzpunktserniedrigung in den erfindungsgemäßen Phasen geeignete Verbindungsklasse ist diejenige der Formel

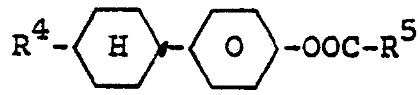

worin $R^4$ und $R^5$ die für Vc, Vh und Vi angegebene bevorzugte Bedeutung haben.

Als weitere Komponenten mit negativer dielektrischer Anisotropie eignen sich weiterhin Verbindungen enthaltend das Strukturelement B oder C.

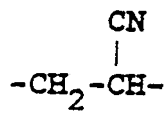

B

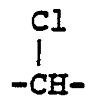

C

Bevorzugte Verbindungen dieser Art entsprechen den Formeln VIb und VIc:

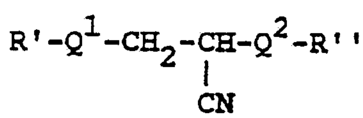

VIb

$$R^1\text{-}Q^3\text{-}Q^4\text{-}R''' \qquad \text{VIc}$$

R′ und R″ bedeuten jeweils vorzugsweise geradkettige Alkyl- oder Alkoxy-Gruppen mit jeweils 2 bis 10 C-Atomen. $Q^1$ und $Q^2$ bedeuten jeweils 1,4-Phenylen, trans-1,4-Cyclohexylen, 4,4′-Biphenylyl, 4-(trans-4-Cyclohexyl)-phenyl, trans,trans-4,4′-Bicyclohexyl oder eine der Gruppen $Q^1$ und $Q^2$ auch eine Einfachbindung.

$Q^3$ und $Q^4$ bedeuten jeweils 1,4-Phenylen, 4,4′-Biphenylyl oder trans-1,4-Cyclohexylen. Eine der Gruppen $Q^3$ und $Q^4$ kann auch 1,4-Phenylen bedeuten, worin mindestens eine CH-Gruppe durch N ersetzt ist. R‴ ist ein optisch aktiver Rest mit einem asymmetrischen Kohlenstoffatom der Struktur

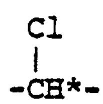

oder

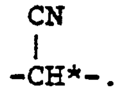

Besonders bevorzugte Verbindungen der Formel VIc sind diejenigen der Formel VIc′:

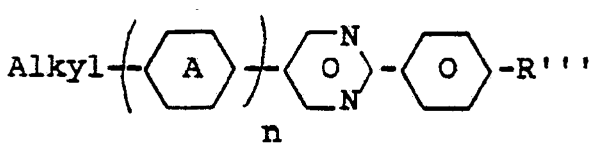

VIc'

worin A 1,4-Phenylen oder trans-1,4-Cyclohexylen und n 0 oder 1 bedeutet.

Die erfindungsgemäßen Phasen enthalten etwa 0,5-40 %, vorzugsweise 5-10 %, einer oder mehrerer Verbindungen der Formel I.

Weiterhin bevorzugt sind erfindungsgemäße Phasen enthaltend 0,3-5 %, vorzugsweise 1-4 %, einer oder mehrerer Verbindungen der Formel I.

Die Herstellung der erfindungsgemäßem Dielektrika erfolgt in an sich üblicher Weise. In der Regel werden

die Komponenten ineinander gelöst, zweckmäßig bei erhöhter Temperatur.

Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Die folgenden Beispiele sollen die Erfindung erläutern, ohne sie zu begrenzen. F. = Schmelzpunkt, K. = Klärpunkt. Vor- und nachstehend bedeuten Prozentangaben Gewichtsprozent; alle Temperaturangaben sind in Grad Celcius angegeben. Die Werte für die spontane Polarisation gelten für Raumtemperatur. Es bedeuten ferner: K: Kristallin-fester Zustand, S: Smektische Phase (der Index kennzeichnet den Phasentyp), N: Nematischer Zustand, Ch: Cholesterische Phase, I: Isotrope Phase. Die zwischen zwei Symbolen stehende Zahl gibt die Umwandlungstemperaturen in Grad celsius an. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Beispiel 1

Eine Lösung von 15,2 g 4-(5-n-Nonylpyrimidyl-2)-phenol, 5,5 g R-2-Chlorpropionsäure und 0,62 g 4-N,N-Dimethylaminopyridin in 100 ml Dichlormethan wird bei 0° langsam mit einer Lösung von 11,6 g Dicyclohexylcarbodiimid in 6 ml Dichlormethan versetzt, auf Raumtemperatur erwärmt und zwei Stunden weiter gerührt. Nach dem Abfiltrieren des ausgefallenen Harnstoffderivates wird das filtrat mit verdünnter Salzsäure und Wasser gewaschen und die organische Phase wie üblich aufgearbeitet. Man erhält R-4-(5-n-Nonylpyrimidyl-2)-phenyl-2-chlorpropionat, F. 69 °.

Analog werden durch Veresterung bzw. Veretherung nach literaturbekanntem Verfahren folgende optisch aktiven Verbindungen hergestellt:

2-p-(2-Methylbutoxyphenyl)-5-butylpyrimidin
2-p-(2-Methylbutoxyphenyl)-5-pentylpyrimidin
2-p-(2-Methylbutoxyphenyl)-5-hexylpyrimidin
2-p-(2-Methylbutoxyphenyl)-5-heptylpyrimidin
2-p-(2-Methylbutoxyphenyl)-5-octylpyrimidin
2-p-(2-Methylbutoxyphenyl)-5-nonylpyrimidin
2-p-(2-Methylbutoxyphenyl)-5-decylpyrimidin
2-p-(2-Methylbutoxyphenyl)-5-undecylpyrimidin, F 40°, K. 47°, $S_C^*/S_A^*$ 41°
2-p-(2-Methylbutoxyphenyl)-5-dodecylpyrimidin
2-p-(2-Methylbutoxyphenyl)-5-heptoxymethylpyrimidin
2-p-(2-Methylbutoxyphenyl)-5-hexoxyethylpyrimidin
2-p-(2-Methylbutoxyphenyl)-5-pentoxypropylpyrimidin
2-p-(3-Methylpentoxyphenyl)-5-butylpyrimidin
2-p-(3-Methylpentoxyphenyl)-5-pentylpyrimidin
2-p-(3-Methylpentoxyphenyl)-5-hexylpyrimidin, F. 13°, K. 32°
2-p-(3-Methylpentoxyphenyl)-5-heptylpyrimidin, F. 39°
2-p-(3-Methylpentoxyphenyl)-5-octylpyrimidin, F. 36°
2-p-(3-Methylpentoxyphenyl)-5-nonylpyrimidin, F. 23, K.46°, $S_C^*/S_A^*$ 39°
2-p-(3-Methylpentoxyphenyl)-5-decylpyrimidin, F. 22°, K. 41°
2-p-(3-Methylpentoxyphenyl)-5-undecylpyrimidin, F 25°, K. 50°, $S_C^*/S_A^*$ 45°
2-p-(3-Methylpentoxyphenyl)-5-dodecylpyrimidin, F 28°, K. 51°, $S_C^*/S_A^*$ 47°
2-p-(3-Methylpentoxyphenyl)-5-heptoxymethylpyrimidin
2-p-(3-Methylpentoxyphenyl)-5-hexoxyethylpyrimidin
2-p-(3-Methylpentoxyphenyl)-5-pentoxypropylpyrimidin
2-p-(4-Methylhexoxyphenyl)-5-butylpyrimidin
2-p-(4-Methylhexoxyphenyl)-5-pentylpyrimidin
2-p-(4-Methylhexoxyphenyl)-5-hexylpyrimidin, F. 25°, K. 42°
2-p-(4-Methylhexoxyphenyl)-5-heptylpyrimidin, F. 28,5°, K. 60°, $S_C^*/S_A^*$ 54°
2-p-(4-Methylhexoxyphenyl)-5-octylpyrimidin, F. 31°, K. 51°, $S_C^*/S_A^*$ 47°
2-p-(4-Methylhexoxyphenyl)-5-nonylpyrimidin, F. 23, K. 52°, $S_C^*/S_A^*$ 30°
2-p-(4-Methylhexoxyphenyl)-5-decylpyrimidin, F. 33°, K. 58°
2-p-(4-Methylhexoxyphenyl)-5-undecylpyrimidin, F 36°, K. 60°
2-p-(4-Methylhexoxyphenyl)-5-dodecylpyrimidin, F 41°, K. 52°

2-p-(4-Methylhexoxyphenyl)-5-heptoxymethylpyrimidin
2-p-(4-Methylhexoxyphenyl)-5-hexoxyethylpyrimidin
2-p-(4-Methylhexoxyphenyl)-5-pentoxypropylpyrimidin
2-p-(5-Methylheptoxyphenyl)-5-butylpyrimidin
2-p-(5-Methylheptoxyphenyl)-5-pentylpyrimidin
2-p-(5-Methylheptoxyphenyl)-5-hexylpyrimidin, F. -5°, K. 42°
2-p-(5-Methylheptoxyphenyl)-5-heptylpyrimidin, F. -6°, K. 49°
2-p-(5-Methylheptoxyphenyl)-5-octylpyrimidin, F. 12°, K. 50°, $S_C^*/S_A^*$ 35°
2-p-(5-Methylheptoxyphenyl)-5-nonylpyrimidin, F. 10°, K. 59°, $S_C^*/S_A^*$ 46°
2-p-(5-Methylheptoxyphenyl)-5-decylpyrimidin, F. 17°, K. 63°, $S_C^*/S_A^*$ 54°
2-p-(5-Methylheptoxyphenyl)-5-undecylpyrimidin, F 20°, K. 59°
2-p-(5-Methylheptoxyphenyl)-5-dodecylpyrimidin, F 23°, K. 61°
2-p-(5-Methylheptoxyphenyl)-5-heptoxymethylpyrimidin
2-p-(5-Methylheptoxyphenyl)-5-hexoxyethylpyrimidin
2-p-(5-Methylheptoxyphenyl)-5-pentoxypropylpyrimidin
2-p-(6-Methyloctoxyphenyl)-5-butylpyrimidin
2-p-(6-Methyloctoxyphenyl)-5-pentylpyrimidin
2-p-(6-Methyloctoxyphenyl)-5-hexylpyrimidin, F. 12°, K. 46°
2-p-(6-Methyloctoxyphenyl)-5-heptylpyrimidin, F. 10°, K. 61°, $S_C^*/S_A^*$ 39°
2-p-(6-Methyloctoxyphenyl)-5-octylpyrimidin, F. 3°, K. 56°, $S_C^*/S_A^*$ 49°
2-p-(6-Methyloctoxyphenyl)-5-nonylpyrimidin, F. 16°, K. 61°, $S_C^*/S_A^*$ 49°
2-p-(6-Methyloctoxyphenyl)-5-decylpyrimidin, F. 41°, K. 61°
2-p-(6-Methyloctoxyphenyl)-5-undecylpyrimidin
2-p-(6-Methyloctoxyphenyl)-5-dodecylpyrimidin, F. 40°, K. 70°
2-p-(6-Methyloctoxyphenyl)-5-heptoxymethylpyrimidin
2-p-(6-Methyloctoxyphenyl)-5-hexoxyethylpyrimidin
2-p-(6-Methyloctoxyphenyl)-5-pentoxypropylpyrimidin
2-p-(2-Methylbutyryloxyphenyl)-5-butylpyrimidin
2-p-(2-Methylbutyryloxyphenyl)-5-pentylpyrimidin
2-p-(2-Methylbutyryloxyphenyl)-5-hexylpyrimidin
2-p-(2-Methylbutyryloxyphenyl)-5-heptylpyrimidin
2-p-(2-Methylbutyryloxyphenyl)-5-octylpyrimidin
2-p-(2-Methylbutyryloxyphenyl)-5-nonylpyrimidin
2-p-(2-Methylbutyryloxyphenyl)-5-decylpyrimidin
2-p-(2-Methylbutyryloxyphenyl)-5-undecylpyrimidin, F. 52°
2-p-(2-Methylbutyryloxyphenyl)-5-dodecylpyrimidin
2-p-(2-Methylbutyryloxyphenyl)-5-heptoxymethylpyrimidin
2-p-(2-Methylbutyryloxyphenyl)-5-hexoxyethylpyrimidin
2-p-(2-Methylbutyryloxyphenyl)-5-pentoxypropylpyrimidin
2-p-(4-Methylhexanoyloxyphenyl)-5-butylpyrimidin
2-p-(4-Methylhexanoyloxyphenyl)-5-pentylpyrimidin
2-p-(4-Methylhexanoyloxyphenyl)-5-hexylpyrimidin
2-p-(4-Methylhexanoyloxyphenyl)-5-heptylpyrimidin
2-p-(4-Methylhexanoyloxyphenyl)-5-octylpyrimidin, F. 54°
2-p-(4-Methylhexanoyloxyphenyl)-5-nonylpyrimidin
2-p-(4-Methylhexanoyloxyphenyl)-5-decylpyrimidin
2-p-(4-Methylhexanoyloxyphenyl)-5-undecylpyrimidin, F. 38°, K. 50°
2-p-(4-Methylhexanoyloxyphenyl)-5-dodecylpyrimidin
2-p-(4-Methylhexanoyloxyphenyl)-5-heptoxymethylpyrimidin
2-p-(4-Methylhexanoyloxyphenyl)-5-hexoxyethylpyrimidin
2-p-(4-Methylhexanoyloxyphenyl)-5-pentoxypropylpyrimidin
2-p-(6-Methyloctanoyloxyphenyl)-5-butylpyrimidin
2-p-(6-Methyloctanoyloxyphenyl)-5-pentylpyrimidin
2-p-(6-Methyloctanoyloxyphenyl)-5-hexylpyrimidin
2-p-(6-Methyloctanoyloxyphenyl)-5-heptylpyrimidin
2-p-(6-Methyloctanoyloxyphenyl)-5-octylpyrimidin, F. 38°, K. 45°

2-p-(6-Methyloctanoyloxyphenyl)-5-nonylpyrimidin
2-p-(6-Methyloctanoyloxyphenyl)-5-decylpyrimidin
2-p-(6-Methyloctanoyloxyphenyl)-5-undecylpyrimidin, F. 62°
2-p-(6-Methyloctanoyloxyphenyl)-5-dodecylpyrimidin
2-p-(6-Methyloctanoyloxyphenyl)-5-heptoxymethylpyrimidin
2-p-(6-Methyloctanoyloxyphenyl)-5-hexoxyethylpyrimidin
2-p-(6-Methyloctanoyloxyphenyl)-5-pentoxypropylpyrimidin
2-p-(6-Methyloctanoylphenyl)-5-butylpyrimidin
2-p-(6-Methyloctanoylphenyl)-5-pentylpyrimidin
2-p-(6-Methyloctanoylphenyl)-5-hexylpyrimidin
2-p-(6-Methyloctanoylphenyl)-5-heptylpyrimidin
2-p-(6-Methyloctanoylphenyl)-5-octylpyrimidin, F. 67°, K. 79°
2-p-(6-Methyloctanoylphenyl)-5-nonylpyrimidin
2-p-(6-Methyloctanoylphenyl)-5-decylpyrimidin
2-p-(6-Methyloctanoylphenyl)-5-undecylpyrimidin
2-p-(6-Methyloctanoylphenyl)-5-dodecylpyrimidin
2-p-(6-Methyloctanoylphenyl)-5-heptoxymethylpyrimidin
2-p-(6-Methyloctanoylphenyl)-5-hexoxyethylpyrimidin
2-p-(6-Methyloctanoylphenyl)-5-pentoxypropylpyrimidin
2-p-(3-Chlorpentoxyphenyl)-5-butylpyrimidin
2-p-(3-Chlorpentoxyphenyl)-5-pentylpyrimidin
2-p-(3-Chlorpentoxyphenyl)-5-hexylpyrimidin
2-p-(3-Chlorpentoxyphenyl)-5-heptylpyrimidin
2-p-(3-Chlorpentoxyphenyl)-5-octylpyrimidin, F. 15°, K. 43°
2-p-(3-Chlorpentoxyphenyl)-5-nonylpyrimidin
2-p-(3-Chlorpentoxyphenyl)-5-decylpyrimidin
2-p-(3-Chlorpentoxyphenyl)-5-undecylpyrimidin
2-p-(3-Chlorpentoxyphenyl)-5-dodecylpyrimidin
2-p-(3-Chlorpentoxyphenyl)-5-heptoxymethylpyrimidin
2-p-(3-Chlorpentoxyphenyl)-5-hexoxyethylpyrimidin
2-p-(3-Chlorpentoxyphenyl)-5-pentoxypropylpyrimidin
2-p-(3-Cyanpentoxyphenyl)-5-butylpyrimidin
2-p-(3-Cyanpentoxyphenyl)-5-pentylpyrimidin
2-p-(3-Cyanpentoxyphenyl)-5-hexylpyrimidin
2-p-(3-Cyanpentoxyphenyl)-5-heptylpyrimidin
2-p-(3-Cyanpentoxyphenyl)-5-octylpyrimidin
2-p-(3-Cyanpentoxyphenyl)-5-nonylpyrimidin
2-p-(3-Cyanpentoxyphenyl)-5-decylpyrimidin
2-p-(3-Cyanpentoxyphenyl)-5-undecylpyrimidin, F. 60°
2-p-(3-Cyanpentoxyphenyl)-5-dodecylpyrimidin
2-p-(3-Cyanpentoxyphenyl)-5-heptoxymethylpyrimidin
2-p-(3-Cyanpentoxyphenyl)-5-hexoxyethylpyrimidin
2-p-(3-Cyanpentoxyphenyl)-5-pentoxypropylpyrimidin
2-p-(1,6-Dioxa-8-methyldecylphenyl)-5-butylpyrimidin
2-p-(1,6-Dioxa-8-methyldecylphenyl)-5-pentylpyrimidin
2-p-(1,6-Dioxa-8-methyldecylphenyl)-5-hexylpyrimidin, F. -8°, K. 28°
2-p-(1,6-Dioxa-8-methyldecylphenyl)-5-heptylpyrimidin
2-p-(1,6-Dioxa-8-methyldecylphenyl)-5-octylpyrimidin, F. 1°, K. 42°
2-p-(1,6-Dioxa-8-methyldecylphenyl)-5-nonylpyrimidin
2-p-(1,6-Dioxa-8-methyldecylphenyl)-5-decylpyrimidin, F. 16°, K. 52°
2-p-(1,6-Dioxa-8-methyldecylphenyl)-5-undecylpyrimidin
2-p-(1,6-Dioxa-8-methyldecylphenyl)-5-dodecylpyrimidin
2-p-(1,6-Dioxa-8-methyldecylphenyl)-5-heptoxymethylpyrimidin
2-p-(1,6-Dioxa-8-methyldecylphenyl)-5-hexoxyethylpyrimidin
2-p-(1,6-Dioxa-8-methyldecylphenyl)-5-pentoxypropylpyrimidin
2-p-Octyloxyphenyl-5-(2-methyloctyl)-pyrimidin
2-p-Octyloxyphenyl-5-(3-methyloctyl)-pyrimidin
2-p-Octyloxyphenyl-5-(4-methyloctyl)-pyrimidin

2-p-Octyloxyphenyl-5-(5-methyloctyl)-pyrimidin
2-p-Octyloxyphenyl-5-(6-methyloctyl)-pyrimidin, F. 14° K. 44°
2-p-Octylphenyl-5-(2-methyloctyloxy)-pyrimidin
2-p-Octylhenyl-5-(3-methyloctyloxy)-pyrimidin 2-p-Octylphenyl-5-(4-methyloctyloxy)-pyrimidin
2-p-Octylphenyl-5-(5-methyloctyloxy)-pyrimidin

2-p-Octylphenyl-5-(6-methyloctyloxy)-pyrimidin, F. 13°, K. 51°
2-p-(2-Methylbutoxy)-phenyl-5-butyloxypyrimidin
2-p-(2-Methylbutoxy)-phenyl-5-pentyloxypyrimidin
2-p-(2-Methylbutoxy)-phenyl-5-hexyloxypyrimidin
2-p-(2-Methylbutoxy)-phenyl-5-heptyloxypyrimidin
2-p-(2-Methylbutoxy)-phenyl-5-octyloxypyrimidin
2-p-(2-Methylbutoxy)-phenyl-5-nonyloxypyrimidin
2-p-(2-Methylbutoxy)-phenyl-5-decyloxypyrimidin
2-p-(2-Methylbutoxy)-phenyl-5-undecyloxypyrimidin, F. 58° K. 77°
2-p-(2-Methylbutoxy)-phenyl-5-dodecyloxypyrimidin
2-p-(2-Methylbutoxy)-phenyl-5-heptoxymethyloxypyrimidin
2-p-(2-Methylbutoxy)-phenyl-5-hexoxyethyloxypyrimidin
2-p-(2-Methylbutoxy)-phenyl-5-pentoxypropyloxypyrimidin
2-p-(3-Methylpentoxy)-phenyl-5-butyloxypyrimidin
2-p-(3-Methylpentoxy)-phenyl-5-pentyloxypyrimidin
2-p-(3-Methylpentoxy)-phenyl-5-hexyloxypyrimidin
2-p-(3-Methylpentoxy)-phenyl-5-heptyloxypyrimidin
2-p-(3-Methylpentoxy)-phenyl-5-octyloxypyrimidin, F. 52°, K. 76°
2-p-(3-Methylpentoxy)-phenyl-5-nonyloxypyrimidin
2-p-(3-Methylpentoxy)-phenyl-5-decyloxypyrimidin
2-p-(3-Methylpentoxy)-phenyl-5-undecyloxypyrimidin, F. 48° K. 78°
2-p-(3-Methylpentoxy)-phenyl-5-dodecyloxypyrimidin
2-p-(3-Methylpentoxy)-phenyl-5-heptoxymethyloxypyrimidin
2-p-(3-Methylpentoxy)-phenyl-5-hexoxyethyloxypyrimidin
2-p-(3-Methylpentoxy)-phenyl-5-pentoxypropyloxypyrimidin
2-p-(6-Methyloctoxy)-phenyl-5-butyloxypyrimidin
2-p-(6-Methyloctoxy)-phenyl-5-pentyloxypyrimidin
2-p-(6-Methyloctoxy)-phenyl-5-hexyloxypyrimidin
2-p-(6-Methyloctoxy)-phenyl-5-heptyloxypyrimidin
2-p-(6-Methyloctoxy)-phenyl-5-octyloxypyrimidin
2-p-(6-Methyloctoxy)-phenyl-5-nonyloxypyrimidin
2-p-(6-Methyloctoxy)-phenyl-5-decyloxypyrimidin
2-p-(6-Methyloctoxy)-phenyl-5-undecyloxypyrimidin, F. 41° K. 89°
2-p-(6-Methyloctoxy)-phenyl-5-dodecyloxypyrimidin
2-p-(6-Methyloctoxy)-phenyl-5-heptoxymethyloxypyrimidin
2-p-(6-Methyloctoxy)-phenyl-5-hexoxyethyloxypyrimidin
2-p-(6-Methyloctoxy)-phenyl-5-pentoxypropyloxypyrimidin
2-p-(2-Methyloctoxy)-phenyl-5-butyloxypyrimidin
2-p-(2-Methyloctoxy)-phenyl-5-pentyloxypyrimidin
2-p-(2-Methyloctoxy)-phenyl-5-hexyloxypyrimidin
2-p-(2-Methyloctoxy)-phenyl-5-heptyloxypyrimidin
2-p-(2-Methyloctoxy)-phenyl-5-octyloxypyrimidin, F. 36°, K. 62°, $S^*_C/S^*_A$ 41°
2-p-(2-Methyloctoxy)-phenyl-5-nonyloxypyrimidin
2-p-(2-Methyloctoxy)-phenyl-5-decyloxypyrimidin
2-p-(2-Methyloctoxy)-phenyl-5-undecyloxypyrimidin
2-p-(2-Methyloctoxy)-phenyl-5-dodecyloxypyrimidin
2-p-(2-Methyloctoxy)-phenyl-5-heptoxymethyloxypyrimidin
2-p-(2-Methyloctoxy)-phenyl-5-hexoxyethyloxypyrimidin
2-p-(2-Methyloctoxy)-phenyl-5-pentoxypropyloxypyrimidin
2-p-(2-Methylbutyryloxy)-phenyl-5-butyloxypyrimidin
2-p-(2-Methylbutyryloxy)-phenyl-5-pentyloxypyrimidin
2-p-(2-Methylbutyryloxy)-phenyl-5-hexyloxypyrimidin

2-p-(2-Methylbutyryloxy)-phenyl-5-heptyloxypyrimidin
2-p-(2-Methylbutyryloxy)-phenyl-5-octyloxypyrimidin, F. 66°
2-p-(2-Methylbutyryloxy)-phenyl-5-nonyloxypyrimidin
2-p-(2-Methylbutyryloxy)-phenyl-5-decyloxypyrimidin
2-p-(2-Methylbutyryloxy)-phenyl-5-undecyloxypyrimidin F. 43°, K. 67°, $S_C^*/S_A^*$ 64°
2-p-(2-Methylbutyryloxy)-phenyl-5-dodecyloxypyrimidin
2-p-(2-Methylbutyryloxy)-phenyl-5-heptoxymethyloxypyrimidin
2-p-(2-Methylbutyryloxy)-phenyl-5-hexoxyethyloxypyrimidin
2-p-(2-Methylbutyryloxy)-phenyl-5-pentoxypropyloxypyrimidin
2-p-(3-Methylpentanoyloxy)-phenyl-5-butyloxypyrimidin
2-p-(3-Methylpentanoyloxy)-phenyl-5-pentyloxypyrimidin
2-p-(3-Methylpentanoyloxy)-phenyl-5-hexyloxypyrimidin
2-p-(3-Methylpentanoyloxy)-phenyl-5-heptyloxypyrimidin
2-p-(3-Methylpentanoyloxy)-phenyl-5-octyloxypyrimidin, F. 70°, K. 73°
2-p-(3-Methylpentanoyloxy)-phenyl-5-nonyloxypyrimidin
2-p-(3-Methylpentanoyloxy)-phenyl-5-decyloxypyrimidin
2-p-(3-Methylpentanoyloxy)-phenyl-5-undecyloxypyrimidin, F. 55°, K. 78°
2-p-(3-Methylpentanoyloxy)-phenyl-5-dodecyloxypyrimidin
2-p-(3-Methylpentanoyloxy)-phenyl-5-heptoxymethyloxypyrimidin
2-p-(3-Methylpentanoyloxy)-phenyl-5-hexoxyethyloxypyrimidin
2-p-(3-Methylpentanoyloxy)-phenyl-5-pentoxypropyloxypyrimidin
2-p-(4-Methylhexanoyloxy)-phenyl-5-butyloxypyrimidin
2-p-(4-Methylhexanoyloxy)-phenyl-5-pentyloxypyrimidin
2-p-(4-Methylhexanoyloxy)-phenyl-5-hexyloxypyrimidin
2-p-(4-Methylhexanoyloxy)-phenyl-5-heptyloxypyrimidin
2-p-(4-Methylhexanoyloxy)-phenyl-5-octyloxypyrimidin, F. 78°
2-p-(4-Methylhexanoyloxy)-phenyl-5-nonyloxypyrimidin
2-p-(4-Methylhexanoyloxy)-phenyl-5-decyloxypyrimidin
2-p-(4-Methylhexanoyloxy)-phenyl-5-undecyloxypyrimidin
2-p-(4-Methylhexanoyloxy)-phenyl-5-dodecyloxypyrimidin
2-p-(4-Methylhexanoyloxy)-phenyl-5-heptoxymethyloxypyrimidin
2-p-(4-Methylhexanoyloxy)-phenyl-5-hexoxyethyloxypyrimidin
2-p-(4-Methylhexanoyloxy)-phenyl-5-pentoxypropyloxypyrimidin
2-p-(6-Methyloctanoyloxy)-phenyl-5-butyloxypyrimidin
2-p-(6-Methyloctanoyloxy)-phenyl-5-pentyloxypyrimidin
2-p-(6-Methyloctanoyloxy)-phenyl-5-hexyloxypyrimidin
2-p-(6-Methyloctanoyloxy)-phenyl-5-heptyloxypyrimidin
2-p-(6-Methyloctanoyloxy)-phenyl-5-octyloxypyrimidin, F. 76°,K. 80°
2-p-(6-Methyloctanoyloxy)-phenyl-5-nonyloxypyrimidin
2-p-(6-Methyloctanoyloxy)-phenyl-5-decyloxypyrimidin
2-p-(6-Methyloctanoyloxy)-phenyl-5-undecyloxypyrimidin
2-p-(6-Methyloctanoyloxy)-phenyl-5-dodecyloxypyrimidin
2-p-(6-Methyloctanoyloxy)-phenyl-5-heptoxymethyloxypyrimidin
2-p-(6-Methyloctanoyloxy)-phenyl-5-hexoxyethyloxypyrimidin
2-p-(6-Methyloctanoyloxy)-phenyl-5-pentoxypropyloxypyrimidin
2-p-(6-Methyloctyloxy)-phenyl-5-butyloxycarbonylpyrimidin
2-p-(6-Methyloctyloxy)-phenyl-5-pentyloxycarbonylpyrimidin
2-p-(6-Methyloctyloxy)-phenyl-5-hexyloxypcarbonylyrimidin
2-p-(6-Methyloctyloxy)-phenyl-5-heptyloxycarbonylpyrimidin
2-p-(6-Methyloctyloxy)-phenyl-5-octyloxycarbonylpyrimidin, F. 78°,K. 80°
2-p-(6-Methyloctyloxy)-phenyl-5-nonyloxycarbonylpyrimidin
2-p-(6-Methyloctyloxy)-phenyl-5-decyloxycarbonylpyrimidin
2-p-(6-Methyloctyloxy)-phenyl-5-undecyloxycarbonylpyrimidin
2-p-(6-Methyloctyloxy)-phenyl-5-dodecyloxycarbonylpyrimidin
2-p-(6-Methyloctyloxy)-phenyl-5-heptoxymethyloxycarbonylpyrimidin
2-p-(6-Methyloctyloxy)-phenyl-5-hexoxyethyloxycarbonylpyrimidin
2-p-(6-Methyloctyloxy)-phenyl-5-pentoxypropyloxycarbonylpyrimidin

2-p-Octyloxyphenyl-5-(2-methyloctyloxycarbonyl)-pyrimidin
2-p-Octyloxyphenyl-5-(3-methyloctyloxycarbonyl)-pyrimidin
2-p-Octyloxyphenyl-5-(4-methyloctyloxycarbonyl)-pyrimidin
2-p-Octyloxyphenyl-5-(5-methyloctyloxycarbonyl)-pyrimidin
2-p-Octyloxyphenyl-5-(6-methyloctyloxycarbonyl)-pyrimidin, F. 80°
2-p-Dodecyloxyphenyl-5-(2-methyloctyloxycarbonyl)-pyrimidin
2-p-Undecyloxyphenyl-5-(2-methylbutoxy)-pyrimidin, F. 51°
2-p-Decyloxyphenyl-5-(2-methylbutoxy)-pyrimidin
2-p-Nonyloxyphenyl-5-(2-methylbutoxy)-pyrimidin
2-p-Octyloxyphenyl-5-(2-methylbutoxy)-pyrimidin
2-p-Heptyloxyphenyl-5-(2-methylbutoxy)-pyrimidin
2-p-Dodecanoyloxyphenyl-5-(2-methylbutoxy)-pyrimidin
2-p-Undecanoyloxyphenyl-5-(2-methylbutoxy)-pyrimidin, F. 58°
2-p-Decanoyloxyphenyl-5-(2-methylbutoxy)-pyrimidin
2-p-Nonanoyloxyphenyl-5-(2-methylbutoxy)-pyrimidin
2-p-Octanoyloxyphenyl-5-(2-methylbutoxy)-pyrimidin
2-p-Heptanoyloxyphenyl-5-(2-methylbutoxy)-pyrimidin
R-4-(5-Dodecylpyrimidyl-2)-phenyl-2-chlorpropionat
R-4-(5-Undecylpyrimidyl-2)-phenyl-2-chlorpropionat
R-4-(5-Decylpyrimidyl-2)-phenyl-2-chlorpropionat
R-4-(5-Octylpyrimidyl-2)-phenyl-2-chlorpropionat
R-4-(5-Heptylpyrimidyl-2)-phenyl-2-chlorpropionat, F. 93 °
R-4-(5-Hexylpyrimidyl-2)-phenyl-2-chlorpropionat, F. 82 °
R-4-(5-Pentylpyrimidyl-2)-phenyl-2-chlorpropionat
R-4-(5-Butylpyrimidyl-2)-phenyl-2-chlorpropionat
R-4-(5-Propylpyrimidyl-2)-phenyl-2-chlorpropioriat

Durch Veresterung von 5-(p-Alkylphenyl)-pyrazin-2-ol (erhältlich durch $SeO_2$-Oxidation von p-Alkylacetophenon und nachfolgende Umsetzung mit Glycinamidhydrochlorid nach literaturbekannten Methoden) erhält man in analoger Weise:

R-5-(p-Propylphenyl)-pyrazin-2-yl-2-chlorpropionat
R-5-(p-Butylphenyl)-pyrazin-2-yl-2-chlorpropionat
R-5-(p-Pentylphenyl)-pyrazin-2-yl-2-chlorpropionat
R-5-(p-Hexylphenyl)-pyrazin-2-yl-2-chlorpropionat
R-5-(p-Heptylphenyl)-pyrazin-2-yl-2-chlorpropionat
R-5-(p-Octylphenyl)-pyrazin-2-yl-2-chlorpropionat
R-5-(p-Nonylphenyl)-pyrazin-2-yl-2-chlorpropionat
R-5-(p-Decylphenyl)-pyrazin-2-yl-2-chlorpropionat
R-5-(p-Undecylphenyl)-pyrazin-2-yl-2-chlorpropionat
R-5-(p-Dodecylphenyl)-pyrazin-2-yl-2-chlorpropionat

Die eingesetzten optisch aktiven Alkohole bzw. Carbonsäuren sind entweder bekannt oder können in Analogie zu bekannten Verbindungen z.B. durch Homologisierung nach literaturbekannten Methoden erhalten werden.

Beispiel 2

Zu einer Suspension von 7,5 g 4-(5-n-Heptylpyrimidin-2-yl)-benzoesäure [erhältlich durch alkalische Verseifung von 2-(4-Cyanphenyl)-5-n-heptylpyrimidin] in 80 ml Dichlormethan gibt man 3,3 g d-2-Octanol und 0,3 g 4-N,N-(Dimethylamino)-pyridin, versetzt bei 5-10 ° unter Rühren mit 5,8 g Dicyclohexylcarbodiimid, rührt noch 30 Minuten bei 10 ° und anschließend über Nacht bei Raumtemperatur, filtriert vom ausgefallenen Harnstoff ab und arbeitet wie üblich auf. Man erhält 5-n-Heptyl-2-(4-carbo-d-2-octyloxyphenyl)-pyrimidin, F. 19 °.

Analog werden hergestellt:

5-Dodecyl-2-(4-carbo-d-2-octyloxyphenyl)-pyrimidin
5-Undecyl-2-(4-carbo-d-2-octyloxyphenyl)-pyrimidin
5-Decyl-2-(4-carbo-d-2-octyloxyphenyl)-pyrimidin
5-Nonyl-2-(4-carbo-d-2-octyloxyphenyl)-pyrimidin, F. 15°
5-Octyl-2-(4-carbo-d-2-octyloxyphenyl)-pyrimidin
5-Hexyl-2-(4-carbo-d-2-octyloxyphenyl)-pyrimidin
5-Pentyl-2-(4-carbo-d-2-octyloxyphenyl)-pyrimidin

5-Butyl-2-(4-carbo-d-2-octyloxyphenyl)-pyrimidin
5-Propyl-2-(4-carbo-d-2-octyloxyphenyl)-pyrimidin
5-Ethyl-2-(4-carbo-d-2-octyloxyphenyl)-pyrimidin
5-Dodecyl-2-[4-carbo-(S-2-methyl-1-butoxy)-phenyl]-pyrimidin
5-Undecyl-2-[4-carbo-(S-2-methyl-1-butoxy)-phenyl]-pyrimidin
5-Decyl-2-[4-carbo-(S-2-methyl-1-butoxy)-phenyl]-pyrimidin
5-Nonyl-2-[4-carbo-(S-2-methyl-1-butoxy)-phenyl]-pyrimidin
5-Octyl-2-[4-carbo-(S-2-methyl-1-butoxy)-phenyl]-pyrimidin
5-Heptyl-2-[4-carbo-(S-2-methyl-1-butoxy)-phenyl]-pyrimidin, F. 55,5 °
5-Hexyl-2-[4-carbo-(S-2-methyl-1-butoxy)-phenyl]-pyrimidin
5-Pentyl-2-[4-carbo-(S-2-methyl-1-butoxy)-phenyl]-pyrimidin
5-Butyl-2-[4-carbo-(S-2-methyl-1-butoxy)-phenyl]-pyrimidin
5-Propyl-2-[4-carbo-(S-2-methyl-1-butoxy)-phenyl]-pyrimidin
5-Ethyl-2-[4-carbo-(S-2-methyl-1-butoxy)-phenyl]-pyrimidin
R-(2-Chlorpropyl)-p-(5-nonylpyrimidin-2-yl)-benzoat
R-(2-Chlorpropyl)-p-(5-decylpyrimidin-2-yl)-benzoat
R-(2-Chlorpropyl)-p-(5-undecylpyrimidin-2-yl)-benzoat
R-(2-Chlorpropyl)-p-(5-dodecylpyrimidin-2-yl)-benzoat
R-(2-Chlorpropyl)-p-(5-octylpyrimidin-2-yl)-benzoat
R-(2-Chlorpropyl)-p-(5-heptylpyrimidin-2-yl)-benzoat
R-(2-Chlorpropyl)-p-(5-hexylpyrimidin-2-yl)-benzoat
R-(2-Chlorpropyl)-p-(5-pentylpyrimidin-2-yl)-benzoat
R-(2-Chlorpropyl)-p-(5-butylpyrimidin-2-yl)-benzoat

Beispiel 3

Durch Veresterung von 27,2 g p-(5-n-Hexylpyrimidyl-2)-phenol mit S-4-(2-Methylbutyl)-benzoesäure in Dichlormethan unter Zusatz von 25,0 g Dicyclohexylcarbodiimid erhält man nach üblicher Aufarbeitung S-4-(5-n-Hexylpyrimidyl-2)-phenyl-4-(2-methylbutyl)-benzoat.

Analog werden hergestellt:
S-4-(5-Propylpyrimidyl-2)-phenyl-4-(2-methylbutyl)-benzoat
S-4-(5-Butylpyrimidyl-2)-phenyl-4-(2-methylbutyl)-benzoat
S-4-(5-Pentylpyrimidyl-2)-phenyl-4-(2-methylbutyl)-benzoat
S-4-(5-Heptylpyrimidyl-2)-phenyl-4-(2-methylbutyl)-benzoat
S-4-(5-Octylpyrimidyl-2)-phenyl-4-(2-methylbutyl)-benzoat
S-4-(5-Nonylpyrimidyl-2)-phenyl-4-(2-methylbutyl)-benzoat
S-4-(5-Decylpyrimidyl-2)-phenyl-4-(2-methylbutyl)-benzoat
S-4-(5-Undecylpyrimidyl-2)-phenyl-4-(2-methylbutyl)-benzoat
S-4-(5-Dodecylpyrimidyl-2)-phenyl-4-(2-methylbutyl)-benzoat
S-4-(5-Propylpyrimidyl-2)-benzoesäure-4-(2-methylbutylphenyl)-ester
S-4-(5-Butylpyrimidyl-2)-benzoesäure-4-(2-methylbutylphenyl)-ester
S-4-(5-Pentylpyrimidyl-2)-benzoesäure-4-(2-methylbutylphenyl)-ester
S-4-(5-Hexylpyrimidyl-2)-benzoesäure-4-(2-methylbutylphenyl)-ester
S-4-(5-Heptylpyrimidyl-2)-benzoesäure-4-(2-methylbutylphenyl)-ester
S-4-(5-Octylpyrimidyl-2)-benzoesäure-4-(2-methylbutylphenyl)-ester
S-4-(5-Nonylpyrimidyl-2)-benzoesäure-4-(2-methylbutylphenyl)-ester
S-4-(5-Decylpyrimidyl-2)-benzoesäure-4-(2-methylbutylphenyl)-ester
S-4-(5-Undecylpyrimidyl-2)-benzoesäure-4-(2-methylbutylphenyl)-ester
S-4-(5-Dodecylpyrimidyl-2)-benzoesäure-4-(2-methylbutylphenyl)-ester

Beispiel 4

Durch Veresterung von 17,9 5-n-Butylpyrimidin-2-carbonsäure mit 16,4 g S-4-(2-Methylbutyl)-phenol in Dichlormethan unter Zusatz von 25,0 g Dicyclohexylcarbodiimid erhält man nach üblicher Aufarbeitung S-4-(2-Methylbutyl)-phenyl-5-butylpyrimidin-2-carboxylat.

Analog wurden hergestellt:
S-4-(2-Methylbutyl)-phenyl-5-propylpyridin-2-carboxylat
S-4-(2-Methylbutyl)-phenyl-5-pentylpyridin-2-carboxylat

S-4-(2-Methylbutyl)-phenyl-5-hexylpyridin-2-carboxylat
S-4-(2-Methylbutyl)-phenyl-5-heptylpyridin-2-carboxylat
S-4-(2-Methylbutyl)-phenyl-5-octylpyridin-2-carboxylat
S-4-(2-Methylbutyl)-phenyl-5-nonylpyridin-2-carboxylat
S-4-(2-Methylbutyl)-phenyl-5-decylpyridin-2-carboxylat
S-4-(2-Methylbutyl)-phenyl-5-undecylpyridin-2-carboxylat
S-4-(2-Methylbutyl)-phenyl-5-dodecylpyridin-2-carboxylat
S-4-(2-Methylbutyl)-phenyl-5-propylpyrimidyl-2-carboxylat
S-4-(2-Methylbutyl)-phenyl-5-pentylpyrimidyl-2-carboxylat
S-4-(2-Methylbutyl)-phenyl-5-hexylpyrimidyl-2-carboxylat
S-4-(2-Methylbutyl)-phenyl-5-heptylpyrimidyl-2-carboxylat
S-4-(2-Methylbutyl)-phenyl-5-octylpyrimidyl-2-carboxylat
S-4-(2-Methylbutyl)-phenyl-5-nonylpyrimidyl-2-carboxylat
S-4-(2-Methylbutyl)-phenyl-5-decylpyrimidyl-2-carboxylat
S-4-(2-Methylbutyl)-phenyl-5-undecylpyrimidyl-2-carboxylat
S-4-(2-Methylbutyl)-phenyl-5-dodecylpyrimidyl-2-carboxylat

Beispiel 5

Nach der in Beispiel 3 genannten Weise werden 28,0 g der aus dem bekannten 4-(5-n-Hexylpyrazinyl-2)-benzonitril (Japanische Offenlegungsschrift 43 961/1983) durch Verseifen mit alkoholischer Kalilauge und anschließendem Neutralisieren mit verdünnter Salzsäure erhältlichen Carbonsäure mit 25,0 g Dicyclohexylcarbodiimid und 13,0 g R-2-Octanol angesetzt. Man erhält nach üblicher Aufarbeitung R-2-Octyl-4-(5-n-Hexylpyrazinyl-2)-benxoat.

Analog werden hergestellt:
R-2-Octyl-4-(5-Propylpyrazinyl-2)-benzoat
R-2-Octyl-4-(5-Butylpyrazinyl-2)-benzoat
R-2-Octyl-4-(5-Pentylpyrazinyl-2)-benzoat
R-2-Octyl-4-(5-Heptylpyrazinyl-2)-benzoat
R-2-Octyl-4-(5-Octylpyrazinyl-2)-benzoat
R-2-Octyl-4-(5-Nonylpyrazinyl-2)-benzoat
R-2-Octyl-4-(5-Decylpyrazinyl-2)-benzoat
R-2-Octyl-4-(5-Undecylpyrazinyl-2)-benzoat
R-2-Octyl-4-(5-Dodecylpyrazinyl-2)-benzoat

Beispiel 6

Die aus 2,4 g Magnesium, 15,1 g S-2-Methylbutylbromid und 200 ml THF nach Grignard hergestellte Lösung wird mit einer Lösung von 26,1 g 4-(5-n-Hexylpyrazinyl-2)-benzonitril umgesetzt. Der aus der organischen Phase nach dem Abdestillieren des Lösungsmittels verbleibende Rückstand wird wie üblich aufgearbeitet. Man erhält optisch aktives 3-Methylvaleryl-4-(5-n-hexylpyrazinyl-2)-benzol.

Analog werden hergestellt:
3-Methylvaleryl-4-(5-n-propylpyrazinyl-2)-benzol
3-Methylvaleryl-4-(5-n-butylpyrazinyl-2)-benzol
3-Methylvaleryl-4-(5-n-pentylpyrazinyl-2)-benzol
3-Methylvaleryl-4-(5-n-heptylpyrazinyl-2)-benzol
3-Methylvaleryl-4-(5-n-octylpyrazinyl-2)-benzol
3-Methylvaleryl-4-(5-n-nonylpyrazinyl-2)-benzol
3-Methylvaleryl-4-(5-n-decylpyrazinyl-2)-benzol
3-Methylvaleryl-4-(5-n-undecylpyrazinyl-2)-benzol
3-Methylvaleryl-4-(5-n-dodecylpyrazinyl-2)-benzol

Beispiel 7

Eine flüssigkristalline Phase bestehend aus
38,3 % 2-p-Nonyloxyphenyl-5-nonylpyrimidin,
2,0 % 2-p-Hexyloxyphenyl-5-nonylpyrimidin,
36,1 % 2-p-Decyloxyphenyl-5-heptylpyrimidin,

5,9 % 2-p-Nonyloxyphenyl-5-heptylpyrimidin,
5,9 % 2-p-Octyloxyphenyl-5-heptylpyrimidin,
5,9 % 2-p-Heptyloxyphenyl-5-heptylpyrimidin und
5,9 % 2-p-Hexyloxyphenyl-5-heptylpyrimidin
wird mit verschiedenen Mengen der chiralen Verbindung R-4-(5-hexylpyrimidyl-2)-phenyl-2-chlorpropionat dotiert. Die Phasenübergangstemperaturen sowie die Werte der spontanen Polarisation der somit erhaltenen Mischungen sind in der folgenden Tabelle angegeben. Die Werte für P gelten jeweils für 10° unterhalb des $S_C^*/S_A^*$-Übergangs.

Zugabe von X % des chiralen Dotierstoffes

| X | $S_C^*$ | $S_A^*$ | Ch | I | P |
|---|---|---|---|---|---|
| 3 | 48,1 | 65 | 68,7 | | 1,08 |
| 6 | 41,0 | 64,1 | 68,1 | | 2,14 |
| 10 | 34,5 | 63 | 68 | | 3,12 |

Beispiel 8

Ein Gemisch von 23,6 g p-(5-n-Heptylpyrimidin-2-yl)-zimtsäure (F. 201°, erhältlich durch 48-stündiges Kochen eines Gemisches von 22,2 g 2-p-Bromphenyl-5-n-heptylpyrimidin, 18,4 Triethylamin, 9,2 g Acrylsäure, 0,17 g Pd(II)-acetat, 0,6 g Tritolylphosphin und 75 ml Acetonitril, Abkühlen auf 0°, Absaugen, Waschen des Niederschlages mit Acetonitril und Wasser und Umkristallisation aus Toluol), 13,0 g R-2-Octanol und 25,0 g Dicyclohexylcarbodiimid in THF wird in Gegenwart von Dimethylaminopyridin 24 Stunden gekocht und wie üblich aufgearbeitet. Man erhält R-p-(5-n-Heptylpyrimidin-2-yl)-zimtsäure-2-octylester, F. 40°, K. 58°.

Analog werden hergestellt:
R-p-(5-Propylpyrimidin-2-yl)-zimtsäure-2-octylester
R-p-(5-Butylpyrimidin-2-yl)-zimtsäure-2-octylester
R-p-(5-Pentylpyrimidin-2-yl)-zimtsäure-2-octylester
R-p-(5-Hexylpyrimidin-2-yl)-zimtsäure-2-octylester
R-p-(5-Octylpyrimidin-2-yl)-zimtsäure-2-octylester
R-p-(5-Nonylpyrimidin-2-yl)-zimtsäure-2-octylester
R-p-(5-Decylpyrimidin-2-yl)-zimtsäure-2-octylester
R-p-(5-Undecylpyrimidin-2-yl)-zimtsäure-2-octylester
R-p-(5-Dodecylpyrimidin-2-yl)-zimtsäure-2-octylester
R-p-(5-Propylpyrimidin-2-yl)-zimtsäure-2-methylbutylester
R-p-(5-Butylpyrimidin-2-yl)-zimtsäure-2-methylbutylester
R-p-(5-Pentylpyrimidin-2-yl)-zimtsäure-2-methylbutylester
R-p-(5-Hexylpyrimidin-2-yl)-zimtsäure-2-methylbutylester
R-p-(5-Heptylpyrimidin-2-yl)-zimtsäure-2-methylbutylester, F. 58°, K. 96°
R-p-(5-Octylpyrimidin-2-yl)-zimtsäure-2-methylbutylester
R-p-(5-Nonylpyrimidin-2-yl)-zimtsäure-2-methylbutylester
R-p-(5-Decylpyrimidin-2-yl)-zimtsäure-2-methylbutylester
R-p-(5-Undecylpyrimidin-2-yl)-zimtsäure-2-methylbutylester
R-p-(5-Dodecylpyrimidin-2-yl)-zimtsäure-2-methylbutylester.

Beispiel 9

Zu einer Suspension von 40 g Kaliumcarbonat in 200 ml Aceton werden 26,3 g des aus p-Toluolsulfonsäurechlorid und (S)-3-Chlorbutanol-1 herstellbaren (S)-3-Chlorbutyl-1-tosylats und 27,2 g 4-(5-n-Hexylpyrimidin-2-yl)-phenol gegeben. Nach 24-stündigem Kochen am Rückfluß wird die Reaktionslösung filtriert, das Filtrat vom Lösungsmittel befreit und der Rückstand aus Ethanol zweimal umkristallisiert. Man erhält (S)-2-[p-(3-Chlorbutyloxy)-phenyl]-5-n-hexylpyrimidin.

Analog werden hergestellt:

2-[p-(3-Chlorbutyloxy)-phenyl]-5-n-butylpyrimidin
2-[p-(3-Chlorbutyloxy)-phenyl]-5-n-pentylpyrimidin
2-[p-(3-Chlorbutyloxy)-phenyl]-5-n-heptylpyrimidin
2-[p-(3-Chlorbutyloxy)-phenyl]-5-n-octylpyrimidin
2-[p-(3-Chlorbutyloxy)-phenyl]-5-n-nonylpyrimidin
2-[p-(3-Chlorbutyloxy)-phenyl]-5-n-decylpyrimidin
2-[p-(3-Chlorbutyloxy)-phenyl]-5-n-undecylpyrimidin
2-[p-(3-Chlorbutyloxy)-phenyl]-5-n-dodecylpyrimidin
2-[p-(3-Cyanbutyloxy)-phenyl]-5-n-butylpyrimidin
2-[p-(3-Cyanbutyloxy)-phenyl]-5-n-pentylpyrimidin
2-[p-(3-Cyanbutyloxy)-phenyl]-5-n-hexylpyrimidin
2-[p-(3-Cyanbutyloxy)-phenyl]-5-n-heptylpyrimidin
2-[p-(3-Cyanbutyloxy)-phenyl]-5-n-octylpyrimidin
2-[p-(3-Cyanbutyloxy)-phenyl]-5-n-nonylpyrimidin
2-[p-(3-Cyanbutyloxy)-phenyl]-5-n-decylpyrimidin
2-[p-(3-Cyanbutyloxy)-phenyl]-5-n-undecylpyrimidin
2-[p-(3-Cyanbutyloxy)-phenyl]-5-n-dodecylpyrimidin

Beispiel 10

Ein Gemisch von 40 g (S)-4,6-Dichlor-2-(p-heptoxyloxyphenyl)-5-(2-methylbutyl)-pyrimidin [erhältlich durch Umsetzung von (S)-2-Methylbutylbromid mit Malonsäurediethylester in Gegenwart von Natriummethanolat, Kondensation des erhaltenen (S)-2-Methylbutyl-malonsäurediethylesters mit p-Heptyloxybenzamidinhydrochlorid und Umsetzung des erhaltenen (S)-4,6-Dihydroxy-2-(p-heptyloxyphenyl)-5-(2- methylbutyl)-pyrimidin mit Phosphoroxytrichlorid und Dimethylanilin], 400 ml Methanol, 40 ml Triethylamin und 20 g Pd-C (5 %) wird bei Raumtemperatur unter Normaldruck bis zur Aufnahme der theoretisch benötigten Wasserstoffmenge hydriert. Nach dem Abfiltrieren des Katalysators und dem Abdestillieren des Lösungsmittels wird der Rückstand aus Ethanol umkristallisiert. Man erhält (S)-2-(p-Heptyloxyphenyl) -5-(2-methylbutyl)-pyrimidin.

Analog werden hergestellt:

2-(p-Pentyloxyphenyl)-5-(2-methylbutyl)-pyrimidin
2-(p-Hexyloxyphenyl)-5-(2-methylbutyl)-pyrimidin
2-(p-Octyloxyphenyl)-5-(2-methylbutyl)-pyrimidin
2-(p-Nonyloxyphenyl)-5-(2-methylbutyl)-pyrimidin
2-(p-Decyloxyphenyl)-5-(2-methylbutyl)-pyrimidin
2-(p-Undecyloxyphenyl)-5-(2-methylbutyl)-pyrimidin
2-(p-Dodecyloxyphenyl)-5-(2-methylbutyl)-pyrimidin

Beispiel 11

Ein Gemisch aus 8 g (S,S)-3-Methyl-2-chlorpentansäure, 16 g p-(5-n-Octylpyrimidin-2-yl)phenol, 11,6 g N,N-Dicyclohexylcarbodiimid, 0,6 g 4-N,N-Dimethylaminopyridin und 300 ml Dichlormethan wird über Nacht bei Raumtemperatur gerührt. Nach Abfiltrieren des ausgefallenen Harnstoffderivates wird das Filtrat mit verd. Salzsäure und $H_2O$ gewaschen und die org. Phase wie üblich aufgearbeitet. Man erhält (S,S)-3-Methyl-2-chlorpentansäure-p-(5-n-octylpyrimidin-2-yl)-phenylester.

Analog werden hergestellt:

3-Methyl-2-chlorpentansäure-p-(5-pentylpyrimidin-2-yl)-phenylester
3-Methyl-2-chlorpentansäure-p-(5-hexylpyrimidin-2-yl)-phenylester
3-Methyl-2-chlorpentansäure-p-(5-heptylpyrimidin-2-yl)-phenylester
3-Methyl-2-chlorpentansäure-p-(5-nonylpyrimidin-2-yl)-phenylester
3-Methyl-2-chlorpentansäure-p-(5-decylpyrimidin-2-yl)-phenylester

3-Methyl-2-brompentansäure-p-(5-pentylpyrimidin-2-yl)-phenylester
3-Methyl-2-brompentansäure-p-(5-hexylpyrimidin-2-yl)-phenylester
3-Methyl-2-brompentansäure-p-(5-heptylpyrimidin-2-yl)-phenylester
3-Methyl-2-brompentansäure-p-(5-octylpyrimidin-2-yl)-phenylester
3-Methyl-2-brompentansäure-p-(5-nonylpyrimidin-2-yl)-phenylester
3-Methyl-2-brompentansäure-p-(5-decylpyrimidin-2-yl)-phenylester
3-Methyl-2-chlorpentansäure-p-(5-pentylpyrazin-2-yl)-phenylester
3-Methyl-2-chlorpentansäure-p-(5-hexylpyrazin-2-yl)-phenylester
3-Methyl-2-chlorpentansäure-p-(5-heptylpyrazin-2-yl)-phenylester
3-Methyl-2-chlorpentansäure-p-(5-octylpyrazin-2-yl)-phenylester
3-Methyl-2-chlorpentansäure-p-(5-nonylpyrazin-2-yl)-phenylester
3-Methyl-2-chlorpentansäure-p-(5-decylpyrazin-2-yl)-phenylester
3-Methyl-2-cyanpentansäure-p-(5-pentylpyrazin-2-yl)-phenylester
3-Methyl-2-cyanpentansäure-p-(5-hexylpyrazin-2-yl)-phenylester
3-Methyl-2-cyanpentansäure-p-(5-heptylpyrazin-2-yl)-phenylester
3-Methyl-2-cyanpentansäure-p-(5-octylpyrazin-2-yl)-phenylester
3-Methyl-2-cyanpentansäure-p-(5-nonylpyrazin-2-yl)-phenylcster
3-Methyl-2-cyanpentansäure-p-(5-decylpyrazin-2-yl)-phenylester
3-Methyl-2-chlorhexancarbonsäure-p-(5-pentylpyrimidin-2-yl)-phenylester
3-Methyl-2-chlorhexancarbonsäure-p-(5-hexylpyrimidin-2-yl)-phenylester
3-Methyl-2-chlorhexancarbonsäure-p-(5-heptylpyrimidin-2-yl)-phenylester
3-Methyl-2-chlorhexancarbonsäure-p-(5-octylpyrimidin-2-yl)-phenylester
3-Methyl-2-chlorhexancarbonsäure-p-(5-nonylpyrimidin-2-yl)-phenylester
3-Methyl-2-chlorhexancarbonsäure-p-(5-decylpyrimidin-2-yl)-phenylester
3-Methyl-2-cyanhexancarbonsäure-p-(5-pentylpyrimidin-2-yl)-phenylester
3-Methyl-2-cyanhexancarbonsäure-p-(5-hexylpyrimidin-2-yl)-phenylester
3-Methyl-2-cyanhexancarbonsäure-p-(5-heptylpyrimidin-2-yl)-phenylester
3-Methyl-2-cyanhexancarbonsäure-p-(5-octylpyrimidin-2-yl)-phenylester
3-Methyl-2-cyanhexancarbonsäure-p-(5-nonylpyrimidin-2-yl)-phenylester
3-Methyl-2-cyanhexancarbonsäure-p-(5-decylpyrimidin-2-yl)-phenylester
3-Chlor-2-methylpentansäure-p-(5-pentylpyrimidin-2-yl)-phenylester
3-Chlor-2-methylpentansäure-p-(5-hexylpyrimidin-2-yl)-phenylester
3-Chlor-2-methylpentansäure-p-(5-heptylpyrimidin-2-yl)-phenylester
3-Chlor-2-methylpentansäure-p-(5-octylpyrimidin-2-yl)-phenylester
3-Chlor-2-methylpentansäure-p-(5-nonylpyrimidin-2-yl)-phenylester
3-Chlor-2-methylpentansäure-p-(5-decylpyrimidin-2-yl)-phenylester
3-Brom-2-methylpentansäure-p-(5-pentylpyrazin-2-yl)-phenylester
3-Brom-2-methylpentansäure-p-(5-hexylpyrazin-2-yl)-phenylester
3-Brom-2-methylpentansäure-p-(5-heptylpyrazin-2-yl)-phenylester
3-Brom-2-methylpentansäure-p-(5-octylpyrazin-2-yl)-phenylester
3-Brom-2-methylpentansäure-p-(5-nonylpyrazin-2-yl)-phenylester
3-Brom-2-methylpentansäure-p-(5-decylpyrazin-2-yl)-phenylester
3-Cyan-2-methylhexansäure-p-(5-pentylpyrimidin-2-yl)-phenylester
3-Cyan-2-methylhexansäure-p-(5-hexylpyrimidin-2-yl)-phenylester
3-Cyan-2-methylhexansäure-p-(5-heptylpyrimidin-2-yl)-phenylester
3-Cyan-2-methylhexansäure-p-(5-octylpyrimidin-2-yl)-phenylester
3-Cyan-2-methylhexansäure-p-(5-nonylpyrimidin-2-yl)-phenylester
3-Cyan-2-methylhexansäure-p-(5-decylpyrimidin-2-yl)-phenylester.

Beispiel 12:

0,01 Mol optisch aktives 1-[4-(5-Heptylpyrimidin-(2)-yl)-phenyl-(1)]propanol-(2) (hergestellt aus käuflichem R-Propylenoxid und 4-(5-Heptylpyrimidin-(2)-yl)-phenylmagnesiumbromid) wird zusammen mit 0,01 Mol Buttersäure, 2,3 g Dicyclohexylcarbodiimid, 0,2 g 4-N,N-Dimethylaminopyridin und 25 ml Dichlormethan bei Zimmertemperatur 48 Stunden gerührt.

Darauf wird im Eisbad abgekühlt, der Niederschlag von Dicyclohexylharnstoff abgesaugt und mit Dichlormethan gewaschen. Die vereinigten Filtrate werden eingedampft und über Kieselgel chromatographiert. Nach Umkristallisation erhält man optisch aktives 1-[4-(5-Heptylpyrimidin-(2)-yl)phenyl]-2-propylbutyrat.

Analog werden hergestellt:
1-[4-(5-Propylpyrimidin-(2)-yl)-phenyl]-2-propylbutyrat
1-[4-(5-Butylpyrimidin-(2)-yl)-phenyl]-2-propylbutyrat
1-[4-(5-Pentylpyrimidin-(2)-yl)-phenyl]-2-propylbutyrat
1-[4-(5-Hexylpyrimidin-(2)-yl)-phenyl]-2-propylbutyrat
1-[4-(5-Octylpyrimidin-(2)-yl)-phenyl]-2-propylbutyrat
1-[4-(5-Nonylpyrimidin-(2)-yl)-phenyl]-2-propylbutyrat
1-[4-(5-Propyloxypyrimidin-(2)-yl)-phenyl]-2-propylbutyrat
1-[4-(5-Butyloxypyrimidin-(2)-yl)-phenyl]-2-propylbutyrat
1-[4-(5-Pentyloxypyrimidin-(2)-yl)-phenyl]-2-propylbutyrat
1-[4-(5-Hexyloxypyrimidin-(2)-yl)-phenyl]-2-propylbutyrat
1-[4-(5-Heptyloxypyrimidin-(2)-yl)-phenyl]-2-propylbutyrat
1-[4-(5-Octyloxypyrimidin-(2)-yl)-phenyl]-2-propylbutyrat
1-[4-(5-Nonyloxypyrimidin-(2)-yl)-phenyl]-2-propylbutyrat
1-[4-(5-Propylpyrimidin-(2)-yl)-phenyl]-2-propylpropionat
1-[4-(5-Butylpyrimidin-(2)-yl)-phenyl]-2-propylpropionat
1-[4-(5-Pentylpyrimidin-(2)-yl)-phenyl]-2-propylpropionat
1-[4-(5-Hexylpyrimidin-(2)-yl)-phenyl]-2-propylpropionat
1-[4-(5-Heptylpyrimidin-(2)-yl)-phenyl]-2-propylpropionat
1-[4-(5-Octylpyrimidin-(2)-yl)-phenyl]-2-propylpropionat
1-[4-(5-Nonylpyrimidin-(2)-yl)-phenyl]-2-propylpropionat
1-[4-(5-Propyloxypyrimidin-(2)-yl)-phenyl]-2-propylpropionat
1-[4-(5-Butyloxypyrimidin-(2)-yl)-phenyl]-2-propylpropionat
1-[4-(5-Pentyloxypyrimidin-(2)-yl)-phenyl]-2-propylpropionat
1-[4-(5-Hexyloxypyrimidin-(2)-yl)-phenyl]-2-propylpropionat
1-[4-(5-Heptyloxypyrimidin-(2)-yl)-phenyl]-2-propylpropionat
1-[4-(5-Octyloxypyrimidin-(2)-yl)-phenyl]-2-propylpropionat
1-[4-(5-Nonyloxypyrimidin-(2)-yl)-phenyl]-2-propylpropionat
1-[4-(5-Propylpyrimidin-(2)-yl)-phenyl]-2-propylvalerat
1-[4-(5-Butylpyrimidin-(2)-yl)-phenyl]-2-propylvalerat
1-[4-(5-Pentylpyrimidin-(2)-yl)-phenyl]-2-propylvalerat
1-[4-(5-Hexylpyrimidin-(2)-yl)-phenyl]-2-propylvalerat
1-[4-(5-Heptylpyrimidin-(2)-yl)-phenyl]-2-propylvalerat
1-[4-(5-Octylpyrimidin-(2)-yl)-phenyl]-2-propylvalerat
1-[4-(5-Nonylpyrimidin-(2)-yl)-phenyl]-2-propylvalerat
1-[4-(5-Propyloxypyrimidin-(2)-yl)-phenyl]-2-propylvalerat
1-[4-(5-Butyloxypyrimidin-(2)-yl)-phenyl]-2-propylvalerat
1-[4-(5-Pentyloxypyrimidin-(2)-yl)-phenyl]-2-propylvalerat
1-[4-(5-Hexyloxypyrimidin-(2)-yl)-phenyl]-2-propylvalerat
1-[4-(5-Heptyloxypyrimidin-(2)-yl)-phenyl]-2-propylvalerat
1-[4-(5-Octyloxypyrimidin-(2)-yl)-phenyl]-2-propylvalerat
1-[4-(5-Nonyloxypyrimidin-(2)-yl)-phenyl]-2-propylvalerat
1-[4-(5-Propylpyrimidin-(2)-yl)-phenyl]-2-propylacetat
1-[4-(5-Butylpyrimidin-(2)-yl)-phenyl]-2-propylacetat
1-[4-(5-Pentylpyrimidin-(2)-yl)-phenyl]-2-propylacetat
1-[4-(5-Hexylpyrimidin-(2)-yl)-phenyl]-2-propylacetat
1-[4-(5-Heptylpyrimidin-(2)-yl)-phenyl]-2-propylacetat
1-[4-(5-Octylpyrimidin-(2)-yl)-phenyl]-2-propylacetat
1-[4-(5-Nonylpyrimidin-(2)-yl)-phenyl]-2-propylacetat
1-[4-(5-Propyloxypyrimidin-(2)-yl)-phenyl]-2-propylacetat
1-[4-(5-Butyloxypyrimidin-(2)-yl)-phenyl]-2-propylacetat
1-[4-(5-Pentyloxypyrimidin-(2)-yl)-phenyl]-2-propylacetat
1-[4-(5-Hexyloxypyrimidin-(2)-yl)-phenyl]-2-propylacetat
1-[4-(5-Heptyloxypyrimidin-(2)-yl)-phenyl]-2-propylacetat
1-[4-(5-Octyloxypyrimidin-(2)-yl)-phenyl]-2-propylacetat
1-[4-(5-Nonyloxypyrimidin-(2)-yl)-phenyl]-2-propylacetat
1-[4-(5-Propylphenyl)pyrazin-(2)-yl]-2-propylacetat
1-[4-(5-Butylphenyl)pyrazin-(2)-yl]-2-propylacetat

1-[4-(5-Pentylphenyl)pyrazin-(2)-yl]-2-propylacetat
1-[4-(5-Hexylphenyl)pyrazin-(2)-yl]-2-propylacetat
1-[4-(5-Heptylphenyl)pyrazin-(2)-yl]-2-propylacetat
1-[4-(5-Octylphenyl)pyrazin-(2)-yl]-2-propylacetat
1-[4-(5-Nonylphenyl)pyrazin-(2)-yl]-2-propylacetat
1-[4-(5-Propyloxyphenyl)pyrazin-(2)-yl]-2-propylacetat
1-[4-(5-Butyloxyphenyl)pyrazin-(2)-yl]-2-propylacetat
1-[4-(5-Pentyloxyphenyl)pyrazin-(2)-yl]-2-propylacetat
1-[4-(5-Hexyloxyphenyl)pyrazin-(2)-yl]-2-propylacetat
1-[4-(5-Heptyloxyphenyl)pyrazin-(2)-yl]-2-propylacetat
1-[4-(5-Octyloxyphenyl)pyrazin-(2)-yl]-2-propylacetat
1-[4-(5-Nonyloxyphenyl)pyrazin-(2)-yl]-2-propylacetat
1-[4-(5-Propylphenyl)pyrazin-(2)-yl]-2-propylpropionat
1-[4-(5-Butylphenyl)pyrazin-(2)-yl]-2-propylpropionat
1-[4-(5-Pentylphenyl)pyrazin-(2)-yl]-2-propylpropionat
1-[4-(5-Hexylphenyl)pyrazin-(2)-yl]-2-propylpropionat
1-[4-(5-Heptylphenyl)pyrazin-(2)-yl]-2-propylpropionat
1-[4-(5-Octylphenyl)pyrazin-(2)-yl]-2-propylpropionat
1-[4-(5-Nonylphenyl)pyrazin-(2)-yl]-2-propylpropionat
1-[4-(5-Propyloxyphenyl)pyrazin-(2)-yl]-2-propylpropionat
1-[4-(5-Butyloxyphenyl)pyrazin-(2)-yl]-2-propylpropionat
1-[4-(5-Pentyloxyphenyl)pyrazin-(2)-yl]-2-propylpropionat
1-[4-(5-Hexyloxyphenyl)pyrazin-(2)-yl]-2-propylpropionat
1-[4-(5-Heptyloxyphenyl)pyrazin-(2)-yl]-2-propylpropionat
1-[4-(5-Octyloxyphenyl)pyrazin-(2)-yl]-2-propylpropionat
1-[4-(5-Nonyloxyphenyl)pyrazin-(2)-yl]-2-propylpropionat
1-[4-(5-Propylphenyl)pyrazin-(2)-yl]-2-propylbutyrat
1-[4-(5-Butylphenyl)pyrazin-(2)-yl]-2-propylbutyrat
1-[4-(5-Pentylphenyl)pyrazin-(2)-yl]-2-propylbutyrat
1-[4-(5-Hexylphenyl)pyrazin-(2)-yl]-2-propylbutyrat
1-[4-(5-Heptylphenyl)pyrazin-(2)-yl]-2-propylbutyrat
1-[4-(5-Octylphenyl)pyrazin-(2)-yl]-2-propylbutyrat
1-[4-(5-Nonylphenyl)pyrazin-(2)-yl]-2-propylbutyrat
1-[4-(5-Propyloxyphenyl)pyrazin-(2)-yl]-2-propylbutyrat
1-[4-(5-Butyloxyphenyl)pyrazin-(2)-yl]-2-propylbutyrat
1-[4-(5-Pentyloxyphenyl)pyrazin-(2)-yl]-2-propylbutyrat
1-[4-(5-Hexyloxyphenyl)pyrazin-(2)-yl]-2-propylbutyrat
1-[4-(5-Heptyloxyphenyl)pyrazin-(2)-yl]-2-propylbutyrat
1-[4-(5-Octyloxyphenyl)pyrazin-(2)-yl]-2-propylbutyrat
1-[4-(5-Nonyloxyphenyl)pyrazin-(2)-yl]-2-propylbutyrat
1-[4-(5-Propylphenyl)pyrazin-(2)-yl]-2-propylvalerat
1-[4-(5-Butylphenyl)pyrazin-(2)-yl]-2-propylvalerat
1-[4-(5-Pentylphenyl)pyrazin-(2)-yl]-2-propylvalerat
1-[4-(5-Hexylphenyl)pyrazin-(2)-yl]-2-propylvalerat
1-[4-(5-Heptylphenyl)pyrazin-(2)-yl]-2-propylvalerat
1-[4-(5-Octylphenyl)pyrazin-(2)-yl]-2-propylvalerat
1-[4-(5-Nonylphenyl)pyrazin-(2)-yl]-2-propylvalerat
1-[4-(5-Propyloxyphenyl)pyrazin-(2)-yl]-2-propylvalerat
1-[4-(5-Butyloxyphenyl)pyrazin-(2)-yl]-2-propylvalerat
1-[4-(5-Pentyloxyphenyl)pyrazin-(2)-yl]-2-propylvalerat
1-[4-(5-Hexyloxyphenyl)pyrazin-(2)-yl]-2-propylvalerat
1-[4-(5-Heptyloxyphenyl)pyrazin-(2)-yl]-2-propylvalerat
1-[4-(5-Octyloxyphenyl)pyrazin-(2)-yl]-2-propylvalerat
1-[4-(5-Nonyloxyphenyl)pyrazin-(2)-yl]-2-propylvalerat.

Beispiel 13

0,01 Mol rechtsdrehende 3-[4-(5-Heptylpyrimidin-(2)-yl) phenyl]-buttersäure, erhalten durch Hydrierung

der entsprechenden 2-Butensäure über 5 % Pd/Kohle in Tetrahydrofuran und Racematspaltung mit (+)-Ephedrin (2-Methylamino-1-phenylpropanol-(1)), $\alpha^{20}_D$ = + 3,3°, wird analog Beispiel 12 mit 0,01 Mol optisch aktivem 2-Octanol verestert. Man erhält nach Reinigung durch Chromatographie und Umkristallisation 3-[4-(5-Heptylpyrimidin-(2)-yl)phenyl]-buttersäure-2-octylester.

Analog werden hergestellt:

3-[4-(5-Propylpyrimidin-(2)-yl)phenyl]-buttersäure-2-octylester
3-[4-(5-Butylpyrimidin-(2)-yl)phenyl]-buttersäure-2-octylester
3-[4-(5-Pentylpyrimidin-(2)-yl)phenyl]-buttersäure-2-octylester
3-[4-(5-Hexylpyrimidin-(2)-yl)phenyl]-buttersäure-2-octylester
3-[4-(5-Octylpyrimidin-(2)-yl)phenyl]-buttersäure-2-octylester
3-[4-(5-Nonylpyrimidin-(2)-yl)phenyl]-buttersäure-2-octylester
3-[4-(5-Propyloxypyrimidin-(2)-yl)phenyl]-buttersäure-2-octylester
3-[4-(5-Butyloxypyrimidin-(2)-yl)phenyl]-buttersäure-2-octylester
3-[4-(5-Pentyloxypyrimidin-(2)-yl)phenyl]-buttersäure-2-octylester
3-[4-(5-Hexyloxypyrimidin-(2)-yl)phenyl]-buttersäure-2-octylester
3-[4-(5-Heptyloxypyrimidin-(2)-yl)phenyl]-buttersäure-2-octylester
3-[4-(5-Octyloxypyrimidin-(2)-yl)phenyl]-buttersäure-2-octylester
3-[4-(5-Nonyloxypyrimidin-(2)-yl)phenyl]-buttersäure-2-octylester
3-[4-(5-Propylpyrimidin-(2)-yl)phenyl]-buttersäure-hexylester
3-[4-(5-Butylpyrimidin-(2)-yl)phenyl]-buttersäure-hexylester
3-[4-(5-Pentylpyrimidin-(2)-yl)phenyl]-buttersäure-hexylester
3-[4-(5-Hexylpyrimidin-(2)-yl)phenyl]-buttersäure-hexylester
3-[4-(5-Heptylpyrimidin-(2)-yl)phenyl]-buttersäure-hexylester
3-[4-(5-Octylpyrimidin-(2)-yl)phenyl]-buttersäure-hexylester
3-[4-(5-Nonylpyrimidin-(2)-yl)phenyl]-buttersäure-hexylester
3-[4-(5-Propyloxypyrimidin-(2)-yl)phenyl]-buttersäure-hexylester
3-[4-(5-Butyloxypyrimidin-(2)-yl)phenyl]-buttersäure-hexylester
3-[4-(5-Pentyloxypyrimidin-(2)-yl)phenyl]-buttersäure-hexylester
3-[4-(5-Hexyloxypyrimidin-(2)-yl)phenyl]-buttersäure-hexylester
3-[4-(5-Heptyloxypyrimidin-(2)-yl)phenyl]-buttersäure-hexylester
3-[4-(5-Octyloxypyrimidin-(2)-yl)phenyl]-buttersäure-hexylester
3-[4-(5-Nonyloxypyrimidin-(2)-yl)phenyl]-buttersäure-hexylester
3-[4-(5-Propylpyrimidin-(2)-yl)phenyl]-buttersäure-2-methyl-butylester
3-[4-(5-Butylpyrimidin-(2)-yl)phenyl]-buttersäure-2-methyl-butylester
3-[4-(5-Pentylpyrimidin-(2)-yl)phenyl]-buttersäure-2-methyl-butylester
3-[4-(5-Hexylpyrimidin-(2)-yl)phenyl]-buttersäure-2-methyl-butylester
3-[4-(5-Heptylpyrimidin-(2)-yl)phenyl]-buttersäure-2-methyl-butylester
3-[4-(5-Octylpyrimidin-(2)-yl)phenyl]-buttersäure-2-methyl-butylester
3-[4-(5-Nonylpyrimidin-(2)-yl)phenyl]-buttersäure-2-methyl-butylester
3-[4-(5-Propyloxypyrimidin-(2)-yl)phenyl]-buttersäure-2-methyl-butylester
3-[4-(5-Butyloxypyrimidin-(2)-yl)phenyl]-buttersäure-2-methyl-butylester
3-[4-(5-Pentyloxypyrimidin-(2)-yl)phenyl]-buttersäure-2-methyl-butylester
3-(4-(5-Hexyloxypyrimidin-(2)-yl)phenyl]-buttersäure-2-methyl-butylester
3-[4-(5-Heptyloxypyrimidin-(2)-yl)phenyl]-buttersäure-2-methyl-butylester
3-[4-(5-Octyloxypyrimidin-(2)-yl)phenyl]-buttersäure-2-methyl-butylester
3-[4-(5-Nonyloxypyrimidin-(2)-yl)phenyl]-buttersäure-2-methyl-butylester.

Beispiel 14

0,01 Mol α-Chlorpropionsäure-4-(5-hexylpyrimidin-(2)-yl)-phenylester (Beispiel 1) wird mit 0,01 Mol Natriumheptanolat in 20 ml N-Methylpyrrolidon bei 80° 2 Stunden erwärmt. Darauf wird auf Wasser gegossen, mit Toluol extrahiert und eingedampft. Man erhält nach Reinigung durch Chromatographie und Umkristallisation α-Heptyloxypropionsäure-4-(5-hexylpyrimidin-(2)-yl)-phenylester.

Analog werden hergestellt:

α-Heptyloxypropionsäure-4-(5-propylpyrimidin-(2)-yl)-phenylester
α-Heptyloxypropionsäure-4-(5-butylpyrimidin-(2)-yl)-phenylester
α-Heptyloxypropionsäure-4-(5-pentylpyrimidin-(2)-yl)-phenylester
α-Heptyloxypropionsäure-4-(5-heptylpyrimidin-(2)-yl)-phenylester

α-Heptyloxypropionsäure-4-(5-octylpyrimidin-(2)-yl)-phenylester
α-Heptyloxypropionsäure-4-(5-nonylpyrimidin-(2)-yl)-phenylester
α-Heptyloxypropionsäure-4-(5-propyloxypyrimidin-(2)-yl)-phenylester
α-Heptyloxypropionsäure-4-(5-butyloxypyrimidin-(2)-yl)-phenylester
α-Heptyloxypropionsäure-4-(5-pentyloxypyrimidin-(2)-yl)-phenylester
α-Heptyloxypropionsäure-4-(5-hexyloxypyrimidin-(2)-yl)-phenylester
α-Heptyloxypropionsäure-4-(5-heptyloxypyrimidin-(2)-yl)-phenylester
α-Heptyloxypropionsäure-4-(5-octyloxypyrimidin-(2)-yl)-phenylester
α-Heptyloxypropionsäure-4-(5-nonyloxypyrimidin-(2)-yl)-phenylester
α-Octyloxypropionsäure-4-(5-propylpyrimidin-(2)-yl)-phenylester
α-Octyloxypropionsäure-4-(5-butylpyrimidin-(2)-yl)-phenylester
α-Octyloxypropionsäure-4-(5-pentylpyrimidin-(2)-yl)-phenylester
α-Octyloxypropionsäure-4-(5-hexylpyrimidin-(2)-yl)-phenylester
α-Octyloxypropionsäure-4-(5-heptylpyrimidin-(2)-yl)-phenylester
α-Octyloxypropionsäure-4-(5-octylpyrimidin-(2)-yl)-phenylester
α-Octyloxypropionsäure-4-(5-nonylpyrimidin-(2)-yl)-phenylester
α-Octyloxypropionsäure-4-(5-piopyloxypyrimidin-(2)-yl)-phenylester
α-Octyloxypropionsäure-4-(5-butyloxypyrimidin-(2)-yl)-phenylester
α-Octyloxypropionsäure-4-(5-pentyloxypyrimidin-(2)-yl)-phenylester
α-Octyloxypropionsäure-4-(5-hexyloxypyrimidin-(2)-yl)-phenylester
α-Octyloxypropionsäure-4-(5-heptyloxypyrimidin-(2)-yl)-phenylester
α-Octyloxypropionsäure-4-(5-octyloxypyrimidin-(2)-yl)-phenylester
α-Octyloxypropionsäure-4-(5-nonyloxypyrimidin-(2)-yl)-phenylester
α-Hexyloxypropionsäure-4-(5-propylpyrimidin-(2)-yl)-phenylester
α-Hexyloxypropionsäure-4-(5-butylpyrimidin-(2)-yl)-phenylester
α-Hexyloxypropionsäure-4-(5-pentylpyrimidin-(2)-yl)-phenylester
α-Hexyloxypropionsäure-4-(5-hexylpyrimidin-(2)-yl)-phenylester
α-Hexyloxypropionsäure-4-(5-heptylpyrimidin-(2)-yl)-phenylester
α-Hexyloxypropionsäure-4-(5-octylpyrimidin-(2)-yl)-phenylester
α-Hexyloxypropionsäure-4-(5-nonylpyrimidin-(2)-yl)-phenylester
α-Hexyloxypropionsäure-4-(5-propyloxypyrimidin-(2)-yl)-phenylester
α-Hexyloxypropionsäure-4-(5-butyloxypyrimidin-(2)-yl)-phenylester
α-Hexyloxyropionsäure-4-(5-pentyloxypyrimidin-(2)-yl)-phenylester
α-Hexyloxypropionsäure-4-(5-hexyloxypyrimidin-(2)-yl)-phenylester
α-Hexyloxypropionsäure-4-(5-heptyloxypyrimidin-(2)-yl)-phenylester
α-Hexyloxypropionsäure-4-(5-octyloxypyrimidin-(2)-yl)-phenylester
α-Hexyloxypropionsäure-4-(5-nonyloxypyrimidin-(2)-yl)-phenylester
α-Pentyloxypropionsäure-4-(5-propylpyrimidin-(2)-yl)-phenylester
α-Pentyloxypropionsäure-4-(5-butylpyrimidin-(2)-yl)-phenylester
α-Pentyloxypropionsäure-4-(5-pentylpyrimidin-(2)-yl)-phenylester
α-Pentyloxypropionsäure-4-(5-hexylpyrimidin-(2)-yl)-phenylester
α-Pentyloxypropionsäure-4-(5-heptylpyrimidin-(2)-yl)-phenylester
α-Pentyloxypropionsäure-4-(5-octylpyrimidin-(2)-yl)-phenylester
α-Pentyloxypropionsäure-4-(5-nonylpyrimidin-(2)-yl)-phenylester
α-Pentyloxypropionsäure-4-(5-propyloxypyrimidin-(2)-yl)-phenylester
α-Pentyloxypropionsäure-4-(5-butyloxypyrimidin-(2)-yl)-phenylester
α-Pentyloxypropionsäure-4-(5-pentyloxypyrimidin-(2)-yl)-phenylester
α-Pentyloxypropionsäure-4-(5-hexyloxypyrimidin-(2)-yl)-phenylester
α-Pentyloxypropionsäure-4-(5-heptyloxypyrimidin-(2)-yl)-phenylester
α-Pentyloxypropionsäure-4-(5-octyloxypyrimidin-(2)-yl)-phenylester
α-Pentyloxypropionsäure-4-(5-nonyloxypyrimidin-(2)-yl)-phenylester
α-Butyloxypropionsäure-4-(5-propylpyrimidin-(2)-yl)-phenylester
α-Butyloxypropionsäure-4-(5-butylpyrimidin-(2)-yl)-phenylester
α-Butyloxypropionsäure-4-(5-pentylpyrimidin-(2)-yl)-phenylester
α-Butyloxypropionsäure-4-(5-hexylpyrimidin-(2)-yl)-phenylester
α-Butyloxypropionsäure-4-(5-heptylpyrimidin-(2)-yl)-phenylester
α-Butyloxypropionsäure-4-(5-octylpyrimidin-(2)-yl)-phenylester
α-Butyloxypropionsäure-4-(5-nonylpyrimidin-(2)-yl)-phenylester

α-Butyloxypropionsäure-4-(5-propyloxypyrimidin-(2)-yl)-phenylester
α-Butyloxypropionsäure-4-(5-butyloxypyrimidin-(2)-yl)-phenylester
α-Butyloxypropionsäure-4-(5-pentyloxypyrimidin-(2)-yl)-phenylester
α-Butyloxypropionsäure-4-(5-hexyloxypyrimidin-(2)-yl)-phenylester
α-Butyloxypropionsäure-4-(5-heptyloxypyrimidin-(2)-yl)-phenylester
α-Butyloxypropionsäure-4-(5-octyloxypyrimidin-(2)-yl)-phenylester
α-Butyloxypropionsäure-4-(5-nonyloxypyrimidin-(2)-yl)-phenylester
α-Propyloxypropionsäure-4-(5-propylpyrimidin-(2)-yl)-phenylester
α-Propyloxypropionsäure-4-(5-butylpyrimidin-(2)-yl)-phenylester
α-Propyloxypropionsäure-4-(5-pentylpyrimidin-(2)-yl)-phenylester
α-Propuloxypropionsäure-4-(5-hexylpyrimidin-(2)-yl)-phenylester
α-Propyloxypropionsäure-4-(5-heptylpyrimidin-(2)-yl)-phenylester
α-Propyloxypropionsäure-4-(5-octylpyrimidin-(2)-yl)-phenylester
α-Propyloxypropionsäure-4-(5-nonylpyrimidin-(2)-yl)-phenylester
α-Propyloxypropionsäure-4-(5-propyloxypyrimidin-(2)-yl)-phenylester
α-Propyloxypropionsäure-4-(5-butyloxypyrimidin-(2)-yl)-phenylester
α-Propyloxypropionsäure-4-(5-pentyloxypyrimidin-(2)-yl)-phenylester
α-Propyloxypropionsäure-4-(5-hexyloxypyrimidin-(2)-yl)-phenylester
α-Propyloxypropionsäure-4-(5-heptyloxypyrimidin-(2)-yl)-phenylester
α-Propyloxypropionsäure-4-(5-octyloxypyrimidin-(2)-yl)-phenylester
α-Propyloxypropionsäure-4-(5-nonyloxypyrimidin-(2)-yl)-phenylester
α-Ethyloxypropionsäure-4-(5-propylpyrimidin-(2)-yl)-phenylester
α-Ethyloxypropionsäure-4-(5-butylpyrimidin-(2)-yl)-phenylester
α-Ethyloxypropiönsäure-4-(5-pentylpyrimidin-(2)-yl)-phenylester
α-Ethyloxypropionsäure-4-(5-hexylpyrimidin-(2)-yl)-phenylester
α-Ethyloxypropionsäure-4-(5-heptylpyrimidin-(2)-yl)-phenylester
α-Ethyloxypropionsäure-4-(5-octylpyrimidin-(2)-yl)-phenylester
α-Ethyloxypropionsäure-4-(5-nonylpyrimidin-(2)-yl)-phenylester
α-Ethyloxypropionsäure-4-(5-propyloxypyrimidin-(2)-yl)-phenylester
α-Ethyloxypropionsäure-4-(5-butyloxypyrimidin-(2)-yl)-phenylester
α-Ethyloxypropionsäure-4-(5-pentyloxypyrimidin-(2)-yl)-phenylester
α-Ethyloxypropionsäure-4-(5-hexyloxypyrimidin-(2)-yl)-phenylester
α-Ethyloxypropionsäure-4-(5-heptyloxypyrimidin-(2)-yl)-phenylester
α-Ethyloxypropionsäure-4-(5-octyloxypyrimidin-(2)-yl)-phenylester
α-Ethyloxypropionsäure-4-(5-nonyloxypyrimidin-(2)-yl)- phenylester
α-Methyloxypropionsäure-4-(5-propylpyrimidin-(2)-yl)-phenylester
α-Methyloxypropionsäure-4-(5-butylpyrimidin-(2)-yl)-phenylester
α-Methyloxypropionsäure-4-(5-pentylpyrimidin-(2)-yl)-phenylester
α-Methyloxypropionsäure-4-(5-hexylpyrimidin-(2)-yl)-phenylester
α-Methyloxypropionsäure-4-(5-heptylpyrimidin-(2)-yl)-phenylester
α-Methyloxypropionsäure-4-(5-octylpyrimidin-(2)-yl)-phenylester
α-Methyloxyropionsäure-4-(5-nonylpyrimidin-(2)-yl)-phenylester
α-Methyloxypropionsäure-4-(5-propyloxypyrimidin-(2)-yl)-phenylester
α-Methyloxypropionsäure-4-(5-butyloxypyrimidin-(2)-yl)-phenylester
α-Methyloxypropionsäure-4-(5-pentyloxypyrimidin-(2)-yl)-phenylester
α-Methyloxypropionsäure-4-(5-hexyloxypyrimidin-(2)-yl)-phenylester
α-Methyloxypropionsäure-4-(5-heptyloxypyrimidin-(2)-yl)-phenylester
α-Methyloxypropionsäure-4-(5-octyloxypyrimidin-(2)-yl)-phenylester
α-Methyloxypropionsäure-4-(5-nonyloxypyrimidin-(2)-yl)-phenylester.

Beispiel 15

0,01 Mol 1-{[4-(5-Heptylpyrimidin-(2)-yl)-phenyl-(1)]-ethylenyl-(2)}-4-octyl-(2)-benzoat ($\alpha_D^{20}$: + 39,0°, c = 2 in $CH_2Cl_2$) wird in 50 ml Tetrahydrofuran mit 2 g 5 % Pd auf Kohle bis zur berechneten Wasserstoffaufnahme bei Zimmertemperatur unter Normaldruck hydriert. Man erhält nach Filtration, Eindampfen und Umkristallisation 1-{[4-(5-Heptylpyrimidin-(2)-yl)-phenyl-(1)]-ethyl-(2)}-4-octyl-(2)-benzoat, Fp. 44,5°, $\alpha_D^{20}$ = 18,9°, $S_A$/I 71°.

Analog werden hergestellt:

1-{[4-(5-Propylpyrimidin-(2)-yl)-phenyl-(1)]-ethyl-(2)}-4-octyl-(2)}-benzoat
1-{[4-(5-Butylpyrimidin-(2)-yl)-phenyl-(1)]-ethyl-(2)}-4-octyl-(2)}-benzoat
1-{[4-(5-Pentylpyrimidin-(2)-yl)-phenyl-(1)]-ethyl-(2)}-4-octyl-(2)}-benzoat
1-{[4-(5-Hexylpyrimidin-(2)-yl)-phenyl-(1)]-ethyl-(2)}-4-octyl-(2)}-benzoat
1-{[4-(5-Octylpyrimidin-(2)-yl)-phenyl-(1)]-ethyl-(2)}-4-octyl-(2)}-benzoat
1-{[4-(5-Nonylpyrimidin-(2)-yl)-phenyl-(1)]-ethyl-(2)}-4-octyl-(2)}-benzoat
1-{[4-(5-Propyloxypyrimidin-(2)-yl)-phenyl-(1)]-ethyl-(2)}-4-octyl-(2)}-benzoat
1-{[4-(5-Butyloxypyrimidin-(2)-yl)-phenyl-(1)]-ethyl-(2)}-4-octyl-(2)}-benzoat
1-{[4-(5-Pentyloxypyrimidin-(2)-yl)-phenyl-(1)]-ethyl-(2)}-4-octyl-(2)}-benzoat
1-{[4-(5-Hexyloxypyrimidin-(2)-yl)-phenyl-(1)]-ethyl-(2)}-4-octyl-(2)}-benzoat
1-{[4-(5-Heptyloxypyrimidin-(2)-yl)-phenyl-(1)]-ethyl-(2)}-4-octyl-(2)}-benzoat
1-{[4-(5-Octyloxypyrimidin-(2)-yl)-phenyl-(1)]-ethyl-(2)}-4-octyl-(2)}-benzoat
1-{[4-(5-Nonyloxypyrimidin-(2)-yl)-phenyl-(1)]-ethyl-(2)}-4-octyl-(2)}-benzoat

Beispiel 16

0,01 Mol linksdrehende 3-[4-(5-Heptylpyrimidin-(2)-yl)-phenyl-(1)]-2-methylpropionsäure ($\alpha_D^{20}$ = - 2,3°), erhalten durch Racematspaltung mit (+)-Ephedrin und Synthese der racemischen Säure durch Hydrieren der entsprechenden α-Methylzimtsäure über 5 % Pd/Kohle in Tetrahydrofuran, wird mit 0,01 Mol 1-Hexanol analog Beispiel 12 verestert. Man erhält nach Aufarbeitung und Reinigung den 3-[4-(5-Heptylpyrimidin-(2)-yl)-phenyl-(1)]-2-methylpropionsäure-n-hexylester.

Analog werden hergestellt:
3-[4-(5-Propylpyrimidin-(2)-yl)-phenyl-(1)]-2-methylpropionsäure-hexylester
3-[4-(5-Butylpyrimidin-(2)-yl)-phenyl-(1)]-2-methylpropionsäure-hexylester
3-[4-(5-Pentylpyrimidin-(2)-yl)-phenyl-(1)]-2-methylpropionsäure-hexylester
3-[4-(5-Hexylpyrimidin-(2)-yl)-phenyl-(1)]-2-methylpropionsäure-hexylester
3-[4-(5-Octylpyrimidin-(2)-yl)-phenyl-(1)]-2-methylpropionsäure-hexylester
3-[4-(5-Nonylpyrimidin-(2)-yl)-phenyl-(1)]-2-methylpropionsäure-hexylester
3-[4-(5-Propyloxypyrimidin-(2)-yl)-phenyl-(1)]-2-methylpropionsäure-hexylester
3-[4-(5-Butyloxypyrimidin-(2)-yl)-phenyl-(1)]-2-methylpropionsäure-hexylester
3-[4-(5-Pentyloxypyrimidin-(2)-yl)-phenyl-(1)]-2-methylpropionsäure-hexylester
3-[4-(5-Hexyloxypyrimidin-(2)-yl)-phenyl-(1)]-2-methylpropionsäure-hexylester
3-[4-(5-Heptyloxypyrimidin-(2)-yl)-phenyl-(1)]-2-methylpropionsäure-hexylester
3-[4-(5-Octyloxypyrimidin-(2)-yl)-phenyl-(1)]-2-methylpropionsäure-hexylester
3-[4-(5-Nonyloxypyrimidin-(2)-yl)-phenyl-(1)]-2-methylpropionsäure-hexylester.
3-[4-(5-Nonylpyrimidin-(2)-phenyl-(1)-2-methylpropionsäure-2-methylbutylester
3-[4-(5-Propyloxypyrimidin-(2)-phenyl-(1)-2-methylpropionsäure-2-methylbutylester
3-[4-(5-Butyloxypyrimidin-(2)-phenyl-(1)-2-methylpropionsäure-2-methylbutylester
3-[4-(5-Pentyloxypyrimidin-(2)-phenyl-(1)-2-methylpropionsäure-2-methylbutylester
3-[4-(5-Hexyloxypyrimidin-(2)-phenyl-(1)-2-methylpropionsäure-2-methylbutylester
3-[4-(5-Heptyloxypyrimidin-(2)-phenyl-(1)-2-methylpropionsäure-2-methylbutylester
3-[4-(5-Octyloxypyrimidin-(2)-phenyl-(1)-2-methylpropionsäure-2-methylbutylester
3-[4-(5-Nonyloxypyrimidin-(2)-phenyl-(1)-2-methylpropionsäure-2-methylbutylester

Beispiel 17

0,01 Mol R-p-(5-Heptylpyrimidin-2-yl)-zimtsäure-2-methylbutylester (vgl. Beispiel 8) werden in 50 ml Tetrahydrofuran mit 2 g 5 % Pd auf Kohle bis zur berechneten Wasserstoffaufnahme bei Zimmertemperatur unter Normaldruck hydriert. Darauf wird filtriert, eingedampft, umkristallisiert und man erhält R-p-(5-Heptylpyrimidin-2-yl)phenylpropionsäure-2-methylbutylester, F. 16°.

Analog werden hergestellt:
R-p-(5-Propylpyrimidin-2-yl)phenylpropionsäure-2-methylbutylester
R-p-(5-Butylpyrimidin-2-yl)phenylpropionsäure-2-methylbutylester
R-p-(5-Pentylpyrimidin-2-yl)phenylpropionsäure-2-methylbutylester
R-p-(5-Hexylpyrimidin-2-yl)phenylpropionsäure-2-methylbutylester
R-p-(5-Octylpyrimidin-2-yl)phenylpropionsäure-2-methylbutylester
R-p-(5-Decylpyrimidin-2-yl)phenylpropionsäure-2-methylbutylester

R-p-(5-Propylpyrimidin-2-yl)phenylpropionsäure-2-octylester
R-p-(5-Butylpyrimidin-2-yl)phenylpropionsäure-2-octylester
R-p-(5-Pentylpyrimidin-2-yl)phenylpropionsäure-2-octylester
R-p-(5-Hexylpyrimidin-2-yl)phenylpropionsäure-2-octylester
R-p-(5-Heptylpyrimidin-2-yl)phenylpropionsäure-2-octylester
R-p-(5-Octylpyrimidin-2-yl)phenylpropionsäure-2-octylester
R-p-(5-Decylpyrimidin-2-yl)phenylpropionsäure-2-octylester

Beispiel 18

7,9 ml Azodicarbonsäurediethylester werden bei Raumtemperatur zu einer Lösung von 0,05 Mol 2-(4-Hydroxyphenyl)-5-nonylpyrimidin, 5,8 ml (s)-Milchsäureethylester und 3,1 g Triphenylphosphin in 150 ml Tetrahydrofuran gegeben. Das Gemisch wird über Nacht gerührt und eingedampft. Der Rückstand wird mit 30 ml Methanol (90 %) und 50 ml Methylenchlorid aufgenommen, und 5 ml $H_2O_2$ werden zugegeben. Nach 15 Minuten werden wasserhaltiges $Na_2S_2O_5$ und nachfolgend 100 ml Wasser zugegeben. Die organische Phase wird gewaschen und getrocknet.
Nach Eindampfen und Chromatographieren an Kieselgel erhält man optisch aktives Ethyl-2-[p-(5-nonylpyrimidin-2-yl)phenoxy]-propanoat.

Analog werden hergestellt:
Ethyl-2-[p-(5-propylpyrimidin-2-yl)phenoxy]-propanoat
Ethyl-2-[p-(5-butylpyrimidin-2-yl)phenoxy]-propanoat
Ethyl-2-[p-(5-pentylpyrimidin-2-yl)phenoxy]-propanoat
Ethyl-2-[p-(5-hexylpyrimidin-2-yl)phenoxy]-propanoat
Ethyl-2-[p-(5-heptylpyrimidin-2-yl)phenoxy]-propanoat
Ethyl-2-[p-(5-octylpyrimidin-2-yl)phenoxy]-propanoat
Ethyl-2-[p-(5-propyloxypyrimidin-2-yl)phenoxy]-propanoat
Ethyl-2-[p-(5-butyloxypyrimidin-2-yl)phenoxy]-propanoat
Ethyl-2-[p-(5-pentyloxypyrimidin-2-yl)phenoxy]-propanoat
Ethyl-2-[p-(5-hexyloxypyrimidin-2-yl)phenoxy]-propanoat
Ethyl-2-[p-(5-heptyloxypyrimidin-2-yl)phenoxy]-propanoat
Ethyl-2-[p-(5-octyloxypyrimidin-2-yl)phenoxy]-propanoat
Ethyl-2-[p-(5-nonyloxypyrimidin-2-yl)phenoxy]-propanoat

Beispiel 19

Veretherung von 2-(4-Hydroxyphenyl)-5-nonylpyrimidin mit 2-Octylbromid in Dimethylformamid in Gegenwart von Kaliumcarbonat liefert 2-[4-(2-Octyloxy)phenyl]-5-nonylpyrimidin.

Analog werden hergestellt:
2-[4-(2-Octyloxy)phenyl]-5-propylpyrimidin
2-[4-(2-Octyloxy)phenyl]-5-butylpyrimidin
2-[4-(2-Octyloxy)phenyl]-5-pentylpyrimidin
2-[4-(2-Octyloxy)phenyl]-5-hexylpyrimidin
2-[4-(2-Octyloxy)phenyl]-5-heptylpyrimidin
2-[4-(2-Octyloxy)phenyl]-5-octylpyrimidin
2-[4-(2-Octyloxy)phenyl]-5-propyloxypyrimidin
2-[4-(2-Octyloxy)phenyl]-5-butyloxypyrimidin
2-[4-(2-Octyloxy)phenyl]-5-pentyloxypyrimidin
2-[4-(2-Octyloxy)phenyl]-5-hexyloxypyrimidin
2-[4-(2-Octyloxy)phenyl]-5-heptyloxypyrimidin
2-[4-(2-Octyloxy)phenyl]-5-octyloxypyrimidin
2-[4-(2-Octyloxy)phenyl]-5-nonyloxypyrimidin
2-[4-(2-Methylbutyloxy)phenyl]-5-propylpyrimidin
2-[4-(2-Methylbutyloxy)phenyl]-5-butylpyrimidin
2-[4-(2-Methylbutyloxy)phenyl]-5-pentylpyrimidin
2-[4-(2-Methylbutyloxy)phenyl]-5-hexylpyrimidin
2-[4-(2-Methylbutyloxy)phenyl]-5-heptylpyrimidin
2-[4-(2-Methylbutyloxy)phenyl]-5-octylpyrimidin
2-[4-(2-Methylbutyloxy)phenyl]-5-nonylpyrimidin

2-[4-(2-Methylbutyloxy)phenyl]-5-propyloxypyrimidin
2-[4-(2-Methylbutyloxy)phenyl]-5-butyloxypyrimidin
2-[4-(2-Methylbutyloxy)phenyl]-5-pentyloxypyrimidin
2-[4-(2-Methylbutyloxy)phenyl]-5-hexyloxypyrimidin
2-[4-(2-Methylbutyloxy)phenyl]-5-heptyloxypyrimidin
2-[4-(2-Methylbutyloxy)phenyl]-5-octyloxypyrimidin
2-[4-(2-Methylbutyloxy)phenyl]-5-nonyloxypyrimidin

Beispiel 20

Eine Mischung von 11,9 g 2-p-Hydroxyphenyl-5-n-nonylpyrimidin, 10,2 g p-(2-Methylbutoxy)-benzylbromid (herstellbar aus 4-Hydroxybenzaldehyd und 2-Methylbutylmesylat in Gegenwart von Kaliumcarbonat in DMF, anschließend Reduktion des Benzaldehyds zum Benzylalkohol und Umsetzung mit $PBr_3$ zum entsprechenden Benzylbromid), 8,6 g Kaliumcarbonat und 50 ml Dimethylformamid wird 10 Stunden auf 90° erwärmt. Übliche Aufarbeitung liefert optisch aktives 4-(5-n-Nonylpyrimidin-2-yl)-phenyl-p-(2-methylbutoxy)benzylether, S-Ch 103°, Ch-I 105°.

Analog werden hergestellt:
4-(5-Hexylpyrimidin-2-yl)-phenyl-p-(2-methylbutoxy)-benxylether
4-(5-Heptylpyrimidin-2-yl)-phenyl-p-(2-methylbutoxy)-benzylether
4-(5-Octylpyrimidin-2-yl)-phenyl-p-(2-methylbutoxy)-benzylether
4-(5-Decylpyrimidin-2-yl)-phenyl-p-(2-methylbutoxy)-benzylether
4-(5-Pentylpyrimidin-2-yl)-phenyl-p-(2-methylbutoxy)-benzylether
4-(5-Butylpyrimidin-2-yl)-phenyl-p-(2-methylbutoxy)-benzylether
4-(5-Propylpyrimidin-2-yl)-phenyl-p-(2-methylbutoxy)-benzylether.

Beispiel 21

Analog Beispiel 20 erhält man aus 2-p-Hydroxyphenyl-5-n-nonylpyrimidin und p-Brommethylbenzoesäure-2-methylbutylester (darstellbar durch azeotrope Veresterung der p-Brommethylbenzoesäure mit 2-Methyl-1-butanol) 4-(5-n-Nonylpyrimidin-2-yl)-phenyl-p-(2-methylbutoxy-carbonyl)-benzylether, F. 29°.

Analog wurden hergestellt:
4-(5-Hexylpyrimidin-2-yl)-phenyl-p-(2-methylbutoxy-carbonyl)-benzylether
4-(5-Heptylpyrimidin-2-yl)-phenyl-p-(2-methylbutoxy-carbonyl)-benzylether
4-(5-Octylpyrimidin-2-yl)-phenyl-p-(2-methylbutoxy-carbonyl)-benzylether
4-(5-Decylpyrimidin-2-yl)-phenyl-p-(2-methylbutoxy-carbonyl)-benzylether
4-(5-Pentylpyrimidin-2-yl)-phenyl-p-(2-methylbutoxy-carbonyl)-benzylether
4-(5-Butylpyrimidin-2-yl)-phenyl-p-(2-methylbutoxy-carbonyl)-benzylether
4-(5-Propylpyrimidin-2-yl)-phenyl-p-(2-methylbutoxy-carbonyl)-benzylether
4-(5-Propylpyrimidin-2-yl)-phenyl-p-(2-octyloxy-carbonyl)-benzylether
4-(5-Butylpyrimidin-2-yl)-phenyl-p-(2-octyloxy-carbonyl)-benzylether
4-(5-Pentylpyrimidin-2-yl)-phenyl-p-(2-octyloxy-carbonyl)-benzylether
4-(5-Hexylpyrimidin-2-yl)-phenyl-p-(2-octyloxy-carbonyl)-benzylether
4-(5-Heptylpyrimidin-2-yl)-phenyl-p-(2-octyloxy-carbonyl)-benzylether
4-(5-Octylpyrimidin-2-yl)-phenyl-p-(2-octyloxy-carbonyl)-benzylether
4-(5-Nonylpyrimidin-2-yl)-phenyl-p-(2-octyloxy-carbonyl)-benzylether
4-(5-Decylpyrimidin-2-yl)-phenyl-p-(2-octyloxy-carbonyl)-benzylether.

Beispiel 22

0,04 Mol 5-Heptyl-2-(4-styryl)-pyrimidin, hergestellt nach an sich bekannten Verfahren, und 0,04 Mol 4-Brombenzoesäure-2-octylester, hergestellt aus 4-Brombenzoesäure und d-2-Octanol, werden mit 0,08 Mol Triethylamin, 0,1 g Palladium-II-acetat und 0,24 g o-Tolyl-phosphin in 50 ml ml Acetonitril 24 Stunden am Rückfluß erwärmt. Nach Eindampfen, Chromatographieren an Kieselgel und Umkristallisation erhält man 1-{[4-(5-Heptylpyrimidin-2-yl)phenyl]-ethylenyl-(2)}-4-octyl-(2)-benzoat, $\alpha_D^{20}$ = + 39,0°.

Analog werden hergestellt:
1-{[4-(5-Propylpyrimidin-2-yl)phenyl]-ethylenyl-(2)}-4-octyl-(2)-benzoat
1-{[4-(5-Butylpyrimidin-2-yl)phenyl]-ethylenyl-(2)}-4-octyl-(2)-benzoat

1-{[4-(5-Pentylpyrimidin-2-yl)phenyl]-ethylenyl-(2)}-4-octyl-(2)-benzoat
1-{[4-(5-Hexylpyrimidin-2-yl)phenyl]-ethylenyl-(2)}-4-octyl-(2)-benzoat
1-{[4-(5-Heptylpyrimidin-2-yl)phenyl]-ethylenyl-(2)}-4-octyl-(2)-benzoat
1-{[4-(5-Octylpyrimidin-2-yl)phenyl]-ethylenyl-(2)}-4-octyl-(2)-benzoat
1-{[4-(5-Propyloxypyrimidin-2-yl)phenyl]-ethylenyl-(2)}-4-octyl-(2)-benzoat
1-{[4-(5-Butyloxypyrimidin-2-yl)phenyl]-ethylenyl-(2)}-4-octyl-(2)-benzoat
1-{[4-(5-Pentyloxypyrimidin-2-yl)phenyl]-ethylenyl-(2)}-4-octyl-(2)-benzoat
1-{[4-(5-Hexyloxypyrimidin-2-yl)phenyl]-ethylenyl-(2)}-4-octyl-(2)-benzoat
1-{[4-(5-Heptyloxypyrimidin-2-yl)phenyl]-ethylenyl-(2)}-4-octyl-(2)-benzoat
1-{[4-(5-Octyloxypyrimidin-2-yl)phenyl]-ethylenyl-(2)}-4-octyl-(2)-benzoat
1-{[4-(5-Nonyloxypyrimidin-2-yl)phenyl]-ethylenyl-(2)}-4-octyl-(2)-benzoat

Beispiel 23

Ein Gemisch von 0,7 g optisch aktiven 5-[p-(2-Methylbutoxy)-phenyl]-pyrazin-2-ol, 0,6 g p-Octylbenzoesäure-chlorid und 5 ml Pyridin wird über Nacht gerührt. Nach üblicher Aufarbeitung erhält man optisch aktives 2-p-Octylbenzoyloxy-5-(2-methylbutoxy)-phenylpyrazin,F. 53°, K. 134°.

Analog werden hergestellt:

2-p-Propylbenzoyloxy-5-(2-methylbutoxy)-phenylpyrazin
2-p-Butylbenzoyloxy-5-(2-methylbutoxy)-phenylpyrazin, F. 78°, K. 142°
2-p-Pentylbenzoyloxy-5-(2-methylbutoxy)-phenylpyrazin, F. 66°, K. 148°
2-p-Hexylbenzoyloxy-5-(2-methylbutoxy)-phenylpyrazin
2-p-Heptylbenzoyloxy-5-(2-methylbutoxy)-phenylpyrazin, F. 63°, K. 140°
2-p-Nonylbenzoyloxy-5-(2-methylbutoxy)-phenylpyrazin
2-p-Decylbenzoyloxy-5-(2-methylbutoxy)-phenylpyrazin, F. 68°, K. 130°
2-p-Undecylbenzoyloxy-5-(2-methylbutoxy)-phenylpyrazin
2-p-Dodecylbenzoyloxy-5-(2-methylbutoxy)-phenylpyrazin
2-p-Propoxybenzoyloxy-5-(2-methylbutoxy)-phenylpyrazin
2-p-Butoxybenzoyloxy-5-(2-methylbutoxy)-phenylpyrazin
2-p-Pentoxybenzoyloxy-5-(2-methylbutoxy)-phenylpyrazin
2-p-Hexoxybenzoyloxy-5-(2-methylbutoxy)-phenylpyrazin
2-p-Heptoxybenzoyloxy-5-(2-methylbutoxy)-phenylpyrazin
2-p-Octoxybenzoyloxy-5-(2-methylbutoxy)-phenylpyrazin, F. 116°, K. 162°
2-p-Nonoxybenzoyloxy-5-(2-methylbutoxy)-phenylpyrazin
2-p-Decoxybenzoyloxy-5-(2-methylbutoxy)-phenylpyrazin
2-p-Undecoxybenzoyloxy-5-(2-methylbutoxy)-phenylpyrazin, F. 104°, K. 151°
2-p-Dodecoxybenzoyloxy-5-(2-methylbutoxy)-phenylpyrazin
2-p-Propionyloxybenzoyloxy-5-(2-methylbutoxy)-phenylpyrazin
2-p-Butyryloxybenzoyloxy-5-(2-methylbutoxy)-phenylpyrazin
2-p-Hexanoyloxybenzoyloxy-5-(2-methylbutoxy)-phenylpyrazin
2-p-Octanoyloxybenzoyloxy-5-(2-methylbutoxy)-phenylpyrazin
2-p-Nonanoyloxybenzoyloxy-5-(2-methylbutoxy)-phenylpyrazin
2-p-Decanoyloxybenzoyloxy-5-(2-methylbutoxy)-phenylpyrazin
2-p-Butoxycarbonyloxybenzoyloxy-5-(2-methylbutoxy)-phenylpyrazin
2-p-Pentoxycarbonyloxybenzoyloxy-5-(2-methylbutoxy)-phenylpyrazin
2-p-Hexoxycarbonyloxybenzoyloxy-5-(2-methylbutoxy)-phenylpyrazin
2-p-Octoxycarbonyloxybenzoyloxy-5-(2-methylbutoxy)-phenylpyrazin
2-p-Nonoxycarbonyloxybenzoyloxy-5-(2-methylbutoxy)-phenylpyrazin, F. 84°, K. 148°
2-p-Decoxycarbonyloxybenzoyloxy-5-(2-methylbutoxy)-phenylpyrazin
2-p-Propoxy-5-(2-methylbutoxy)-phenylpyrazin
2-p-Butoxy-5-(2-methylbutoxy)-phenylpyrazin
2-p-Pentoxy-5-(2-methylbutoxy)-phenylpyrazin
2-p-Hexoxy-5-(2-methylbutoxy)-phenylpyrazin
2-p-Heptoxy-5-(2-methylbutoxy)-phenylpyrazin
2-p-Nonoxy-5-(2-methylbutoxy)-phenylpyrazin
2-p-Decoxy-5-(2-methylbutoxy)-phenylpyrazin
2-p-Propionyloxy-5-(2-methylbutoxy)-phenylpyrazin
2-p-Butyryloxy-5-(2-methylbutoxy)-phenylpyrazin

2-p-Pentanoyloxy-5-(2-methylbutoxy)-phenylpyrazin
2-p-Hexanoyloxy-5-(2-methylbutoxy)-phenylpyrazin
2-p-Heptanoyloxy-5-(2-methylbutoxy)-phenylpyrazin
2-p-Octanoyloxy-5-(2-methylbutoxy)-phenylpyrazin
2-p-Nonanoyloxy-5-(2-methylbutoxy)-phenylpyrazin
2-p-Decanoyloxy-5-(2-methylbutoxy)-phenylpyrazin
2-p-Butoxycarbonyloxy-5-(2-methylbutoxy)-phenylpyrazin
2-p-Pentoxycarbonyloxy-5-(2-methylbutoxy)-phenylpyrazin
2-p-Hexoxycarbonyloxy-5-(2-methylbutoxy)-phenylpyrazin
2-p-Heptoxycarbonyloxy-5-(2-methylbutoxy)-phenylpyrazin
2-p-Octoxycarbonyloxy-5-(2-methylbutoxy)-phenylpyrazin, F. 42°
2-p-Nonoxycarbonyloxy-5-(2-methylbutoxy)-phenylpyrazin
2-p-Decoxycarbonyloxy-5-(2-methylbutoxy)-phenylpyrazin

Beispiel 24

Ein Gemisch von 6,3 g S-2-Methylbutanol und 50 ml Benzol wird mit 0,65 g Natrium versetzt. Zu der erhaltenen Alkoholatlösung werden 8,1 g 3-Chlor-6-(p-nonylphenyl)-pyridazin gegeben. Nach vier Stunden Kochen am Rückfluß wird wie üblich aufgearbeitet. Man erhält optisch aktives 3-(2-Methylbutoxy)-6-(p-nonylphenyl)-pyridazin, F. 78°.

Analog werden hergestellt:
3-(2-Methylbutoxy)-6-(p-butylphenyl)-pyridazin
3-(2-Methylbutoxy)-6-(p-pentylphenyl)-pyridazin, F. 94°
3-(2-Methylbutoxy)-6-(p-hexylphenyl)-pyridazin
3-(2-Methylbutoxy)-6-(p-heptylphenyl)-pyridazin
3-(2-Methylbutoxy)-6-(p-octylphenyl)-pyridazin
3-(2-Methylbutoxy)-6-(p-decylphenyl)-pyridazin
3-(2-Methylbutoxy)-6-(p-undecylphenyl)-pyridazin
3-(2-Methylbutoxy)-6-(p-dodecylphenyl)-pyridazin, F. 78°
3-(2-Methylbutoxy)-6-(p-butoxyphenyl)-pyridazin
3-(2-Methylbutoxy)-6-(p-pentoxyphenyl)-pyridazin
3-(2-Methylbutoxy)-6-(p-hexoxyphenyl)-pyridazin
3-(2-Methylbutoxy)-6-(p-heptoxyphenyl)-pyridazin
3-(2-Methylbutoxy)-6-(p-octoxyphenyl)-pyridazin, F. 91°
3-(2-Methylbutoxy)-6-(p-nonoxyphenyl)-pyridazin
3-(2-Methylbutoxy)-6-(p-decoxyphenyl)-pyridazin, F. 87°
3-(2-Methylbutoxy)-6-(p-undecoxyphenyl)-pyridazin
3-(2-Methylbutoxy)-6-(p-dodecoxyhenyl)-pyridazin
3-(2-Octyl)-6-(p-butylphenyl)-pyridazin
3-(2-Octyl)-6-(p-pentylphenyl)-pyridazin, F. 55°
3-(2-Octyl)-6-(p-hexylphenyl)-pyridazin
3-(2-Octyl)-6-(p-heptylphenyl)-pyridazin
3-(2-Octyl)-6-(p-octylphenyl)-pyridazin
3-(2-Octyl)-6-(p-decylphenyl)-pyridazin
3-(2-Octyl)-6-(p-undecylphenyl)-pyridazin
3-(2-Octyl)-6-(p-dodecylphenyl)-pyridazin, F. 56°
3-(2-Octyl)-6-(p-butoxyphenyl)-pyridazin
3-(2-Octyl)-6-(p-pentoxyphenyl)-pyridazin
3-(2-Octyl)-6-(p-hexoxyphenyl)-pyridazin
3-(2-Octyl)-6-(p-heptoxyphenyl)-pyridazin
3-(2-Octyl)-6-(p-octoxyphenyl)-pyridazin, F. 85°
3-(2-Octyl)-6-(p-nonoxyphenyl)-pyridazin
3-(2-Octyl)-6-(p-decoxyphenyl)-pyridazin, F. 87°
3-(2-Octyl)-6-(p-undecoxyphenyl)-pyridazin
3-(2-Octyl)-6-(p-dodecoxyphenyl)-pyridazin
3-(p-2-Methylbutoxyphenyl)-6-butylpyridazin
3-(p-2-Methylbutoxyphenyl)-6-pentylpyridazin
3-(p-2-Methylbutoxyphenyl)-6-hexylpyridazin

3-(p-2-Methylbutoxyphenyl)-6-heptylpyridazin
3-(p-2-Methylbutoxyphenyl)-6-octylpyridazin
3-(p-2-Methylbutoxyphenyl)-6-decylpyridazin
3-(p-2-Methylbutoxyphenyl)-6-undecylpyridazin
3-(p-2-Methylbutoxyphenyl)-6-dodecylpyridazin
3-(p-2-Methylbutoxyphenyl)-6-butoxypyridazin
3-(p-2-Methylbutoxyphenyl)-6-pentoxypyridazin
3-(p-2-Methylbutoxyphenyl)-6-hexoxypyridazin
3-(p-2-Methylbutoxyphenyl)-6-heptoxypyridazin
3-(p-2-Methylbutoxyphenyl)-6-octoxypyridazin
3-(p-2-Methylbutoxyphenyl)-6-nonoxypyridazin
3-(p-2-Methylbutoxyphenyl)-6-decoxypyridazin
3-(p-2-Methylbutoxyphenyl)-6-undecoxypyridazin
3-(p-2-Methylbutoxyphenyl)-6-dodecoxypyridazin
3-(p-2-Octyloxyphenyl)-6-butylpyridazin
3-(p-2-Octyloxyphenyl)-6-pentylpyridazin
3-(p-2-Octyloxyphenyl)-6-hexylpyridazin
3-(p-2-Octyloxyphenyl)-6-heptylpyridazin
3-(p-2-Octyloxyphenyl)-6-octylpyridazin
3-(p-2-Octyloxyphenyl)-6-decylpyridazin
3-(p-2-Octyloxyphenyl)-6-undecylpyridazin
3-(p-2-Octyloxyphenyl)-6-dodecylpyridazin
3-(p-2-Octyloxyphenyl)-6-butoxypyridazin
3-(p-2-Octyloxyphenyl)-6-pentoxypyridazin
3-(p-2-Octyloxyphenyl)-6-hexoxypyridazin
3-(p-2-Octyloxyphenyl)-6-heptoxypyridazin
3-(p-2-Octyloxyphenyl)-6-octoxypyridazin
3-(p-2-Octyloxyphenyl)-6-nonoxypyridazin
3-(p-2-Octyloxyphenyl)-6-decoxypyridazin
3-(p-2-Octyloxyphenyl)-6-undecoxypyridazin
3-(p-2-Octyloxyphenyl)-6-dodecoxypyridazin

Beispiel 25

Man erhitzt 6,5 g Heptylmalondialdehydtetraethylacetal, 5,1 g optisch aktives 4-(2-Methylbutyl)-mercaptobenzamidin-hydrochlorid und 10 ml Dimethylformamid 12 Std. auf 150°. Anschließend wird das Reaktionsgemisch in Dichlormethan aufgenommen, mit Natriumhydrogencarbonatlösung und Wasser neutral gewaschen, getrocknet und das Lösungsmittel abdestilliert. Man erhält optisch aktives 2-[4-(2-Methylbutyl)-mercaptophenyl]-5-n-heptylpyrimidin.

Analog werden hergestellt:
2-[4-(2-Methylbutyl)-mercaptophenyl]-5-octylpyrimidin
2-[4-(2-Methylbutyl)-mercaptophenyl]-5-nonylpyrimidin
2-[4-(2-Methylbutyl)-mercaptophenyl]-5-decylpyrimidin
2-[4-(2-Methylbutyl)-mercaptophenyl]-5-undecylpyrimidin
2-[4-(2-Methylbutyl)-mercaptophenyl]-5-dodecylpyrimidin
2-[4-(2-Octyl)-mercaptophenyl]-5-heptylpyrimidin
2-[4-(2-Octyl)-mercaptophenyl]-5-octylpyrimidin
2-[4-(2-Octyl)-mercaptophenyl]-5-nonylpyrimidin
2-[4-(2-Octyl)-mercaptophenyl]-5-decylpyrimidin
2-[4-(2-Octyl)-mercaptophenyl]-5-undecylpyrimidin
2-[4-(2-Octyl)-mercaptophenyl]-5-dodecylpyrimidin
2-[4-Dihydrocitronellylmercaptophenyl]-5-heptylpyrimidin
2-[4-Dihydrocitronellylmercaptophenyl]-5-octylpyrimidin
2-[4-Dihydrocitronellylmercaptophenyl]-5-nonylpyrimidin
2-[4-Dihydrocitronellylmercaptophenyl]-5-decylpyrimidin
2-[4-Dihydrocitronellylmercaptophenyl]-5-undecylpyrimidin
2-[4-Dihydrocitronellylmercaptophenyl]-5-dodecylpyrimidin.

Beispiel 26

Zu einer Lösung von 0,01 m 3-Pentylpyridin (welches durch Kopplung von Pentylmagnesiumbromid und 3-Brompyridin erhältlich ist) in 30 ml Toluol tropft man bei -20° unter Stickstoff eine Lösung von 0,01 m optisch aktivem 4-(2-Methylbutoxy)-phenyllithium (dargestellt aus 4-Brom-(2-methylbutoxy)-benzol und Lithium)in 30 ml Toluol. Die Reaktionsmischung wird 4 h zum Sieden erhitzt und nach dem Abkühlen vorsichtig mit 10 ml Wasser hydrolysiert. Die organische Phase wird mit Wasser und gesättigter NaCl-Lösung gewaschen, mit Magnesiumsulfat getrocknet und eingedampft. Der Rückstand wird über eine Kieselgelsäule mit Diisopropylether als Laufmittel chromatographisch gereinigt. Man erhält optisch aktives 2-p-(2-Methylbutoxy)-phenyl-5-pentylpyridin.

Analog werden hergestellt:

2-p-(2-Methylbutoxyphenyl)-5-butylpyridin
2-p-(2-Methylbutoxyphenyl)-5-pentylpyridin
2-p-(2-Methylbutoxyphenyl)-5-hexylpyridin
2-p-(2-Methylbutoxyphenyl)-5-heptylpyridin
2-p-(2-Methylbutoxyphenyl)-5-octylpyridin
2-p-(2-Methylbutoxyphenyl)-5-nonylpyridin
2-p-(2-Methylbutoxyphenyl)-5-decylpyridin
2-p-(2-Methylbutoxyphenyl)-5-undecylpyridin
2-p-(2-Methylbutoxyphenyl)-5-dodecylpyridin
2-p-(2-Methylbutoxyphenyl)-5-heptoxymethylpyridin
2-p-(2-Methylbutoxyphenyl)-5-hexoxyethylpyridin
2-p-(2-Methylbutoxyphenyl)-5-pentoxypropylpyridin
2-p-(3-Methylpentoxyphenyl)-5-butylpyridin
2-p-(3-Methylpentoxyphenyl)-5-pentylpyridin
2-p-(3-Methylpentoxyphenyl)-5-hexylpyridin
2-p-(3-Methylpentoxyphenyl)-5-heptylpyridin
2-p-(3-Methylpentoxyphenyl)-5-octylpyridin
2-p-(3-Methylpentoxyphenyl)-5-nonylpyridin
2-p-(3-Methylpentoxyphenyl)-5-decylpyridin
2-p-(3-Methylpentoxyphenyl)-5-undecylpyridin
2-p-(3-Methylpentoxyphenyl)-5-dodecylpyridin
2-p-(3-Methylpentoxyphenyl)-5-heptoxymethylpyridin
2-p-(3-Methylpentoxyphenyl)-5-hexoxyethylpyridin
2-p-(3-Methylpentoxyphenyl)-5-pentoxypropylpyridin
2-p-(4-Methylhexoxyphenyl)-5-butylpyridin
2-p-(4-Methylhexoxyphenyl)-5-pentylpyridin
2-p-(4-Methylhexoxyphenyl)-5-hexylpyridin
2-p-(4-Methylhexoxyphenyl)-5-heptylpyridin
2-p-(4-Methylhexoxyphenyl)-5-octylpyridin
2-p-(4-Methylhexoxyphenyl)-5-nonylpyridin
2-p-(4-Methylhexoxyphenyl)-5-decylpyridin
2-p-(4-Methylhexoxyphenyl)-5-undecylpyridin
2-p-(4-Methylhexoxyphenyl)-5-dodecylpyridin
2-p-(4-Methylhexoxyphenyl)-5-heptoxymethylpyridin
2-p-(4-Methylhexoxyphenyl)-5-hexoxyethylpyridin
2-p-(4-Methylhexoxyphenyl)-5-pentoxypropylpyridin
2-p-(5-Methylheptoxyphenyl)-5-butylpyridin
2-p-(5-Methylheptoxyphenyl)-5-pentylpyridin
2-p-(5-Methylheptoxyphenyl)-5-hexylpyridin
2-p-(5-Methylheptoxyphenyl)-5-heptylpyridin
2-p-(5-Methylheptoxyphenyl)-5-octylpyridin
2-p-(5-Methylheptoxyphenyl)-5-nonylpyridin
2-p-(5-Methylheptoxyphenyl)-5-decylpyridin
2-p-(5-Methylheptoxyphenyl)-5-undecylpyridin
2-p-(5-Methylheptoxyphenyl)-5-dodecylpyridin
2-p-(5-Methylheptoxyphenyl)-5-heptoxymethylpyridin
2-p-(5-Methylheptoxyphenyl)-5-hexoxyethylpyridin

2-p-(5-Methylheptoxyphenyl)-5-pentoxypropylpyridin
2-p-(6-Methyloctoxyphenyl)-5-butylpyridin
2-p-(6-Methyloctoxyphenyl)-5-pentylpyridin
2-p-(6-Methyloctoxyphenyl)-5-hexylpyridin
2-p-(6-Methyloctoxyphenyl)-5-heptylpyridin
2-p-(6-Methyloctoxyphenyl)-5-octylpyridin
2-p-(6-Methyloctoxyphenyl)-5-nonylpyridin
2-p-(6-Methyloctoxyphenyl)-5-decylpyridin
2-p-(6-Methyloctoxyphenyl)-5-undecylpyridin
2-p-(6-Methyloctoxyphenyl)-5-dodecylpyridin
2-p-(6-Methyloctoxyphenyl)-5-heptoxymethylpyridin
2-p-(6-Methyloctoxyphenyl)-5-hexoxyethylpyridin
2-p-(6-Methyloctoxyphenyl)-5-pentoxypropylpyridin
2-p-(2-Methylbutyryloxyphenyl)-5-butylpyridin
2-p-(2-Methylbutyryloxyphenyl)-5-pentylpyridin
2-p-(2-Methylbutyryloxyphenyl)-5-hexylpyridin
2-p-(2-Methylbutyryloxyphenyl)-5-heptylpyridin
2-p-(2-Methylbutyryloxyphenyl)-5-octylpyridin
2-p-(2-Methylbutyryloxyphenyl)-5-nonylpyridin
2-p-(2-Methylbutyryloxyphenyl)-5-decylpyridin
2-p-(2-Methylbutyryloxyphenyl)-5-undecylpyridin
2-p-(2-Methylbutyryloxyphenyl)-5-dodecylpyridin
2-p-(2-Methylbutyryloxyphenyl)-5-heptoxymethylpyridin
2-p-(2-Methylbutyryloxyphenyl)-5-hexoxyethylpyridin
2-p-(2-Methylbutyryloxyphenyl)-5-pentoxypropylpyridin
2-p-(4-Methylhexanoyloxyphenyl)-5-butylpyridin
2-p-(4-Methylhexanoyloxyphenyl)-5-pentylpyridin
2-p-(4-Methylhexanoyloxyphenyl)-5-hexylpyridin
2-p-(4-Methylhexanoyloxyphenyl)-5-heptylpyridin
2-p-(4-Methylhexanoyloxyphenyl)-5-octylpyridin
2-p-(4-Methylhexanoyloxyphenyl)-5-nonylpyridin
2-p-(4-Methylhexanoyloxyphenyl)-5-decylpyridin
2-p-(4-Methylhexanoyloxyphenyl)-5-undecylpyridin
2-p-(4-Methylhexanoyloxyphenyl)-5-dodecylpyridin
2-p-(4-Methylhexanoyloxyphenyl)-5-heptoxymethylpyridin
2-p-(4-Methylhexanoyloxyphenyl)-5-hexoxyethylpyridin
2-p-(4-Methylhexanoyloxyphenyl)-5-pentoxypropylpyridin
2-p-(6-Methyloctanoyloxyphenyl)-5-butylpyridin
2-p-(6-Methyloctanoyloxyphenyl)-5-pentylpyridin
2-p-(6-Methyloctanoyloxyphenyl)-5-hexylpyridin
2-p-(6-Methyloctanoyloxyphenyl)-5-heptylpyridin
2-p-(6-Methyloctanoyloxyphenyl)-5-octylpyridin
2-p-(6-Methyloctanoyloxyphenyl)-5-nonylpyridin
2-p-(6-Methyloctanoyloxyphenyl)-5-decylpyridin
2-p-(6-Methyloctanoyloxyphenyl)-5-undecylpyridin
2-p-(6-Methyloctanoyloxyphenyl)-5-dodecylpyridin
2-p-(6-Methyloctanoyloxyphenyl)-5-heptoxymethylpyridin
2-p-(6-Methyloctanoyloxyphenyl)-5-hexoxyethylpyridin
2-p-(6-Methyloctanoyloxyphenyl)-5-pentoxypropylpyridin
2-p-(6-Methyloctanoylphenyl)-5-butylpyridin
2-p-(6-Methyloctanoylphenyl)-5-pentylpyridin
2-p-(6-Methyloctanoylphenyl)-5-hexylpyridin
2-p-(6-Methyloctanoylphenyl)-5-heptylpyridin
2-p-(6-Methyloctanoylphenyl)-5-octylpyridin
2-p-(6-Methyloctanoylphenyl)-5-nonylpyridin
2-p-(6-Methyloctanoylphenyl)-5-decylpyridin
2-p-(6-Methyloctanoylphenyl)-5-undecylpyridin
2-p-(6-Methyloctanoylphenyl)-5-dodecylpyridin

2-p-(6-Methyloctanoylphenyl)-5-heptoxymethylpyridin
2-p-(6-Methyloctanoylphenyl)-5-hexoxyethylpyridin
2-p-(6-Methyloctanoylphenyl)-5-pentoxypropylpyridin
2-p-(3-Chlorpentoxyphenyl)-5-butylpyridin
2-p-(3-Chlorpentoxyphenyl)-5-pentylpyridin
2-p-(3-Chlorpentoxyphenyl)-5-hexylpyridin
2-p-(3-Chlorpentoxyphenyl)-5-heptylpyridin
2-p-(3-Chlorpentoxyphenyl)-5-octylpyridin,
2-p-(3-Chlorpentoxyphenyl)-5-nonylpyridin
2-p-(3-Chlorpentoxyphenyl)-5-decylpyridin
2-p-(3-Chlorpentoxyphenyl)-5-undecylpyridin
2-p-(3-Chlorpentoxyphenyl)-5-dodecylpyridin
2-p-(3-Chlorpentoxyphenyl)-5-heptoxymethylpyridin
2-p-(3-Chlorpentoxyphenyl)-5-hexoxyethylpyridin
2-p-(3-Chlorpentoxyphenyl)-5-pentoxypropylpyridin
2-p-(3-Cyanpentoxyphenyl)-5-butylpyridin
2-p-(3-Cyanpentoxyphenyl)-5-pentylpyridin
2-p-(3-Cyanpentoxyphenyl)-5-hexylpyridin
2-p-(3-Cyanpentoxyphenyl)-5-heptylpyridin
2-p-(3-Cyanpentoxyphenyl)-5-octylpyridin
2-p-(3-Cyanpentoxyphenyl)-5-nonylpyridin
2-p-(3-Cyanpentoxyphenyl)-5-decylpyridin
2-p-(3-Cyanpentoxyphenyl)-5-undecylpyridin
2-p-(3-Cyanpentoxyphenyl)-5-dodecylpyridin
2-p-(3-Cyanpentoxyphenyl)-5-heptoxymethylpyridin
2-p-(3-Cyanpentoxyphenyl)-5-hexoxyethylpyridin
2-p-(3-Cyanpentoxyphenyl)-5-pentoxypropylpyridin
2-p-(1,6-Dioxa-8-methyldecylphenyl)-5-butylpyridin
2-p-(1,6-Dioxa-8-methyldecylphenyl)-5-pentylpyridin
2-p-(1,6-Dioxa-8-methyldecylphenyl)-5-hexylpyridin
2-p-(1,6-Dioxa-8-methyldecylphenyl)-5-heptylpyridin
2-p-(1,6-Dioxa-8-methyldecylphenyl)-5-octylpyridin
2-p-(1,6-Dioxa-8-methyldecylphenyl)-5-nonylpyridin
2-p-(1,6-Dioxa-8-methyldecylphenyl)-5-decylpyridin
2-p-(1,6-Dioxa-8-methyldecylphenyl)-5-undecylpyridin
2-p-(1,6-Dioxa-8-methyldecylphenyl)-5-dodecylpyridin
2-p-(1,6-Dioxa-8-methyldecylphenyl)-5-heptoxymethylpyridin
2-p-(1,6-Dioxa-8-methyldecylphenyl)-5-hexoxyethylpyridin
2-p-(1,6-Dioxa-8-methyldecylphenyl)-5-pentoxypropylpyridin
2-p-Octyloxyphenyl-5-(2-methyloctyl)-pyridin
2-p-Octyloxyphenyl-5-(3-methyloctyl)-pyridin
2-p-Octyloxyphenyl-5-(4-methyloctyl)-pyridin
2-p-Octyloxyphenyl-5-(5-methyloctyl)-pyridin
2-p-Octyloxyphenyl-5-(6-methyloctyl)-pyridin
2-p-Octylphenyl-5-(2-methyloctyl)-pyridin
2-p-Octylhenyl-5-(3-methyloctyl)-pyridin
2-p-Octylphenyl-5-(4-methyloctyl)-pyridin
2-p-Octylphenyl-5-(5-methyloctyl)-pyridin
2-p-Octylphenyl-5-(6-methyloctyl)-pyridin
2-p-(2-Methylbutoxy)-phenyl-5-butyloxypyridin
2-p-(2-Methylbutoxy)-phenyl-5-pentyloxypyridin
2-p-(2-Methylbutoxy)-phenyl-5-hexyloxypyridin
2-p-(2-Methylbutoxy)-phenyl-5-heptyloxypyridin
2-p-(2-Methylbutoxy)-phenyl-5-octyloxypyridin
2-p-(2-Methylbutoxy)-phenyl-5-nonyloxypyridin
2-p-(2-Methylbutoxy)-phenyl-5-decyloxypyridin
2-p-(2-Methylbutoxy)-phenyl-5-undecyloxypyridin
2-p-(2-Methylbutoxy)-phenyl-5-dodecyloxypyridin

2-p-(2-Methylbutoxy)-phenyl-5-heptoxymethyloxypyridin
2-p-(2-Methylbutoxy)-phenyl-5-hexoxyethyloxypyridin
2-p-(2-Methylbutoxy)-phenyl-5-pentoxypropyloxypyridin
2-p-(3-Methylpentoxy)-phenyl-5-butyloxypyridin
2-p-(3-Methylpentoxy)-phenyl-5-pentyloxypyridin
2-p-(3-Methylpentoxy)-phenyl-5-hexyloxypyridin
2-p-(3-Methylpentoxy)-phenyl-5-heptyloxypyridin
2-p-(3-Methylpentoxy)-phenyl-5-octyloxypyridin
2-p-(3-Methylpentoxy)-phenyl-5-nonyloxypyridin
2-p-(3-Methylpentoxy)-phenyl-5-decyloxypyridin
2-p-(3-Methylpentoxy)-phenyl-5-undecyloxypyridin
2-p-(3-Methylpentoxy)-phenyl-5-dodecyloxypyridin
2-p-(3-Methylpentoxy)-phenyl-5-heptoxymethyloxypyridin
2-p-(3-Methylpentoxy)-phenyl-5-hexoxyethyloxypyridin
2-p-(3-Methylpentoxy)-phenyl-5-pentoxypropyloxypyridin
2-p-(6-Methyloctoxy)-phenyl-5-butyloxypyridin
2-p-(6-Methyloctoxy)-phenyl-5-pentyloxypyridin
2-p-(6-Methyloctoxy)-phenyl-5-hexyloxypyridin
2-p-(6-Methyloctoxy)-phenyl-5-heptyloxypyridin
2-p-(6-Methyloctoxy)-phenyl-5-octyloxypyridin
2-p-(6-Methyloctoxy)-phenyl-5-nonyloxypyridin
2-p-(6-Methyloctoxy)-phenyl-5-decyloxypyridin
2-p-(6-Methyloctoxy)-phenyl-5-undecyloxypyridin
2-p-(6-Methyloctoxy)-phenyl-5-dodecyloxypyridin
2-p-(6-Methyloctoxy)-phenyl-5-heptoxymethyloxypyridin
2-p-(6-Methyloctoxy)-phenyl-5-hexoxyethyloxypyridin
2-p-(6-Methyloctoxy)-phenyl-5-pentoxypropyloxypyridin
2-p-(2-Methyloctoxy)-phenyl-5-butyloxypyridin
2-p-(2-Methyloctoxy)-phenyl-5-pentyloxypyridin
2-p-(2-Methyloctoxy)-phenyl-5-hexyloxypyridin
2-p-(2-Methyloctoxy)-phenyl-5-heptyloxypyridin
2-p-(2-Methyloctoxy)-phenyl-5-octyloxypyridin
2-p-(2-Methyloctoxy)-phenyl-5-nonyloxypyridin
2-p-(2-Methyloctoxy)-phenyl-5-decyloxypyridin
2-p-(2-Methyloctoxy)-phenyl-5-undecyloxypyridin
2-p-(2-Methyloctoxy)-phenyl-5-dodecyloxypyridin
2-p-(2-Methyloctoxy)-phenyl-5-heptoxymethyloxypyridin
2-p-(2-Methyloctoxy)-phenyl-5-hexoxyethyloxypyridin
2-p-(2-Methyloctoxy)-phenyl-5-pentoxypropyloxypyridin
2-p-(2-Methylbutyryloxy)-phenyl-5-butyloxypyridin
2-p-(2-Methylbutyryloxy)-phenyl-5-pentyloxypyridin
2-p-(2-Methylbutyryloxy)-phenyl-5-hexyloxypyridin
2-p-(2-Methylbutyryloxy)-phenyl-5-heptyloxypyridin
2-p-(2-Methylbutyryloxy)-phenyl-5-octyloxypyridin
2-p-(2-Methylbutyryloxy)-phenyl-5-nonyloxypyridin
2-p-(2-Methylbutyryloxy)-phenyl-5-decyloxypyridin
2-p-(2-Methylbutyryloxy)-phenyl-5-undecyloxypyridin
2-p-(2-Methylbutyryloxy)-phenyl-5-dodecyloxypyridin
2-p-(2-Methylbutyryloxy)-phenyl-5-heptoxymethyloxypyridin
2-p-(2-Methylbutyryloxy)-phenyl-5-hexoxyethyloxypyridin
2-p-(2-Methylbutyryloxy)-phenyl-5-pentoxypropyloxypyridin
2-p-(3-Methylpentanoyloxy)-phenyl-5-butyloxypyridin
2-p-(3-Methylpentanoyloxy)-phenyl-5-pentyloxypyridin
2-p-(3-Methylpentanoyloxy)-phenyl-5-hexyloxypyridin
2-p-(3-Methylpentanoyloxy)-phenyl-5-heptyloxypyridin
2-p-(3-Methylpentanoyloxy)-phenyl-5-octyloxypyridin
2-p-(3-Methylpentanoyloxy)-phenyl-5-nonyloxypyridin
2-p-(3-Methylpentanoyloxy)-phenyl-5-decyloxypyridin

2-p-(3-Methylpentanoyloxy)-phenyl-5-undecyloxypyridin
2-p-(3-Methylpentanoyloxy)-phenyl-5-dodecyloxypyridin
2-p-(3-Methylpentanoyloxy)-phenyl-5-heptoxymethyloxypyridin
2-p-(3-Methylpentanoyloxy)-phenyl-5-hexoxyethyloxypyridin
2-p-(3-Methylpentanoyloxy)-phenyl-5-pentoxypropyloxypyridin
2-p-(4-Methylhexanoyloxy)-phenyl-5-butyloxypyridin
2-p-(4-Methylhexanoyloxy)-phenyl-5-pentyloxypyridin
2-p-(4-Methylhexanoyloxy)-phenyl-5-hexyloxypyridin
2-p-(4-Methylhexanoyloxy)-phenyl-5-heptyloxypyridin
2-p-(4-Methylhexanoyloxy)-phenyl-5-octyloxypyridin
2-p-(4-Methylhexanoyloxy)-phenyl-5-nonyloxypyridin
2-p-(4-Methylhexanoyloxy)-phenyl-5-decyloxypyridin
2-p-(4-Methylhexanoyloxy)-phenyl-5-undecyloxypyridin
2-p-(4-Methylhexanoyloxy)-phenyl-5-dodecyloxypyridin
2-p-(4-Methylhexanoyloxy)-phenyl-5-heptoxymethyloxypyridin
2-p-(4-Methylhexanoyloxy)-phenyl-5-hexoxyethyloxypyridin
2-p-(4-Methylhexanoyloxy)-phenyl-5-pentoxypropyloxypyridin
2-p-(6-Methyloctanoyloxy)-phenyl-5-butyloxypyridin
2-p-(6-Methyloctanoyloxy)-phenyl-5-pentyloxypyridin
2-p-(6-Methyloctanoyloxy)-phenyl-5-hexyloxypyridin
2-p-(6-Methyloctanoyloxy)-phenyl-5-heptyloxypyridin
2-p-(6-Methyloctanoyloxy)-phenyl-5-octyloxypyridin
2-p-(6-Methyloctanoyloxy)-phenyl-5-nonyloxypyridin
2-p-(6-Methyloctanoyloxy)-phenyl-5-decyloxypyridin
2-p-(6-Methyloctanoyloxy)-phenyl-5-undecyloxypyridin
2-p-(6-Methyloctanoyloxy)-phenyl-5-dodecyloxypyridin
2-p-(6-Methyloctanoyloxy)-phenyl-5-heptoxymethyloxypyridin
2-p-(6-Methyloctanoyloxy)-phenyl-5-hexoxyethyloxypyridin
2-p-(6-Methyloctanoyloxy)-phenyl-5-pentoxypropyloxypyridin
2-p-(6-Methyloctyloxy)-phenyl-5-butyloxypyridin
2-p-(6-Methyloctyloxy)-phenyl-5-pentyloxypyridin
2-p-(6-Methyloctyloxy)-phenyl-5-hexyloxypyridin
2-p-(6-Methyloctyloxy)-phenyl-5-heptyloxypyridin
2-p-(6-Methyloctyloxy)-phenyl-5-octyloxypyridin
2-p-(6-Methyloctyloxy)-phenyl-5-nonyloxypyridin
2-p-(6-Methyloctyloxy)-phenyl-5-decyloxypyridin
2-p-(6-Methyloctyloxy)-phenyl-5-undecyloxypyridin
2-p-(6-Methyloctyloxy)-phenyl-5-dodecyloxypyridin
2-p-(6-Methyloctyloxy)-phenyl-5-heptoxymethyloxypyridin
2-p-(6-Methyloctyloxy)-phenyl-5-hexoxyethyloxypyridin
2-p-(6-Methyloctyloxy)-phenyl-5-pentoxypropyloxypyridin
2-p-Octyloxyphenyl-5-(2-methyloctyloxycarbonyl)-pyridin
2-p-Octyloxyphenyl-5-(3-methyloctyloxycarbonyl)-pyridin
2-p-Octyloxyphenyl-5-(4-methyloctyloxycarbonyl)-pyridin
2-p-Octyloxyphenyl-5-(5-methyloctyloxycarbonyl)-pyridin
2-p-Octyloxyphenyl-5-(6-methyloctyloxycarbonyl)-pyridin
2-p-Dodecyloxyphenyl-5-(2-methyloctyloxycarbonyl)-pyridin
2-p-Undecyloxyphenyl-5-(2-methylbutoxy)-pyridin
2-p-Decyloxyphenyl-5-(2-methylbutoxy)-pyridin
2-p-Nonyloxyphenyl-5-(2-methylbutoxy)-pyridin
2-p-Octyloxyphenyl-5-(2-methylbutoxy)-pyridin
2-p-Heptyloxyphenyl-5-(2-methylbutoxy)-pyridin
2-p-Dodecanoyloxyphenyl-5-(2-methylbutoxy)-pyridin
2-p-Undecanoyloxyphenyl-5-(2-methylbutoxy)-pyridin
2-p-Decanoyloxyphenyl-5-(2-methylbutoxy)-pyridin
2-p-Nonanoyloxyphenyl-5-(2-methylbutoxy)-pyridin
2-p-Octanoyloxyphenyl-5-(2-methylbutoxy)-pyridin
2-p-Heptanoyloxyphenyl-5-(2-methylbutoxy)-pyridin

Beispiel 27

Zu einer Lösung von 0,01 mol 3-[4-(R-2-Octyloxy)-phenyl]-pyridin (erhältlich durch Kopplung von 4-(R-2-Octyloxy)-phenylmagnesiumbromid und 3-Brompyridin) in 30 ml Toluol tropft man bei 10-15 °C eine Lösung von 0,01 m n-Butyllithium (5 % in n-Pentan) und erhitzt anschließend 4 h unter Rückfluß. Nach Aufarbeitung und Reinigung durch Chromatographie erhält man 2-n-Butyl-5-[4-(R-2-octyloxy)-phenyl]-pyridin.

Analog werden hergestellt:

2-Pentyl-5-[4-(R-2-octyloxy)-phenyl]-pyridin
2-Hexyl-5-[4-(R-2-octyloxy)-phenyl]-pyridin
2-Heptyl-5-[4-(R-2-octyloxy)-phenyl]-pyridin
2-Octyl-5-[4-(R-2-octyloxy)-phenyl]-pyridin
2-Nonyl-5-[4-(R-2-octyloxy)-phenyl]-pyridin
2-Decyl-5-[4-(R-2-octyloxy)-phenyl]-pyridin
2-Undecyl-5-[4-(R-2-octyloxy)-phenyl]-pyridin
2-Dodecyl-5-[4-(R-2-octyloxy)-phenyl]-pyridin

Beispiel 28

Eine flüssigkristalline Phase bestehend aus

10 % 2-p-(4-Methylhexyloxy)-phenyl-5-heptylpyrimidin,
4 % 2-p-(4-Methylhexyloxy)-phenyl-5-decylpyrimidin,
3 % 2-p-(4-Methylhexyloxy)-phenyl-5-dodecylpyrimidin,
8 % 2-p-(5-Methylheptyloxy)-phenyl-5-nonylpyrimidin,
8 % 2-p-(5-Methylheptyloxy)-phenyl-5-undecylpyrimidin,
6 % 2-p-(5-Methylheptyloxy)-phenyl-5-dodecylpyrimidin,
6 % 2-p-(6-Methyloctyloxy)-phenyl-5-octylpyrimidin,
30 % r-1-Cyan-cis-4-(4'-octyloxybiphenyl-4-yl)-1-octylcyclohexan,
15 % r-1-Cyan-cis-4-(4'-octylbiphenyl-4-yl)-1-butylcyclohexan und
10 % 4-(5-Hexylpyrimidin-2-yl)-phenyl-2-chlorpropionat

zeigt K. -18° $S_C$* 66° $S_A$* 70° Ch 82° I.

Beispiel 29

Eine flüssigkristalline Phase bestehend aus

10 % 2-p-(4-Methylhexyloxy)-phenyl-5-heptylpyrimidin,
4 % 2-p-(4-Methylhexyloxy)-phenyl-5-decylpyrimidin,
3 % 2-p-(4-Methylhexyloxy)-phenyl-5-dodecylpyrimidin,
8 % 2-p-(5-Methylheptyloxy)-phenyl-5-nonylpyrimidin,
8 % 2-p-(5-Methylheptyloxy)-phenyl-5-undecylpyrimidin,
6 % 2-p-(5-Methylheptyloxy)-phenyl-5-dodecylpyrimidin,
6 % 2-p-(6-Methyloctyloxy)-phenyl-5-octylpyrimidin,
30 % r-1-Cyan-cis-4-(4'-octyloxybiphenyl-4-yl)-1-octylcyclohexan,
15 % r-1-Cyan-cis-4-(4'-octylbiphenyl-4-yl)-1-butylcyclohexan und
6 % 1-(4'-Pentylbiphenyl-4-yl)-2-(1-cyan-3-methylcyclohexyl)-ethan und
4 % 4-(5-Nonylpyrimidin-2-yl)-phenyl-p-(2-octyloxycarbonyl)-benzylether

zeigt K. -15° $S_C$* 60° $S_A$* 66° Ch 80° I.

Beispiel 30

Ein flüssigkristallines Material bestehend aus einer achiralen Basismischung enthaltend

3,3 % 2-p-Hexyloxyphenyl-5-heptylpyrimidin,
7,7 % 2-p-Hexyloxyphenyl-5-nonylpyrimidin,
3,3 % 2-p-Heptyloxyphenyl-5-heptylpyrimidin,
3,3 % 2-p-Octyloxyphenyl-5-heptylpyrimidin,
3,3 % 2-p-Nonyloxyphenyl-5-heptylpyrimidin,
25,6 % 2-p-Nonyloxyphenyl-5-nonylpyrimidin,
31,2 % r-1-Cyan-cis-4-(4'-octyloxybiphenyl-4-yl)-1-butylcyclohexan,
15,6 % r-1-Cyan-cis-4-(4'-heptylbiphenyl-4-yl)-1-hexylcyclohexan

und 10 % optisch aktives Ethyl-2-[p-nonylpyrimidin-2-yl)-phenoxy)-propanoat
zeigt K. -5° $S_C$* 63° $S_A$* 64° Ch 89° I und eine spontane Polarisation von 10,4 nC/cm² bei 20°.

Beispiel 31

Eine flüssigkristalline Phase bestehend aus der achiralen Basismischung aus Beispiel 30 und 10 % optisch aktivem 2-p-(2-Octyloxy)-phenyl-5-nonylpyrimidin zeigt K. -12° $S_C$* 61° $S_A$* 67° Ch 82° I. Dieses Material weist ein besonders günstiges Tieftemperaturverhalten auf.

Beispiel 32

Eine flüssigkristalline Phase bestehend aus der achiralen Basismischung aus Beispiel 30 und 10 % optisch aktivem 2,5-Dimethyl-hex-4-enyloxyphenyl-5-hexylpyrimidin zeigt K. 0° $S_C$* 68° $S_A$* 72° Ch 91° I.

Beispiel 33

Eine flüssigkristalline Phase bestehend aus der achiralen Basismischung aus Beispiel 30 und 10 % optisch aktivem p-(5-Heptylpyrimidin-2-yl)-zimtsäure-(2-methylbutylester) zeigt K. 5° $S_C$* 70° $S_A$* 86° Ch 89° I.

Beispiel 34

Eine flüssigkristalline Phase bestehend aus der achiralen Basismischung aus Beispiel 30 und 10 % optisch aktivem p-(5-Heptylpyrimidin-2-yl)-zimtsäure-(2-octylester) zeigt K. 3° $S_C$* 64° $S_A$* 83° Ch 91° I und eine spontane Polarisation von 3,3 nC/cm² bei 20°.

Beispiel 35

Eine flüssigkristalline Phase bestehend aus der achiralen Basismischung aus Beispiel 30 und 10 % optisch aktivem p-(5-Heptylpyrimidin-2-yl)-phenylpropionsäure-2-methylbutylester zeigt K. -2° $S_C$* 70° $S_A$* 79° Ch 93° I.

Beispiel 36

Eine flüssigkristalline Phase bestehend aus der achiralen Basismischung aus Beispiel 30 und 10 % optisch aktivem 2-p-(3,7-Dimethyloctyloxy)-phenyl-5-hexylpyrimidin zeigt K. 3° $S_C$* 64° $S_A$* 67° Ch 88° I und eine spontane Polarisation von 1,2 nC/cm² bei 20°.

Beispiel 37

Eine flüssigkristalline Phase bestehend aus

| | |
|---|---|
| 3 % | 2-p-Hexyloxyphenyl-5-heptylpyrimidin, |
| 3 % | 2-p-Heptyloxyphenyl-5-heptylpyrimidin, |
| 3 % | 2-p-Octyloxyphenyl-5-heptylpyrimidin, |
| 3 % | 2-p-Nonyloxyphenyl-5-heptylpyrimidin, |
| 7 % | 2-p-Hexyloxyphenyl-5-nonylpyrimidin, |
| 25 % | 2-p-Nonyloxyphenyl-5-nonylpyrimidin, |
| 28 % | r-1-Cyan-cis-4-(4′-octyloxybiphenyl-4-yl)-1-butylcyclohexan, |
| 4 % | r-1-Cyan-cis-4-(4′-octylbiphenyl-4-yl)-1-butylcyclohexan, |
| 6 % | r-1-Cyan-cis-4-(trans-4-pentylcyclohexyl)-1-(trans-4-pentylcyclohexyl)-cyclohexan |
| 10 % | optisch aktives 1-(4′-Pentylbiphenyl-4-yl)-2-(1-cyan-3-methylcyclohexyl)-ethan und |
| 8 % | optisch aktives 2-p-Dodecylphenyl-5-(2-octyloxy)-pyridazin |
| zeigt | K -15° $S_C$* 58° $S_A$* 64° Ch 82° I und eine spontane Polarisation von 19 nC/cm². |

Beispiel 38

Eine flüssigkristalline Phase bestehend aus

| | |
|---|---|
| 3 % | 2-p-Hexyloxyphenyl-5-heptylpyrimidin, |
| 3 % | 2-p-Heptyloxyphenyl-5-heptylpyrimidin, |

3 % 2-p-Octyloxyphenyl-5-heptylpyrimidin,
3 % 2-p-Nonyloxyhenyl-5-hetpylpyrimidin,
7 % 2-p-Hexyloxyphenyl-5-nonylpyrimidin,
23 % 2-p-Nonyloxyphenyl-5-nonylpyrimidin,
28 % r-1-Cyan-cis-4-(4'-octyloxybiphenyl-4-yl)-1-butylcyclohexan,
14 % r-1-Cyan-cis-4-(4'-heptylbiphenyl-4-yl)-1-hexylcyclohexan,
6 % r-1-Cyan-cis-4-(trans-4-pentylcyclohexyl)-1-(trans-4-pentylcyclohexyl)-cyclohexan und
10 % optisch aktives 2-p-(2-Octyloxycarbonylphenyl)-5-nonylpyrimidin
zeigt $S_C$* 68° $S_A$* 81° Ch 96 I.

Beispiel 39

Eine flüssigkristalline Phase bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
7 % 2-p-Hexyloxyphenyl-5-nonylpyrimidin,
23 % 2-p-Nonyloxyphenyl-5-nonylpyrimidin,
28 % r-1-Cyan-cis-4-(4'-octyloxybiphenyl-4-yl)-1-butylcyclohexan,
14 % r-1-Cyan-cis-4-(4'-heptylbiphenyl-4-yl)-1-hexylcyclohexan,
6 % r-1-Cyan-cis-4-(trans-4-pentylcyclohexyl)-1-(trans-4-pentylcyclohexyl)-cyclohexan und
10 % optisch aktives p-[p-(5-Nonylpyrimidin-2-yl)-phenoxymethyl]-benzoesäure-(2-octylester)
zeigt $S_C$* 72° $S_A$* 83° Ch 93° I und eine spontane Polarisation von 5 nC/cm².

Beispiel 40

Eine flüssigkristalline Phase bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
7 % 2-p-Hexyloxyphenyl-5-nonylpyrimidin,
23 % 2-p-Nonyloxyphenyl-5-nonylpyrimidin,
28 % r-1-Cyan-cis-4-(4'-octyloxybiphenyl-4-yl)-1-butylcyclohexan,
14 % r-1-Cyan-cis-4-(4'-heptylbiphenyl-4-yl)-1-hexylcyclohexan,
6 % r-1-Cyan-cis-4-(trans-4-pentylcyclohexyl)-1-(trans-4-pentylcyclohexyl)-cyclohexan und
10 % optisch aktives 3-Chlor-4-(2-octyloxy)-benzoesäure-p-(5-nonyl-pyrimidin-2-yl)-phenylester
zeigt $S_C$* 70° Ch 93° I.

Beispiel 41

Eine flüssigkristalline Phase bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
7 % 2-p-Hexyloxyphenyl-5-nonylpyrimidin,
23 % 2-p-Nonyloxyphenyl-5-nonylpyrimidin,
28 % r-1-Cyan-cis-4-(4'-octyloxybiphenyl-4-yl)-1-butylcyclohexan,
14 % r-1-Cyan-cis-4-(4'-heptylbiphenyl-4-yl)-1-hexylcyclohexan,
6 % r-1-Cyan-cis-4-(trans-4-pentylcyclohexyl)-1-(trans-4-pentylcyclohexyl)-cyclohexan und
10 % optisch aktives 2-p-(2-Methylbutoxycarbonyl)-phenyl-5-heptylpyrimidin
zeigt $S_C$* 55° $S_A$* 77° Ch 91° I.

Beispiel 42

Eine flüssigkristalline Phase bestehend aus

3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
7 % 2-p-Hexyloxyphenyl-5-nonylpyrimidin,
23 % 2-p-Nonyloxyphenyl-5-nonylpyrimidin,
28 % r-1-Cyan-cis-4-(4'-octyloxybiphenyl-4-yl)-1-butylcyclohexan,
14 % r-1-Cyan-cis-4-(4'-heptylbiphenyl-4-yl)-1-hexylcyclohexan,
6 % r-1-Cyan-cis-4-(trans-4-pentylcyclohexyl)-1-(trans-4-pentylcyclohexyl)-cyclohexan und
10 % optisch aktives p-[p-(5-Nonylpyrimidin-2-yl)-phenoxymethyl]-benzoesäure-(2-methylbutylester)
zeigt $S_C$* 78° $S_A$* 84° Ch 90° I und eine spontane Polarisation von 4 nC/cm².

Beispiel 43

Eine flüssigkristalline Phase bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
7 % 2-p-Hexyloxyphenyl-5-nonylpyrimidin,
23 % 2-p-Nonyloxyphenyl-5-nonylpyrimidin,
28 % r-1-Cyan-cis-4-(4'-octyloxybiphenyl-4-yl)-1-butylcyclohexan,
14 % r-1-Cyan-cis-4-(4'-heptylbiphenyl-4-yl)-1-hexylcyclohexan,
6 % r-1-Cyan-cis-4-(trans-4-pentylcyclohexyl)-1-(trans-4-pentylcyclohexyl)-cyclohexan und
10 % optisch aktives 2-p-(2-Octyloxycarbonyl)-phenyl-5-heptylpyrimidin
zeigt $S_C$* 50° $S_A$* 78° Ch 86° I.

Beispiel 44

Eine flüssigkristalline Phase bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
7 % 2-p-Hexyloxyphenyl-5-nonylpyrimidin,
23 % 2-p-Nonyloxyphenyl-5-nonylpyrimidin,
28 % r-1-Cyan-cis-4-(4'-octyloxybiphenyl-4-yl)-1-butylcyclohexan,
14 % r-1-Cyan-cis-4-(4'-heptylbiphenyl-4-yl)-1-hexylcyclohexan,
6 % r-1-Cyan-cis-4-(trans-4-pentylcyclohexyl)-1-(trans-4-pentylcyclohexyl)-cyclohexan und
10 % optisch aktives p-[p-(5-Nonylpyrimidin-2-yl)-phenoxymethyl]-(2-methylbutoxy)-benzol
zeigt $S_C$* 80° Ch 100° I.

Beispiel 45

Eine flüssigkristalline Phase bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
7 % 2-p-Hexyloxyphenyl-5-nonylpyrimidin,
23 % 2-p-Nonyloxyphenyl-5-nonylpyrimidin,
28 % r-1-Cyan-cis-4-(4'-octyloxybiphenyl-4-yl)-1-butylcyclohexan,
14 % r-1-Cyan-cis-4-(4'-heptylbiphenyl-4-yl)-1-hexylcyclohexan,
6 % r-1-Cyan-cis-4-(trans-4-pentylcyclohexyl)-1-(trans-4-pentylcyclohexyl)-cyclohexan und
10 % optisch aktives 1-[p-(5-Heptylpyrimidin-2-yl)-phenyl]-2-p-(2-octyloxycarbonylphenyl)-ethan
zeigt $S_C$* 77° $S_A$* 80° Ch 89° I.

Beispiel 46

Eine flüssigkristalline Phase bestehend aus

| | |
|---|---|
| 3 % | 2-p-Hexyloxyphenyl-5-heptylpyrimidin, |
| 3 % | 2-p-Heptyloxyphenyl-5-heptylpyrimidin, |
| 3 % | 2-p-Octyloxyphenyl-5-heptylpyrimidin, |
| 3 % | 2-p-Nonyloxyphenyl-5-heptylpyrimidin, |
| 7 % | 2-p-Hexyloxyphenyl-5-nonylpyrimidin, |
| 23 % | 2-p-Nonyloxyphenyl-5-nonylpyrimidin, |
| 28 % | r-1-Cyan-cis-4-(4'-octyloxybiphenyl-4-yl)-1-butylcyclohexan, |
| 14 % | r-1-Cyan-cis-4-(4'-heptylbiphenyl-4-yl)-1-hexylcyclohexan, |
| 6 % | r-1-Cyan-cis-4-(trans-4-pentylcyclohexyl)-1-(trans-4-pentylcyclohexyl)-cyclohexan und |
| 10 % | optisch aktives 1-[p-(5-Heptylpyrimidin-2-yl)-phenyl]-2-p-(2-octyloxycarbonylphenyl)-ethen |
| zeigt | $S_C$* 73° $S_A$* 78° Ch 91° I. |

Beispiel 47

Eine flüssigkristalline Phase bestehend aus

| | |
|---|---|
| 3 % | 2-p-Hexyloxyphenyl-5-heptylpyrimidin, |
| 3 % | 2-p-Heptyloxyphenyl-5-heptylpyrimidin, |
| 3 % | 2-p-Octyloxyphenyl-5-heptylpyrimidin, |
| 3 % | 2-p-Nonyloxyphenyl-5-heptylpyrimidin, |
| 7 % | 2-p-Hexyloxyphenyl-5-nonylpyrimidin, |
| 23 % | 2-p-Nonyloxyphenyl-5-nonylpyrimidin, |
| 28 % | r-1-Cyan-cis-4-(4'-octyloxybiphenyl-4-yl)-1-butylcyclohexan, |
| 14 % | r-1-Cyan-cis-4-(4'-heptylbiphenyl-4-yl)-1-hexylcyclohexan, |
| 6 % | r-1-Cyan-cis-4-(trans-4-pentylcyclohexyl)-1-(trans-4-pentylcyclohexyl)-cyclohexan und |
| 10 % | optisch aktives 2-p-(2-Methylbutylmercaptophenyl)-5-heptylpyrimidin |
| zeigt | $S_C$* 63° $S_A$* 65° Ch 88° I. |

Die in den Beispielen 38 bis 47 angegebenen Phasen zeigen alle K/$S_C$*-Phasenübergänge unterhalb Raumtemperatur.

Beispiel 48

Eine flüssigkristalline Phase bestehend aus

| | |
|---|---|
| 3 % | 2-p-Hexyloxyphenyl-5-octylpyrimidin, |
| 3 % | 2-p-Heptyloxyphenyl-5-octylpyrimidin, |
| 3 % | 2-p-Octyloxyphenyl-5-octylpyrimidin, |
| 3 % | 2-p-Nonyloxyphenyl-5-octylpyrimidin, |
| 3 % | 2-p-Hexyloxyphenyl-5-nonylpyrimidin, |
| 23 % | 2-p-Nonyloxyphenyl-5-nonylpyrimidin, |
| 25 % | r-1-Cyan-cis-4-(4'-octyloxybiphenyl-4-yl)-1-octylcyclohexan, |
| 13 % | r-1-Cyan-cis-4-(4'-heptylbiphenyl-4-yl)-1-hexylcyclohexan, |
| 4 % | r-1-Cyan-cis-4-(trans-4-pentylcyclohexyl)-1-(trans-4-pentylcyclohexyl)-cyclohexan |
| 10 % | optisch aktives 1-(4'-Pentylbiphenyl-4-yl)-2-(1-cyan-3-methyl-cyclohexyl)-ethan und |
| 10 % | optisch aktives 2-p-(2-Methylbutoxy)-phenyl-5-octyloxycarbonyloxypyrazin |
| zeigt | K -18° $S_C$* 61° $S_A$* 67° Ch 84° I und eine spontane Polarisation von 22 $nC/cm^2$. |

Beispiel 49

Eine flüssigkristalline Phase bestehend aus

| | |
|---|---|
| 3 % | 2-p-Hexyloxyphenyl-5-heptylpyrimidin, |
| 3 % | 2-p-Heptyloxyphenyl-5-heptylpyrimidin, |
| 3 % | 2-p-Octyloxyphenyl-5-heptylpyrimidin, |
| 3 % | 2-p-Nonyloxyphenyl-5-heptylpyrimidin, |
| 7 % | 2-p-Hexyloxyphenyl-5-nonylpyrimidin, |
| 23 % | 2-p-Nonyloxyphenyl-5-nonylpyrimidin, |
| 32 % | r-1-Cyan-cis-4-(4'-octyloxybiphenyl-4-yl)-1-octylcyclohexan, |

13 % r-1-Cyan-cis-4-(4'-heptylbiphenyl-4-yl)-1-hexylcyclohexan,
3 % r-1-Cyan-cis-4-(trans-4-pentylcyclohexyl)-1-(trans-4-pentylcyclohexyl)-cyclohexan und
10 % optisch aktives Ethyl-2-[p-(5-nonylpyrimidin-2-yl)-phenoxy]-propanoat
zeigt K <-30° $S_C$* 61° $S_A$* 66° Ch 85° I und eine spontane Polarisation von 9,7 nC/cm².

Beispiel 50

Man stellt eine flüssigkristalline Phase her bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
7 % 2-p-Hexyloxyphenyl-5-nonylpyrimidin,
23 % 2-p-Nonyloxyphenyl-5-nonylpyrimidin,
32 % r-1-Cyan-cis-4-(4'-octyloxybiphenyl-4-yl)-1-octylcyclohexan,
13 % r-1-Cyan-cis-4-(4'-heptylbiphenyl-4-yl)-1-hexylcyclohexan,
3 % r-1-Cyan-cis-4-(trans-4-pentylcyclohexyl)-1-(trans-4-pentylcyclohexyl)-cyclohexan und
10 % optisch aktives 2-p-(5-Methylheptyl)-phenyl-5-nonylpyrazin.

Beispiel 51

Man stellt eine flüssigkristalline Phase her bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
7 % 2-p-Hexyloxyphenyl-5-nonylpyrimidin,
23 % 2-p-Nonyloxyphenyl-5-nonylpyrimidin,
32 % r-1-Cyan-cis-4-(4'-octyloxybiphenyl-4-yl)-1-octylcyclohexan,
13 % r-1-Cyan-cis-4-(4'-heptylbiphenyl-4-yl)-1-hexylcyclohexan,
3 % r-1-Cyan-cis-4-(trans-4-pentylcyclohexyl)-1-(trans-4-pentylcyclohexyl)-cyclohexan und
10 % optisch aktives 2-p-(5-Methylheptyl)-phenyl-5-(1,4-dioxanonyl)-pyrazin.

Beispiel 52

Man stellt eine flüssigkristalline Phase her bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
7 % 2-p-Hexyloxyphenyl-5-nonylpyrimidin,
23 % 2-p-Nonyloxyphenyl-5-nonylpyrimidin,
32 % r-1-Cyan-cis-4-(4'-octyloxybiphenyl-4-yl)-1-octylcyclohexan,
13 % r-1-Cyan-cis-4-(4'-heptylbiphenyl-4-yl)-1-hexylcyclohexan,
3 % r-1-Cyan-cis-4-(trans-4-pentylcyclohexyl)-1-(trans-4-pentylcyclohexyl)-cyclohexan und
10 % optisch aktives Ethyl-2-[p-(5-heptyloxypyrazin-2-yl)-phenoxy]-propanoat.

Beispiel 53

Man stellt eine flüssigkristalline Phase her bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
7 % 2-p-Hexyloxyphenyl-5-nonylpyrimidin,
23 % 2-p-Nonyloxyphenyl-5-nonylpyrimidin,
32 % r-1-Cyan-cis-4-(4'-octyloxybiphenyl-4-yl)-1-octylcyclohexan,
13 % r-1-Cyan-cis-4-(4'-heptylbiphenyl-4-yl)-1-hexylcyclohexan,

3 % r-1-Cyan-cis-4-(trans-4-pentylcyclohexyl)-1-(trans-4-pentylcyclohexyl)-cyclohexan und
10 % optisch aktives Ethyl-2-[-p-(5-heptylpyridin-2-yl)-phenoxy]-propanoat.

Beispiel 54

Man stellte eine flüssigkristalline Phase her bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
7 % 2-p-Hexyloxyphenyl-5-nonylpyrimidin,
23 % 2-p-Nonyloxyphenyl-5-nonylpyrimidin,
32 % r-1-Cyan-cis-4-(4'-octyloxybiphenyl-4-yl)-1-octylcyclohexan,
13 % r-1-Cyan-cis-4-(4'-heptylbiphenyl-4-yl)-1-hexylcyclohexan,
3 % r-1-Cyan-cis-4-(trans-4-pentylcyclohexyl)-1-(trans-4-pentylcyclohexyl)-cyclohexan und
10 % optisch aktives Butyl-2-[-p-(5-nonylpyridin-2-yl)-phenoxy]-propanoat.

Beispiel 55

Man stellt einer flüssigkristalline Phase her bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
7 % 2-p-Hexyloxyphenyl-5-nonylpyrimidin,
23 % 2-p-Nonyloxyphenyl-5-nonylpyrimidin,
32 % r-1-Cyan-cis-4-(4'-octyloxybiphenyl-4-yl)-1-octylcyclohexan,
13 % r-1-Cyan-cis-4-(4'-heptylbiphenyl-4-yl)-1-hexylcyclohexan,
3 % r-1-Cyan-cis-4-(trans-4-pentylcyclohexyl)-1-(trans-4-pentylcyclohexyl)-cyclohexan und
10 % optisch aktives Ethyl-2-[-p-(5-nonylpyridazin-2-yl)-phenoxy]-propanoat.

Beispiel 56

Man stellt eine flüssigkristalline Phase her bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
7 % 2-p-Hexyloxyphenyl-5-nonylpyrimidin,
23 % 2-p-Nonyloxyphenyl-5-nonylpyrimidin,
32 % r-1-Cyan-cis-4-(4'-octyloxybiphenyl-4-yl)-1-octylcyclohexan,
13 % r-1-Cyan-cis-4-(4'-heptylbiphenyl-4-yl)-1-hexylcyclohexan,
3 % r-1-Cyan-cis-4-(trans-4-pentylcyclohexyl)-1-(trans-4-pentylcyclohexyl)-cyclohexan und
10 % optisch aktives Ethyl-2-[-p-(5-nonylpyridin-2-yl)-benzoyloxy]-propanoat.

Bei den in den Beispielen 37 und 48 eingesetzten Gemischen von optisch aktiven Materialien ist jeweils ein Zusatz bestrebt, eine rechtshändige Verdrillung zu erzeugen, während der andere Zusatz bestrebt ist, eine linkshändige Verdrillung zu erzeugen.

## Patentansprüche

### Patentansprüche für folgenden Vertragsstaat: AT

1. Ferroelektrische flüssigkristalline Phase mit mindestens zwei Komponenten, dadurch gekennzeichnet, daß mindestens eine Komponente ein stickstoffhaltiger Heterocylus der Formel I ist,

$$R^1\text{-}A^1\text{-}Z^1\text{-}A^2\text{-}R^2 \quad I$$

worin
einer der Reste

$R^1$ und $R^2$ H, eine unsubstituierte oder substituierte Alkylgruppe mit 1-15 C-Atomen, worin auch eine oder zwei nicht benacbbarte $CH_2$-Gruppen durch mindestens eine Gruppierung aus der Gruppe -O-, -CO-, -O-CO-, -CO-O- und -CH=CH- ersetzt sein können, F, Cl, Br, -CN, -NCS oder $R^3$-$(A^3)_p$-$Z^2$-,

der andere Rest

$R^1$ oder $R^2$ ein optisch aktiver organischer Rest der Formel

$$-X-Q-\overset{*}{C}H(Y)-R$$

worin

X -CO-O-, -O-CO-, -O-CO-O-, -CO-, -O-, -S-, -CH=CH-, -CH=CH-COO- oder eine Einfachbindung,

Q Alkylen mit 1 bis 5 C-Atomen, worin auch eine nicht mit X verknüpfte $CH_2$-Gruppe durch -O-, -CO-, -O-CO-, -CO-O- oder -CH=CH- ersetzt sein kann, oder eine Einfachbindung,

Y CN, Halogen, Methyl oder Methoxy, und

R eine von Y verschiedene Alkylgruppe mit 1 bis 10 C-Atomen, worin auch eine oder zwei nicht benachbarte $CH_2$-Gruppen durch -O-, -CO-, -O-CO-, -CO-O- und/oder -CH=CH- ersetzt sein können,

bedeutet,

$A^1$ -A-, -$A^4$-$Z^3$-A- oder -A-$Z^3$-$A^4$-,

A eine 1,4-Phenylengruppe, worin mindestens eine CH-Gruppe durch N ersetzt ist,

$A^2$, $A^3$ und $A^4$ jeweils unsubstituiertes oder durch ein oder zwei F- und/oder Cl-Atome und/oder $CH_3$-Gruppen und/oder CN-Gruppen substituiertes 1,4-Phenylen, worin auch eine oder zwei CH-Gruppen durch N-Atome ersetzt sein können, 1,4-Cyclohexylen, worin auch eine oder zwei nicht benachbarte $CH_2$-Gruppen durch O-Atome und/oder S-Atome ersetzt sein können, Piperidin-1,4-diyl, 1,4-Bicyclo(2,2,2)-octylen, unsubstituiertes oder durch CN unsubstituiertes Decahydronaphtalin-2,6-diyl oder 1,2,3,4-Tetrahydronaphthalin-2,6-diyl,

$Z^1$, $Z^2$ und $Z^3$ jeweils -CO-O-, -O-CO-, -$OCH_2$-, -$CH_2O$-, -$CH_2CH_2$-, substituiertes Ethylen oder eine Einfachbindung,

$R^3$ H, eine unsubstituierte oder substituierte Alkylgruppe mit 1-15 C-Atomen, worin auch eine oder zwei nicht benachbarte $CH_2$- Gruppen durch eine Gruppierung aus der Gruppe -O-, -CO-, -O-CO-, -CO-O und -CH=CH- ersetzt sein können, F, Cl, Br, -NCS oder -CN, und

P 1 oder 2

bedeutet, wobei für p = 2 die Gruppen $A^3$ gleich der voneinander verschieden sein können, mit den Maßgaben, daß im Falle A = Pyrimidin-2,5-diyl einer der Reste $R^1$ und $R^2$ ein optisch aktiver organischer Rest und der andere Rest $R^1$ oder $R^2$ eine unsubstituierte oder substituierte Alkylgruppe mit 1-15 C-Atomen, worin auch eine oder zwei nicht benachbarte $CH_2$-Gruppen durch mindestens eine Gruppierung aus der Gruppe -O-, -CO-, -O-CO-, -CO-O- und -CH=CH- ersetzt sein können, bedeutet.

2. Ferroelektrische Phase nach Anspruch 1, dadurch gekennzeichnet, daß mindestens eine Komponente ein stickstoffhaltiger Heterocyclus der Formel I1 ist,

$$R^1-A-A^2-X-Q-\overset{*}{C}H(Y)-R \qquad \text{I1}$$

worin

$R^1$ Alkyl oder Alkoxy mit 1-15 C-Atomen,

A Pyrimidin-2,5-diyl oder Pyridin-2,5-diyl,

$A^2$ unsubstituiertes oder durch ein Fluoratom substituiertes 1,4-Phenylen,

X -O-CO- oder -O-,

Q Alkylen mit 1 bis 5 C-Atomen oder eine Einfachbindung,

Y Halogen, und

R Alkyl mit 1 bis 10 C-Atomen

bedeuten.

3. Ferroelektrische Phase nach Anspruch 1 oder 2, dadurch gekennzeichnet, daß mindestens eine Komponente ein stickstoffhaltiger Heterocyclus der Formel I ist,

worin

$-A^1-Z^1-A^2-$ ein Strukturelement ausgewählt aus der Gruppe der Formeln 1 bis 4

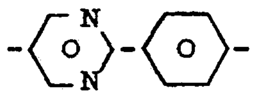 , 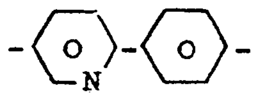 , 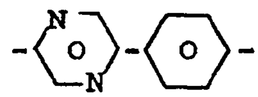

1 2 3

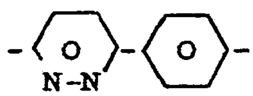

4

bedeutet.

4. Ferroelektrische Phase nach einem der Ansprüche 1 bis 3, dadurch gekennzeichnet, daß mindestens eine Komponente ein stickstoffhaltige Heterocyclus der Formel I032, I048, I066, I083 und I084 ist:

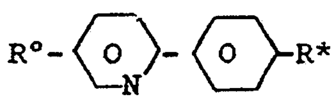 I032

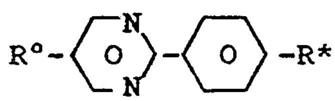 I048

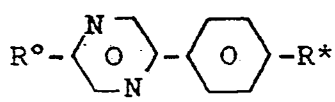 I066

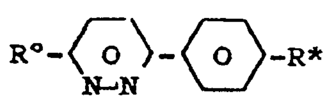 I083

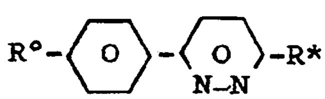 I084

worin

R° geradkettiges Alkyl oder Alkoxy mit jeweils 2 bis 12 C-Atomen bedeutet,

und

R* eine der für den optisch aktiven organischen Rest in Anspruch 1 angegebenen Bedeutungen hat.

5. Ferroelektrische flüssigkristalline Phase nach einem der Asprüche 1 bis 4, dadurch gekennzeichnet, daß mindestens eine Komponente eine Verbindung der Formel I und daß diese Phase mindestens eine andere Komponente mit betragsmäßig kleiner positiver oder negativer dielektrischer Anisotropie ausgewählt aus den Verbindungen der Teilformeln Va bis Vp enthält:

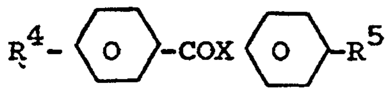

Va

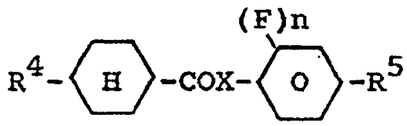

Vb

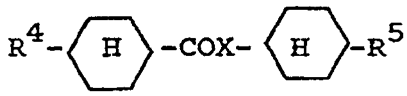

Vc

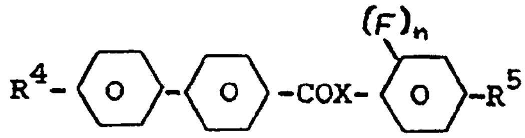

Vd

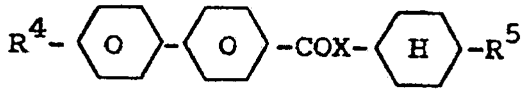

Ve

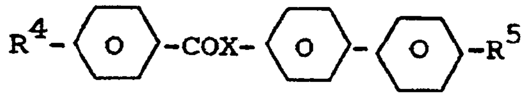

Vf

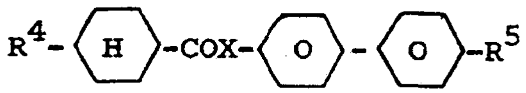

Vg

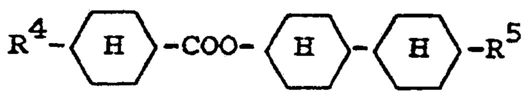

Vh

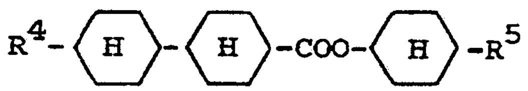

Vi

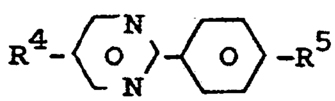

Vj

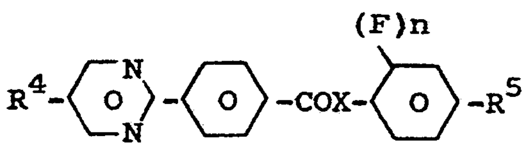

Vk

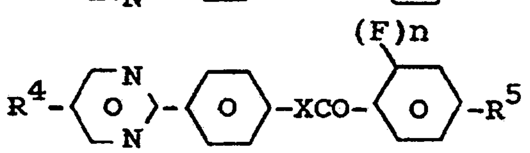

Vl

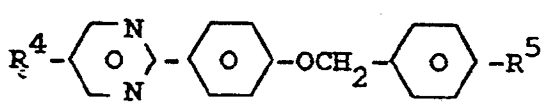

Vm

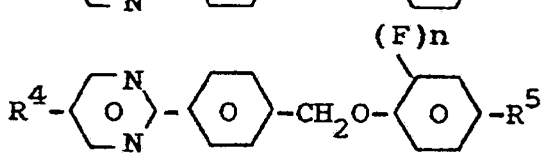

Vn

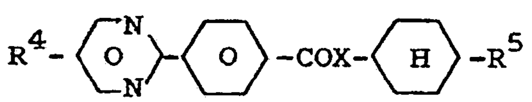

Vo

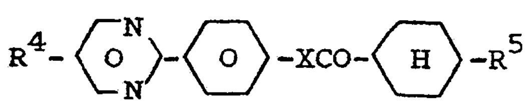

Vp

worin

$R^4$ und $R^5$ jeweils geradkettiges Alkyl, Alkoxy, Alkanoyloxy oder Alkoxycarbonyl mit jeweils 3 bis 12 C-Atomen,

X O, und

n 0 oder 1 bedeuten.

6. Ferroelektrische Phase nach Anspruch 5, dadurch gekennzeichnet, daß mindestens eine Komponente der Verbindung der Formel I ist, worin $-A^1-Z^1-A^2-$

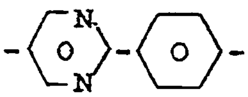

ist,

einer der Reste

$R^1$ und $R^2$ eine unsubstituierte Alkylgruppe mit 1 bis 15 C-Atomen, worin auch eine $CH_2$- Gruppe durch -O-, -COO- oder -OCO- ersetzt sein kann

und der andere Rest $R^1$ oder $R^2$

eine optisch aktive Gruppe der Formel

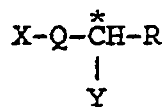

worin

X -CO-O-, -O-, -O-CO- oder eine Einfachbindung,

Q Alkylen mit 1 bis 5 C-Atomen, worin auch eine nicht mit X verknüpfte $CH_2$-Gruppe durch -O- ersetzt sein kann,

Y $CH_3$, CN oder Halogen, und

R geradkettiges Alkyl mit 1 bis 10 C-Atomen

bedeutet,

und daß

mindestens eine andere Komponente eine Verbindung der Formel II ist,

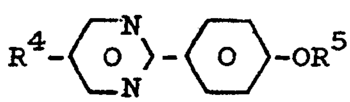

II

worin

$R^4$ und $R^5$ jeweils unabhängig voneinander n-Alkyl mit 5 bis 12 C-Atomen bedeutet.

7. Elektrooptisches Anzeigelement auf der Basis einer Flüssigkristallzelle, dadurch gekennzeichnet, daß es eine Phase nach einem der Ansprüche 1 bis 6 enthält.

**Patentansprüche für folgenden Vertragsstaaten: BE, IT**

1. Stickstoffhaltige Heterocylen der Formel I

$$R^1-A^1-Z^1-A^2-R^2 \quad I$$

worin

einer der Reste

$R^1$ und $R^2$ H, eine substituierte oder substituierte Alkylgruppe mit 1-15 C-Atomen, worin auch eine oder zwei nicht benachbarte $CH_2$-Gruppen durch mindestens eine Gruppierung aus der Gruppe -O-, -CO-, -O-CO-, -CO-O- und -CH=CH- ersetzt sein können, F, Cl, Br, -CN, -NCS oder $R^3-(A^3)_p-Z^2-$,

der andere Rest

$R^1$ oder $R^2$ ein optisch aktiver organischer Rest der Formel

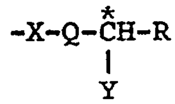

worin

X -CO-O-, O-CO-, -O-CO-O-, -CO-, -O-, -S-, -CH=CH-, -CH=CH-COO- oder eine Einfachbindung,

Q Alkylen mit 1 bis 5 C-Atomen, worin auch eine nicht mit X verknüpfte $CH_2$-Gruppe durch -O-, -CO-, -O-CO-, -CO-O- oder -CH=CH- ersetzt sein kann, oder eine Einfachbindung,

Y CN, Halogen, Methyl oder Methoxy, und

R eine von Y verschiedene Alkylgruppe mit 1 bis 10 C-Atomen, worin auch eine oder zwei nicht benachbarte $CH_2$-Gruppen durch -O-, -CO-, -O-CO-, -CO-O- und/oder -CH=CH- ersetzt sein können,

bedeutet,

$A^1$ -A-, -$A^4$-$Z^3$-A- oder -A-$Z^3$-$A^4$-,

A - eine 1,4-Phenylengruppe, worin mindestens eine CH-Gruppe durch N ersetzt ist,

$A^2$, $A^3$ und $A^4$ jeweils unsubstituiertes oder durch ein oder zwei F- und/oder Cl-Atome und/oder $CH_3$-Gruppen und/oder CN-Gruppen substituiertes 1,4-Phenylen, worin auch eine oder zwei CH-Gruppen durch N-Atome ersetzt sein können, 1,4-Cyclohexylen, worin auch eine oder zwei nicht benachbarte $CH_2$-Gruppen durch O-Atome und/oder S-Atome ersetzt sein können, Piperidin-1,4-diyl, 1,4-Bicyclo(2,2,2)-octylen, unsubstituiertes oder durch CN unsubstituiertes Decahydronaphthalin-2,6-diyl oder 1,2,3,4-Tetrahydronaphthalin-2,6-diyl,

$Z^1$, $Z^2$ und $Z^3$ jeweils -CO-O-, -O-CO-, -$OCH_2$-, -$CH_2O$-, -$CH_2CH_2$-, substituiertes Ethylen oder eine Einfachbindung,

$R^3$ H, eine unsubstituierte oder substituierte Alkylgruppe mit 1-15 C-Atomen, worin auch eine oder zwei nicht benachbarte $CH_2$- Gruppen durch eine Gruppierung aus der Gruppe -O-, -CO-, -O-CO-, -CO-O und -CH=CH- ersetzt sein können, F, Cl, Br, -NCS oder -CN, und

P 1 oder 2

bedeutet, wobei für p = 2 die Gruppen $A^3$ gleich der voneinander verschieden sein können, mit der Maßgabe, daß

im Falle A = Pyrimidin-2,5-diyl einer der Reste $R^1$ und $R^2$ ein optisch aktiver organischer Rest und der andere Rest $R^1$ oder $R^2$ eine unsubstituierte oder substituierte Alkylgruppe mit 1-15 C-Atomen, worin auch eine oder zwei nicht benachbarte $CH_2$-Gruppen durch mindestens eine Gruppierung aus dei Gruppe -O-, -CO-, -O-CO-, -CO-O- und -CH=CH- ersetzt sein können, bedeutet.

2. Stickstoffhaltige Heterocyclen nach Anspruch 1 der Formel I1

$$R^1\text{-A-}A^2\text{-X-Q-}\overset{*}{\underset{\underset{Y}{|}}{CH}}\text{-R} \qquad \text{I1}$$

worin

$R^1$ Alkyl oder Alkoxy mit 1-15 C-Atomen,

A Pyrimidin-2,5-diyl oder Pyridin-2,5-diyl,

$A^2$ unsubstituiertes oder durch ein Fluoratom substituiertes 1,4-Phenylen,

X -O-CO- oder -O-,

Q Alkylen mit 1 bis 5 C-Atomen oder eine Einfachbindung,

Y Halogen, und

R Alkyl mit 1 bis 10 C-Atomen

bedeuten.

3. Stickstoffhaltige Heterocyclen der Formel I nach Anspruch 1,

worin

-$A^1$-$Z^1$-$A^2$- ein Strukturelement ausgewählt aus der Gruppe der Formeln 1 bis 4

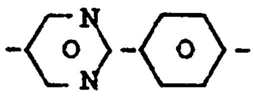 , 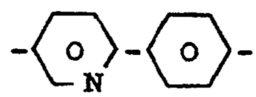 , 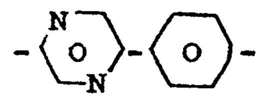

1 2 3

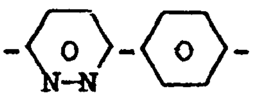

4

bedeutet.

4. Stickstoffhaltige Heterocyclen nach einem der Ansprüche 1 bis 3 der Formel I032, I048, I066, I083 und I084

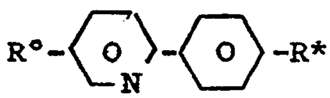 I032

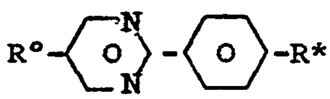 I048

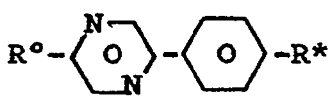 I066

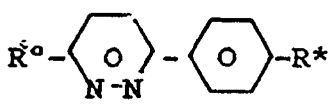 I083

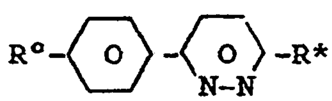 I084 ,

worin

R° geradkettiges Alkyl oder Alkoxy mit jeweils 2 bis 12 C-Atomen bedeutet,

und

R* eine der für den optisch aktiven organischen Rest in Anspruch 1 angegebenen Bedeutungen hat.

5. Verwendung der Verbindungen der Formel I nach einem der Ansprüche 1 bis 4 als Komponenten ferroelektrischer flüssigkristalliner Phasen.

6. Ferroelektrische flüssigkristalline Phase mit mindestens zwei flüssigkristallinen Komponenten, dadurch gekennzeichnet, daß mindestens eine Komponente eine Verbindung der Formel I nach Anspruch 1 ist.

7. Elektrooptisches Anzeigelement auf der Basis einer Flüssigkristallzelle, dadurch gekennzeichnet, daß es eine Phase nach Anspruch 6 enthält.

**Patentansprüche für folgenden Vertragsstaat: NL**

1. Stickstoffhaltige Heterocylen der Formel I

$$R^1\text{-}A^1\text{-}Z^1\text{-}A^2\text{-}R^2 \quad I$$

worin

einer der Reste

$R^1$ und $R^2$ H, eine unsubstituierte oder substituierte Alkylgruppe mit 1-15 C-Atomen, worin auch eine oder

zwei nicht benachbarte $CH_2$-Gruppen durch mindestens eine Gruppierung aus der Gruppe -O-, -CO-, -O-CO-, -CO-O- und -CH=CH- ersetzt sein können, F, Cl, Br, -CN, -NCS oder $R^3$-$(A^3)_p$-$Z^2$-,

der andere Rest

$R^1$oder $R^2$ ein optisch aktiver organischer Rest der Formel

$$-X-Q-\overset{*}{C}H(Y)-R$$

worin

X -CO-O-, -O-CO-, -O-CO-O-, -CO-, -O-, -S-, -CH=CH-, -CH=CH-COO- oder eine Einfachbindung,

Q Alkylen mit 1 bis 5 C-Atomen, worin auch eine nicht mit X verknüpfte $CH_2$-Gruppe durch -O-, -CO-, -O-CO-, -CO-O- oder -CH=CH- ersetzt sein kann, oder eine Einfachbindung,

Y CN, Halogen, Methyl oder Methoxy, und

R eine von Y verschiedene Alkylgruppe mit 1 bis 10 C-Atomen, worin auch eine oder zwei nicht benachbarte $CH_2$-Gruppen durch -O-, -CO-, -O-CO-, -CO-O- und/oder -CH=CH- ersetzt sein können,

bedeutet,

$A^1$ -A-, -$A^4$-$Z^3$-A- oder -A-$Z^3$-$A^4$-,

A eine 1,4-Phenylengruppe, worin mindestens eine CH-Gruppe durch N ersetzt ist,

$A^2$, $A^3$ und $A^4$ jeweils unsubstituiertes oder durch ein oder zwei F- und/oder Cl-Atome und/oder $CH_3$-Gruppen und/oder CN-Gruppen substituiertes 1,4-Phenylen, worin auch eine oder zwei CH-Gruppen durch N-Atome ersetzt sein können, 1,4-Cyclohexylen, worin auch eine oder zwei nicht benachbarte $CH_2$-Gruppen durch O-Atome und(oder S-Atome ersetzt sein können, Piperidin-1,4-diyl, 1,4-Bicyclo(2,2,2)-octylen, unsubstituiertes oder durch CN unsubstituiertes Decahydronaphthalin-2,6-diyl oder 1,2,3,4-Tetrahydronaphthalin-2,6-diyl,

$Z^1$, $Z^2$ und $Z^3$ jeweils -CO-O-, -O-CO-, -$OCH_2$-, -$CH_2O$-, -$CH_2CH_2$-, substituiertes Ethylen oder eine Einfachbindung,

$R^3$ H, eine unsubstituierte oder substituierte Alkylgruppe mit 1-15 C-Atomen, worin auch eine oder zwei nicht benachbarte $CH_2$- Gruppen durch eine Gruppierung aus der Gruppe -O-, -CO-, -O-CO-, -CO-O und -CH=CH- ersetzt sein können, F, Cl, Br, -NCS oder -CN, und

P 1 oder 2

bedeutet, wobei für p = 2 die Gruppen $A^3$ gleich der voneinander verschieden sein können, mit den Maßgaben, daß

a) im Falle A = Pyrimidin-2,5-diyl einer der Reste $R^1$ und $R^2$ ein optisch aktiver organischer Rest und der andere Rest $R^1$ oder $R^2$ eine unsubstituierte oder substituierte Alkylgruppe mit 1-15 C-Atomen, worin auch eine oder zwei nicht benachbarte $CH_2$-Gruppen durch mindestens eine Gruppierung aus der Gruppe -O-, -CO-, -O-CO-, -CO-O- und -CH=CH- ersetzt sein können, bedeutet.

b) 4-[5-(4'-Butylphenyl)pyrimidin-2-yl]benzoesäure-(2'-dodecyloxypropyl-ester) und 4-(5-Heptylpyrimidin-2-yl)-benzoesäure-(2'-dodecyloxypropyl-ester)

ausgenommen sind,

2. Stickstoffhaltige Heterocyclen nach Anspruch 1 der Formel I1

$$R^1-A-A^2-X-Q-CH(Y)-R \qquad I1$$

worin

$R^1$ Alkyl oder Alkoxy mit 1-15 C-Atomen,

A Pyrimidin-2,5-diyl oder Pyridin-2,5-diyl,

$A^2$ unsubstituiertes oder durch ein Fluoratom substituiertes 1,4-Phenylen,

X -O-CO- oder -O-,

Q Alkylen mit 1 bis 5 C-Atomen oder eine Einfachbindung,

Y Halogen, und

R Alkyl mit 1 bis 10 C-Atomen

bedeuten.

3. Stickstoffhaltige Heterocyclen der Formel I nach Anspruch 1,

worin
$-A^1-Z^1-A^2-$ ein Strukturelement ausgewählt aus der Gruppe der Formeln 1 bis 4

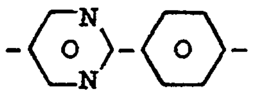 , 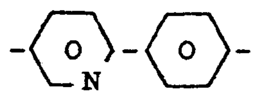 , 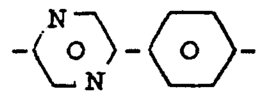

1 2 3

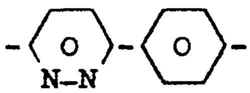

4

bedeutet.

4. Stickstoffhaltige Heterocyclen nach einem der Ansprüche 1 bis 3 der Formel I032, I048, I066, I083 und I084

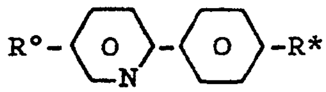 I032

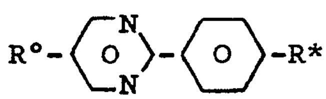 I048

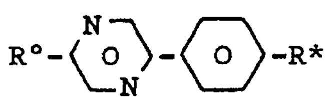 I066

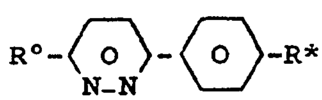 I083

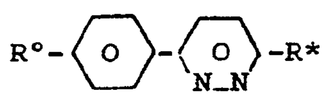 I084

worin
R° geradkettiges Alkyl oder Alkoxy mit jeweils 2 bis 12 C-Atomen bedeutet,
und
R* eine der für den optisch aktiven organischen Rest in Anspruch 1 angegebenen Bedeutungen hat.

5. Verwendung der Verbindungen der Formel I nach einem der Ansprüche 1 bis 4 als Komponenten ferroelektrischer flüssigkristalliner Phasen.

6. Ferroelektrische flüssigkristalline Phase mit mindestens zwei flüssigkristallinen Komponenten, dadurch gekennzeichnet, daß mindestens eine Komponente eine Verbindung der Formel I nach Anspruch 1 ist.

7. Chiral getiltete smektische flüssigkristalline Phasen mit mindestens zwei flüssigkristallinen Komponenten, dadurch gekennzeichnet, daß mindestens eine Komponente eine Verbindung der Formel I ist, worin $R^1$, $R^2$, $R^3$, R, $A^1$, $A^2$, $A^3$, $A^4$, $Z^1$, $Z^2$, $Z^3$, X, Q, Y und p die in Anspruch 1 gegebene Bedeutung besitzen, und daß diese Phase mindestens eine andere Komponente mit betragsmäßig kleiner positiver oder negativer dielektrischer Anisotropie ausgewählt aus den Verbindungen der Teilformeln Va bis Vp enthält:

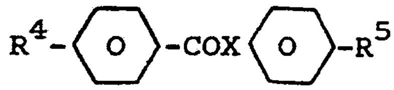

Va

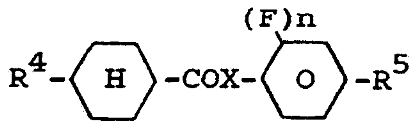

Vb

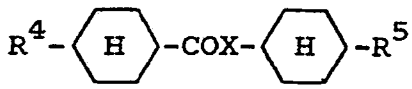

Vc

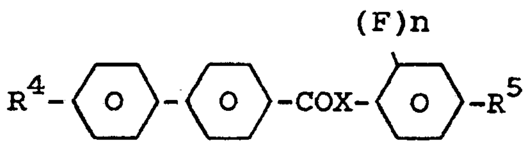

Vd

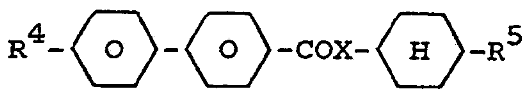

Ve

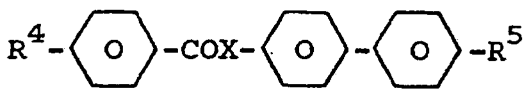

Vf

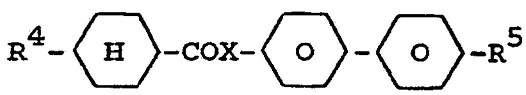

Vg

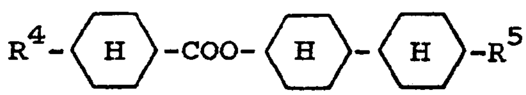

Vh

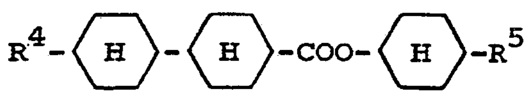

Vi

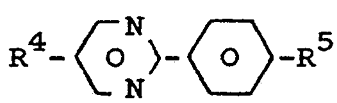

Vj

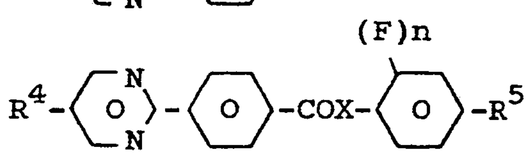

Vk

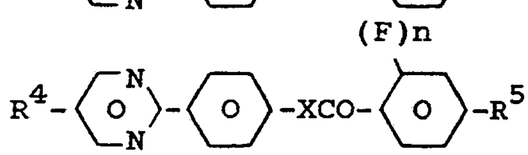

Vl

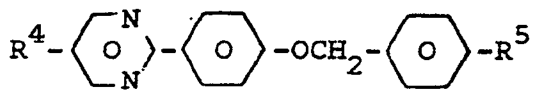 Vm

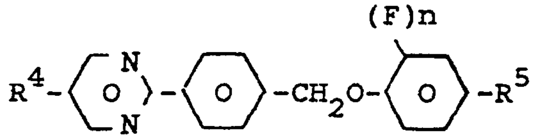 Vn

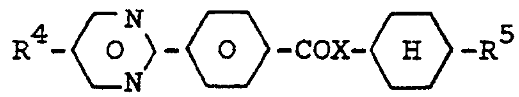 Vo

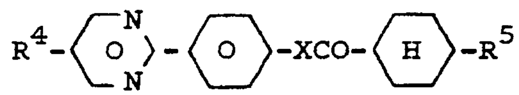 Vp

worin

$R^4$ und $R^5$ jeweils geradkettiges Alkyl, Alkoxy, Alkanoyloxy oder Alkoxycarbonyl mit jeweils 3 bis 12 C-Atomen,

X O, und

n 0 oder 1 bedeuten.

8. Chiral getiltete smektische flüssigkristalline Phase nach Anspruch 7, dadurch gekennzeichnet, daß mindestens eine Komponente der Verbindung der Formel I ist, worin $-A^1-Z^1-A^2-$

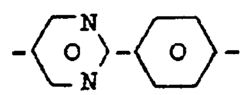

ist,

einer der Reste

$R^1$ und $R^2$ eine unsubstituierte Alkylgruppe mit 1 bis 15 C-Atomen, worin auch eine $CH_2$- Gruppe durch -O-, -COO- oder -OCO- ersetzt sein kann

und der andere Rest $R^1$ oder $R^2$

eine optisch aktive Gruppe der Formel

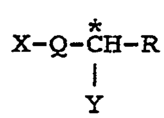

worin

X -CO-O-, -O-, -O-CO- oder eine Einfachbindung,

Q Alkylen mit 1 bis 5 C-Atomen, worin auch eine nicht mit X verknüpfte $CH_2$-Gruppe durch -O- ersetzt sein kann,

Y $CH_3$, CN oder Halogen, und

R geradkettiges Alkyl mit 1 bis 10 C-Atomen

bedeutet,

und daß

mindestens eine andere Komponente eine Verbindung der Formel II ist,

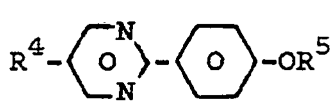 II

worin

$R^4$ und $R^5$ jeweils unabhängig voneinander n-Alkyl mit 5 bis 12 C-Atomen bedeutet.

9. Elektrooptisches Anzeigelement auf der Basis einer Flüssigkristallzelle, dadurch gekennzeichnet, daß es eine Phase nach einem der Ansprüche 6 bis 8 enthält.

**Patentansprüche Für folgenden Vertragsstaat: SE**

1. Stickstoffhaltige Heterocylen der Formel I

$$R^1\text{-}A^1\text{-}Z^1\text{-}A^2\text{-}R^2 \quad I$$

worin

einer der Reste

$R^1$ und $R^2$ H, eine unsubstituierte oder substituierte Alkylgruppe mit 1-15 C-Atomen, worin auch eine oder zwei nicht benachbarte $CH_2$-Gruppen durch mindestens eine Gruppierung aus der Gruppe -O-, -CO-, -O-CO-, -CO-O- und -CH=CH- ersetzt sein können, F, Cl, Br, -CN, -NCS oder $R^3\text{-}(A^3)_p\text{-}Z^2\text{-}$,

der andere Rest

$R^1$ oder $R^2$ ein optisch aktiver organischer Rest der Formel

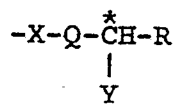

worin

X -CO-O-, -O-CO-, -O-CO-O-, -CO-, -O-, -S-, -CH=CH-, -CH=CH-COO- oder eine Einfachbindung,

Q Alkylen mit 1 bis 5 C-Atomen, worin auch eine nicht mit X verknüpfte $CH_2$-Gruppe durch -O-, -CO-, -O-CO-, -CO-O- oder -CH=CH- ersetzt sein kann, oder eine Einfachbindung,

Y CN, Halogen, Methyl oder Methoxy, und

R eine von Y verschiedene Alkylgruppe mit 1 bis 10 C-Atomen, worin auch eine oder zwei nicht benachbarte $CH_2$-Gruppen durch -O-, -CO-, -O-CO-, -CO-O- und/oder -CH=CH- ersetzt sein können,

bedeutet,

$A^1$ -A-, $-A^4\text{-}Z^3\text{-}A\text{-}$ oder $-A\text{-}Z^3\text{-}A^4\text{-}$,

A eine 1,4-Phenylengruppe, worin mindestens eine CH-Gruppe durch N ersetzt ist,

$A^2$, $A^3$ und $A^4$ jeweils unsubstituiertes oder durch ein oder zwei F- und/oder Cl-Atome und/oder $CH_3$-Gruppen und/oder CN-Gruppen substituiertes 1,4-Phenylen, worin auch eine oder zwei CH-Gruppen durch N-Atome ersetzt sein können, 1,4-Cyclohexylen, worin auch eine oder zwei nicht benachbarte $CH_2$-Gruppen durch O-Atome und/oder S-Atome ersetzt sein können, Piperidin-1,4-diyl, 1,4-Bicyclo(2,2,2)-octylen, unsubstituiertes oder durch CN unsubstituiertes Decahydronaphthalin-2,6-diyl oder 1,2,3,4-Tetrahydronaphthalin-2,6-diyl,

$Z^1$, $Z^2$ und $Z^3$ jeweils -CO-O-, -O-CO-, $-OCH_2-$, $-CH_2O-$, $-CH_2CH_2-$, substituiertes Ethylen oder eine Einfachbindung,

$R^3$ H, eine unsubstituierte oder substituierte Alkylgruppe mit 1-15 C-Atomen, worin auch eine oder zwei nicht benachbarte $CH_2$- Gruppen durch eine Gruppierung aus der Gruppe -O-, -CO-, -O-CO-, -CO-O und -CH=CH- ersetzt sein können, F, Cl, Br, -NCS oder -CN, und

P 1 oder 2

bedeutet, wobei für p = 2 die Gruppen $A^3$ gleich der voneinander verschieden sein können, mit den Maßgaben, daß

a) im Falle A = Pyrimidin-2,5-diyl einer der Reste $R^1$ und $R^2$ ein optisch aktiver organischer Rest und der andere Rest $R^1$ oder $R^2$ eine unsubstituierte oder substituierte Alkylgruppe mit 1-15 C-Atomen, worin auch eine oder zwei nicht benachbarte $CH_2$-Gruppen durch mindestens eine Gruppierung aus der Gruppe -O-, -CO-, -O-CO-, -CO-O- und -CH=CH- ersetzt sein können, bedeutet.

b) Verbindungen der Formel

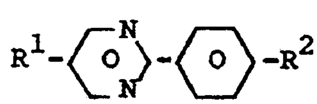

wobei einer der Reste $R^1$ und $R^2$ Alkyl, Alkoxy oder Alkoxycarbonyl mit 1 bis 15 C-Atomen und der andere Rest $R^1$ oder $R^2$ ein optisch aktiver Rest der Formel,

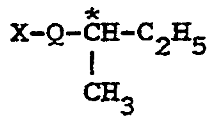

worin
X -CO-O-, -O-, -O-CO-, -CO- oder eine Einfachbindung und
Q Alkylen mit 1 bis 5 C-Atomen, worin auch eine nicht mit X verknüpfte $CH_2$-Gruppe durch -O- ersetzt sein kann,
bedeutet,
ausgenommen sind,
c) (S)-5-n-Octyl-2-[4-(3-chlorpentyloxy)phenyl] pyrimidin,
(S)-5-n-Undecyl-2-[4-(3-cyanopentyloxy)phenyl] pyrimidin, und
(S)-5-(n-Octyloxy)-2-[4-(2-methyloctyl)-phenyl] pyrimidin
ausgenommen sind,
2. Stickstoffhaltige Heterocyclen nach Anspruch 1 der Formel I1

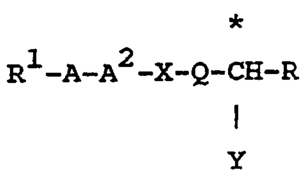

I1

worin
$R^1$ Alkyl oder Alkoxy mit 1-15 C-Atomen,
A Pyrimidin-2,5-diyl oder Pyridin-2,5-diyl,
$A^2$ unsubstituiertes oder durch ein Fluoratom substituiertes 1,4-Phenylen,
X -O-CO- oder -O-,
Q Alkylen mit 1 bis 5 C-Atomen oder eine Einfachbindung,
Y Halogen, und
R Alkyl mit 1 bis 10 C-Atomen
bedeuten.
3. Stickstoffhaltige Heterocyclen der Formel I nach Anspruch 1,
worin
$-A^1-Z^1-A^2-$ ein Strukturelement ausgewählt aus der Gruppe der Formeln 1 bis 4

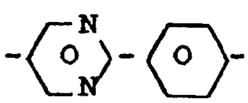 , 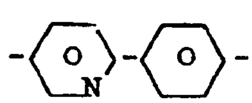 , 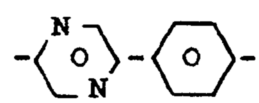

1 2 3

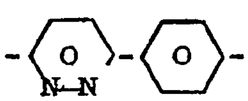

4

bedeutet.

4. Stickstoffhaltige Heterocyclen nach einem der Ansprüche 1 bis 3 der Formel I032, I048, I066, I083 und I084

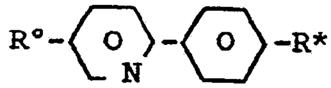

I032

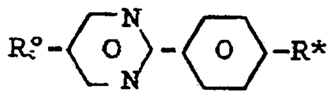

I048

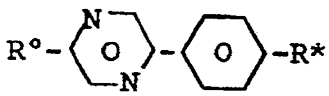

I066

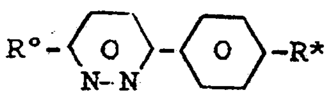

I083

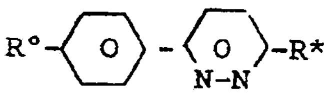

I084

worin

R° geradkettiges Alkyl oder Alkoxy mit jeweils 2 bis 12 C-Atomen bedeutet,

und

R* eine der für den optisch aktiven organischen Rest in Anspruch 1 angegebenen Bedeutungen hat.

5. Verwendung der Verbindungen der Formel I nach einem der Ansprüche 1 bis 4 als Komponenten ferroelektrischer flüssigkristalliner Phasen.

6. Ferroelektrische flüssigkristalline Phase mit mindestens zwei flüssigkristallinen Komponenten, dadurch gekennzeichnet, daß mindestens eine Komponente eine Verbindung der Formel I nach Anspruch 1 ist.

7. Chiral getiltete smektische flüssigkristalline Phasen mit mindestens zwei flüssigkristallinen Komponenten, dadurch gekennzeichnet, daß mindestens eine Komponente eine Verbindung der Formel I ist, worin $R^1$, $R^2$, $R^3$, R, $A^1$, $A^2$, $A^3$, $A^4$, $Z^1$, $Z^2$, $Z^3$, X, Q, Y und p die in Anspruch 1 gegebene Bedeutung besitzen, und daß diese Phase mindestens eine andere Komponente mit betragsmäßig kleiner positiver oder negativer dielektrischer Anisotropie ausgewählt aus den Verbindungen der Teilformeln Va bis Vp enthält:

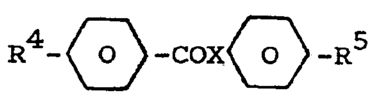

Va

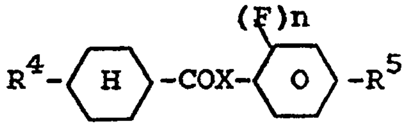 Vb

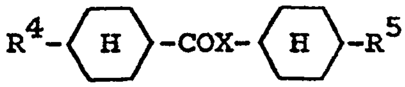 Vc

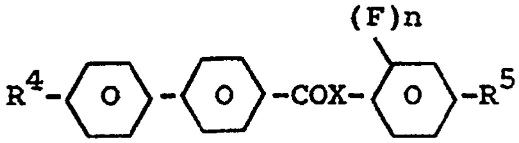 Vd

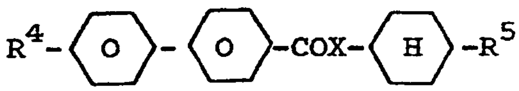 Ve

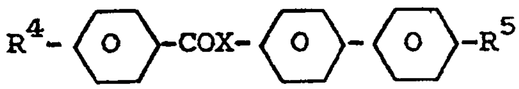 Vf

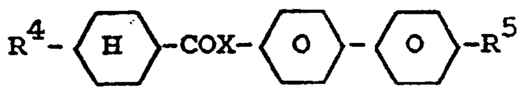 Vg

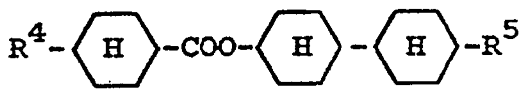 Vh

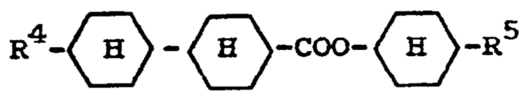 Vi

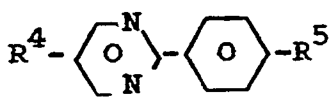 Vj

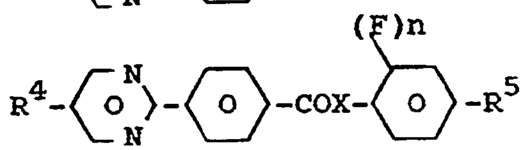 Vk

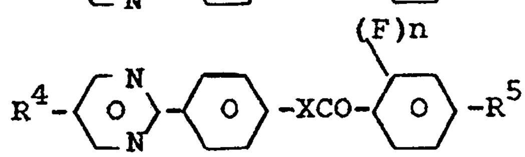 Vl

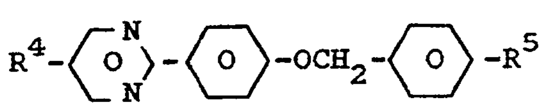 Vm

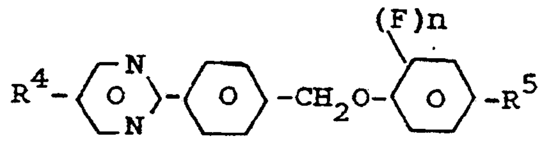

Vn

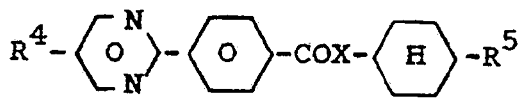

Vo

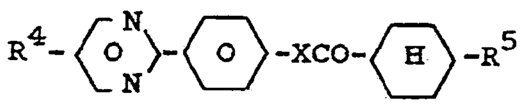

Vp

worin

$R^4$ und $R^5$ jeweils geradkettiges Alkyl, Alkoxy, Alkanoyloxy oder Alkoxycarbonyl mit jeweils 3 bis 12 C-Atomen,

X O, und

n 0 oder 1 bedeuten.

8. Chiral getiltete smektische flüssigkristalline Phase nach Anspruch 7, dadurch gekennzeichnet, daß mindestens eine Komponente der Verbindung der Formel I ist, worin $-A^1-Z^1-A^2-$

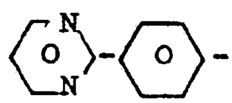

ist,

einer der Reste

$R^1$ und $R^2$ eine unsubstituierte Alkylgruppe mit 1 bis 15 C-Atomen, worin auch eine $CH_2$- Gruppe durch -O-, -COO- oder -OCO- ersetzt sein kann

und der andere Rest $R^1$ oder $R^2$

eine optisch aktive Gruppe der Formel

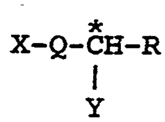

worin

X -CO-O-, -O-, -O-CO- oder eine Einfachbindung,

Q Alkylen mit 1 bis 5 C-Atomen, worin auch eine nicht mit X verknüpfte $CH_2$-Gruppe durch -O- ersetzt sein kann,

Y $CH_3$, CN oder Halogen, und

R geradkettiges Alkyl mit 1 bis 10 C-Atomen

bedeutet,

und daß

mindestens eine andere Komponente eine Verbindung der Formel II ist,

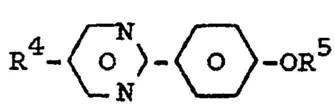

II

worin

$R^4$ und $R^5$ jeweils unabhängig voneinander n-Alkyl mit 5 bis 12 C-Atomen bedeutet.

9. Elektrooptisches Anzeigelement auf der Basis einer Flüssigkristallzelle, dadurch gekennzeichnet, daß es eine Phase nach einem der Ansprüche 6 bis 8 enthält.

**Patentansprüche für folgenden Vertragsstaat: FR**

1. Stickstoffhaltige Heterocylen der Formel I

$R^1$-$A^1$-$Z^1$-$A^2$-$R^2$    I

worin

einer der Reste

$R^1$ und $R^2$ H, eine unsubstituierte oder substituierte Alkylgruppe mit 1-15 C-Atomen, worin auch eine oder zwei nicht benacbbarte $CH_2$-Gruppen durch mindestens eine Gruppierung aus der Gruppe -O-, -CO-, -O-CO-, -CO-O- und -CH=CH- ersetzt sein können, F, Cl, Br, -CN, -NCS oder $R^3$-$(A^3)_p$-$Z^2$-,

der andere Rest

$R^1$ oder $R^2$ ein optisch aktiver organischer Rest der Formel

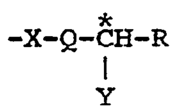

worin

X -CO-O-, -O-CO-, -O-CO-O-, -CO-, -O-, -S-, -CH=CH-, -CH=CH-COO- oder eine Einfachbindung,

Q Alkylen mit 1 bis 5 C-Atomen, worin auch eine nicht mit X verknüpfte $CH_2$-Gruppe durch -O-, -CO-, -O-CO-, -CO-O- oder -CH=CH- ersetzt sein kann, oder eine Einfachbindung,

Y CN, Halogen, Methyl oder Methoxy, und

R eine von Y verschiedene Alkylgruppe mit 1 bis 10 C-Atomen, worin auch eine oder zwei nicht benachbarte $CH_2$-Gruppen durch -O-, -CO-, -O-CO-, -CO-O- und/oder -CH=CH- ersetzt sein können,

bedeutet,

$A^1$ -A-, -$A^4$-$2^3$-A- oder -A-$Z^3$-$A^4$-,

A eine 1,4-Phenylengruppe, worin mindestens eine CH-Gruppe durch N ersetzt ist,

$A^2$, $A^3$ und $A^4$ jeweils unsubstituiertes oder durch ein oder zwei F- und/oder Cl-Atome und/oder $CH_3$-Gruppen und/oder CN-Gruppen substituiertes 1,4-Phenylen, worin auch eine oder zwei CH-Gruppen durch N-Atome ersetzt sein können, 1,4-Cyclohexylen, worin auch eine oder zwei nicht benachbarte $CH_2$-Gruppen durch O-Atome und/oder S-Atome ersetzt sein können, Piperidin-1,4-diyl, 1,4-Bicyclo(2,2,2)-octylen, unsubstituiertes oder durch CN unsubstituiertes Decahydronaphthalin-2,6-diyl oder 1,2,3,4-Tetrahydronaphthalin-2,6-diyl,

$Z^1$, $Z^2$ und $Z^3$ jeweils -CO-O-, -O-CO-, -$OCH_2$-, -$CH_2O$-, -$CH_2CH_2$-, substituiertes Ethylen oder eine Einfachbindung,

$R^3$ H, eine unsubstituierte oder substituierte Alkylgruppe mit 1-15 C-Atomen, worin auch eine oder zwei nicht benachbarte $CH_2$- Gruppen durch eine Gruppierung aus der Gruppe -O-, -CO-, -O-CO-, -CO-O und -CH=CH- ersetzt sein können, F, Cl, Br, -NCS oder -CN, und

P 1 oder 2

bedeutet, wobei für p = 2 die Gruppen $A^3$ gleich der voneinander verschieden sein können, mit den Maßgaben, daß

a) im Falle A = Pyrimidin-2,5-diyl einer der Reste $R^1$ und $R^2$ ein optisch aktiver organischer Rest und der andere Rest $R^1$ oder $R^2$ eine unsubstituierte oder substituierte Alkylgruppe mit 1-15 C-Atomen, worin auch eine oder zwei nicht benachbarte $CH_2$-Gruppen durch mindestens eine Gruppierung aus der Gruppe -O-, -CO-, -O-CO-, -CO-O- und -CH=CH- ersetzt sein können, bedeutet.

b) Verbindungen der Formel

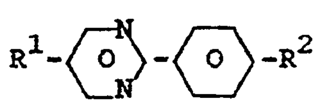

wobei einer der Reste $R^1$ und $R^2$ Alkyl, Alkoxy oder Alkoxycarbonyl mit 1 bis 15 C-Atomen und der andere Rest $R^1$ oder $R^2$ ein optisch aktiver Rest der Formel,

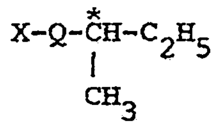

worin
X -CO-O-, -O-, -O-CO-, -CO- oder eine Einfachbindung und
Q Alkylen mit 1 bis 5 C-Atomen, worin auch eine nicht mit X verknüpfte $CH_2$-Gruppe durch -O- ersetzt sein kann,
bedeutet,
ausgenommen sind,
c) (S)-5-n-Octyl-2-[4-(3-chlorpentyloxy)phenyl] pyrimidin,
(S)-5-n-Undecyl-2-[4-(3-cyanopentyloxy)phenyl] pyrimidin, und
(S)-5-(n-Octyloxy)-2-[4-(2-methyloctyl)-phenyl] pyrimidin
d) 4-[5-(4'-Butylphenyl)pyrimidin-2-yl]benzoesäure-(2'-dodecyloxypropyl-ester) und
4-(5-Heptylpyrimidin-2-yl)benzoesäure-(2'-decyloxypropyl-ester)
ausgenommen sind, und
e) die Verbindungen der Formel

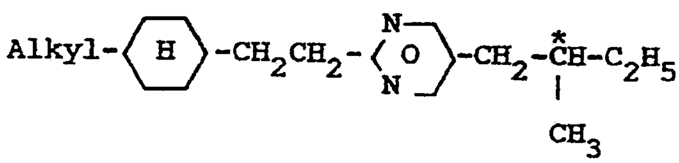

worin
Alkyl Alkyl mit 1 bis 12 C-Atomen bedeutet,
ausgenommen sind.
2. Stickstoffhaltige Heterocyclen nach Anspruch 1 der Formel I1

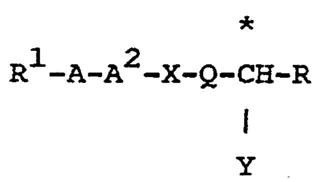

I1

worin
$R^1$ Alkyl oder Alkoxy mit 1-15 C-Atomen,
A Pyrimidin-2,5-diyl oder Pyridin-2,5-diyl,
$A^2$ unsubstituiertes oder durch ein Fluoratom substituiertes 1,4-Phenylen,
X -O-CO- oder -O-,
Q Alkylen mit 1 bis 5 C-Atomen oder eine Einfachbindung,
Y Halogen, und
R Alkyl mit 1 bis 10 C-Atomen
bedeuten.
3. Stickstoffhaltige Heterocyclen der Formel I nach Anspruch 1,
worin
$-A^1-Z^1-A^2-$ ein Strukturelement ausgewählt aus der Gruppe der Formeln 1 bis 4

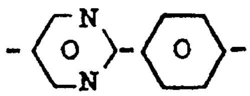 , 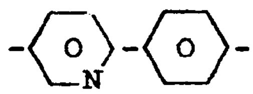 , 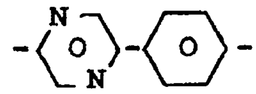

1 2 3

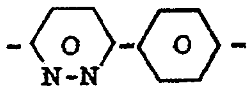

4

bedeutet.

4. Stickstoffhaltige Heterocyclen nach einem der Ansprüche 1 bis 3 der Formel I032, I048, I066, I083 und I084

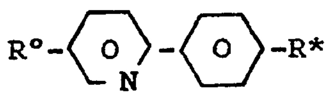 I032

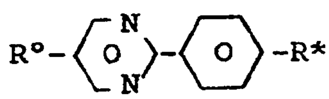 I048

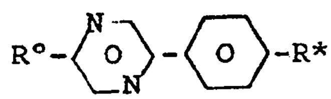 I066

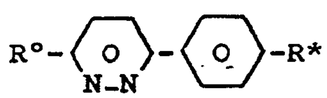 I083

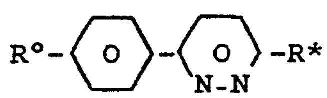 I084

worin

R° geradkettiges Alkyl oder Alkoxy mit jeweils 2 bis 12 C-Atomen bedeutet,

und

R* eine der für den optisch aktiven organischen Rest in Anspruch 1 angegebenen Bedeutungen hat.

5. Verwendung der Verbindungen der Formel I nach einem der Ansprüche 1 bis 4 als Komponenten ferroelektrischer flüssigkristalliner Phasen.

6. Ferroelektrische flüssigkristalline Phase mit mindestens zwei flüssigkristallinen Komponenten, dadurch gekennzeichnet, daß mindestens eine Komponente eine Verbindung der Formel I nach Anspruch 1 ist.

7. Chiral getiltete smektische flüssigkristalline Phasen mit mindestens zwei flüssigkristallinen Komponenten, dadurch gekennzeichnet, daß mindestens eine Komponente eine Verbindung der Formel I ist, worin $R^1$, $R^2$, $R^3$, R, $A^1$, $A^2$, $A^3$, $A^4$, $Z^1$, $Z^2$, $Z^3$, X, Q, Y und p die in Anspruch 1 gegebene Bedeutung besitzen, und daß diese Phase mindestens eine andere Komponente mit betragsmäßig kleiner positiver oder negativer dielektrischer Anisotropie ausgewählt aus den Verbindungen der Teilformeln Va bis Vp enthält:

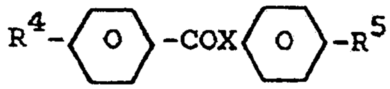 Va

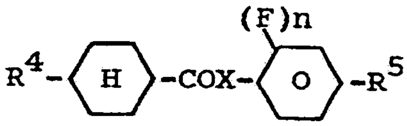 Vb

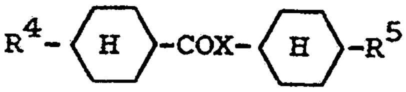 Vc

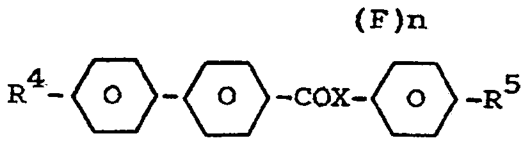 Vd

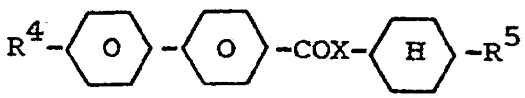 Ve

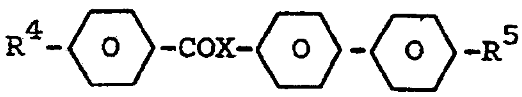 Vf

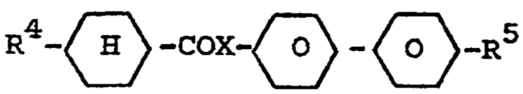 Vg

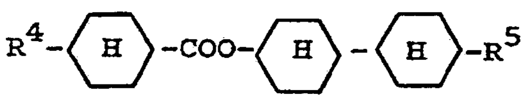 Vh

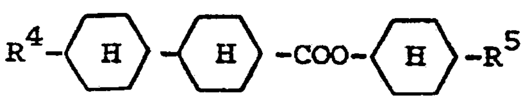 Vi

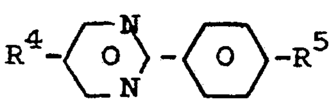 Vj

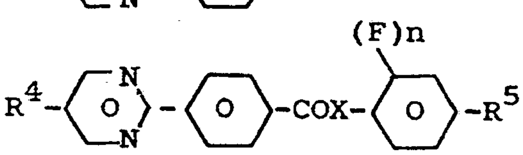 Vk

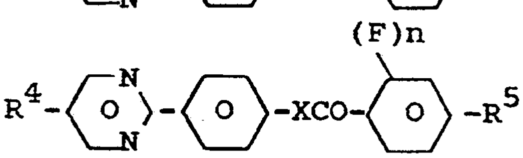 Vl

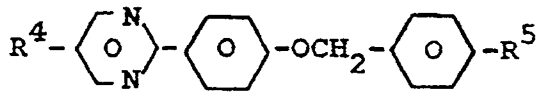

Vm

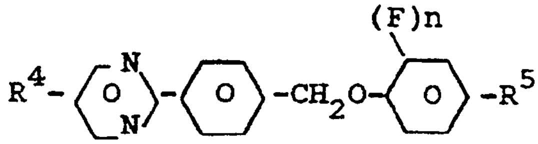

Vn

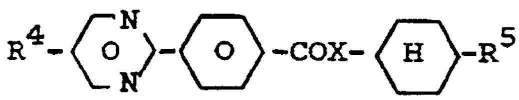

Vo

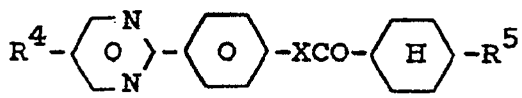

Vp

worin

$R^4$ und $R^5$ jeweils geradkettiges Alkyl, Alkoxy, Alkanoyloxy oder Alkoxycarbonyl mit jeweils 3 bis 12 C-Atomen,

X O, und

n 0 oder 1 bedeuten.

8. Chiral getiltete smektische flüssigkristalline Phase nach Anspruch 7, dadurch gekennzeichnet, daß mindestens eine Komponente der Verbindung der Formel I ist, worin $-A^1-Z^1-A^2-$

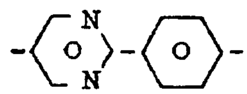

einer der Reste

$R^1$ und $R^2$ eine unsubstituierte Alkylgruppe mit 1 bis 15 C-Atomen, worin auch eine $CH_2$Gruppe durch -O-, -COO- oder -OCO- ersetzt sein kann und der andere Rest $R^1$ oder $R^2$

eine optisch aktive Gruppe der Formel

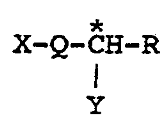

worin

X -CO-O-, -O-, -O-CO- oder eine Einfachbindung,

Q Alkylen mit 1 bis 5 C-Atomen, worin auch eine nicht mit X verknüpfte $CH_2$-Gruppe durch -O- ersetzt sein kann,

Y $CH_3$, CN oder Halogen, und

R geradkettiges Alkyl mit 1 bis 10 C-Atomen

bedeutet,

und daß

mindestens eine andere Komponente eine Verbindung der Formel II ist,

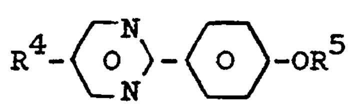

II

worin

$R^4$ und $R^5$ jeweils unabhängig voneinander n-Alkyl mit 5 bis 12 C-Atomen bedeutet.

9. Elektrooptisches Anzeigelement auf der Basis einer Flüssigkristallzelle, dadurch gekennzeichnet, daß

es eine Phase nach einem der Ansprüche 6 bis 8 enthält.

**Patentansprüche für folgenden Vertragsstaat: DE, GB, CH, LI**

1. Stickstoffhaltige Heterocylen der Formel I

$$R^1\text{-}A^1\text{-}Z^1\text{-}A^2\text{-}R^2 \quad I$$

worin

einer der Reste

$R^1$ und $R^2$ H, eine unsubstituierte oder substituierte Alkylgruppe mit 1-15 C-Atomen, worin auch eine oder zwei nicht benachbarte $CH_2$-Gruppen durch mindestens eine Gruppierung aus der Gruppe -O-, -CO-, -O-CO-, -CO-O- und -CH=CH- ersetzt sein können, F, Cl, Br, -CN, -NCS oder $R^3\text{-}(A^3)_p\text{-}Z^2\text{-}$,

der andere Rest

$R^1$ oder $R^2$ ein optisch aktiver organischer Rest der Formel

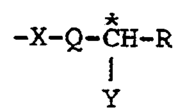

worin

X -CO-O-, -O-CO-, -O-CO-O-, -CO-, -O-, -S-, -CH=CH-, -CH=CH-COO- oder eine Einfachbindung,

Q Alkylen mit 1 bis 5 C-Atomen, worin auch eine nicht mit X verknüpfte $CH_2$-Gruppe durch -O-, -CO-, -O-CO-, -CO-O- oder -CH=CH- ersetzt sein kann, oder eine Einfachbindung,

Y CN, Halogen, Methyl oder Methoxy, und

R eine von Y verschiedene Alkylgruppe mit 1 bis 10 C-Atomen, worin auch eine oder zwei nicht benachbarte $CH_2$-Gruppen durch -O-, -CO-, -O-CO-, -CO-O- und/oder -CH=CH- ersetzt sein können,

bedeutet,

$A^1$ -A-, $-A^4\text{-}Z^3\text{-}A\text{-}$ oder $-A\text{-}Z^3\text{-}A^4\text{-}$,

A eine 1,4-Phenylengruppe, worin mindestens eine CH-Gruppe durch N ersetzt ist,

$A^2$, $A^3$ und $A^4$ jeweils unsubstituiertes oder durch ein oder zwei F- und/oder Cl-Atome und/oder $CH_3$-Gruppen und/oder CN-Gruppen substituiertes 1,4-Phenylen, worin auch eine oder zwei CH-Gruppen durch N-Atome ersetzt sein können, 1,4-Cyclohexylen, worin auch eine oder zwei nicht benachbarte $CH_2$-Gruppen durch O-Atome und/oder S-Atome ersetzt sein können, Piperidin-1,4-diyl, 1,4-Bicyclo(2,2,2)-octylen, unsubstituiertes oder durch CN unsubstituiertes Decahydronaphthalin-2,6-diyl oder 1,2,3,4-Tetrahydronaphthalin-2,6-diyl,

$Z^1$, $Z^2$ und $Z^3$ jeweils -CO-O-, -O-CO-, $-OCH_2-$, $-CH_2O-$, $-CH_2CH_2-$, substituiertes Ethylen oder eine Einfachbindung,

$R^3$ H, eine unsubstituierte oder substituierte Alkylgruppe mit 1-15 C-Atomen, worin auch eine oder zwei nicht benachbarte $CH_2$- Gruppen durch eine Gruppierung aus der Gruppe -O-, -CO-, -O-CO-, -CO-O und -CH=CH- ersetzt sein können, F, Cl, Br, -NCS oder -CN, und

P 1 oder 2

bedeutet, wobei für p = 2 die Gruppen $A^3$ gleich der voneinander verschieden sein können, mit den Maßgaben, daß

a) im Falle A = Pyrimidin-2,5-diyl einer der Reste $R^1$ und $R^2$ ein optisch aktiver organischer Rest und der andere Rest $R^1$ oder $R^2$ eine unsubstituierte oder substituierte Alkylgruppe mit 1-15 C-Atomen, worin auch eine oder zwei nicht benachbarte $CH_2$-Gruppen durch mindestens eine Gruppierung aus der Gruppe -O-, -CO-, -O-CO-, -CO-O- und -CH=CH- ersetzt sein können, bedeutet.

b) Verbindungen der Formel

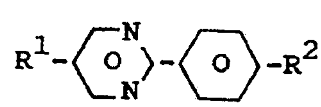

wobei einer der Reste $R^1$ und $R^2$ Alkyl, Alkoxy oder Alkoxycarbonyl mit 1 bis 15 C-Atomen und der andere Rest $R^1$ oder $R^2$ ein optisch aktiver Rest der Formel,

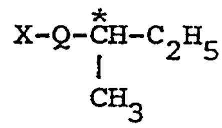

worin
X -CO-O-, -O-, -O-CO-, -CO- oder eine Einfachbindung und
Q Alkylen mit 1 bis 5 C-Atomen, worin auch eine nicht mit X verknüpfte $CH_2$-Gruppe durch -O- ersetzt sein kann,
bedeutet,
ausgenommen sind,
c) (S)-5-n-Octyl-2-[4-(3-chlorpentyloxy)phenyl] pyrimidin,
(S)-5-n-Undecyl-2-[4-(3-cyanopentyloxy)phenyl] pyrimidin, und
(S)-5-(n-Octyloxy)-2-[4-(2-methyloctyl)-phenyl] pyrimidin
ausgenommen sind,
d) 3-(2-Methylbutoxy)-6-(4-pentylphenyl)-pyridazin,
3-(2-Methylbutoxy)-6-(4-nonylphenyl)-pyridazin,
3-(2-Methylbutoxy)-6-(4-dodecylphenyl)-pyridazin,
3-(2-Methylbutoxy)-6-(4-pentadecylphenyl)-pyridazin,
3-(2-Methylbutoxy)-6-(4-octyloxyphenyl)-pyridazin,
3-(2-Methylbutoxy)-6-(4-decyloxyphenyl)-pyridazin,
3-(2-Methylbutoxy)-6-(4-tetradecyloxyphenyl)-pyridazin,
3-(1-Methylheptyloxy)-6-(4-pentylphenyl)-pyridazin,
3-(1-Methylheptyloxy)-6-(4-dodecylphenyl)-pyridazin,
3-(1-Methylheptyloxy)-6-(4-decyloxyphenyl)-pyridazin und
3-(1-Methylbutoxy)-6-(4-octyloxyphenyl)-pyridazin
ausgenommen sind,
e) 4-[5-(4′-Butylphenyl)pyrimidin-2-yl]benzoesäure-(2′-dodecyloxypropyl-ester) und 4-(5-Heptylpyrimidin-2-yl)benzoesäure-(2′-decyloxypropyl-ester)
ausgenommen sind, und
f) die Verbindungen der Formel

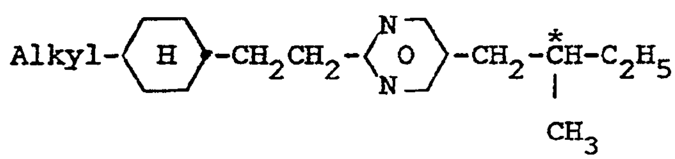

worin
Alkyl Alkyl mit 1 bis 12 C-Atomen bedeutet,
ausgenommen sind.
2. Stickstoffhaltige Heterocyclen nach Anspruch 1 der Formel I1

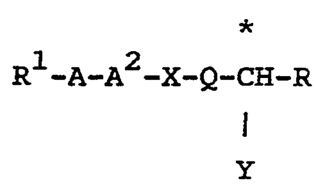

I1

worin
$R^1$ Alkyl oder Alkoxy mit 1-15 C-Atomen,
A Pyrimidin-2,5-diyl oder Pyridin-2,5-diyl,
$A^2$ unsubstituiertes oder durch ein Fluoratom substituiertes 1,4-Phenylen,
X -O-CO- oder -O-,
Q Alkylen mit 1 bis 5 C-Atomen oder eine Einfachbindung,
Y Halogen, und

R Alkyl mit 1 bis 10 C-Atomen bedeuten.

3. Stickstoffhaltige Heterocyclen der Formel I nach Anspruch 1, worin $-A^1-Z^1-A^2-$ ein Strukturelement ausgewählt aus der Gruppe der Formeln 1 bis 4

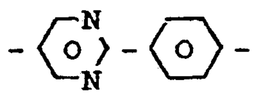
,
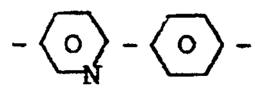
,
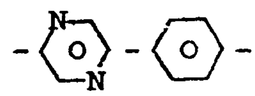

1 2 3

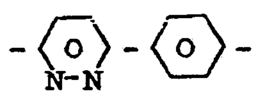

4

bedeutet.

4. Stickstoffhaltige Heterocyclen nach einem der Ansprüche 1 bis 3 der Formel I032, I048, I066, I083 und I084

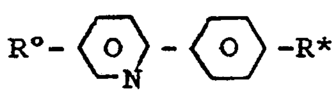
I032

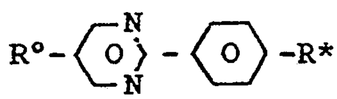
I048

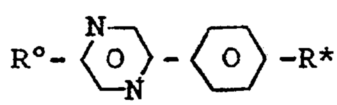
I066

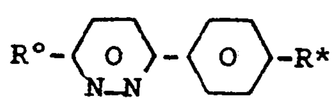
I083

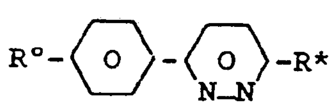
I084

worin
R° geradkettiges Alkyl oder Alkoxy mit jeweils 2 bis 12 C-Atomen bedeutet,
und
R* eine der für den optisch aktiven organischen Rest in Anspruch 1 angegebenen Bedeutungen hat.

5. Verwendung der Verbindungen der Formel I nach einem der Ansprüche 1 bis 4 als Komponenten ferroelektrischer flüssigkristalliner Phasen.

6. Ferroelektrische flüssigkristalline Phase mit mindestens zwei flüssigkristallinen Komponenten, dadurch gekennzeichnet, daß mindestens eine Komponente eine Verbindung der Formel I nach Anspruch 1 ist.

7. Chiral getiltete smektische flüssigkristalline Phasen mit mindestens zwei flüssigkristallinen Komponenten, dadurch gekennzeichnet, daß mindestens eine Komponente eine Verbindung der Formel I ist, worin $R^1$, $R^2$, $R^3$, R, $A^1$, $A^2$, $A^3$, $A^4$, $Z^1$, $Z^2$, $Z^3$, X, Q, Y und p die in Anspruch 1 gegebene Bedeutung besitzen, und daß

diese Phase mindestens eine andere Komponente mit betragsmäßig kleiner positiver oder negativer dielektrischer Anisotropie ausgewählt aus den Verbindungen der Teilformeln Va bis Vp enthält:

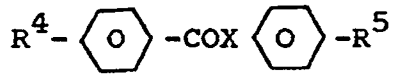 Va

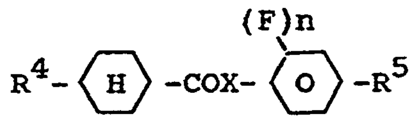 Vb

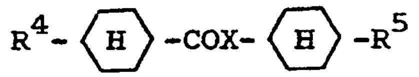 Vc

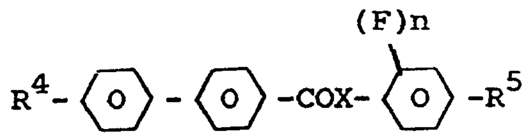 Vd

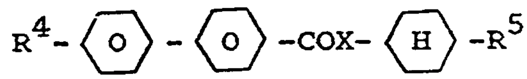 Ve

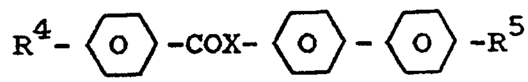 Vf

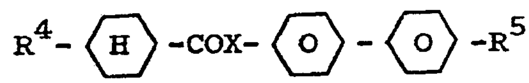 Vg

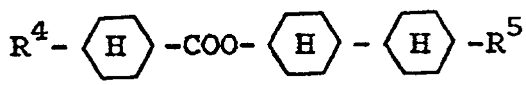 Vh

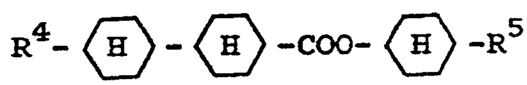 Vi

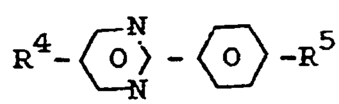 Vj

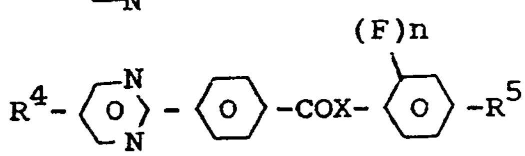 Vk

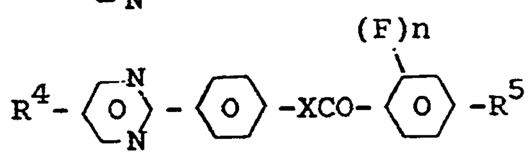 Vl

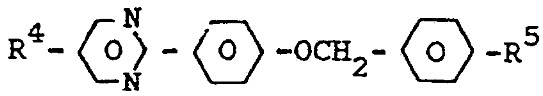 Vm

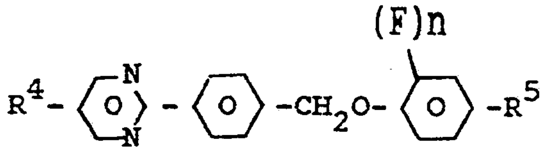 Vn

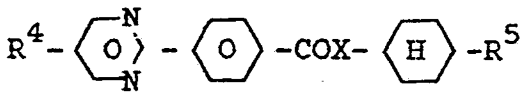 Vo

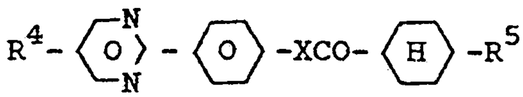 Vp

worin

$R^4$ und $R^5$ jeweils geradkettiges Alkyl, Alkoxy, Alkanoyloxy oder Alkoxycarbonyl mit jeweils 3 bis 12 C-Atomen,

X O, und

n 0 oder 1 bedeuten.

8. Chiral getiltete smektische flüssigkristalline Phase nach Anspruch 7, dadurch gekennzeichnet, daß mindestens eine Komponente der Verbindung der Formel I ist, Worin

$-A^1-Z^1-A^2-$ 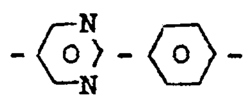 ist,

einer der Reste

$R^1$ und $R^2$ eine unsubstituierte Alkylgruppe mit 1 bis 15 C-Atomen, worin auch eine $CH_2$Gruppe durch -O-, -COO- oder -OCO- ersetzt sein kann

und der andere Rest $R^1$ oder $R^2$

eine optisch aktive Gruppe der Formel

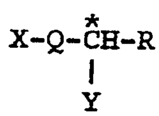

worin

X -CO-O-, -O-, -O-CO-, oder eine Einfachbindung,

Q Alkylen mit 1 bis 5 C-Atomen, worin auch eine nicht mit X verknüpfte $CH_2$-Gruppe durch -O- ersetzt sein kann,

Y $CH_3$, CN oder Halogen, und

R geradkettiges Alkyl mit 1 bis 10 C-Atomen

bedeutet,

und daß

mindestens eine andere Komponente eine Verbindung der Formel II ist,

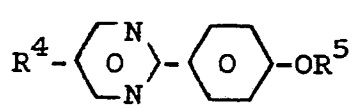 II

worin

$R^4$ und $R^5$ jeweils unabhängig voneinander n-Alkyl mit 5 bis 12 C-Atomen bedeutet.

9. Elektrooptisches Anzeigelement auf der Basis einer Flüssigkristallzelle, dadurch gekennzeichnet, daß es eine Phase nach einem der Ansprüche 6 bis 8 enthält.

## Claims

### Claims for the following Contracting State: AT

1. Ferroelectric liquid crystalline phase with at least two components, characterised in that at least one component is a nitrogen-containing heterocycle of the formula I

$$R^1\text{-}A^1\text{-}Z^1\text{-}A^2\text{-}R^2 \quad \text{I}$$

in which

one of the radicals $R^1$ and $R^2$ is H; an unsubstituted or substituted alkyl group of 1-15 carbon atoms in which one or two non-adjacent $CH_2$ groups can also be replaced by at least one member of the group -O-, -CO-, -O-CO-, -CO-O- and -CH=CH-; F, Cl, Br, -CN, -NCS or $R^3\text{-}(A^3)p\text{-}Z^2\text{-}$,

the other radical $R^1$ or $R^2$ is an optically active organic radical of the formula

$$\begin{array}{l}-X-Q-\overset{*}{C}H-R\\ \qquad\quad\;\, |\\ \qquad\quad\;\, Y\end{array}$$

in which

X is -CO-O-, -O-CO-, -O-CO-O-, -CO-, -O-, -S-, -CH=CH-, -CH=CH-COO- or a single bond,

Q is alkylene of 1-5 carbon atoms, in which a $CH_2$ group not linked to X can also be replaced by -O-, -CO-, -O-CO-, -CO-O- or -CH=CH-, or a single bond,

Y is CN, halogen, methyl or methoxy, and

R is an alkyl group of 1-10 carbon atoms which is different from Y and in which one or two non-adjacent $CH_2$ groups can also be replaced by -O-, -CO-, -O-CO-, -CO-O- and/or -CH=CH-,

$A^1$ is -A-, $-A^4\text{-}Z^3\text{-}A\text{-}$ or $-A\text{-}Z^3\text{-}A^4\text{-}$,

A is a 1,4-phenylene group in which at least one CH group is replaced by N,

$A^2$, $A^3$ and $A^4$ each are 1,4-phenylene which is unsubstituted or substituted by one or two F atoms and/or Cl atoms and/or $CH_3$ groups and/or CN groups, in which one or two CH groups can also be replaced by N atoms; 1,4-cyclohexylene in which one or two non-adjacent $CH_2$ groups can also be replaced by O atoms and/or S atoms; piperidine-1,4-diyl, 1,4-bicyclo(2,2,2)octylene; unsubstituted or CN-substituted decahydronaphthalene-2,6-diyl or 1,2,3,4-tetrahydronaphthalene-2,6-diyl,

$Z^1$, $Z^2$ and $Z^3$ each are -CO-O, -O-CO-, $-OCH_2-$, $-CH_2O-$, $-CH_2CH_2-$, substituted ethylene or a single bond,

$R^3$ is H; an unsubstituted or substituted alkyl group of 1-15 carbon atoms in which one or two non-adjacent $CH_2$ groups can also be replaced by a member of the group -O-, -CO-, -O-CO-, -CO-O and -CH=CH-; F, Cl, Br, -NCS or -CN, and

p is 1 or 2,

in which the groups $A^3$ can be the same or can differ from each other, when p = 2, with the provisos that when A = pyrimidine-2,5-diyl, one of the radicals $R^1$ and $R^2$ is an optically active organic radical and the other radical $R^1$ or $R^2$ is an unsubstituted or substituted alkyl group of 1-15 carbon atoms, in which one or two non-adjacent $CH_2$ groups can also be replaced by at least one member of the group -O-, -CO-, -O-CO-, -CO-O- and -CH=CH-.

2. Ferroelectric phase according to Claim 1, characterised in that at least one component is a nitrogen-containing heterocycle of the formula I1

$$\begin{array}{l}R^1-A-A^2-X-Q-\overset{*}{C}H-R\\ \qquad\qquad\qquad\qquad\;\; |\\ \qquad\qquad\qquad\qquad\;\; Y\end{array} \qquad \text{I1}$$

in which

$R^1$ is alkyl or alkoxy of 1-15 carbon atoms,

A is pyrimidine-2,5-diyl or pyridine-2,5-diyl,
$A^2$ is 1,4-phenylene which is unsubstituted or substituted by one fluorine atom,
X is -O-CO- or -O-,
Q is alkylene of 1 to 5 carbon atoms or a single bond,
Y is halogen, and
R is alkyl of 1 to 10 carbon atoms.

3. Ferroelectric phase according to Claim 1 or 2, characterised in that at least one component is a nitrogen-containing heterocycle of the formula I, in which
$-A^1-Z^1-A^2-$ is a structural element selected from the group of the formulae 1 to 4

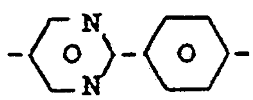 , 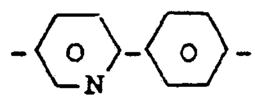 , 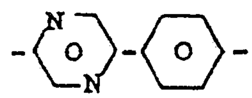

1 2 3

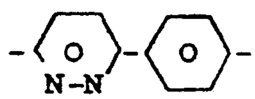

4

4. Ferroelectric phase according to one of Claims 1 to 3, characterised in that at least one component is a nitrogen-containing heterocycle of the formula I032, I048, I066, I083 and I084:

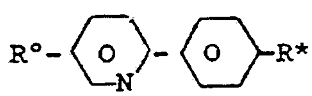 I032

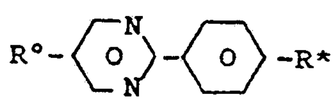 I048

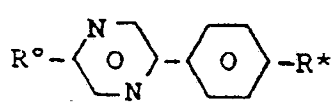 I066

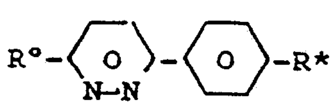 I083

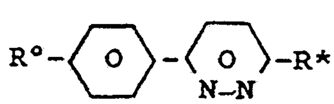 I084

in which
R° is straight-chain alkyl or alkoxy each having 2 to 12 carbon atoms, and

R* has one of the meanings given for the optically active organic radical in Claim 1.

5. Ferroelectric liquid-crystalline phase according to one of Claims 1 to 4, characterised in that at least one component is a compound of the formula I and in that this phase contains at least one other component which has a small positive or negative dielectric anisotropy in its absolute magnitude and is selected from the compounds of partial formulae Va to Vp:

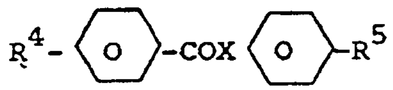 Va

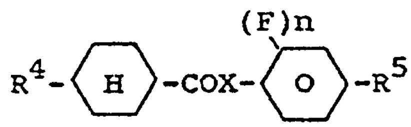 Vb

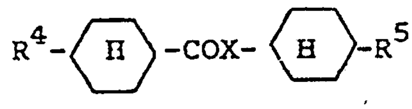 Vc

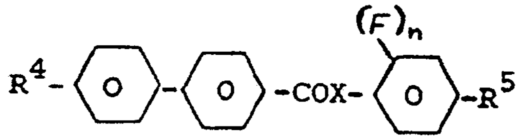
Vd

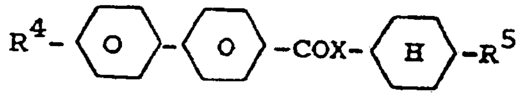
Ve

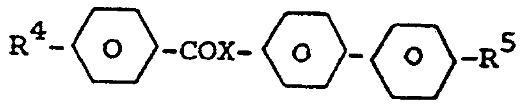
Vf

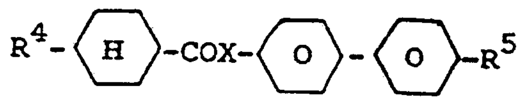
Vg

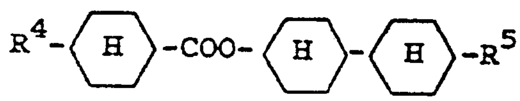
Vh

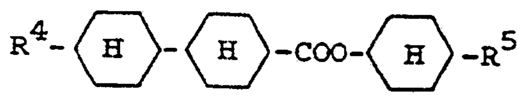
Vi

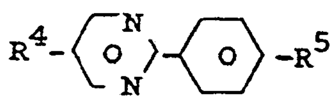
Vj

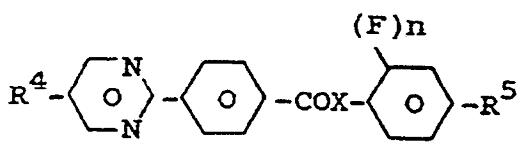
Vk

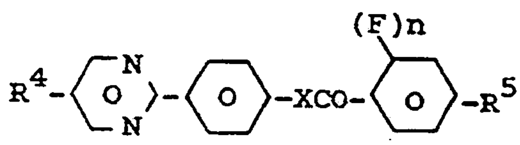
Vl

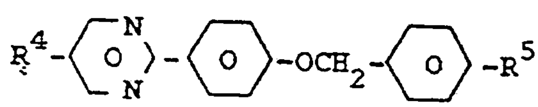
Vm

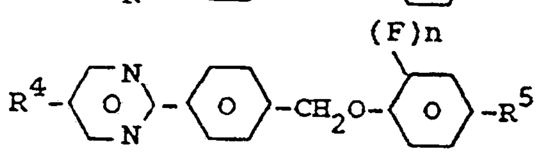
Vn

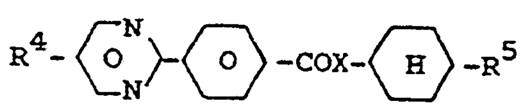
Vo

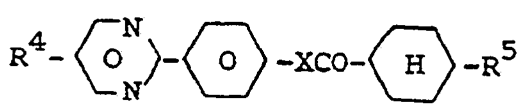
Vp

in which

$R^4$ and $R^5$ are each straight-chain alkyl, alkoxy, alkanoyloxy or alkoxycarbonyl each having 3 to 12 carbon atoms,

X is O, and

n is 0 or 1.

6. Ferroelectric phase according to Claim 5, characterised in that at least one component is the compound of the formula I, in which $-A^1-Z^1-A^2-$ is

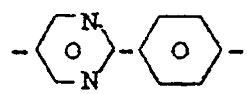

one of the radicals

$R^1$ and $R^2$ is an unsubstituted alkyl group of 1 to 15 carbon atoms, in which one $CH_2$ group can also be replaced by -O-, -COO- or -OCO-,

and the other radical $R^1$ or $R^2$ is an optically active group of the formula

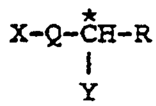

in which

X is -CO-O-, -O-, -O-CO-, or a single bond,

Q is alkylene of 1 to 5 carbon atoms, in which a $CH_2$ group not linked to X can also be replaced by -O-,

Y is $CH_3$, CN or halogen, and

R is straight-chain alkyl of 1 to 10 carbon atoms, and in that at least one other component is a compound of the formula II

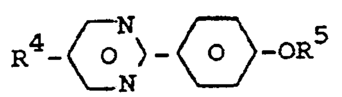

II

in which

$R^4$ and $R^5$, independently of one another, are each n-alkyl of 5 to 12 carbon atoms.

7. Electro-optical display element on the basis of a liquid crystal cell, characterised in that it contains a phase according to one of Claims 1 to 6.

**Claims for the following Contracting States: BE, IT**

1. Nitrogen-containing heterocycles of the formula I

$$R^1\text{-}A^1\text{-}Z^1\text{-}A^2\text{-}R^2 \quad I$$

in which

one of the radicals $R^1$ and $R^2$ is H; an unsubstituted or substituted alkyl group of 1-15 carbon atoms in which one or two non-adjacent $CH_2$ groups can also be replaced by at least one member of the group -O-, -CO-, -O-CO-, -CO-O- and -CH=CH-; F, Cl, Br, -CN, -NCS or $R^3\text{-}(A^3)_p\text{-}Z^2\text{-}$,

the other radical $R^1$ or $R^2$ is an optically active organic radical of the formula

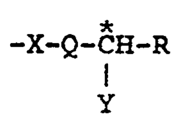

in which

X is -CO-O-, -O-CO-, -O-CO-O-, -CO-, -O-, -S-, -CH=CH-, -CH=CH-COO- or a single bond,

Q is alkylene of 1-5 carbon atoms, in which a $CH_2$ group not linked to X can also be replaced by -O-, -CO-, -O-CO-, -CO-O- or -CH=CH-, or a single bond,

Y is CN, halogen, methyl or methoxy, and

R is an alkyl group of 1-10 carbon atoms which is different from Y and in which one or two non-adjacent $CH_2$ groups can also be replaced by -O-, -CO-, -O-CO-, -CO-O- and/or -CH=CH-,

$A^1$ is -A-, $-A^4-Z^3-A-$ or $-A-Z^3-A^4-$,

A is a 1,4-phenylene group in which at least one CH group is replaced by N,

$A^2$, $A^3$ and $A^4$ each are 1,4-phenylene which is unsubstituted or substituted by one or two F atoms and/or Cl atoms and/or $CH_3$ groups and/or CN groups, in which one or two CH groups can also be replaced by N atoms; 1,4-cyclohexylene in which one or two non-adjacent $CH_2$ groups can also be replaced by O atoms and/or S atoms; piperidine-1,4-diyl, 1,4-bicyclo(2,2,2)octylene; unsubstituted or CN-substituted decahydronaphthalene-2,6-diyl or 1,2,3,4-tetrahydronaphthalene-2,6-diyl,

$Z^1$, $Z^2$ and $Z^3$ each are -CO-O, -O-CO-, $-OCH_2-$, $-CH_2O-$, $-CH_2CH_2-$, substituted ethylene or a single bond,

$R^3$ is H; an unsubstituted or substituted alkyl group of 1-15 carbon atoms in which one or two non-adjacent $CH_2$ groups can also be replaced by a member of the group -O-, -CO-, -O-CO-, -CO-O and -CH=CH-; F, Cl, Br, -NCS or -CN, and

p is 1 or 2,

in which the groups $A^3$ can be the same or can differ from each other, when p = 2, with the proviso that when A = pyrimidine-2,5-diyl, one of the radicals $R^1$ and $R^2$ is an optically active organic radical and the other radical $R^1$ or $R^2$ is an unsubstituted or substituted alkyl group of 1-15 carbon atoms, in which one or two non-adjacent $CH_2$ groups can also be replaced by at least one member of the group -O-, -CO-, -O-CO-, -CO-O- and -CH=CH-.

2. Nitrogen-containing heterocycles according to Claim 1 of the formula I1

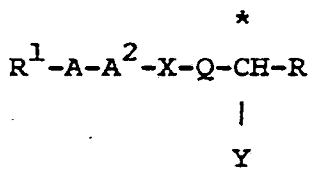

I1

in which

$R^1$ is alkyl or alkoxy of 1-15 carbon atoms,

A is pyrimidine-2,5-diyl or pyridine-2,5-diyl,

$A^2$ is 1,4-phenylene which is unsubstituted or substituted by one fluorine atom,

X is -O-CO- or -O-,

Q is alkylene of 1 to 5 carbon atoms or a single bond,

Y is halogen, and

R is alkyl of 1 to 10 carbon atoms.

3. Nitrogen-containing heterocycles of the formula I according to Claim 1,

in which

$-A^1-Z^1-A^2-$ is a structural element selected from the group of the formulae 1 to 4

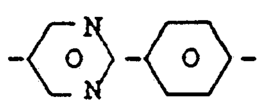 , 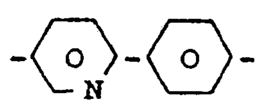 , 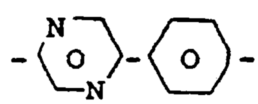

1 2 3

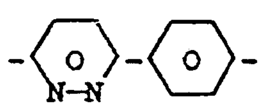

4

4. Nitrogen-containing heterocycles according to one of Claims 1 to 3 of the formula I032, I048, I066, I083 and I084

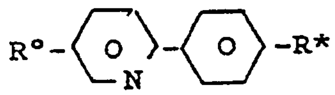

I032

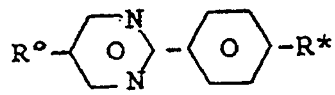

I048

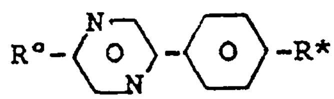

I066

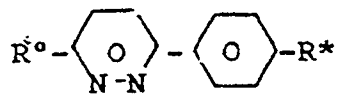

I083

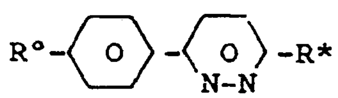

I084

in which

R° is straight-chain alkyl or alkoxy each having 2 to 12 carbon atoms, and

R* has one of the meanings given for the optically active organic radical in Claim 1.

5. Use of the compounds of the formula I according to one of Claims 1 to 4 as components of ferroelectric liquid crystalline phases.

6. Ferroelectric liquid crystalline phase with at least two liquid crystalline components, characterised in that at least one component is a compound of the formula I according to Claim 1.

7. Electro-optical display element on the basis of a liquid crystal cell, characterised in that it contains a phase according to Claim 6.

**Claims for the following Contracting State: NL**

1. Nitrogen-containing heterocycles of the formula I

$$R^1\text{-}A^1\text{-}Z^1\text{-}A^2\text{-}R^2 \quad I$$

in which

one of the radicals $R^1$ and $R^2$ is H; an unsubstituted or substituted alkyl group of 1-15 carbon atoms in which one or two non-adjacent $CH_2$ groups can also be replaced by at least one member of the group -O-, -CO-, -O-CO-, -CO-O- and -CH=CH-; F, Cl, Br, -CN, -NCS or $R^3$-$(A^3)_p$-$Z^2$-,

the other radical $R^1$ or $R^2$ is an optically active organic radical of the formula

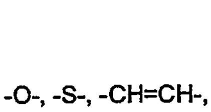

in which

X is -CO-O-, -O-CO-, -O-CO-O-, -CO-, -O-, -S-, -CH=CH-, -CH=CH-COO- or a single bond,

Q is alkylene of 1-5 carbon atoms, in which a $CH_2$ group not linked to X can also be replaced by -O-, -CO-, -O-CO-, -CO-O- or -CH=CH-, or a single bond,

Y is CN, halogen, methyl or methoxy, and

R is an alkyl group of 1-10 carbon atoms which is different from Y and in which one or two non-adjacent $CH_2$ groups can also be replaced by -O-, -CO-, -O-CO-, -CO-O- and/or -CH=CH-,

$A^1$ is -A-, $-A^4-Z^3-A-$ or $-A-Z^3-A^4-$,

A is a 1,4-phenylene group in which at least one CH group is replaced by N,

$A^2$, $A^3$ and $A^4$ each are 1,4-phenylene which is unsubstituted or substituted by one or two F atoms and/or Cl atoms and/or $CH_3$ groups and/or CN groups, in which one or two CH groups can also be replaced by N atoms; 1,4-cyclohexylene in which one or two non-adjacent $CH_2$ groups can also be replaced by O atoms and/or S atoms; piperidine-1,4-diyl, 1,4-bicyclo-(2,2,2)octylene; unsubstituted or CN-substituted decahydronaphthalene-2,6-diyl or 1,2,3,4-tetrahydronaphthalene-2,6-diyl,

$Z^1$, $Z^2$ and $Z^3$ each are -CO-O, -O-CO-, $-OCH_2-$, $-CH_2O-$, $-CH_2CH_2-$, substituted ethylene or a single bond,

$R^3$ is H; an unsubstituted or substituted alkyl group of 1-15 carbon atoms in which one or two non-adjacent $CH_2$ groups can also be replaced by a member of the group -O-, -CO-, -O-CO-, -CO-O and -CH=CH-; F, Cl, Br, -NCS or -CN, and

p is 1 or 2,

in which the groups $A^3$ can be the same or can differ from each other, when p = 2, <u>with the provisos</u> that

a) when A = pyrimidine-2,5-diyl, one of the radicals $R^1$ and $R^2$ is an optically active organic radical and the other radical $R^1$ or $R^2$ is an unsubstituted or substituted alkyl group of 1-15 carbon atoms, in which one or two non-adjacent $CH_2$ groups can also be replaced by at least one member of the group -O-, -CO-, -O-CO-, -CO-O- and -CH=CH-.

b) (2′-Dodecyloxypropyl) 4-[5-(4′-butylphenyl)-pyrimidin-2-yl]benzoate and (2′-decyloxypropyl) 4-(5-heptylpyrimidin-2-yl)benzoate are excepted,

2. Nitrogen-containing heterocycles according to Claim 1 of the formula I1

```
                      *
R1-A-A2-X-Q-CH-R                  I1
                      |
                      Y
```

in which

$R^1$ is alkyl or alkoxy of 1-15 carbon atoms,

A is pyrimidine-2,5-diyl or pyridine-2,5-diyl,

$A^2$ is 1,4-phenylene which is unsubstituted or substituted by one fluorine atom,

X is -O-CO- or -O-,

Q is alkylene of 1 to 5 carbon atoms or a single bond,

Y is halogen, and

R is alkyl of 1 to 10 carbon atoms.

3. Nitrogen-containing heterocycles of the formula I according to Claim 1,

in which

$-A^1-Z^1-A^2-$ is a structural element selected from the group of the formulae 1 to 4

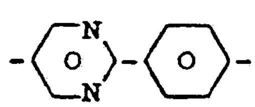 , 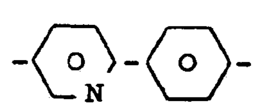 , 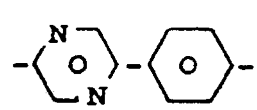

1 2 3

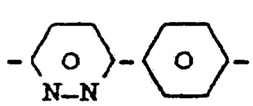

4

4. Nitrogen-containing heterocycles according to one of Claims 1 to 3 of the formula I032, I048, I066, I083 and I084

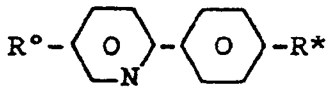

I032

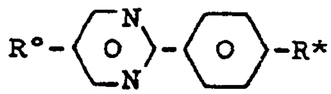

I048

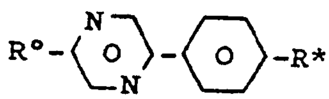

I066

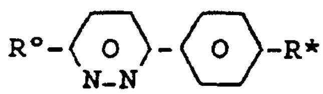

I083

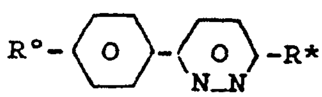

I084

in which

R° is straight-chain alkyl or alkoxy each having 2 to 12 carbon atoms, and

R* has one of the meanings given for the optically active organic radical in Claim 1.

5. Use of the compounds of the formula I according to one of Claims 1 to 4 as components of ferroelectric liquid crystalline phases.

6. Ferroelectric liquid crystalline phase with at least two liquid crystalline components, characterised in that at least one component is a compound of the formula I according to Claim 1.

7. Chirally tilted smectic liquid-crystalline phases containing at least two liquid crystalline components, characterised in that at least one component is a compound of the formula I, in which $R^1$, $R^2$, $R^3$, R, $A^1$, $A^2$, $A^3$, $A^4$, $Z^1$, $Z^2$, $Z^3$, X, Q, Y and p have the meaning given in Claim 1 and in that this phase contains at least one other component which has a small positive or negative dielectric anisotropy in its absolute magnitude and is selected from the compounds of partial formulae Va to Vp:

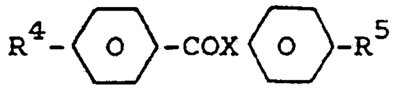 Va

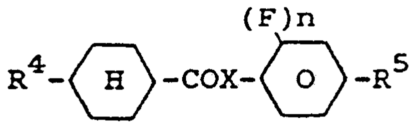 Vb

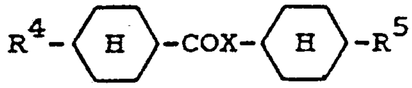 Vc

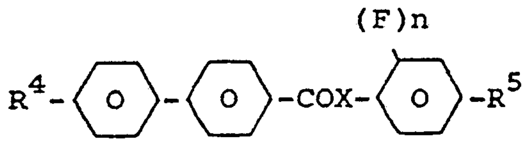 Vd

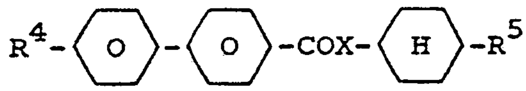 Ve

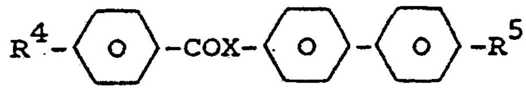 Vf

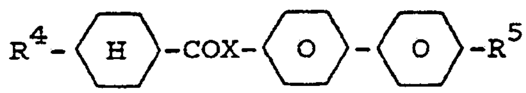 Vg

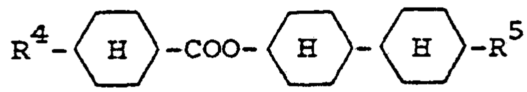 Vh

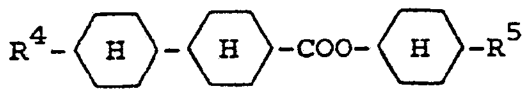 Vi

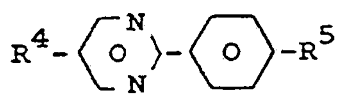 Vj

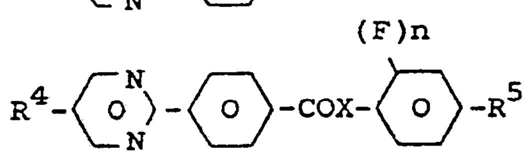 Vk

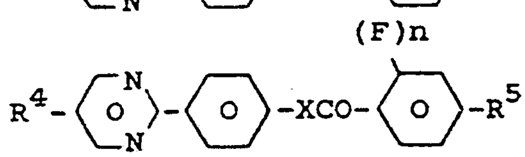 Vl

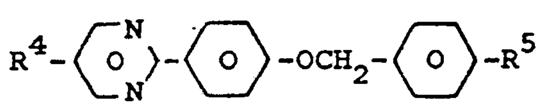 Vm

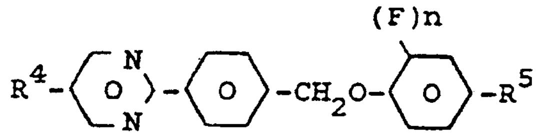

Vn

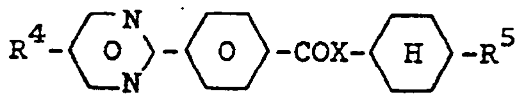

Vo

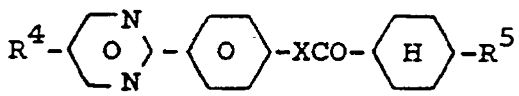

Vp

in which

$R^4$ and $R^5$ are each straight-chain alkyl, alkoxy, alkanoyloxy or alkoxycarbonyl each having 3 to 12 carbon atoms,

X is O, and

n is 0 or 1.

8. Chirally tilted smectic liquid-crystalline phase according to Claim 7, characterised in that at least one component is the compound of the formula I, in which $-A^1-Z^1-A^2-$ is

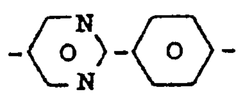

one of the radicals

$R^1$ and $R^2$ is an unsubstituted alkyl group of 1 to 15 carbon atoms, in which one $CH_2$ group can also be replaced by -O-, -COO- or -OCO-,

and the other radical $R^1$ or $R^2$ is an optically active group of the formula

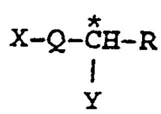

in which

X is -CO-O-, -O-, -O-CO-, or a single bond,

Q is alkylene of 1 to 5 carbon atoms, in which a $CH_2$ group not linked to X can also be replaced by -O-,

Y is $CH_3$, CN or halogen, and

R is straight-chain alkyl of 1 to 10 carbon atoms, and in that at least one other component is a compound of the formula II

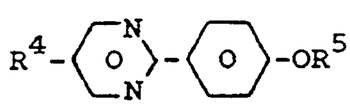

II

in which

$R^4$ and $R^5$, independently of one another, are each n-alkyl of 5 to 12 carbon atoms.

9. Electro-optical display element on the basis of a liquid crystal cell, characterised in that it contains a phase according to one of Claims 6 to 8.

**Claims for the following Contracting State: SE**

1. Nitrogen-containing heterocycles of the formula I

$$R^1\text{-}A^1\text{-}Z^1\text{-}A^2\text{-}R^2 \quad I$$

in which

one of the radicals $R^1$ and $R^2$ is H; an unsubstituted or substituted alkyl group of 1-15 carbon atoms in which one or two non-adjacent $CH_2$ groups can also be replaced by at least one member of the group -O-, -CO-, -O-CO-, -CO-O- and -CH=CH-; F, Cl, Br, -CN, -NCS or $R^3$-$(A^3)_p$-$Z^2$-,

the other radical $R^1$ or $R^2$ is an optically active organic radical of the formula

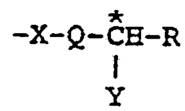

in which

X is -CO-O-, -O-CO-, -O-CO-O-, -CO-, -O-, -S-, -CH=CH-, -CH=CH-COO- or a single bond,

Q is alkylene of 1-5 carbon atoms, in which a $CH_2$ group not linked to X can also be replaced by -O-, -CO-, -O-CO-, -CO-O- or -CH=CH-, or a single bond,

Y is CN, halogen, methyl or methoxy, and

R is an alkyl group of 1-10 carbon atoms which is different from Y and in which one or two non-adjacent $CH_2$ groups can also be replaced by -O-, -CO-, -O-CO-, -CO-O- and/or -CH=CH-,

$A^1$ is -A-, -$A^4$-$Z^3$-A- or -A-$Z^3$-$A^4$-,

A is a 1,4-phenylene group in which at least one CH group is replaced by N,

$A^2$, $A^3$ and $A^4$ each are 1,4-phenylene which is unsubstituted or substituted by one or two F atoms and/or Cl atoms and/or $CH_3$ groups and/or CN groups, in which one or two CH groups can also be replaced by N atoms; 1,4-cyclohexylene in which one or two non-adjacent $CH_2$ groups can also be replaced by O atoms and/or S atoms; piperidine-1,4-diyl, 1,4-bicyclo-(2,2,2)octylene; unsubstituted or CN-substituted decahydronaphthalene-2,6-diyl or 1,2,3,4-tetrahydronaphthalene-2,6-diyl,

$Z^1$, $Z^2$ and $Z^3$ each are -CO-O, -O-CO-, -$OCH_2$-, -$CH_2O$-, -$CH_2CH_2$-, substituted ethylene or a single bond,

$R^3$ is H; an unsubstituted or substituted alkyl group of 1-15 carbon atoms in which one or two non-adjacent $CH_2$ groups can also be replaced by a member of the group -O-, -CO-, -O-CO-, -CO-O and -CH=CH-; F, Cl, Br, -NCS or -CN, and

p is 1 or 2,

in which the groups $A^3$ can be the same or can differ from each other, when p = 2, with the provisos that

a) when A = pyrimidine-2,5-diyl, one of the radicals $R^1$ and $R^2$ is an optically active organic radical and the other radical $R^1$ or $R^2$ is an unsubstituted or substituted alkyl group of 1-15 carbon atoms, in which one or two non-adjacent $CH_2$ groups can also be replaced by at least one member of the group -O-, -CO-, -O-CO-, -CO-O- and -CH=CH-.

b) Compounds of the formula

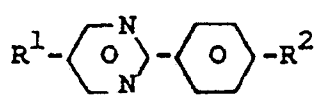

in which one of the radicals $R^1$ and $R^2$ is alkyl, alkoxy or alkoxycarbonyl of 1 to 15 carbon atoms and the other radical $R^1$ or $R^2$ is an optically active radical of the formula

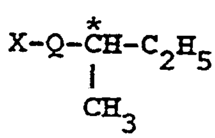

in which

X is -CO-O, -O-, -O-CO-, -CO- or a single bond and

Q is alkylene of 1 to 5 carbon atoms, in which a $CH_2$ group not linked to X can also be replaced by -O-, are excepted,

c) (S)-5-n-Octyl-2-[4-(3-chloropentyloxy)-phenyl]-pyrimidine

(S)-5-n-Undecyl-2-[4-(3-cyanopentyloxy)-phenyl]-pyrimidine and

(S)-5-(n-Octyloxy)-2-[4-(2-methyloctyl)-phenyl]-pyrimidine

are excepted,

2. Nitrogen-containing heterocycles according to Claim 1 of the formula I1

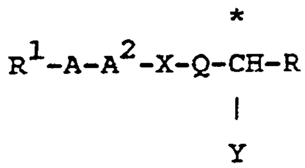

I1

in which

$R^1$ is alkyl or alkoxy of 1-15 carbon atoms,

A is pyrimidine-2,5-diyl or pyridine-2,5-diyl,

$A^2$ is 1,4-phenylene which is unsubstituted or substituted by one fluorine atom,

X is O-CO- or -O-,

Q is alkylene of 1 to 5 carbon atoms or a single bond,

Y is halogen, and

R is alkyl of 1 to 10 carbon atoms.

3. Nitrogen-containing heterocycles of the formula I according to Claim 1, in which

$-A^1-Z^1-A^2-$ is a structural element selected from the group of the formulae 1 to 4

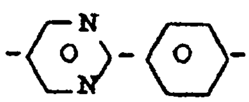 , 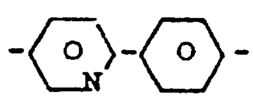 , 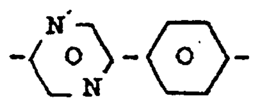

1 2 3

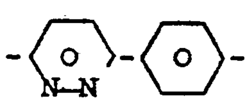

4

4. Nitrogen-containing heterocycles according to one of Claims 1 to 3 of the formula I032, I048, I066, I083 and I084

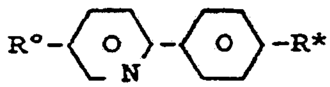

I032

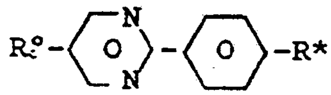

I048

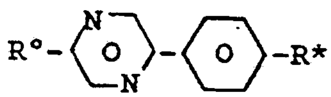

I066

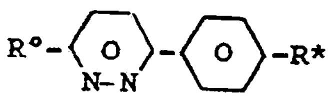

I083

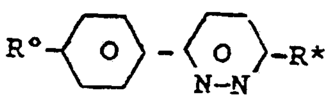

I084

in which

R° is straight-chain alkyl or alkoxy each having 2 to 12 carbon atoms, and

R* has one of the meanings given for the optically active organic radical in Claim 1.

5. Use of the compounds of the formula I according to one of Claims 1 to 4 as components of ferroelectric liquid crystalline phases.

6. Ferroelectric liquid crystalline phase with at least two liquid crystalline components, characterised in that at least one component is a compound of the formula I according to Claim 1.

7. Chirally tilted smectic liquid-crystalline phases containing at least two liquid crystalline components, characterised in that at least one component is a compound of the formula I, in which $R^1$, $R^2$, $R^3$, R, $A^1$, $A^2$, $A^3$, $A^4$, $Z^1$, $Z^2$, $Z^3$, X, Q, Y and p have the meaning given in Claim 1 and in that this phase contains at least one other component which has a small positive or negative dielectric anisotropy in its absolute magnitude and is selected from the compounds of partial formulae Va to Vp:

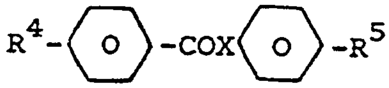

Va

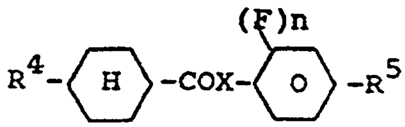

Vb

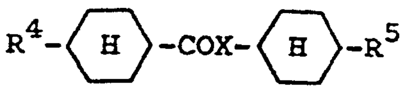

Vc

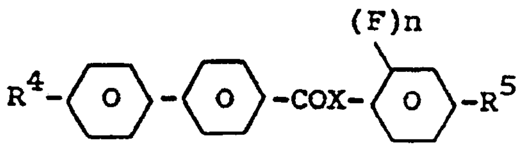

Vd

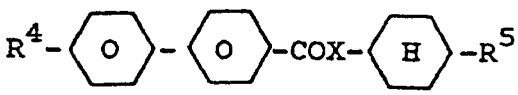

Ve

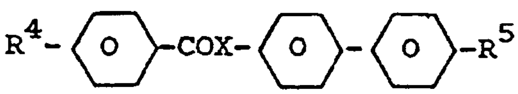

Vf

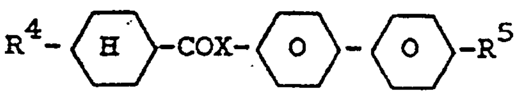

Vg

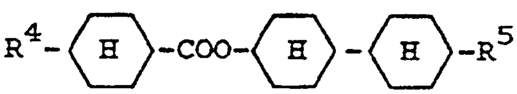

Vh

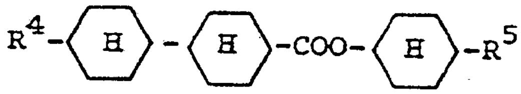

Vi

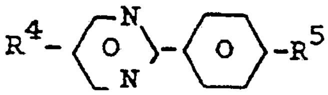

Vj

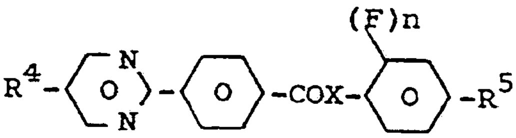

Vk

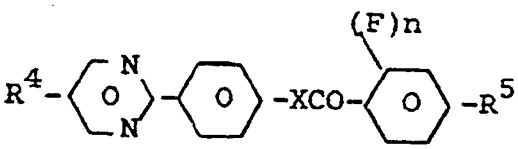

Vl

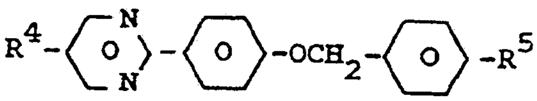

Vm

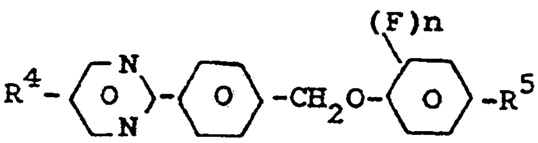

Vn

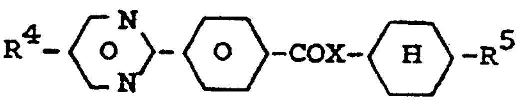

Vo

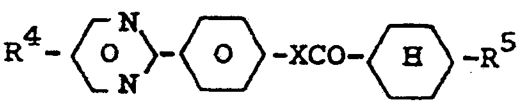

Vp

in which

$R^4$ and $R^5$ are each straight-chain alkyl, alkoxy, alkanoyloxy or alkoxycarbonyl each having 3 to 12 carbon atoms,

X is O, and

n is 0 or 1.

8. Chirally tilted smectic liquid-crystalline phase according to Claim 7, characterised in that at least one component is the compound of the formula I, in which $-A^1-Z^1-A^2-$ is

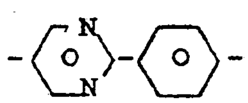

one of the radicals

$R^1$ and $R^2$ is an unsubstituted alkyl group of 1 to 15 carbon atoms, in which one $CH_2$ group can also be replaced by -O-, -COO- or -OCO-,

and the other radical $R^1$ or $R^2$ is an optically active group of the formula

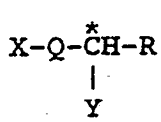

in which

X is -CO-O-, -O-, -O-CO-, or a single bond,

Q is alkylene of 1 to 5 carbon atoms, in which a $CH_2$ group not linked to X can also be replaced by -O-,

Y is $CH_3$, CN or halogen, and

R is straight-chain alkyl of 1 to 10 carbon atoms, and in that at least one other component is a compound of the formula II

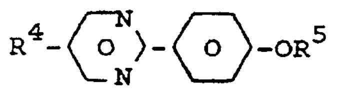

II

in which

$R^4$ and $R^5$, independently of one another, are each n-alkyl of 5 to 12 carbon atoms.

9. Electro-optical display element on the basis of a liquid crystal cell, characterised in that it contains a phase according to one of Claims 6 to 8.

**Claims for the following Contracting State: FR**

1. Nitrogen-containing heterocycles of the formula I

$$R^1\text{-}A^1\text{-}Z^1\text{-}A^2\text{-}R^2 \quad I$$

in which

one of the radicals $R^1$ and $R^2$ is H; an unsubstituted or substituted alkyl group of 1-15 carbon atoms in which one or two non-adjacent $CH_2$ groups can also be replaced by at least one member of the group -O-, -CO-, -O-CO-, -CO-O- and -CH=CH-; F, Cl, Br, -CN, -NCS or $R^3\text{-}(A^3)_p\text{-}Z^2\text{-}$,

the other radical $R^1$ or $R^2$ is an optically active organic radical of the formula

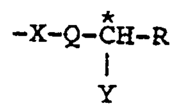

in which

X is -CO-O-, -O-CO-, -O-CO-O-, -CO-, -O-, -S-, -CH=CH-, -CH=CH-COO- or a single bond,

Q is alkylene of 1-5 carbon atoms, in which a $CH_2$ group not linked to X can also be replaced by -O-, -CO-, -O-CO-, -CO-O- or -CH=CH-, or a single bond,

Y is CN, halogen, methyl or methoxy, and

R is an alkyl group of 1-10 carbon atoms which is different from Y and in which one or two non-adjacent $CH_2$ groups can also be replaced by -O-, -CO-, -O-CO-, -CO-O- and/or -CH=CH-,

$A^1$ is -A-, $-A^4\text{-}Z^3\text{-}A\text{-}$ or $-A\text{-}Z^3\text{-}A^4\text{-}$,

A is a 1,4-phenylene group in which at least one CH group is replaced by N,

$A^2$, $A^3$ and $A^4$ each are 1,4-phenylene which is unsubstituted or substituted by one or two F atoms and/or Cl atoms and/or $CH_3$ groups and/or CN groups, in which one or two CH groups can also be replaced by N atoms; 1,4-cyclohexylene in which one or two non-adjacent $CH_2$ groups can also be replaced by O atoms and/or S atoms; piperidine-1,4-diyl, 1,4-bicyclo-(2,2,2)octylene; unsubstituted or CN-substituted decahydronaphthalene-2,6-diyl or 1,2,3,4-tetrahydronaphthalene-2,6-diyl,

$Z^1$, $Z^2$ and $Z^3$ each are -CO-O, -O-CO-, $-OCH_2-$, $-CH_2O-$, $-CH_2CH_2-$, substituted ethylene or a single bond,

$R^3$ is H; an unsubstituted or substituted alkyl group of 1-15 carbon atoms in which one or two non-adjacent $CH_2$ groups can also be replaced by a member of the group -O-, -CO-, -O-CO-, -CO-O and -CH=CH-; F, Cl, Br, -NCS or -CN, and

p is 1 or 2,

in which the groups $A^3$ can be the same or can differ from each other, when p = 2, <u>with the provisos</u> that

a) when A = pyrimidine-2,5-diyl, one of the radicals $R^1$ and $R^2$ is an optically active organic radical and the other radical $R^1$ or $R^2$ is an unsubstituted or substituted alkyl group of 1-15 carbon atoms, in which one or two non-adjacent $CH_2$ groups can also be replaced by at least one member of the group -O-, -CO-, -O-CO-, -CO-O- and -CH=CH-.

b) Compounds of the formula

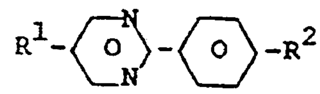

in which one of the radicals $R^1$ and $R^2$ is alkyl, alkoxy or alkoxycarbonyl of 1 to 15 carbon atoms and the other radical $R^1$ or $R^2$ is an optically active radical of the formula

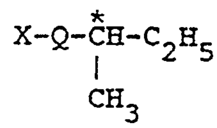

in which
X is -CO-O, -O-, -O-CO-, -CO- or a single bond and
Q is alkylene of 1 to 5 carbon atoms, in which a $CH_2$ group not linked to X can also be replaced by -O-, are excepted,
c) (S)-5-n-Octyl-2-[4-(3-chloropentyloxy)-phenyl]-pyrimidine
(S)-5-n-Undecyl-2-[4-(3-cyanopentyloxy)-phenyl]-pyrimidine and
(S)-5-(n-Octyloxy)-2-[4-(2-methyloctyl)-phenyl]-pyrimidine
are excepted,
d) (2′-Dodecyloxypropyl) 4-[5-(4′-butylphenyl)-pyrimidin-2-yl]benzoate and (2′-decyloxypropyl) 4-(5-heptylpyrimidin-2-yl)benzoate are excepted, and
e) the compounds of the formula

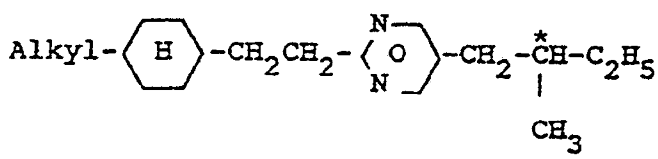

in which
Alkyl is alkyl of 1 to 12 carbon atoms are excepted.

2. Nitrogen-containing heterocycles according to Claim 1 of the formula I1

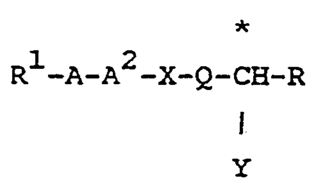

I1

in which
$R^1$ is alkyl or alkoxy of 1-15 carbon atoms,
A is pyrimidine-2,5-diyl or pyridine-2,5-diyl,
$A^2$ is 1,4-phenylene which is unsubstituted or substituted by one fluorine atom,
X is -O-CO- or -O-,
Q is alkylene of 1 to 5 carbon atoms or a single bond,
Y is halogen, and
R is alkyl of 1 to 10 carbon atoms.

3. Nitrogen-containing heterocycles of the formula I according to Claim 1,
in which
$-A^1-Z^1-A^2-$ is a structural element selected from the group of the formulae 1 to 4

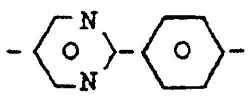 , 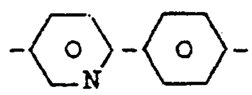 , 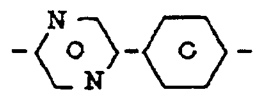

1 2 3

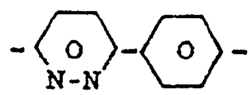

4

4. Nitrogen-containing heterocycles according to one of Claims 1 to 3 of the formula I032, I048, I066, I083 and I084

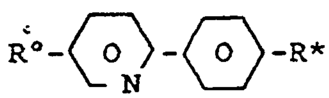

I032

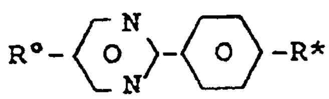

I048

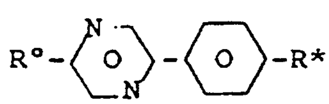

I066

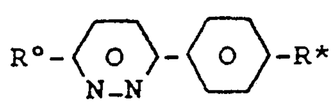

I083

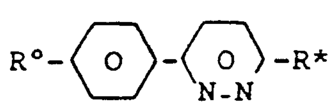

I084

in which

R° is straight-chain alkyl or alkoxy each having 2 to 12 carbon atoms, and

R* has one of the meanings given for the optically active organic radical in Claim 1.

5. Use of the compounds of the formula I according to one of Claims 1 to 4 as components of ferroelectric liquid crystalline phases.

6. Ferroelectric liquid crystalline phase with at least two liquid crystalline components, characterised in that at least one component is a compound of the formula I according to Claim 1.

7. Chirally tilted smectic liquid-crystalline phases containing at least two liquid crystalline components, characterised in that at least one component is a compound of the formula I, in which $R^1$, $R^2$, $R^3$, R, $A^1$, $A^2$, $A^3$, $A^4$, $Z^1$, $Z^2$, $Z^3$, X, Q, Y and p have the meaning given in Claim 1 and in that this phase contains at least one other component which has a small positive or negative dielectric anisotropy in its absolute magnitude and is selected from the compounds of partial formulae Va to Vp:

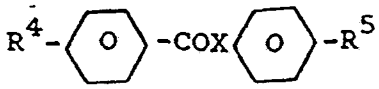 Va

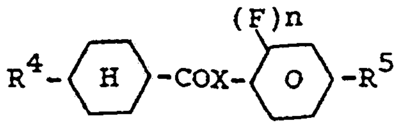 Vb

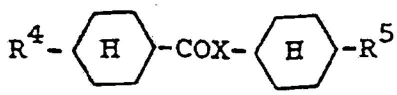 Vc

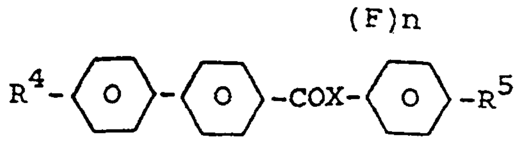
Vd

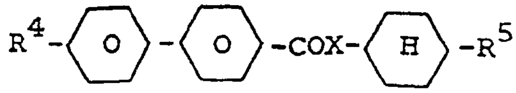
Ve

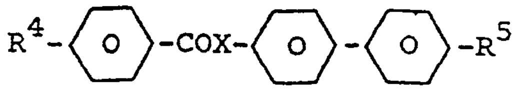
Vf

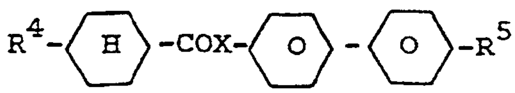
Vg

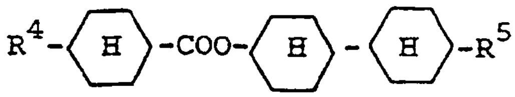
Vh

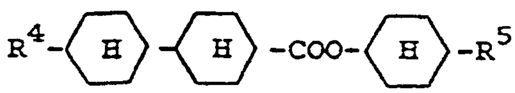
Vi

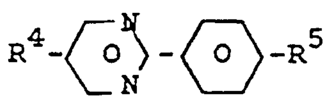
Vj

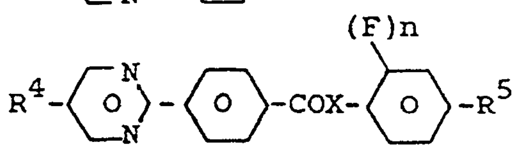
Vk

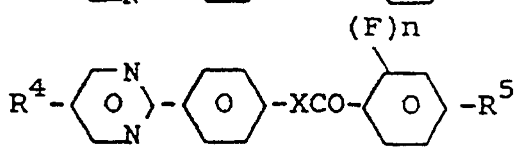
Vl

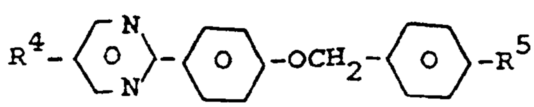
Vm

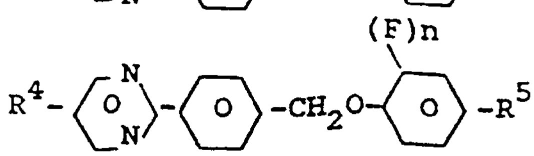
Vn

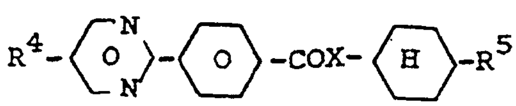
Vo

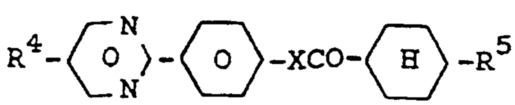
Vp

in which

$R^4$ and $R^5$ are each straight-chain alkyl, alkoxy, alkanoyloxy or alkoxycarbonyl each having 3 to 12 carbon atoms,

X is O, and

n is 0 or 1.

8. Chirally tilted smectic liquid-crystalline phase according to Claim 7, characterised in that at least one component is the compound of the formula I, in which

$-A^1-Z^1-A^2-$ is 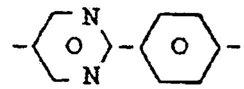

one of the radicals

$R^1$ and $R^2$ is an unsubstituted alkyl group of 1 to 15 carbon atoms, in which one $CH_2$ group can also be replaced by -O-, -COO- or -OCO-,

and the other radical $R^1$ or $R^2$ is an optically active group of the formula

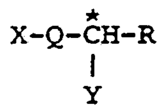

in which

X is -CO-O-, -O-, -O-CO-, or a single bond,

Q is alkylene of 1 to 5 carbon atoms, in which a $CH_2$ group not linked to X can also be replaced by -O-,

Y is $CH_3$, CN or halogen, and

R is straight-chain alkyl of 1 to 10 carbon atoms, and in that at least one other component is a compound of the formula II

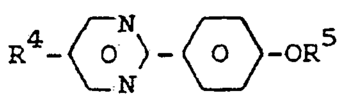 II

in which

$R^4$ and $R^5$, independently of one another, are each n-alkyl of 5 to 12 carbon atoms.

9. Electro-optical display element on the basis of a liquid crystal cell, characterised in that it contains a phase according to one of Claims 6 to 8.

**Claims for the following Contracting States: DE, GB, CH, LI**

1. Nitrogen-containing heterocycles of the formula I

$$R^1-A^1-Z^1-A^2-R^2 \quad I$$

in which

one of the radicals $R^1$ and $R^2$ is H; an unsubstituted or substituted alkyl group of 1-15 carbon atoms in which one or two non-adjacent $CH_2$ groups can also be replaced by at least one member of the group -O-, -CO-, -O-CO-, -CO-O- and -CH=CH-; F, Cl, Br, -CN, -NCS or $R^3-(A^3)_p-Z^2-$,

the other radical $R^1$ or $R^2$ is an optically active organic radical of the formula

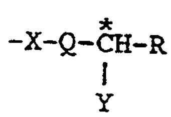

in which

X is -CO-O-, -O-CO-, -O-CO-O-, -CO-, -O-, -S-, -CH=CH-, -CH=CH-COO- or a single bond,

Q is alkylene of 1-5 carbon atoms, in which a $CH_2$ group not linked to X can also be replaced by -O-, -CO-, -O-CO-, -CO-O- or -CH=CH-, or a single bond,

Y is CN, halogen, methyl or methoxy, and

R is an alkyl group of 1-10 carbon atoms which is different from Y and in which one or two non-adjacent

$CH_2$ groups can also be replaced by -O-, -CO-, -O-CO-, -CO-O- and/or -CH=CH-,

$A^1$ is -A-, $-A^4-Z^3-A-$ or $-A-Z^3-A^4-$,

A is a 1,4-phenylene group in which at least one CH group is replaced by N,

$A^2$, $A^3$ and $A^4$ each are 1,4-phenylene which is unsubstituted or substituted by one or two F atoms and/or Cl atoms and/or $CH_3$ groups and/or CN groups, in which one or two CH groups can also be replaced by N atoms; 1,4-cyclohexylene in which one or two non-adjacent $CH_2$ groups can also be replaced by O atoms and/or S atoms; piperidine-1,4-diyl, 1,4-bicyclo-(2,2,2)octylene; unsubstituted or CN-unsubstituted [sic] decahydronaphthalene-2,6-diyl or 1,2,3,4-tetrahydronaphthalene-2,6-diyl,

$Z^1$, $Z^2$ and $Z^3$ each are -CO-O, -O-CO-, $-OCH_2-$, $-CH_2O-$, $-CH_2CH_2-$, substituted ethylene or a single bond,

$R^3$ is H; an unsubstituted or substituted alkyl group of 1-15 carbon atoms in which one or two non-adjacent $CH_2$ groups can also be replaced by a member of the group -O-, -CO-, -O-CO-, -CO-O and -CH=CH-; F, Cl, Br, -NCS or -CN, and

P [sic] is 1 or 2,

in which the groups $A^3$ can be the same or can differ from each other, when p = 2, with the provisos that

a) when A = pyrimidine-2,5-diyl, one of the radicals $R^1$ and $R^2$ is an optically active organic radical and the other radical $R^1$ or $R^2$ is an unsubstituted or substituted alkyl group of 1-15 carbon atoms, in which one or two non-adjacent $CH_2$ groups can also be replaced by at least one member of the group -O-, -CO-, -O-CO-, -CO-O- and -CH=CH-.

b) Compounds of the formula

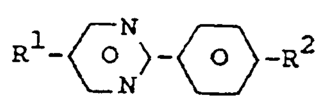

in which one of the radicals $R^1$ and $R^2$ is alkyl, alkoxy or alkoxycarbonyl of 1 to 15 carbon atoms and the other radical $R^1$ or $R^2$ is an optically active radical of the formula

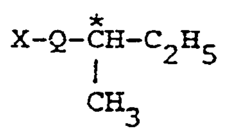

in which

X is -CO-O, -O-, -O-CO-, -CO- or a single bond and

Q is alkylene of 1 to 5 carbon atoms, in which a $CH_2$ group not linked to X can also be replaced by -O-, are excepted,

c) (S)-5-n-Octyl-2-[4-(3-chloropentyloxy)-phenyl]-pyrimidine
(S)-5-n-Undecyl-2-[4-(3-cyanopentyloxy)-phenyl]-pyrimidine and
(S)-5-(n-Octyloxy)-2-[4-(2-methyloctyl)-phenyl]-pyrimidine
are excepted,

d) 3-(2-Methylbutoxy)-6-(4-pentylphenyl)-pyridazine,
3-(2-Methylbutoxy)-6-(4-nonylphenyl)-pyridazine,
3-(2-Methylbutoxy)-6-(4-dodecylphenyl)-pyridazine,
3-(2-Methylbutoxy)-6-(4-pentadecylphenyl)-pyridazine,
3-(2-Methylbutoxy)-6-(4-octyloxyphenyl)-pyridazine,
3-(2-Methylbutoxy)-6-(4-decyloxyphenyl)-pyridazine,
3-(2-Methylbutoxy)-6-(4-tetradecyloxyphenyl)-pyridazine,
3-(1-Methylheptyloxy)-6-(4-pentylphenyl)-pyridazine,
3-(1-Methylheptyloxy)-6-(4-dodecylphenyl)-pyridazine,
3-(1-Methylheptyloxy)-6-(4-decyloxyphenyl)-pyridazine and
3-(1-Methylbutoxy)-6-(4-octyloxyphenyl)-pyridazine are excepted,

e) (2′-Dodecyloxypropyl) 4-[5-(4′-butylphenyl)-pyrimidin-2-yl]benzoate and (2′-decyloxypropyl) 4-(5-heptylpyrimidin-2-yl)benzoate are excepted and

f) the compounds of the formula

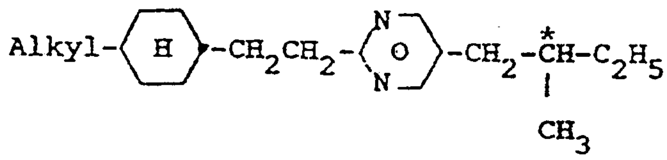

in which
Alkyl is alkyl of 1 to 12 carbon atom are excepted.

2. Nitrogen-containing heterocycles according to Claim 1 of the formula I1

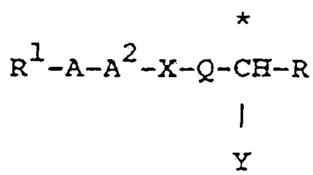

I1

in which
$R^1$ is alkyl or alkoxy of 1-15 carbon atoms,
A is pyrimidine-2,5-diyl or pyridine-2,5-diyl,
$A^2$ is 1,4-phenylene which is unsubstituted or substituted by one fluorine atom,
X is -O-CO- or -O-,
Q is alkylene of 1 to 5 carbon atoms or a single bond,
Y is halogen, and
R is alkyl of 1 to 10 carbon atoms.

3. Nitrogen-containing heterocycles of the formula I according to Claim 1,
in which
$-A^1-Z^1-A^2-$ is a structural element selected from the group of the formulae 1 to 4

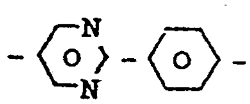 , 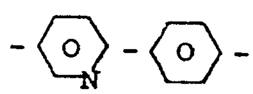 , 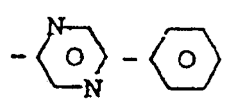

1 2 3

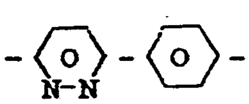

4

4. Nitrogen-containing heterocycles according to one of Claims 1 to 3 of the formula I032, I048, I066, I083 and I084

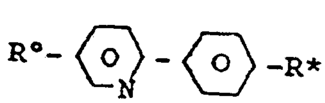

I032

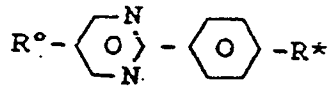

I048

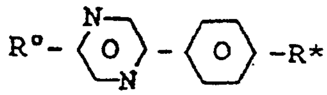

I066

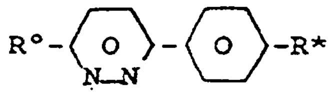

I083

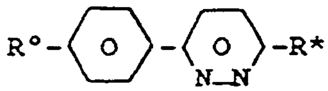

I084

in which

R° is straight-chain alkyl or alkoxy each having 2 to 12 carbon atoms, and

R* has one of the meanings given for the optically active organic radical in Claim 1.

5. Use of the compounds of the formula I according to one of Claims 1 to 4 as components of ferroelectric liquid crystalline phases.

6. Ferroelectric liquid crystalline phase with at least two liquid crystalline components, characterised in that at least one component is a compound of the formula I according to Claim 1.

7. Chirally tilted smectic liquid-crystalline phases containing at least two liquid crystalline components, characterised in that at least one component is a compound of the formula I, in which $R^1$, $R^2$, $R^3$, R, $A^1$, $A^2$, $A^3$, $A^4$, $Z^1$, $Z^2$, $Z^3$, X, Q, Y and p have the meaning given in Claim 1 and in that this phase contains at least one other component which has a small positive or negative dielectric anisotropy in its absolute magnitude and is selected from the compounds of partial formulae Va to Vp:

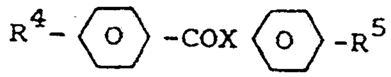
Va

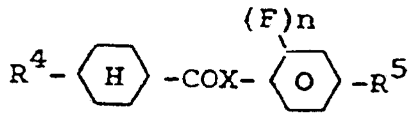
Vb

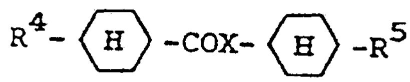
Vc

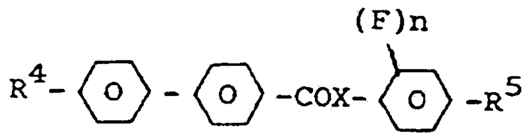
Vd

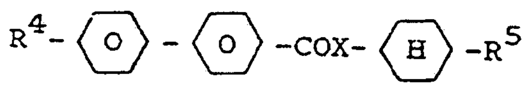
Ve

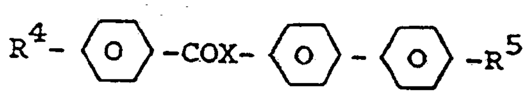
Vf

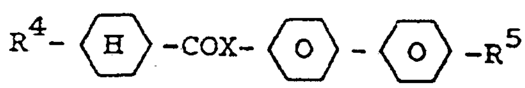
Vg

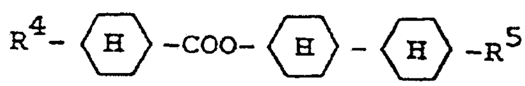
Vh

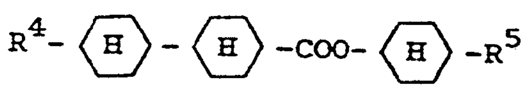
Vi

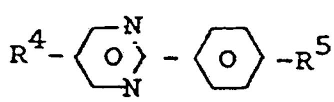
Vj

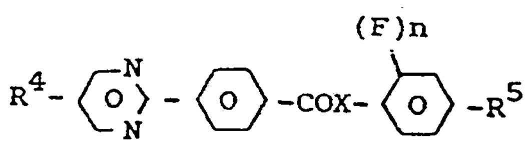
Vk

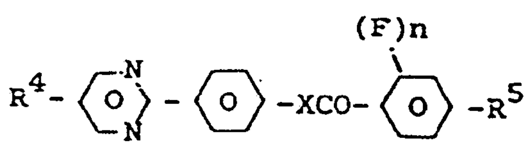
Vl

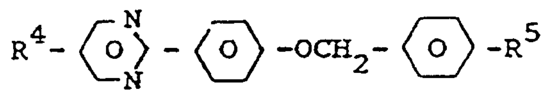 Vm

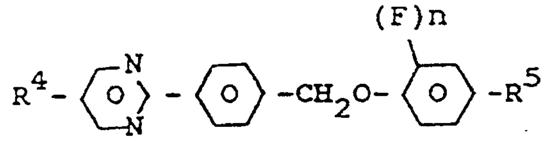 Vn

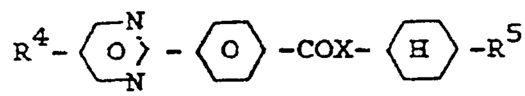 Vo

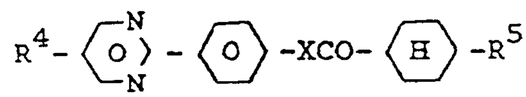 Vp

in which
$R^4$ and $R^5$ are each straight-chain alkyl, alkoxy, alkanoyloxy or alkoxycarbonyl each having 3 to 12 carbon atoms,
X is O, and
n is 0 or 1.

8. Chirally tilted smectic liquid-crystalline phase according to Claim 7, characterised in that at least one component is of [sic] the compound of the formula I, in which
$-A^1-Z^1-A^2-$ is

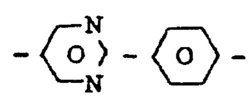

one of the radicals
$R^1$ and $R^2$ is an unsubstituted alkyl group of 1 to 15 carbon atoms, in which one $CH_2$ group can also be replaced by -O-, -COO- or -OCO-,
and the other radical $R^1$ or $R^2$ is an optically active group of the formula

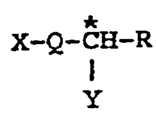

in which
X is -CO-O-, -O-, -O-CO-, or a single bond,
Q is alkylene of 1 to 5 carbon atoms, in which a $CH_2$ group not linked to X can also be replaced by -O-,
Y is $CH_3$, CN or halogen, and
R is straight-chain alkyl of 1 to 10 carbon atoms, and in that at least one other component is a compound of the formula II

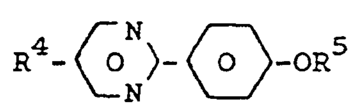 II

in which
$R^4$ and $R^5$, independently of one another, are each n-alkyl of 5 to 12 carbon atoms.

9. Electro-optical display element on the basis of a liquid crystal cell, characterised in that it contains a phase according to one of Claims 6 to 8.

**Revendications**

**Revendications pour l'Etat contractant suivant: AT**

1. Phase à cristaux liquides ferroélectrique à au moins deux composants, caractérisée en ce que l'un au moins des composants est un composé hétérocyclique azoté de formule I

$$R^1\text{-}A^1\text{-}Z^1\text{-}A^2\text{-}R^2 \quad I$$

dans laquelle

l'un des symboles

$R^1$ et $R^2$ représente H, un groupe alkyle non substitué ou substitué en C 1-C 15 dans lequel également un ou deux groupes $CH_2$ non voisins peuvent être remplacés par au moins un groupement choisi parmi -O-, -CO-, -O-CO-, -CO-O-, et -CH=CH-, ou F, Cl, Br, -CN, -NCS ou $R^3\text{-}(A^3)_p\text{-}Z^2\text{-}$,

et l'autre représente

un groupe organique possédant l'activité optique, de formule

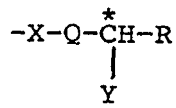

dans laquelle

X représente -CO-O-, -O-CO-, -O-CO-O-, -CO-, -O-, -S-, -CH=CH-, -CH=CH-COO- ou une liaison simple,

Q représente un groupe alkylène en C 1-C 5 dans lequel également un groupe $CH_2$ non relié à X peut être remplacé par -O-, -CO-, -O-CO-, -CO-O- ou -CH=CH-, ou une liaison simple,

Y représente CN, un halogène, un groupe méthyle ou méthoxy, et

R représente un groupe alkyle en C 1-C 10 différent de Y et dans lequel également un ou deux groupes $CH_2$ non voisins peuvent être remplacés par -O-, -CO-, -O-CO-, -CO-O- et/ou -CH=CH-,

$A^1$ représente -A-, $-A^4\text{-}Z^3\text{-}A\text{-}$ ou $-A\text{-}Z^3\text{-}A^4\text{-}$,

A représente un groupe 1,4-phénylène dans lequel au moins un groupe CH est remplacé par N,

$A^2$, $A^3$ et $A^4$ représentent chacun un groupe 1,4-phénylène non substitué ou substitué par un ou deux atomes de F et/ou de Cl et/ou groupes $CH_3$ et/ou groupes CN et dans lequel également un ou deux groupes CH peuvent être remplacés par des atomes de N, un groupe 1,4-cyclohexylène dans lequel également un ou deux groupes $CH_2$ non voisins peuvent être remplacés par des atomes de O et/ou de S, un groupe pipéridine-1,4-diyle, 1,4-bicyclo(2,2,2)-octylène, un groupe décahydronaphtalène-2,6-diyle ou 1,2,3,4-tétrahydronaphtalène-2,6-diyle non substitué ou substitué par CN,

$Z^1$, $Z^2$ et $Z^3$ représentent chacun -CO-O-, -O-CO-, $-OCH_2-$, $-CH_2O-$, $-CH_2CH_2-$, un groupe éthylène substitué ou une liaison simple,

$R^3$ représente H, un groupe alkyle non substitué ou substitué en C 1-C 15 dans lequel également un ou deux groupes $CH_2$ non voisins peuvent être remplacés par un groupement choisi parmi -O-, -CO-, -O-CO-, -CO-O et -CH=CH-, ou bien F, Cl, Br, -NCS ou -CN, et

p est égal à 1 ou 2,

et lorsque p = 2, les groupes $A^3$ peuvent être identiques ou différents, avec les réserves suivantes :

dans le cas où A représente un groupe pyrimidine-2,5-diyle, l'un des symboles $R^1$ et $R^2$ représente un groupe organique possédant l'activité optique et l'autre un groupe alkyle non substitué ou substitué en C 1-C 15 dans lequel également un ou deux groupes $CH_2$ non voisins peuvent être remplacés par au moins un groupement choisi parmi -O-, -CO-, -O-CO-, -CO-O- et -CH=CH-.

2. Phase ferroélectrique selon revendication 1, caractérisée en ce que l'un au moins des composants est un composé hétérocyclique azoté de formule I1

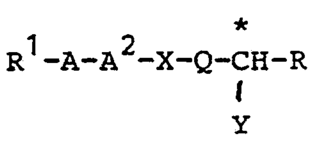

I1

dans laquelle

$R^1$ représente un groupe alkyle ou alcoxy en C 1-C 15,

A représente un groupe pyrimidine-2,5-diyle ou pyridine-2,5-diyle,

$A^2$ représente un groupe 1,4-phénylène non substitué ou substitué par un atome de fluor,
X représente -O-CO- ou -O-,
Q représente un groupe alkylène en C 1-C 5 ou une liaison simple,
Y représente un halogène, et
R représente un groupe alkyle en C 1-C 10.

3. Phase ferroélectrique selon revendication 1 ou 2, caractérisée en ce que l'un au moins des composants est un composé hétérocyclique azoté de formule I dans laquelle
$-A^1-Z^1-A^2-$ représente un élément de structure choisi parmi ceux qui répondent aux formules 1 à 4

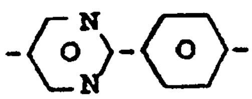 , 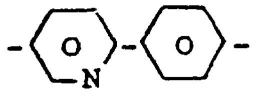 , 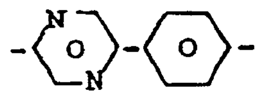

1 2 3

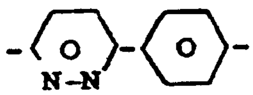

4

4. Phase ferroélectrique selon une des revendications 1 à 3, caractérisée en ce que l'un au moins des composants est un composé hétérocyclique azoté de formule I032, I048, I066, I083 ou I084 :

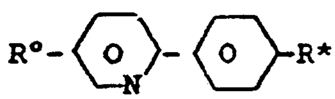 I032

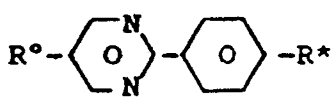 I048

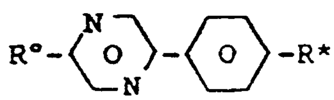 I066

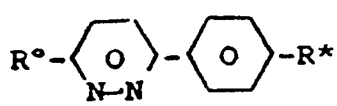 I083

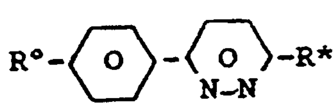 I084

dans lesquelles
R° représente un groupe alkyle ou alcoxy à chaîne droite en C 2-C 12, et
R* a l'une des significations indiquées pour le groupe organique possédant l'activité optique de la revendication 1.

5. Phase à cristaux liquides ferroélectriques selon une des revendications 1 à 4, caractérisée en ce que l'un au moins des composants est un composé de formule I et en ce que la phase contient au moins un autre composant à anisotropie diélectrique relativement peu positive ou négative, choisi parmi les composés répondant aux formules partielles Va à Vp :

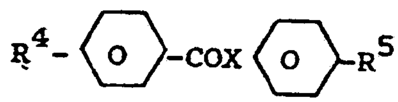 Va

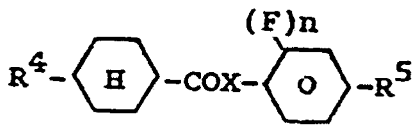 Vb

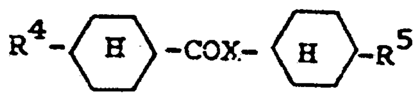 Vc

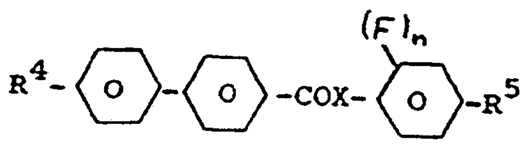 Vd

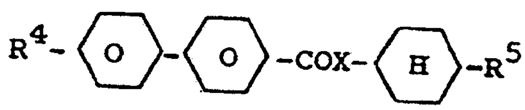 Ve

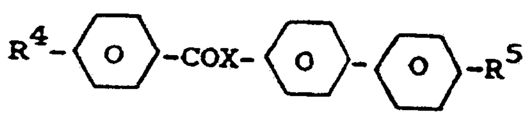 Vf

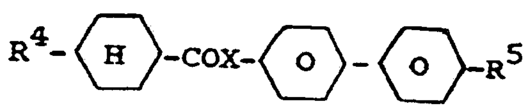 Vg

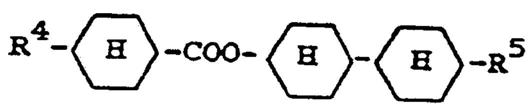 Vh

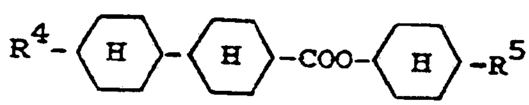 Vi

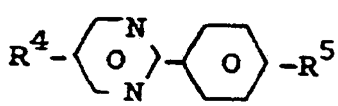 Vj

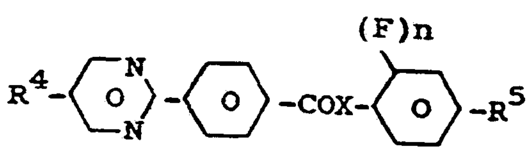 Vk

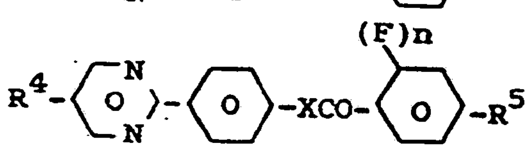 Vl

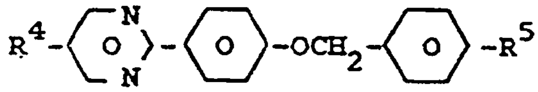

Vm

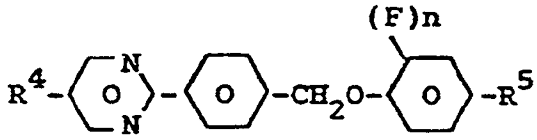

Vn

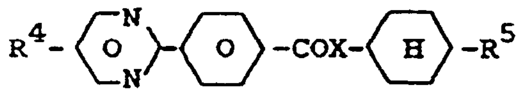

Vo

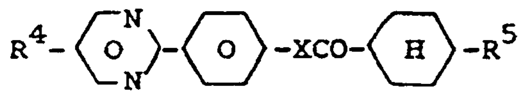

Vp

dans lesquelles

$R^4$ et $R^5$ représentent chacun un groupe alkyle, alcoxy, alcanoyloxy ou alcoxycarbonyle à chaîne droite contenant chacun 3 à 12 atomes de carbone,

X représente 0, et

n est égal à 0 ou 1.

6. Phase ferroélectrique selon revendication 5, caractérisée en ce que l'un au moins des composants est un composé de formule I dans laquelle

$-A^1-Z^1-A^2-$ représente

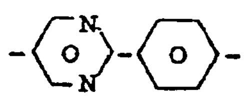

l'un des symboles $R^1$ et $R^2$ représente un groupe alkyle non substitué en C 1-C 15 dans lequel également un groupe $CH_2$ peut être remplacé par -O-, -COO- ou -OCO-, et l'autre représente un groupe possédant l'activité optique, de formule

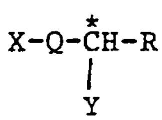

dans laquelle

X représente -CO-O-, -O-, -O-CO- ou une liaison simple,

Q représente un groupe alkylène en C 1-C 5 dans lequel également un groupe $CH_2$ non relié à X peut être remplacé par -O-,

Y représente $CH_3$, CN ou un halogène, et

R représente un groupe alkyle à chaîne droite en C 1-C 10,

et en ce que

au moins un autre composant consiste en un composé de formule II

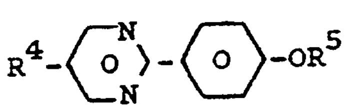

II

dans laquelle $R^4$ et $R^5$ représentent chacun, indépendamment l'un de l'autre, un groupe n-alkyle en C 5-C 12.

7. Elément d'affichage électro-optique à base d'une cellule à cristaux liquides, caractérisé en ce qu'il contient une phase selon l'une des revendications 1 à 6.

**Revendications pour les Etats contractants suivants: BE, IT**

1. Composés hétérocycliques azotés de formule I

$$R^1\text{-}A^1\text{-}Z^1\text{-}A^2\text{-}R^2 \quad I$$

dans laquelle

l'un des symboles

$R^1$ et $R^2$ représente H, un groupe alkyle non substitué ou substitué en C 1-C 15 dans lequel également un ou deux groupes $CH_2$ non voisins peuve nt être remplacés par au moins un groupement choisi parmi -O-, -CO-, -O-CO-, -CO-O- et -CH=CH-, ou F, Cl, Br, -CN, -NCS ou $R^3\text{-}(A^3)_p\text{-}Z^2\text{-}$,

et l'autre représente un groupe organique possédant l'activité optique, de formule

$$-X-Q-\overset{*}{C}H(Y)-R$$

dans laquelle

X représente -CO-O-, -O-CO-, -O-CO-O-, -CO-, -O-, -S-, -CH=CH-, -CH=CH-COO- ou une liaison simple,

Q représente un groupe alkylène en C 1-C 5 dans lequel également un groupe $CH_2$ non relié à X peut être remplacé par -O-, -CO-, -O-CO-, -CO-O- ou -CH=CH-, ou une liaison simple,

Y représente CN, un halogène, un groupe méthyle ou méthoxy, et

R représente un groupe alkyle en C 1-C 10 différent de Y et dans lequel également un ou deux groupes $CH_2$ non voisins peuvent être remplacés par -O-, -CO-, -O-CO-, -CO-O- et/ou -CH=CH-,

$A^1$ représente -A-, $-A^4\text{-}Z^3\text{-}A\text{-}$ ou $-A\text{-}Z^3\text{-}A^4\text{-}$,

A représente un groupe 1,4-phénylène dans lequel au moins un groupe CH est remplacé par N,

$A^2$, $A^3$ et $A^4$ représentent chacun un groupe 1,4-phénylène non substitué ou substitué par un ou deux atomes de F et/ou Cl et/ou groupes $CH_3$ et/ou groupes CN et dans lequel également un ou deux groupes CH peuvent être remplacés par des atomes de N, un groupe 1,4-cyclohexylène dans lequel également un ou deux groupes $CH_2$ non voisins peuvent être remplacés par des atomes de O et/ou de S, un groupe pipéridine-1,4-diyle, 1,4-bicyclo(2,2,2)-octylène, un groupe décahydronaphtalène-2,6-diyle ou 1,2,3,4-tétrahydronaphtalène-2,6-diyle non substitué ou substitué par un groupe CN,

$Z^1$, $Z^2$ et $Z^3$ représentent chacun -CO-O-, -O-CO-, $-OCH_2-$, $-CH_2O-$, $-CH_2CH_2-$, un groupe éthylène substitué ou une liaison simple,

$R^3$ représente H, un groupe alkyle non substitué ou substitué en C 1-C 15 dans lequel également un ou deux groupes $CH_2$ non voisins peuvent être remplacés par un groupement choisi parmi -O-, -CO-, -O-CO-, -CO-O et -CH=CH-, ou F, Cl, Br, -NCS ou -CN, et

p est égal à 1 ou 2,

et lorsque p = 2, les groupes $A^3$ peuvent être identiques ou différents, avec la restriction suivante :

dans le cas où A représente un groupe pyrimidine-2,5-diyle, l'un des symboles $R^1$ et $R^2$ représente un groupe organique possédant l'activité optique et l'autre un groupe alkyle non substitué ou substitué en C 1-C 15 dans lequel également un ou' deux groupes $CH_2$ non voisins peuvent être remplacés par au moins un groupement choisi parmi -O-, -CO-, -O-CO-, -CO-O- et -CH=CH-.

2. Composés hétérocycliques azotés selon revendication 1, de formule I1

$$R^1-A-A^2-X-Q-\overset{*}{C}H(Y)-R \qquad I1$$

dans laquelle

$R^1$ représente un groupe alkyle ou alcoxy en C 1-C 15,

A représente un groupe pyrimidine-2,5-diyle ou pyridine-2,5-diyle,

$A^2$ représente un groupe 1,4-phénylène non substitué ou substitué par un atome de fluor,

X représente -O-CO- ou -O-,

Q représente un groupe alkylène en C 1-C 5 ou une liaison simple,

Y représente un halogène, et

R représente un groupe alkyle en C 1-C 10.

3. Composés hétérocycliques azotés de formule I selon revendication 1, dans laquelle $-A^1-Z^1-A^2-$ représente un élément de structure choisi parmi ceux de formules 1 à 4

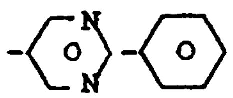 , 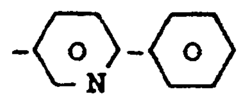 , 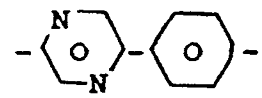

1 2 3

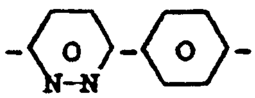

4

4. Composés hétérocycliques azotés selon l'une des revendications 1 à 3, de formules I032, I048, I066, I083 et I084

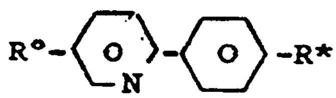 I032

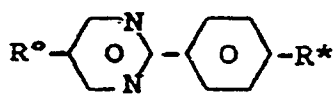 I048

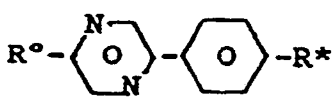 I066

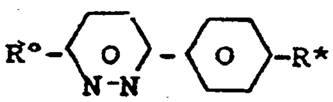 I083

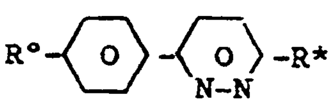 I084

dans lesquelles
R° représente un groupe alkyle ou alcoxy à chaîne droite en C 2-C 12, et
R* a l'une des significations indiquées pour le groupe organique possédant l'activité optique dans la revendication 1.

5. Utilisation des composés de formule I selon une des revendications 1 à 4 en tant que composants de phases à cristaux liquides ferroélectriques.

6. Phase à cristaux liquides ferroélectriques a au moins deux composants à cristaux liquides, caractérisée en ce que l'un au moins des composants est un composé de formule I de la revendication 1.

7. Elément d'affichage électro-optique à base d'une cellule à cristaux liquides, caractérisé en ce qu'il contient une phase selon revendication 6.

**Revendications pour l'Etat contractant suivant: NL**

1. Composés hétérocycliques azotés de formule I

$$R^1-A^1-Z^1-A^2-R^2 \quad I$$

dans laquelle l'un des symboles

$R^1$ et $R^2$ représente H, un groupe alkyle non substitué ou substitué en C 1-C 15 dans lequel également un ou deux groupes $CH_2$ non voisins peuvent être remplacés par au moins un groupement choisi parmi -O-, -CO-, -O-CO-, -CO-O- et -CH=CH-, ou F, Cl, Br, -CN, -NCS ou $R^3$-$(A^3)_p$-$Z^2$-,

et l'autre représente un groupe organique possédant l'activité optique, de formule

$$-X-Q-\underset{\substack{|\\ Y}}{\overset{*}{C}}H-R$$

dans laquelle

X représente -CO-O-, -O-CO-, -O-CO-O-, -CO-, -O-, -S-, -CH=CH-, -CH=CH-COO- ou une liaison simple,

Q représente un groupe alkylène en C 1-C 5 dans lequel également un groupe $CH_2$ non relié à X peut être remplacé par -O-, -CO-, -O-CO-, -CO-O- ou -CH=CH-, ou une liaison simple,

Y représente CN, un halogène, un groupe méthyle ou méthoxy, et

R représente un groupe alkyle en C 1-C 10 différent de Y et dans lequel également un ou deux groupes $CH_2$ non voisins peuvent être remplacés par -O-, -CO-, -O-CO-, -CO-O- et/ou -CH=CH-,

$A^1$ représente -A-, -$A^4$-$Z^3$-A- ou -A-$Z^3$-$A^4$-,

A représente un groupe 1,4-phénylène dans lequel au moins un groupe CH est remplacé par N,

$A^2$, $A^3$ et $A^4$ représentent chacun un groupe 1,4-phénylène non substitué ou substitué par un ou deux atomes de F et/ou Cl et/ou groupes $CH_3$ et/ou groupes CN et dans lequel également un ou deux groupes CH peuvent être remplacés par des atomes de N, un groupe 1,4-cyclohexylène dans lequel également un ou deux groupes $CH_2$ non voisins peuvent être remplacés par des atomes de O et/ou de S, un groupe pipéridine-1,4-diyle, 1,4-bicyclo(2,2,2)-octylène, un groupe décahydronaphtalène-2,6-diyle ou 1,2,3,4-tétrahydronaphtalène-2,6-diyle non substitué ou substitué par CN,

$Z^1$, $Z^2$ et $Z^3$ représentent chacun -CO-O-, -O-CO-, -$OCH_2$-, -$CH_2O$-, -$CH_2CH_2$-, un groupe éthylène substitué ou une liaison simple,

$R^3$ représente H, un groupe alkyle en C 1-C 15 non substitué ou substitué et dans lequel également un ou deux groupes $CH_2$ non voisins peuvent être remplacés par un groupement choisi parmi -O-, -CO-, -O-CO-, -CO-O- et -CH=CH-, ou F, Cl, Br, -NCS ou -CN, et

p est égal à 1 ou 2,

et lorsque p est égal à 2, les groupes $A^3$ peuvent être identiques ou différents, avec les restrictions suivantes:

a) dans le cas où A représente un groupe pyrimidine-2,5-diyle, l'un des symboles $R^1$ et $R^2$ représente un groupe organique possédant l'activité optique et l'autre un groupe alkyle en C 1-C 15 non substitué ou substitué et dans lequel également un ou deux groupes $CH_2$ non voisins peuvent être remplacés par au moins un groupement choisi parmi -O-, -CO-, -O-CO-, -CO-O- et -CH=CH-,

b) le 4-[5-(4'-butylphényl)-pyrimidine-2-yl] -benzoate de 2'-dodécyloxypropyle et

le 4-(5-heptylpyrimidine-2-yl)-benzoate de 2'-dodécyloxypropyle

sont exclus.

2. Composés hétérocycliques azotés selon revendication 1, de formule I1

$$R^1-A-A^2-X-Q-\underset{\substack{\\ Y}}{\overset{*}{C}}H-R \qquad I1$$

dans laquelle

$R^1$ représente un groupe alkyle ou alcoxy en C 1-C 15,

A représente un groupe pyrimidine-2,5-diyle ou pyridine-2,5-diyle,

$A^2$ représente un groupe 1,4-phénylène non substitué ou substitué par un atome de fluor,

X représente -O-CO- ou -O-,

Q représente un groupe alkylène en C 1-C 5 ou une liaison simple,

Y représente un halogène, et

R représente un groupe alkyle en C 1-C 10.

3. Composés hétérocycliques azotés de formule I de la revendication 1, dans laquelle

$-A^1-Z^1-A^2-$ représente un élément de structure choisi parmi ceux qui répondent aux formules 1 à 4

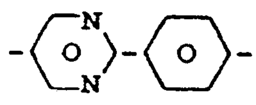

1

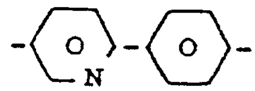

2

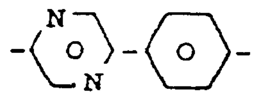

3

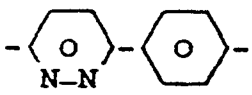

4

4. Composés hétérocycliques azotés selon une des revendications 1 à 3, de formules I032, I048, I066, I083 et I084

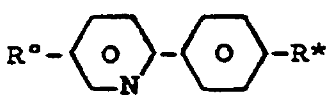

I032

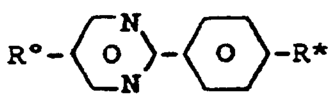

I048

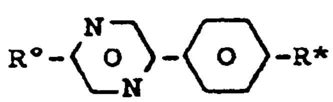

I066

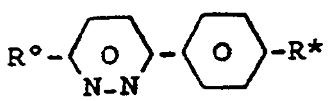

I083

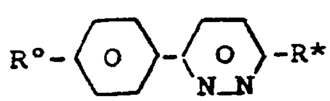

I084

dans lesquelles

R° représente un groupe alkyle ou alcoxy à chaîne droite en C 2-C 12, et

R* a l'une des significations indiquées pour le groupe organique possédant l'activité optique dans la revendication 1.

5. Utilisation des composés de formule I selon une des revendications 1 à 4 en tant que composants de phases à cristaux liquides ferroélectriques.

6. Phase à cristaux liquides ferroélectriques à au moins deux composants à cristaux liquides, caractérisée en ce que l'un au moins des composants est un composé de formule I de la revendication 1.

7. Phases à cristaux liquides smectiques chiralisées à au moins deux composants à cristaux liquides, caractérisées en ce que l'un au moins des composants est un composé de formule I dans laquelle $R^1$, $R^2$, $R^3$, R, $A^1$, $A^2$, $A^3$, $A^4$, $Z^1$, $Z^2$, $Z^3$, X, Q, Y et p ont les significations indiquées dans la revendication 1, et en ce qu'elles contiennent au moins un autre composant à anisotropie diélectrique relativement peu positive ou négative choisi parmi les composés répondant aux formules partielles Va à Vp

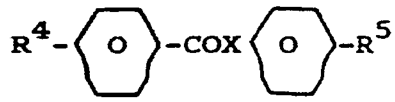

Va

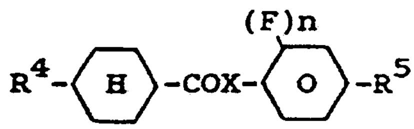

Vb

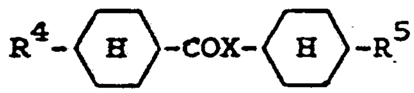

Vc

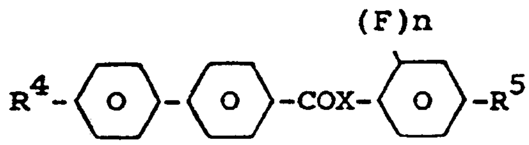

Vd

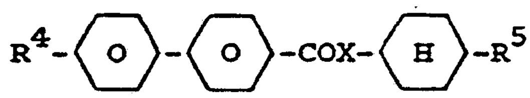

Ve

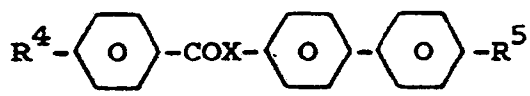

Vf

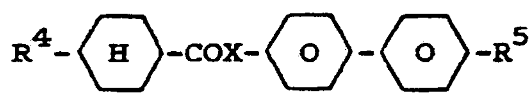

Vg

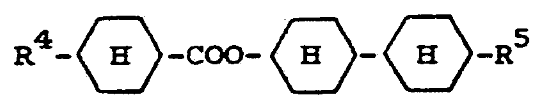

Vh

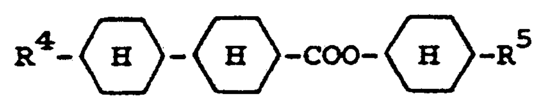

Vi

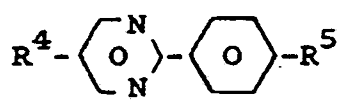

Vj

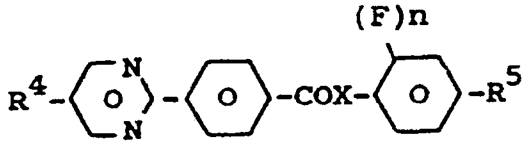

Vk

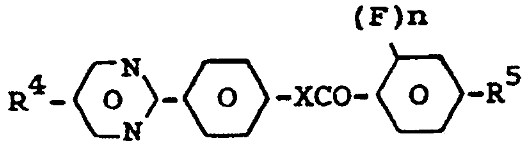

Vl

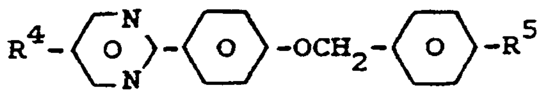

Vm

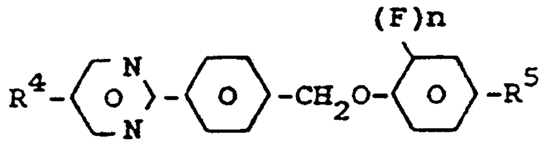

Vn

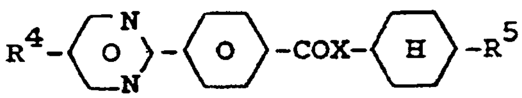

Vo

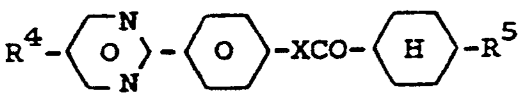

Vp

dans lesquelles

$R^4$ et $R^5$ représentent chacun un groupe alkyle, alcoxy, alcanoyloxy ou alcoxycarbonyle à chaîne droite en C 3-C 12,

X représente 0, et

n est égal à 0 ou 1.

8. Phase à cristaux liquides smectique chiralisée selon revendication 7, caractérisée en ce que l'un au moins des composants est un composé de formule I dans laquelle

$-A^1-Z^1-A^2-$ représente

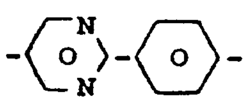

l'un des symboles

$R^1$ et $R^2$ représente un groupe alkyle non substitué en C 1-C 15 dans lequel également un groupe $CH_2$ peut être remplacé par -O-, -COO- ou -OCO-,

et l'autre représente un groupe possédant l'activité optique, de formule

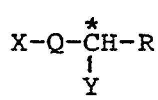

dans laquelle

X représente -CO-O-, -O-, -O-CO- ou une liaison simple,

Q représente un groupe alkylène en C 1-C 5 dans lequel également un groupe $CH_2$ non relié à X peut être remplacé par -O-,

Y représente $CH_3$, CN ou un halogène, et

R représente un groupe alkyle à chaîne droite en C 1-C 10,

et au moins un autre composant consistant en un composé de formule II

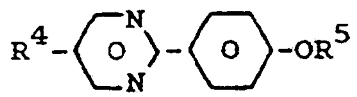

II

dans laquelle

$R^4$ et $R^5$ représentent chacun, indépendamment l'un de l'autre, un groupe n-alkyle en C 5-C 12.

9. Elément d'affichage électro-optique à base d'une cellule à cristaux liquides caractérisé en ce qu'il contient une phase selon une des revendications 6 à 8.

**Revendications pour l'Etat contractant suivant: SE**

1. Composés hétérocycliques azotés de formule I

$$R^1\text{-}A^1\text{-}Z^1\text{-}A^2\text{-}R^2 \quad I$$

dans laquelle l'un des symboles

$R^1$ et $R^2$ représente H, un groupe alkyle non substitué ou substitué en C 1-C 15 et dans lequel également un ou deux groupes $CH_2$ non voisins peuvent être remplacés par au moins un groupement choisi parmi,-O-, -CO-, -O-CO-, -CO-O- et -CH=CH-, ou F, Cl, Br, -CN, -NCS ou $R^3\text{-}(A^3)_p\text{-}Z^2\text{-}$,

et l'autre représente un groupe organique possédant l'activité optique, de formule

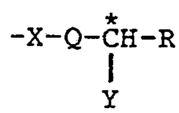

dans laquelle

X représente -CO-O-, -O-CO-, -O-CO-O-, -CO-, -O-, -S-, -CH=CH-, -CH=CH-COO- ou une liaison simple,

Q représente un groupe alkylène en C 1-C 5 dans lequel également un groupe $CH_2$ non relié à X peut être remplacé par -O-, -CO-, -O-CO-, -CO-O- ou -CH=CH-, ou une liaison simple,

Y représente CN, un halogène, un groupe méthyle ou méthoxy, et

R représente un groupe alkyle en C 1-C 10 différent de

Y et dans lequel également un ou deux groupes $CH_2$ non voisins peuvent être remplacés par -O-, -CO-, -O-CO-, -CO-O- et/ou -CH=CH-,

$A^1$ représente -A-, $\text{-}A^4\text{-}Z^3\text{-}A\text{-}$ ou $\text{-}A\text{-}Z^3\text{-}A^4\text{-}$,

A représente un groupe 1,4-phénylène dans lequel au moins un groupe CH est remplacé par N,

$A^2$, $A^3$ et $A^4$ représentent chacun un groupe 1,4-phénylène non substitué ou substitué par un ou deux atomes de F et/ou de Cl et/ou groupes $CH_3$ et/ou groupes CN et dans lequel également un ou deux groupes CH peuvent être remplacés par des atomes de N, un groupe 1,4-cyclohexylène dans lequel également un ou deux groupes $CH_2$ non voisins peuvent être remplacés par des atomes de O et/ou de S, un groupe pipéridine-1,4-diyle, 1,4-bicyclo(2,2,2)-octylène, un groupe décahydronaphtalène-2,5-diyle ou 1,2,3,4-tétrahydronaphtalène-2,6-diyle non substitué ou substitué par CN,

$Z^1$, $Z^2$ et $Z^3$ représentent chacun -CO-O-, -O-CO-, $\text{-}OCH_2\text{-}$, $\text{-}CH_2O\text{-}$, $\text{-}CH_2CH_2\text{-}$, un groupe éthylène substitué ou une liaison simple,

$R^3$ représente H, un groupe alkyle non substitué ou substitué en C 1-C 15 et dans lequel également un ou deux groupes $CH_2$ non voisins peuvent être remplacés par un groupement choisi parmi -O-, -CO-, -O-CO-, -CO-O- et -CH=CH-, ou F, Cl, Br, -NCS ou -CN, et

p est égal à 1 ou 2,

et lorsque p = 2, les groupes $A^3$ peuvent être identiques ou différents, avec les restrictions suivantes :

a) lorsque A représente un groupe pyrimidine-2,5-diyle, l'un des symboles $R^1$ et $R^2$ représente un groupe organique possédant l'activité optique et l'autre un groupe alkyle non substitué ou substitué en C 1-C 15 dans lequel également un ou deux groupes $CH_2$ non voisins peuvent être remplacés par au moins un groupement choisi parmi -O-, -CO-, -O-CO-, -CO-O- et -CH=CH-,

b) les composés de formule

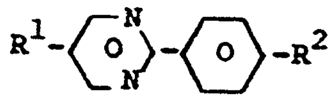

dans laquelle l'un des symboles $R^1$ et $R^2$ représente un groupe alkyle, alcoxy ou alcoxycarbonyle en C 1-C 15 et l'autre un groupe possédant l'activité optique, de formule

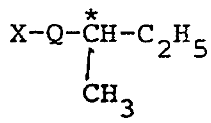

dans laquelle
X représente -CO-O-, -O-, -O-CO-, -CO- ou une liaison simple, et
Q représente un groupe alkylène en C 1-C 5 dans lequel également un groupe $CH_2$ non relié à X peut être remplacé par -O-,
sont exclus,
c) la (S)-5-n-octyl-2-[4-(3-chloropentyloxy)-phényl]-pyrimidine,
la (S)-5-n-undécyl-2-[4-(3-cyanopentyloxy)-phényl]-pyrimidine, et
la (S)-5-(n-octyloxy)-2-[4-(2-méthyloctyl)-phényl]-pyridine
sont exclues.
2. Composés hétérocycliques azotés selon revendication 1, de formule I1

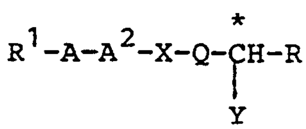 I1

dans laquelle
$R^1$ représente un groupe alkyle ou alcoxy en C 1-C 15,
A représente un groupe pyrimidine-2,5-diyle ou pyridine-2,5-diyle,
$A^2$ représente un groupe 1,4-phénylène non substitué ou substitué par un atome de fluor,
X représente -O-CO- ou -O-,
Q représente un groupe alkylène en C 1-C 5 ou une liaison simple,
Y représente un halogène, et
R représente un groupe alkyle en C 1-C 10.
3. Composés hétérocycliques azotés de formule I de la revendication 1 dans laquelle
$-A^1-Z^1-A^2-$ représente un élément de structure choisi parmi ceux qui répondent aux formules 1 à 4

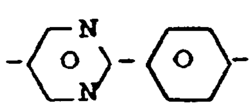 , 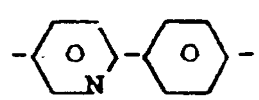 , 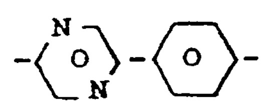

1 2 3

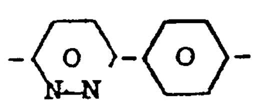

4

4. Composés hétérocycliques azotés selon une des revendications 1 à 3, de formule I032, I048, I066, I083 et I084.

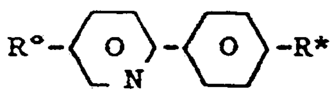 I032

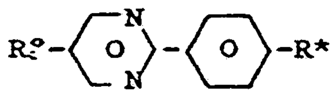 I048

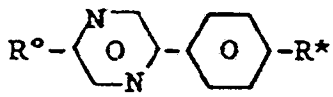 I066

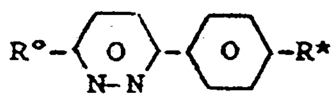 I083

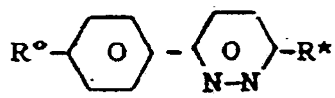 I084

dans lesquelles

R° représente un groupe alkyle ou alcoxy à chaîne droite en C 2-C 12, et

R* a l'une des significations indiquées pour le groupe organique possédant l'activité optique dans la revendication 1.

5. Utilisation des composés de formule I selon une des revendications 1 à 4 en tant que composants de phases à cristaux liquides ferroélectriques.

6. Phase à cristaux liquides ferroélectrique à au moins deux composants à cristaux liquides, caractérisée en ce que l'un au moins des composants est un composé de formule I de la revendication 1.

7. Phases à cristaux liquides smectiques chiralisées à au moins deux composants à cristaux liquides, caractérisées en ce que l'un au moins des composants est un composé de formule I dans laquelle $R^1$, $R^2$, $R^3$, R, $A^1$, $A^2$, $A^3$, $A^4$, $Z^1$, $Z^2$, $Z^3$, X, Y, et p ont les significations indiquées dans la revendication 1, et en ce qu'elles contiennent au moins un autre composant à anisotropie diélectrique relativement peu positive ou négative, choisi parmi les composés répondant aux formules partielles Va à Vp

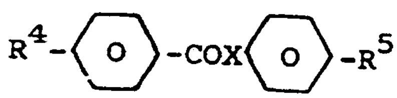 Va

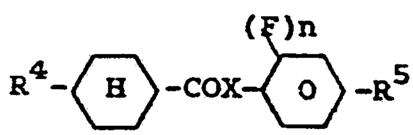 Vb

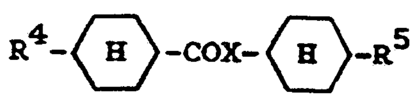 Vc

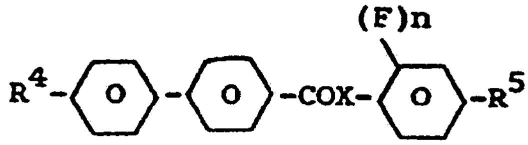 Vd

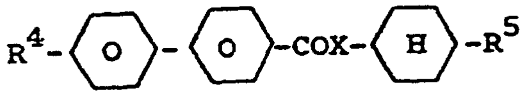
Ve

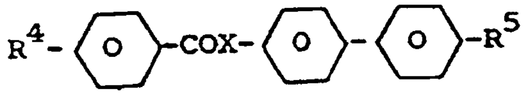
Vf

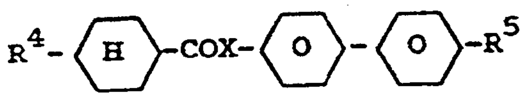
Vg

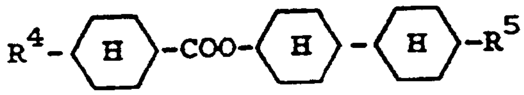
Vh

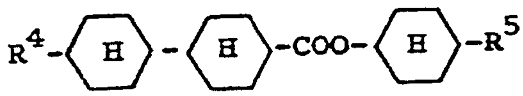
Vi

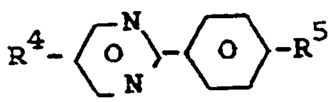
Vj

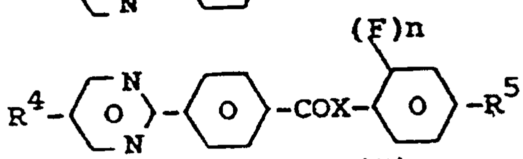
Vk

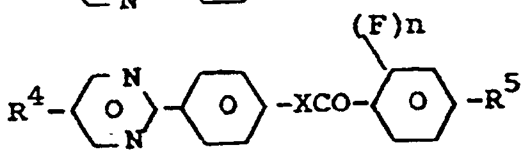
Vl

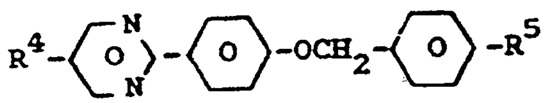
Vm

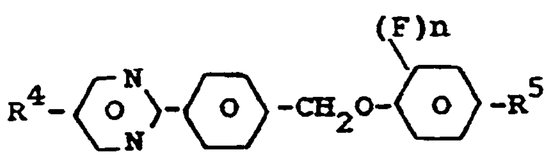
Vn

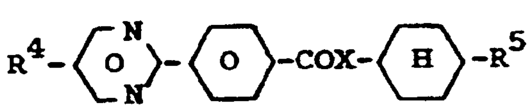
Vo

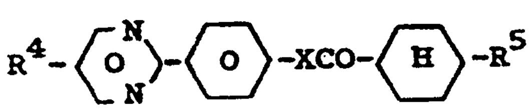
Vp

dans lesquelles

$R^4$ et $R^5$ représentent chacun un groupe alkyle, alcoxy, alcanoyloxy ou alcoxycarbonyle à chaîne droite contenant chacun 3 à 12 atomes de carbone,

X représente 0, et

n est égal à 0 ou 1.

8. Phase à cristaux liquides smectique chiralisée selon revendication 7, caractérisée en ce que l'un au moins des composants consiste en un composé de formule I dans laquelle

$-A^1-Z^1-A^2-$ représente

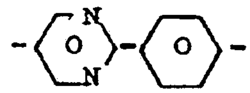

l'un des symboles $R^1$ et $R^2$ représente un groupe alkyle non substitué en C 1-C 15 dans lequel également un groupe $CH_2$ peut être remplacé par -O-, -COO- ou -OCO-, et l'autre représente un groupe possédant l'activité optique, de formule

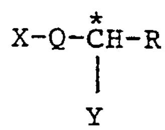

dans laquelle
X représente -CO-O-, -O-, -O-CO- ou une liaison simple,
Q représente un groupe alkylène en C 1-C 5 dans lequel également un groupe $CH_2$ non relié à X peut être remplacé par -O-,
Y représente $CH_3$, CN ou un halogène, et
R représente un groupe alkyle à chaîne droite en C 1-C 10,
et au moins un autre composant consiste en un composé de formule II

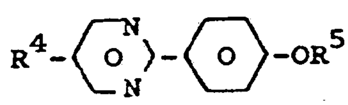

II

dans laquelle
$R^4$ et $R^5$ représentent chacun, indépendamment l'un de l'autre, un groupe n-alkyle en C 5-C 12.

9. Elément d'affichage électro-optique à base d'une cellule à cristaux liquides, caractérisé en ce qu'il contient une phase selon l'une des revendications 6 à 8.

**Revendications pour l'Etat contractant suivant: FR**

1. Composés hétérocycliques azotés de formule I

$$R^1-A^1-Z^1-A^2-R^2 \quad I$$

dans laquelle l'un des symboles
$R^1$ et $R^2$ représente H, un groupe alkyle non substitué ou substitué en C 1-C 15 dans lequel également un ou deux groupes $CH_2$ non voisins peuvent être remplacés par au moins un groupement choisi parmi -O-, -CO-, -O-CO-, -CO-O- et -CH=CH-, ou F, Cl, Br, -CN, -NCS ou $R^3-(A^3)_p-Z^2-$,
et l'autre représente un groupe organique possédant l'activité optique, de formule

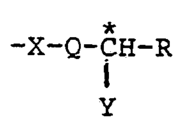

dans laquelle
X représente -CO-O-, -O-CO-, -O-CO-O-, -CO-, -O-, -S-, -CH=CH-, -CH=CH-COO- ou une liaison simple,
Q représente un groupe alkylène en C 1-C 5 dans lequel également un groupe $CH_2$ non relié à X peut être remplacé par -O-, -CO-, -O-CO-, -CO-O- ou -CH=CH-, ou une liaison simple,
Y représente CN, un halogène, un groupe méthyle ou méthoxy, et
R représente un groupe alkyle en C 1-C 10 différent de Y et dans lequel également un ou deux groupes $CH_2$ non voisins peuvent être remplacés par -O-, -CO-, -O-CO-, -CO-O- et/ou -CH=CH-,
$A^1$ représente -A-, $-A^4-Z^3-A-$ ou $-A-Z^3-A^4-$,

A représente un groupe 1,4-phénylène dans lequel au moins un groupe CH est remplacé par N,

$A^2$, $A^3$ et $A^4$ représentent chacun un groupe 1,4-phénylène non substitué ou substitué par un ou deux atomes de F et/ou de Cl et/ou groupes $CH_3$ et/ou groupes CN et dans lequel également un ou deux groupes CH peuvent être remplacés par des atomes de N, un groupe 1,4-cyclohexylène dans lequel également un ou deux groupes $CH_2$ non voisins peuvent être remplacés par des atomes de O et/ou de S, un groupe pipéridine-1,4-diyle, 1,4-bicyclo(2,2,2)-octylène, un groupe décahydronaphtalène-2,6-diyle ou 1,2,3,4-tétrahydronaphtalène-2,6-diyle non substitué ou substitué par CN,

$Z^1$, $Z^2$ et $Z^3$ représentent chacun -CO-O, -O-CO-, $-OCH_2-$, $-CH_2O-$, $-CH_2CH_2-$, un groupe éthylène substitué ou une liaison simple,

$R^3$ représente H, un groupe alkyle non substitué ou substitué en C 1-C 15 dans lequel également un ou deux groupes $CH_2$ non voisins peuvent être remplacés par un groupement choisi parmi -O-, -CO-, -O-CO-, -CO-O et -CH=CH-, ou F, Cl, Br, -NCS ou -CN, et

p est égal à 1 ou 2,

et lorsque p = 2, les groupes $A^3$ peuvent être identiques ou différents, avec les restrictions suivantes :

a) lorsque A représente un groupe pyrimidine-2,5-diyle, l'un des symboles $R^1$ et $R^2$ représente un groupe organique possédant l'activité optique et l'autre un groupe alkyle non substitué ou substitué en C 1-C 15 dans lequel également un ou deux groupes $CH_2$ non voisins peuvent être remplacés par au moins un groupement choisi parmi -O-, -CO-, -O-CO-, -CO-O- et -CH=CH-,

b) les composés de formule

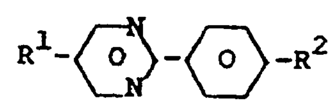

dans laquelle l'un des symboles $R^1$ et $R^2$ représente un groupe alkyle, alcoxy ou alcoxycarbonyle en C 1-C 15 et l'autre un groupe possédant l'activité optique de formule

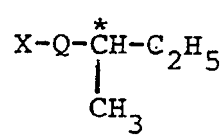

dans laquelle

X représente -CO-O-, -O-, -O-CO-, -CO- ou une liaison simple, et

Q représente un groupe alkylène en C 1-C 5 dans lequel également un groupe $CH_2$ non relié à X peut être remplacé par -O-, sont exclus,

c) la (S)-5-n-octyl-2-[4-(3-chloropentyloxy)phényl] pyrimidine,
la (S)-5-n-undécyl-2-[4-(3-cyanopentyloxy)phényl] pyrimidine, et
la (S)-5-(n-octyloxy)-2-[4-(2-méthyloctyl)-phényl] pyrimidine
sont exclues,

d) le 4-[5-(4'-butylphényl)-pyrimidine-2-yl] -benzoate de 2'-dodécyloxypropyle, et le 4-(5-heptylpyrimidine-2-yl)-benzoate de 2'-décyloxypropyle, sont exclus, et

e) les composés de formule

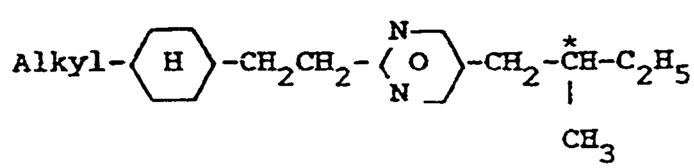

dans laquelle

"Alkyl" représente un groupe alkyle en C 1-C 12, sont exclus.

2. Composés hétérocycliques azotés selon revendication 1, de formule I1

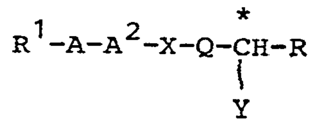

I1

dans laquelle
$R^1$ représente un groupe alkyle ou alcoxy en C 1-C 15,
A représente un groupe pyrimidine-2,5-diyle ou pyridine-2,5-diyle,
$A^2$ représente un groupe 1,4-phénylène non substitué ou substitué par un atome de fluor,
X représente -O-CO- ou -O-,
Q représente un groupe alkylène en C 1-C 5 ou une liaison simple,
Y représente un halogène, et
R représente un groupe alkyle en C 1-C 10.

3. Composés hétérocycliques azotés de formule I de la revendication 1 dans laquelle $-A^1-Z^1-A^2-$ représente un élément de structure choisi parmi ceux qui répondent aux formules 1 à 4

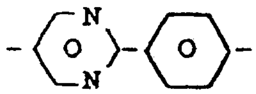 , 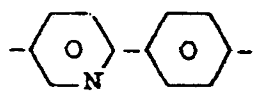 , 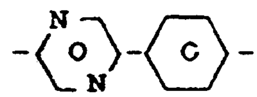

1 2 3

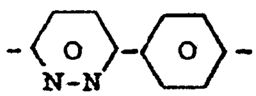

4

4. Composés hétérocycliques azotés selon une des revendications 1 à 3, de formules I032, I048, I066, I083 et I084

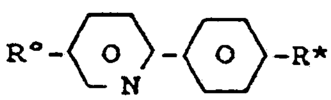

I032

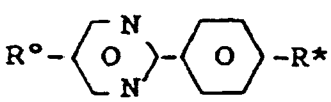

I048

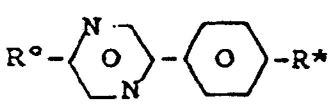

I066

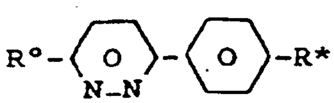

I083

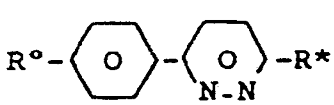

I084

dans lesquelles

R° représente un groupe alkyle ou alcoxy à chaîne droite en C 2-C 12, et

R* représente l'une des significations indiquées dans la revendication 1 pour le groupe organique possédant l'activité optique.

5. Utilisation des composés de formule I selon une des revendications 1 à 4 en tant que composants de phases à cristaux liquides ferroélectriques.

6. Phase à cristaux liquides ferroélectrique à au moins deux composants à cristaux liquides, caractérisée en ce que l'un au moins des composants est un composé de formule I de la revendication 1.

7. Phases à cristaux liquides smectiques chiralisées à au moins deux composants à cristaux liquides, caractérisées en ce que l'un au moins des composants est un composé de formule I dans laquelle $R^1$, $R^2$, $R^3$, R, $A^1$, $A^2$, $A^3$, $A^4$, $Z^1$, $Z^2$, $Z^3$, X, Q, Y et p ont les significations indiquées dans la revendication 1, et ces phases contiennent au moins un autre composant à anisotropie diélectrique relativement peu positive ou négative, choisi parmi les composés répondant aux formules partielles Va à Vp

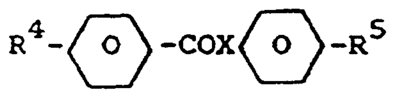 Va

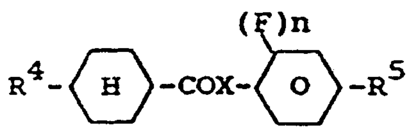 Vb

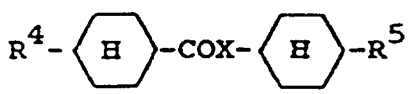 Vc

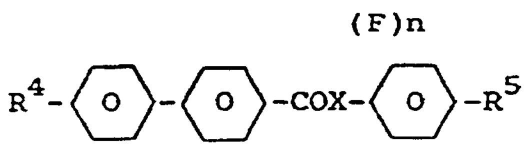 Vd

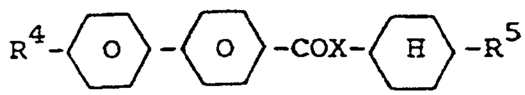 Ve

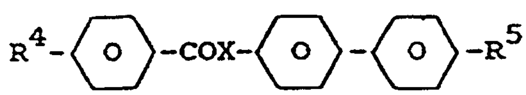 Vf

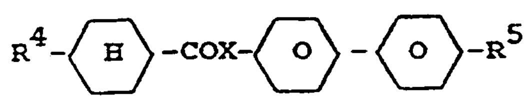 Vg

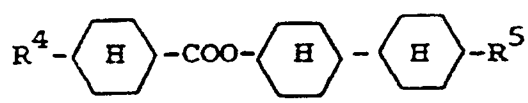 Vh

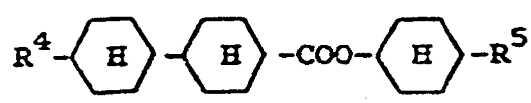 Vi

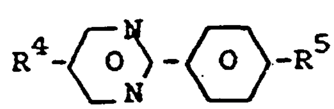 Vj

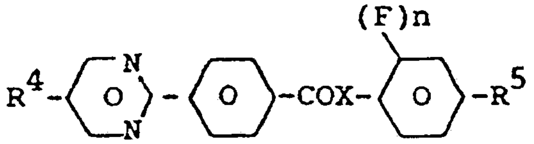
Vk

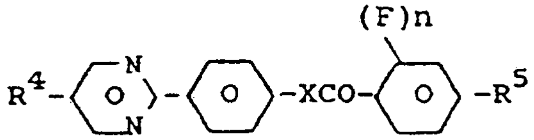
Vl

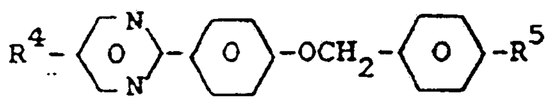
Vm

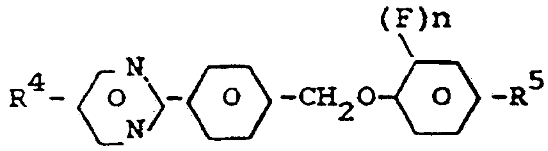
Vn

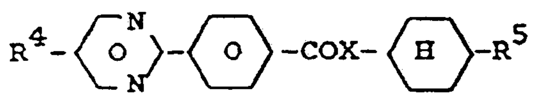
Vo

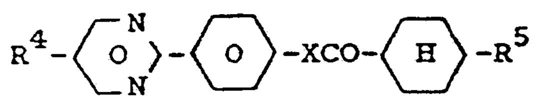
-Vp

dans lesquelles

$R^4$ et $R^5$ représentent chacun un groupe alkyle, alcoxy, alcanoyloxy ou alcoxycarbonyle à chaîne droite contenant chacun 3 à 12 atomes de carbone,

X représente O, et

n est égal à 0 ou 1.

8. Phase à cristaux liquides smectique chiralisée selon revendication 7, caractérisée en ce que l'un au moins des composants est un composé de formule I dans laquelle

$-A^1-Z^1-A^2-$ représente

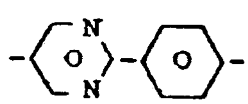

l'un des symboles $R^1$ et $R^2$ représente un groupe alkyle non substitué en C 1-C 15 dans lequel également un groupe $CH_2$ peut être remplacé par -O-, -COO- ou -OCO-, et l'autre représente un groupe possédant l'activité optique, de formule

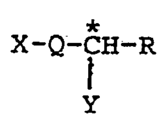

dans laquelle

X représente -CO-O-, -O-, -O-CO- ou une liaison simple,

Q représente un groupe alkylène en C 1-C 5 dans lequel également un groupe $CH_2$ non relié à X peut être remplacé par -O-,

Y représente $CH_3$, CN ou un halogène, et

R représente un groupe alkyle à chaîne droite en C 1-C 10,

et au moins un autre composant consiste en un composé de formule II

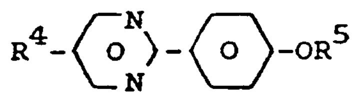

II

dans laquelle

$R^4$ et $R^5$ représentent chacun, indépendamment l'un de l'autre, un groupe n-alkyle en C 5-C 12.

9. Elément d'affichage électro-optique à base d'une cellule à cristaux liquides, caractérisé en ce qu'il contient une phase selon une des revendications 6 à 8.

**Revendications pour les Etats contractants suivants: DE, GB, CH, LI**

1. Composés hétérocycliques azotés de formule I

$$R^1\text{-}A^1\text{-}Z^1\text{-}A^2\text{-}R^2 \quad I$$

dans laquelle l'un des symboles

$R^1$ et $R^2$ représente H, un groupe alkyle non substitué ou substitué en C 1-C 15 dans lequel également un ou deux groupes $CH_2$ non voisins peuvent être remplacés par au moins un groupement choisi parmi -O-, -CO-, -O-CO-, -CO-O- et -CH=CH-, ou F, Cl, Br, -CN, -NCS ou $R^3\text{-}(A^3)_p\text{-}Z^2\text{-}$,

et l'autre représente un groupe organique possédant l'activité optique, de formule

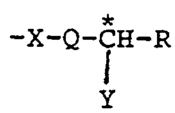

dans laquelle

X représente -CO-O-, -O-CO-, -O-CO-O-, -CO-, -O-, -S-, -CH=CH-, -CH=CH-COO- ou une liaison simple,

Q représente un groupe alkylène en C 1-C 5 dans lequel également un groupe $CH_2$ non relié à X peut être remplacé par -O-, -CO-, -O-CO-, -CO-O- ou -CH=CH-, ou une liaison simple,

Y représente CN, un halogène, un groupe méthyle ou méthoxy, et

R représente un groupe alkyle en C 1-C 10 différent de Y et dans lequel également un ou deux groupes $CH_2$ non voisins peuvent être remplacés par -O-, -CO-, -O-CO-, -CO-O- et/ou -CH=CH-,

$A^1$ représente -A-, $-A^4\text{-}Z^3\text{-}A\text{-}$ ou $-A\text{-}Z^3\text{-}A^4\text{-}$,

A représente un groupe 1,4-phénylène dans lequel au moins un groupe CH est remplacé par N,

$A^2$, $A^3$ et $A^4$ représentent chacun un groupe 1,4-phénylène non substitué ou substitué par'un ou deux atomes de F et/ou de Cl et/ou groupes $CH_3$ et/ou groupes CN et dans lequel également un ou deux groupes CH peuvent être remplacés par des atomes de N, un groupe 1,4- cyclohexylène dans lequel également un ou deux groupes $CH_2$ non voisins peuvent être remplacés par des atomés de O et/ou de S, un groupe pipéridine-1,4-diyle, 1,4-bicyclo(2,2,2)-octylène, un groupe décahydronaphtalène-2,6-diyle ou 1,2,3,4-tétrahydronaphtalène-2,6-diyle non substitué ou substitué par CN,

$Z^1$, $Z^2$ et $Z^3$ représentent chacun -CO-O-, -O-CO-, $-OCH_2-$, $-CH_2O-$, $-CH_2CH_2-$, un groupe éthylène substitué ou une liaison simple,

$R^3$ représente H, un groupe alkyle non substitué ou substitué en C 1-C 15 dans lequel également un ou deux groupes $CH_2$ non voisins peuvent être remplacés par un groupement choisi parmi -O-, -CO-, -O-CO-, -CO-O et -CH=CH-, ou F, Cl, Br, -NCS ou -CN, et

p est égal à 1 ou 2,

et lorsque p = 2, les groupes $A^3$ peuvent être identiques ou différents, avec les restrictions suivantes :

a) lorsque A représente un groupe pyrimidine-2,5-diyle, l'un des symboles $R^1$ et $R^2$ représente un groupe organique possédant l'activité optique et l'autre un groupe alkyle non substitué ou substitué en C 1-C 15 dans lequel également un ou deux groupes $CH_2$ non voisins peuvent être remplacés par au moins un groupement choisi parmi -O-, -CO-, -O-CO-, -CO-O- et -CH=CH-,

b) les composés de formule

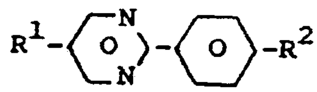

dans laquelle l'un des symboles $R^1$ et $R^2$ représente un groupe alkyle, alcoxy ou alcoxycarbonyle en C 1-C 15 et l'autre un groupe possédant l'activité optique, de formule

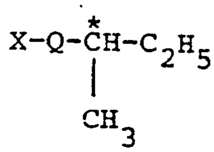

dans laquelle
X représente -CO-O-, -O-, -O-CO-, -CO- ou une liaison simple, et
Q représente un groupe alkylène en C 1-C 5 dans lequel également un groupe $CH_2$ non relié à X peut être remplacé par -O-,
sont exclus,
c) la (S)-5-n-octyl-2-[4 -(3-chloropentyloxy)phényl] pyrimidine,
la (S)-5-n-undécyl-2-[4-(3-cyanopentyloxy)phényl] pyrimidine, et
la (S)-5-(n-octyloxy)-2-[4-(2-méthyloctyl)-phényl] pyrimidine
sont exclues,
d) la 3-(2-méthylbutoxy)-6-(4-pentylphényl)-pyridazine,
la 3-(2-méthylbutoxy)-6-(4-nonylphényl)-pyridazine,
la 3-(2-méthylbutoxy)-6-(4-dodécylphényl)-pyridazine,
la 3-(2-méthylbutoxy)-6-(4-pentadécylphényl)-pyridazine,
la 3-(2-méthylbutoxy)-6-(4-octyloxyphényl)-pyridazine,
la 3-(2-méthylbutoxy)-6-(4-décyloxyphényl)-pyridazine,
la 3-(2-méthylbutoxy)-6-(4-tétradécyloxyphényl)-pyridazine,
la 3-(1-méthylheptyloxy)-6-(4-pentylphényl)-pyridazine,
la 3-(1-méthylheptyloxy)-6-(4-dodécylphényl)-pyridazine,
la 3-(1-méthylheptyloxy)-6-(4-décyloxyphényl)-pyridazine et
la 3-(1-méthylbutoxy)-6-(4-octyloxyphényl)-pyridazine sont exclues,
e) le 4-[5-(4'-butylphényl)-pyrimidine-2-yl]-benzoate de 2'-dodécyloxypropyle, et le 4-(5-heptylpyrimidine-2-yl)-benzoate de 2'-décyloxypropyle sont exclus, et
f) les composés de formule

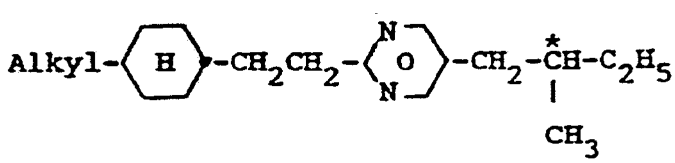

dans laquelle "Alkyl" représente un groupe alkyle en C 1-C 12,
sont exclus.

2. Composés hétérocycliques azotés selon revendication 1, de formule I1

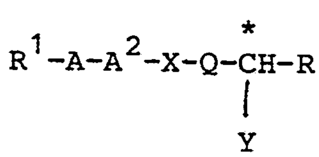

I1

dans laquelle
$R^1$ représente un groupe alkyle ou alcoxy en C 1-C 15,
A représente un groupe pyrimidine-2,5-diyle ou pyridine-2,5-diyle,
$A^2$ représente un groupe 1,4-phénylène non substitué ou substitué par un atome de fluor,

X représente -O-CO- ou -O-,
Q représente un groupe alkylène en C 1-C 5 ou une liaison simple,
Y représente un halogène, et
R représente un groupe alkyle en C 1-C 10.

3. Composés hétérocycliques azotés de formule I de la revendication 1 dans laquelle $-A^1-Z^1-A^2-$ représente un élément de structure choisi parmi ceux qui répondent aux formules 1 à 4

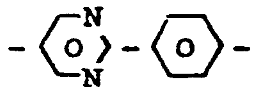 , 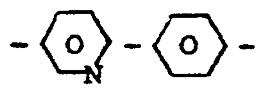 , 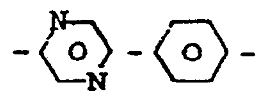

1 2 3

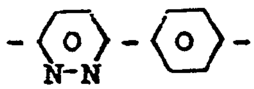

4

4. Composés hétérocycliques azotés selon une des revendications 1 à 3, de formule I032, I048, I066, I083 et I084

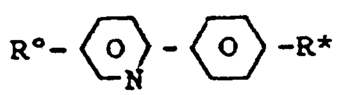 I032

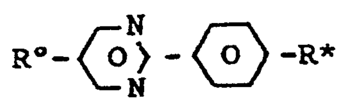 I048

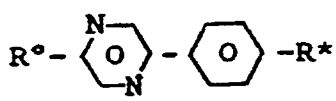 I066

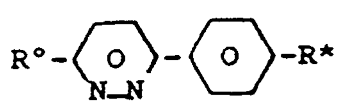 I083

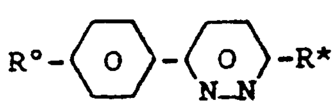 I084

dans lesquelles
R° représente un groupe alkyle ou alcoxy à chaîne droite en C 2-C 12, et
R* a l'une des significations indiquées dans la revendication 1 pour le groupe organique possédant l'activité optique.

5. Utilisation des composés de formule I selon une des revendications 1 à 4 en tant que composants de phases à cristaux liquides ferroélectriques.

6. Phase à cristaux liquides ferroélectrique à au moins deux composants à cristaux liquides, caractérisée en ce que l'un au moins des composants est un composé de formule I de la revendication 1.

7. Phases à cristaux liquides smectiques chiralisées à au moins deux composants à cristaux liquides, caractérisées en ce que l'un au moins des composants est un composé de formule I dans laquelle $R^1$, $R^2$, $R^3$,

R, $A^1$, $A^2$, $A^3$, $A^4$, $Z^1$, $Z^2$, $Z^3$, X, Q, Y et p ont les significations indiquées dans la revendication 1, et les phases contiennent au moins un autre composant à anisotropie diélectrique relativement peu positive ou négative, choisi parmi les composés répondant aux formules partielles Va à Vp

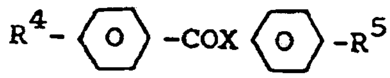

Va

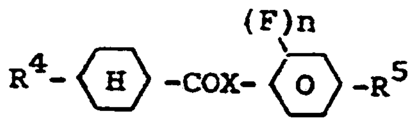

Vb

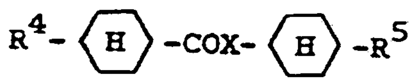

Vc

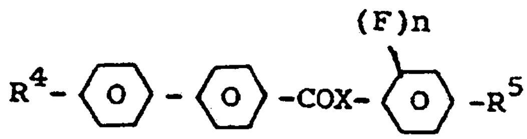

Vd

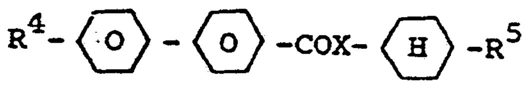

Ve

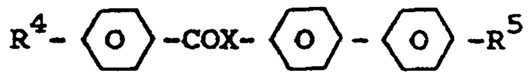 Vf

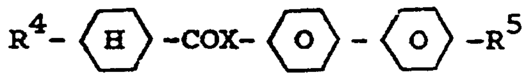 Vg

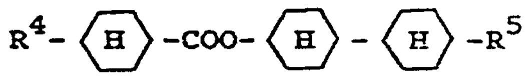 Vh

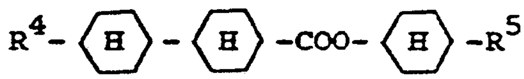 Vi

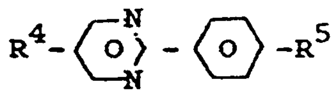 Vj

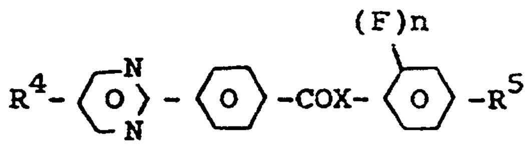 Vk

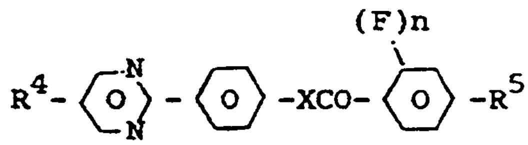 Vl

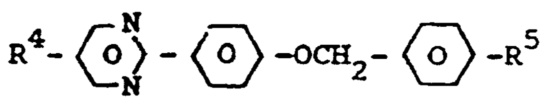 Vm

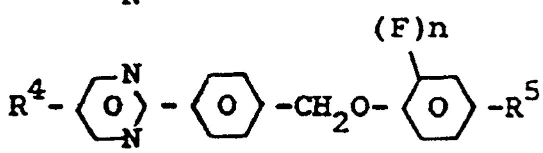 Vn

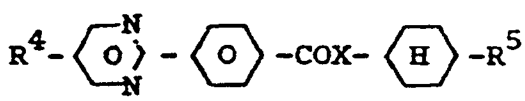 Vo

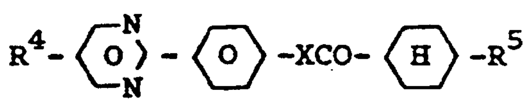 Vp

dans lesquelles

$R^4$ et $R^5$ représentent chacun un groupe alkyle, alcoxy, alcanoyloxy ou alcoxycarbonyle à chaîne droite en C 3-C 12,

X représente 0, et

n est égal à 0 ou 1.

8. Phase à cristaux liquides smectique chiralisée selon revendication 7, caractérisée en ce que l'un au moins des composants est un composé de formule I dans laquelle

$-A^1-Z^1-A^2-$ représente

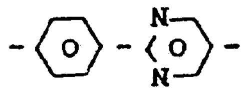

l'un des symboles $R^1$ et $R^2$ représente un groupe alkyle non substitué en C 1-C 15 dans lequel également un groupe $CH_2$ peut être remplacé par -O-, -COO- ou -OCO-, et l'autre représente un groupe possédant l'activité optique de formule

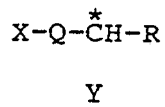

dans laquelle

X représente -CO-O-, -O-, -O-CO- ou une liaison simple,

Q représente un groupe alkylène en C 1-C 5 dans lequel également un groupe $CH_2$ non relié à X peut être remplacé par -O-,

Y représente $CH_3$, CN ou un halogène, et

R représente un groupe alkyle à chaîne droite en C 1-C 10,

et en ce qu'un autre composant au moins consiste en un composé de formule II

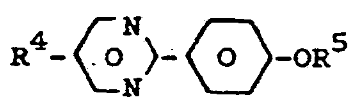 II

dans laquelle

$R^4$ et $R^5$ représentent chacun, indépendamment l'un de l'autre, un groupe n-alkyle en C 5-C 12.

9. Elément d'affichage électro-optique à base d'une cellule à cristaux liquides, caractérisé en ce qu'il contient une phase selon une des revendications 6 à 8.